# EUROPÄISCHE PATENTSCHRIFT

(11) **EP 2 391 620 B1**
(45) Veröffentlichungstag und Bekanntmachung des Hinweises auf die Patenterteilung: **07.05.2014**
(21) Anmeldenummer: 10700508.4
(22) Anmeldetag: 19.01.2010
(51) Int. Cl.: C07D 417/12, C07D 417/14, C07K 5/037, C07K 5/062, A61K 31/4439, A61P 3/06, A61P 3/10

(54) **ALKYLAMINO-SUBSTITUIERTE DICYANOPYRIDINE UND DEREN AMINOSÄUREESTER-PRODRUGS**
ALKYLAMINE-SUBSTITUTED DICYANOPYRIDINE AND AMINO ACID ESTER PRODRUGS THEREOF
DICYANOPYRIDINE À SUBSTITUTION ALKYLAMINO ET SES PROMÉDICAMENTS D'ESTER D'ACIDE AMINÉ

(30) Priorität: 29.01.2009 DE 102009006602
(43) Veröffentlichungstag der Anmeldung: 07.12.2011
(60) Teilanmeldung: 14155583.9
(73) Patentinhaber: Bayer Intellectual Property GmbH, 40789 Monheim (DE)
(72) Erfinder: VAKALOPOULOS, Alexandros, 40721 Hilden (DE); MEIBOM, Daniel, 51373 Leverkusen (DE); ALBRECHT-KÜPPER, Barbara, 42489 Wülfrath (DE); ZIMMERMANN, Katja, 40489 Düsseldorf (DE); KELDENICH, Joerg, 42113 Wuppertal (DE); LERCHEN, Hans-Georg, 51375 Leverkusen (DE); NELL, Peter, Woodside, CA 94062 (US); SÜSSMEIER, Frank, 80992 München (DE); KRENZ, Ursula, 42799 Leichlingen (DE)
(74) Vertreter: BIP Patents
(86) Internationale Anmeldenummer: PCT/EP2010/000262
(87) Internationale Veröffentlichungsnummer: WO 2010/086101

(56) Entgegenhaltungen:
- WO-A1-03/053441
- WO-A1-2006/027142
- WO-A1-2008/028590
- WO-A1-2009/080197
- WO-A2-2009/015812

## Beschreibung

Die vorliegende Anmeldung betrifft neue 6-Alkylamino-substituierte Dicyanopyridine, deren Aminosäureester-Prodrugs, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, vorzugsweise zur Behandlung und/oder Prävention von kardiovaskulären Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung. In Adipozyten ist Adenosin in der Lage, die Lipolyse zu hemmen und somit die Konzentration an freien Fettsäuren und Triglyzeriden im Blut zu senken.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosin-rezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über A1-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A1-Rezeptoren durch spezifische A1-Agonisten führt beim Menschen zu einer frequenzabhängigen Senkung der Herzfrequenz, ohne einen Einfluss auf den Blutdruck zu haben. Selektive A1-Agonisten könnten somit unter anderem für die Behandlung von Angina pectoris und Vorhofflimmern geeignet sein.

Die kardioprotektive Wirkung der A1-Rezeptoren im Herzen kann unter anderem durch die Aktivierung dieser A1-Rezeptoren durch spezifische A1-Agonisten für die Behandlung und Organprotektion bei akutem Myokardinfarkt, akutem Koronarsyndrom, Herzinsuffizienz, Bypass Operationen, Herzkatheteruntersuchungen und Organtransplantationen genutzt werden.

Die Aktivierung von A2b-Rezeptoren durch Adenosin oder spezifische A2b-Agonisten führt über die Erweiterung von Gefäßen zu einer Blutdrucksenkung. Die Blutdrucksenkung ist von einem reflektorischen Herzfrequenzanstieg begleitet. Der Herzfrequenzanstieg kann durch die Aktivierung von A1-Rezeptoren durch spezifische A1-Agonisten reduziert werden.

Die kombinierte Wirkung von selektiven A1/A2b-Agonisten auf das Gefäßsystem und die Herzfrequenz resultiert somit in einer systemischen Blutdrucksenkung ohne relevanten Herzfrequenzanstieg. Mit einem solchen pharmakologischen Profil könnten duale A1/A2b-Agonisten zur Behandlung z.B. der Hypertonie beim Menschen eingesetzt werden.

In Adipozyten bewirkt die Aktivierung von A1- und A2b-Rezeptoren eine Inhibition der Lipolyse. Die selektive bzw. kombinierte Wirkung von A1- und A1/A2b-Agonisten auf den Lipidstoffwechsel führt somit zu einer Senkung von freien Fettsäuren und Triglyzeriden. Eine Senkung der Lipide wiederum führt bei Patienten mit Metabolischem Syndrom und bei Diabetikern zur Verringerung der Insulinresistenz und zur Verbesserung der Symptomatik.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis", J. Biol. Chem. 267 (1992), Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells", Naunyn Schmiedebergs Arch. Pharmacol. 357 (1998), Seiten 1-9, deren Offenbarung hiermit im vollen Umfang durch Bezugnahme eingeschlossen ist).

Bei den aus dem Stand der Technik bekannten, als "Adenosinrezeptor-spezifisch" geltenden Liganden handelt es sich überwiegend um Derivate auf Basis des natürlichen Adenosins [S.-A. Poulsen und R. J. Quinn, "Adenosine receptors: New opportunities for future drugs", Bioorganic and Medicinal Chemistry 6 (1998), Seiten 619-641]. Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin, nach oraler Applikation nur sehr schwach wirksam sind oder unerwünschte Nebenwirkungen auf das Zentralnervensystem (ZNS) haben (A. K. Dhalla et al, Curr. Topics in Med. Chem. 2003, 3, 369-385; [E. Elzein, J. Zablocki, Exp. Opin. Invest. Drugs 2008, 17(12), 1901-1910]. Deshalb werden sie überwiegend nur für experimentelle Zwecke verwendet. In klinischer Entwicklung befindliche Verbindungen dieser Art eignen sich bislang nur zur intravenösen Applikation.

Prodrugs sind Derivate eines Wirkstoffs, die *in vivo* eine ein- oder mehrstufige Biotransformation enzymatischer und/oder chemischer Art durchlaufen, bevor der eigentliche Wirkstoff freigesetzt wird. Ein Prodrug-Rest wird in der Regel genutzt, um das Eigenschaftsprofil des zu Grunde liegenden Wirkstoffs zu verbessern [P. Ettmayer et al., J. Med. Chem. 47, 2393-2404 (2004)]. Um ein optimales Wirkprofil zu erreichen, muss dabei das Design des Prodrug-Restes ebenso wie der angestrebte Freisetzungsmechanismus sehr genau auf den individuellen Wirkstoff, die Indikation, den Wirkort und die Applikationsroute abgestimmt werden. Eine große Zahl von Arzneimitteln wird als Prodrugs verabreicht, die gegenüber dem zu Grunde liegenden Wirkstoff eine verbesserte Bioverfügbarkeit aufweisen, beispielsweise erzielt durch eine Verbesserung des physikochemischen Profils, speziell der Löslichkeit, der aktiven oder passiven Absorptionseigenschaften oder der gewebsspezifischen Verteilung. Aus der umfangreichen Literatur über Prodrugs sei beispielhaft genannt: H. Bundgaard (Ed.), Design of Prodrugs: Bioreversible derivatives for various functional groups and chemical entities, Elsevier Science Publishers B.V., 1985. Eine Übersicht zu Prodrug-Derivaten auf Basis von Carbonsäureestern und möglichen Eigenschaften solcher Verbindungen ist beispielsweise in K. Beaumont et al., Curr. Drug Metab. 4, 461-485 (2003) gegeben. Weiterhin sind Dipeptid-Prodrugs von Acyclovir zur Behandlung von Herpesinfektionen der Augen bekannt (B. S. Anand et al., Curr. Eye Res. 26, No. 3-4, 151-163 (2003)), die den Oligopeptid-Transporter auf der Hornhaut ansprechen, um die Bioverfügbarkeit von Acylovir im Auge zu erhöhen.

In WO 01/25210, WO 02/070484, WO 02/070485, WO 03/053441, WO 2008/028590, WO 2009/100827, WO 2009/015776 und WO 2009/112155 werden verschiedenartig substituierte 3,5-Dicyano-6-aminopyridine als Adenosinrezeptor-Liganden für die Behandlung von kardiovaskulären Erkrankungen offenbart. WO 2009/015811 und WO 2009/015812 beschreiben Aminosäureester-Prodrugs von 3,5-Dicyano-6-aminopyridinen.

Aufgabe der vorliegenden Erfindung ist die Bereitstellung neuer Verbindungen, die als potente und selektive Agonisten des Adenosin A1-Rezeptors oder selektive duale Agonisten des A1- und A2b-Rezeptors wirken, gleiche oder verbesserte physikochemische und/oder pharmakokinetische Eigenschaften sowie ein vorteilhaftes therapeutisches und/oder pharmakologisches Wirkprofil aufweisen, und als solche zur Behandlung und/oder Prävention von Krankheiten, insbesondere zur Behandlung und/oder Prävention von kardiovaskulären Erkrankungen geeignet sind, sowie die Identifizierung geeigneter Aminosäureester-Prodrugs der neuen Verbindungen, die über eine verbesserte Löslichkeit in Wasser, physiologischen Medien und organischen Lösungsmitteln verfügen und/oder eine verbesserte Bioverfügbarkeit nach oraler Applikation besitzen und gleichzeitig nach Applikation eine kontrollierte Freisetzung des Wirkstoffs im Körper des Patienten erlauben. Durch eine verbesserte intravenöse Applizierbarkeit könnten zudem weitere therapeutische Einsatzgebiete für diesen Wirkstoff erschlossen werden.

Gegenstand der vorliegenden Erfmdung sind Verbindungen der Formel (I) 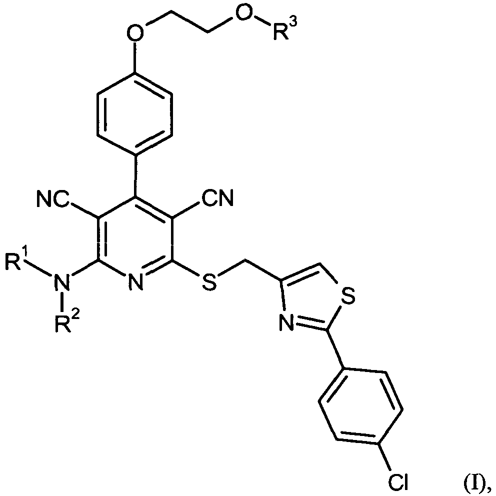 in welcher
- R¹: für Wasserstoff oder (C₁-C₄)-Alkyl steht,
- R²: für (C₁-C₆)-Alkyl, (C₂-C₄)-Alkenyl, (C₂-C₄)-Alkinyl oder (C₃-C₇)-Cycloalkyl steht,
wobei (C₁-C₆)-Alkyl mit 1 bis 3 Substituenten unabhängig voneinander ausgewählt aus der Gruppe Fluor, Chlor, Trifluormethyl, Trifluormethoxy, (C₁-C₄)-Alkoxy, (C₃-C₇)-Cycloalkyl, (C₃-C₇)-Cycloalkoxy, (C₁-C₄)-Alkylsulfanyl oder (C₁-C₄)-Alkylsulfonyl substituiert sein kann,
und
wobei (C₂-C₄)-Alkenyl und (C₂-C₄)-Alkinyl mit 1 oder 2 Substituenten unabhängig voneinander ausgewählt aus der Gruppe Fluor, Trifluormethyl, (C₁-C₄)-Alkyl, Trifluormethoxy und (C₁-C₄)-Alkoxy substituiert sein können,
und
wobei (C₃-C₇)-Cycloalkyl mit 1 oder 2 Substituenten unabhängig voneinander ausgewählt aus der Gruppe Fluor, Chlor, Trifluormethyl, (C₁-C₄)-Alkyl, Trifluormethoxy und (C₁-C₄)-Alkoxy substituiert sein kann,
oder
- R¹ und R²: zusammen mit dem Stickstoffatom, an das sie gebunden sind, einen 4- bis 7-gliedrigen Heterocyclus, der ein weiteres Heteroatom aus der Reihe N, O und S enthalten kann, bilden,
wobei der 4- bis 7-gliedrige Heterocyclus mit 1 oder 2 Substituenten unabhängig voneinander ausgewählt aus der Gruppe Fluor, Chlor, Oxo, Trifluormethyl, (C₁-C₄)-alkyl, Trifluormethoxy und (C₁-C₄)-Alkoxy substituiert sein kann,
- R³: für Wasserstoff oder eine Gruppe der Formel 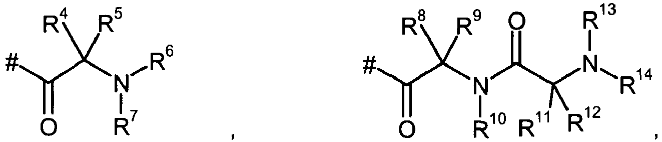 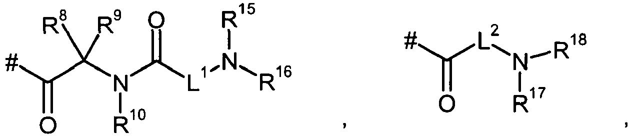 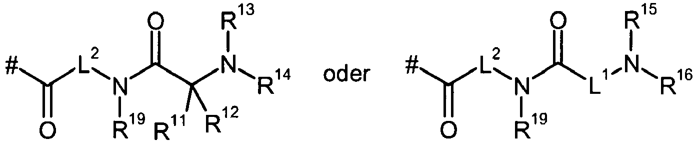 steht, wobei
# für die Anknüpfstelle an das Sauerstoffatom steht,
L¹ für (C₂-C₆)-Alkandiyl steht,
L² für (C₂-C₆)-Alkandiyl steht,
R⁴ für Wasserstoff oder die Seitengruppe einer natürlichen α-Aminosäure oder ihrer Homologe oder Isomere steht,
R⁵ für Wasserstoff oder Methyl steht,
R⁶ für Wasserstoff oder (C₁-C₄)-Alkyl steht,
R⁷ für Wasserstoff oder (C₁-C₄)-Alkyl steht,
oder
- R⁶ und R⁷: zusammen mit dem Stickstoffatom, an das sie gebunden sind, einen 5- oder 6-gliedrigen Heterocyclus bilden,
wobei der 5- oder 6-gliedrige Heterocyclus mit 1 oder 2 Substituenten unäbhangig voneinander ausgewählt aus der Gruppe (C₁-C₄)-Alkyl, Amino, Hydroxy und (C₁-C₄)-Alkoxy substituiert sein kann,
oder
- R⁷: zusammen mit R⁴ und den Atomen, an die sie jeweils gebunden sind, einen Pyrrolidin- oder Piperidinring bildet,
- R⁸: für Wasserstoff oder die Seitengruppe einer natürlichen α-Aminosäure oder ihrer Homologe oder Isomere steht,
- R⁹: für Wasserstoff oder Methyl steht,
- R¹⁰: für Wasserstoff oder Methyl steht,
- R¹¹: für Wasserstoff oder die Seitengruppe einer natürlichen α-Aminosäure oder ihrer Homologe oder Isomere steht,
- R¹²: für Wasserstoff oder Methyl steht,
- R¹³: für Wasserstoff oder (C₁-C₄)-Alkyl steht,
- R¹⁴: für Wasserstoff oder (C₁-C₄)-Alkyl steht,
oder
- R¹³ und R¹⁴: zusammen mit dem Stickstoffatom, an das sie gebunden sind, einen 5- oder 6-gliedrigen Heterocyclus bilden,
wobei der 5- oder 6-gliedrige Heterocyclus mit 1 oder 2 Substituenten unäbhangig voneinander ausgewählt aus der Gruppe (C₁-C₄)-Alkyl, Amino, Hydroxy und (C₁-C₄)-Alkoxy substituiert sein kann,
oder
- R¹⁴: zusammen mit R¹¹ und den Atomen, an die sie jeweils gebunden sind, einen Pyrrolidin- oder Piperidinring bildet,
- R¹⁵: für Wasserstoff oder (C₁-C₄)-Alkyl steht,
- R¹⁶: für Wasserstoff oder (C₁-C₄)-Alkyl steht,
oder
- R¹⁵ und R¹⁶: zusammen mit dem Stickstoffatom, an das sie gebunden sind, einen 5- oder 6-gliedrigen Heterocyclus bilden,
wobei der 5- oder 6-gliedrige Heterocyclus mit 1 oder 2 Substituenten unäbhangig voneinander ausgewählt aus der Gruppe (C₁-C₄)-Alkyl, Amino, Hydroxy und (C₁-C₄)-Alkoxy substituiert sein kann,
- R¹⁷: für Wasserstoff oder (C₁-C₄)-Alkyl steht,
- R¹⁸: für Wasserstoff oder (C₁-C₄)-Alkyl steht,
oder
- R¹⁷ und R¹⁸: zusammen mit dem Stickstoffatom, an das sie gebunden sind, einen 5- oder 6-gliedrigen Heterocyclus bilden,
wobei der 5- oder 6-gliedrige Heterocyclus mit 1 oder 2 Substituenten unäbhangig voneinander ausgewählt aus der Gruppe (C₁-C₄)-Alkyl, Amino, Hydroxy und (C₁-C₄)-Alkoxy substituiert sein kann,
- R¹⁹: für Wasserstoff oder Methyl steht,
sowie ihre N-Oxide, Salze, Solvate, Salze der N-Oxide und Solvate der N-Oxide und Salze.

Erfindungsgemäße Verbindungen sind die Verbindungen der Formel (I) und deren N-Oxide, Salze, Solvate, Salze der N-Oxide und Solvate der Salze und N-Oxide, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 6 bzw. 1 bis 4 Kohlenstoffatomen. Bevorzugt ist ein linearer oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
Alkenyl steht im Rahmen der Erfindung für einen linearen oder verzweigten Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Alkinyl steht im Rahmen der Erfindung für einen linearen oder verzweigten Alkinylrest mit 2 bis 4 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.
Alkandiyl steht im Rahmen der Erfindung für einen linearen oder verzweigten divalenten Alkylrest mit 2 bis 6 Kohlenstoffatomen. Beispielhaft und vorzugsweise seien genannt: Methylen, Ethan-1,1-diyl, Ethan-1,2-diyl, Propan-1,1-diyl, Propan-1,2-diyl, Propan-2,2-diyl, Propan-1,3-diyl, Butan-1,4-diol, Butan-1,2-diyl, Butan-1,3-diyl, Butan-2,3-diyl oder Butan-3,4-diyl.
Cycloalkyl steht im Rahmen der Erfindung für einen monocyclischen, gesättigten Carbocyclus mit 3 bis 7 bzw. 5 bis 6 Ring-Kohlenstoffatomen. Beispielhaft und vorzugsweise seien genannt: Cyclopropyl, Cyclobutyl, Cyclopentyl, Cyclohexyl und Cycloheptyl.
Alkoxy steht im Rahmen der Erfindung für einen linearen oder verzweigten Alkoxyrest mit 1 bis 6 bzw. 1 bis 4 bzw. 2 bis 4 Kohlenstoffatomen. Bevorzugt ist ein linearer oder verzweigter Alkoxyrest mit 1 bis 4 bzw. 2 bis 4 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.
Cycloalkoxy steht im Rahmen der Erfindung für einen monocyclischen, gesättigten Carbocyclus mit 3 bis 7 Kohlenstoffatomen, der über ein Sauerstoffatom gebunden ist. Beispielhaft und vorzugsweise seien genannt: Cyclopropyloxy, Cyclobutyloxy, Cyclopentyloxy, Cyclohexyloxy und Cycloheptyloxy.
Alkylsulfonyl steht in Rahmen der Erfindung für einen linearen oder verzweigten Alkylrest mit 1 bis 4 Kohlenstoffatomen, der über eine Sulfanylgruppe gebunden ist. Beispielhaft und vorzugsweise seinen genannt: Methylsulfanyl, Ethylsulfanyl, n-Propylsulfanyl, iso-Propylsulfanyl, n-Butylsulfanyl und tert.-Butylsulfanyl.
Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.
Heterocyclus steht im Rahmen der Erfmdung für einen gesättigten Heterocyclus mit insgesamt 4 bis 7 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Azetidinyl, Pyrrolidinyl, Pyrazolidinyl, Tetrahydrofuranyl, Piperidinyl, Piperazinyl, Tetrahydropyranyl, Morpholinyl, Thiomorpholinyl und Azepanyl. Bevorzugt sind Azetidinyl, Pyrrolidinyl, Tetrahydrofuranyl, Piperidinyl, Piperazinyl, Tetrahydropyranyl und Morpholinyl. Besonders sind Azetidinyl, Pyrrolidinyl, Piperidinyl und Morpholinyl.
Die Seitengruppe einer α-Aminosäure in der Bedeutung von R³ umfasst sowohl die Seitengruppen der natürlich vorkommenden α-Aminosäuren als auch die Seitengruppen von Homologen und Isomeren dieser α-Aminosäuren. Die α-Aminosäure kann hierbei sowohl in der L- als auch in der D-Konfiguration oder auch als Gemisch der L- und D-Form vorliegen. Als Seitengruppen seien beispielhaft genannt: Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), tert.-Butyl (2-tert.-Butyl-glycin), Phenyl (2-Phenylglycin), Benzyl (Phenylalanin), p-Hydroxybenzyl (Tyrosin), Indol-3-yl-methyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (*S-*Methylcystein), 2-Mercaptoethyl (Homocystein), 2-Methylthioethyl (Methionin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 4-Amino-3-hydroxybutan-1-yl (Hydroxylysin), 3-Aminopropan-1-yl (Ornithin), 2-Aminoethyl (2,4-Diaminobuttersäure), Aminomethyl (2,3-Diaminopropionsäure), 3-Guanidinopropan-1-yl (Arginin), 3-Ureidopropan-1-yl (Citrullin). Bevorzugte α-Aminosäure-Seitengruppen in der Bedeutung von R³ sind Methyl (Alanin), Propan-2-yl (Valin), 2-Methylpropan-1-yl (Leucin), Benzyl (Phenylalanin), Imidazol-4-ylmethyl (Histidin), Hydroxymethyl (Serin), 1-Hydroxyethyl (Threonin), 4-Aminobutan-1-yl (Lysin), 3-Aminopropan-1-yl (Ornithin), 2-Aminoethyl (2,4-Diaminobuttersäure), Aminomethyl (2,3-Diaminopropionsäure), 3-Guanidinopropan-1-yl (Arginin). Bevorzugt ist jeweils die L-Konfiguration.
Eine Oxo-Gruppe steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Bevorzugt sind im Rahmen der vorliegenden Erfindung Verbindungen der Formel (I), in welcher
- R¹: für Wasserstoff, Methyl oder Ethyl steht,
- R²: für (C₁-C₆)-Alkyl oder (C₃-C₆)-Cycloalkyl steht,
wobei (C₁-C₆)-Alkyl mit 1 bis 3 Substituenten unabhängig voneinander ausgewählt aus der Gruppe Fluor, Chlor, Trifluormethyl, Trifluormethoxy und (C₁-C₃)-Alkoxy substituiert sein kann,
oder
- R¹ und R²: zusammen mit dem Stickstoffatom, an das sie gebunden sind, einen 4- bis 6-gliedrigen Heterocyclus, der ein weiteres Heteroatom aus der Reihe N, O und S enthalten kann, bilden,
wobei der 4- bis 6-gliedrige Heterocyclus mit 1 oder 2 Substituenten unabhängig voneinander ausgewählt aus der Gruppe Fluor, Trifluormethyl, (C₁-C₄)-Alkyl, Trifluormethoxy, Methoxy und Ethoxy substituiert sein kann,
- R³: für Wasserstoff oder eine Gruppe der Formel 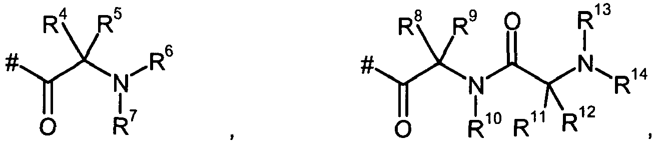 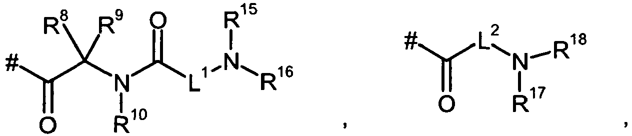 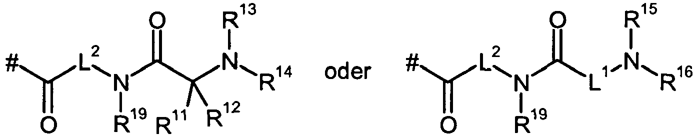 steht, wobei
# für die Anknüpfstelle an das Sauerstoffatom steht,
L¹ für Ethan-1,2-diyl steht,
L² für Ethan-1,2-diyl oder Propan-1,3-diyl steht,
R⁴ für Wasserstoff, Methyl, 2-Methylpropan-1-yl, Hydroxymethyl, 1-Hydroxyethyl, 4-Aminobutan-1-yl oder 3-Aminopropan-1-yl steht,
R⁵ für Wasserstoff steht,
R⁶ für Wasserstoff oder Methyl steht,
R⁷ für Wasserstoff oder Methyl steht, oder
R⁷ zusammen mit R⁴ und den Atomen, an die sie jeweils gebunden sind, einen Pyrrolidinring bildet,
R⁸ für Wasserstoff, Methyl, Propan-2-yl, 1-Methylpropan-1-yl, 2-Methylpropan-1-yl oder 1-Hydroxyethyl steht,
R⁹ für Wasserstoff steht,
R¹⁰ für Wasserstoff steht,
R¹¹ für Methyl, 1-Methylpropan-1-yl, Imidazol-4-ylmethyl, 4-Aminobutan-1-yl, 3-Aminopropan-1-yl, 2-Aminoethyl, Aminomethyl oder 3-Guanidinopropan-1-yl steht,
R¹² für Wasserstoff steht,
R¹³ für Wasserstoff oder Methyl steht,
R¹⁴ für Wasserstoff oder Methyl steht, oder
R¹⁴ zusammen mit R¹¹ und den Atomen, an die sie jeweils gebunden sind, einen Pyrrolidinring bildet,
R¹⁵ für Wasserstoff oder Methyl steht,
R¹⁶ für Wasserstoff oder Methyl steht,
R¹⁷ für Wasserstoff oder Methyl steht,
R¹⁸ für Wasserstoff oder Methyl steht,
R¹⁹ für Wasserstoff oder Methyl steht,
sowie ihre Salze, Solvate, und Solvate der Salze.

Besonders bevorzugt sind im Rahmen der vorliegenden Erfindung Verbindungen der Formel (I), in welcher
- R¹: für Wasserstoff, Methyl oder Ethyl steht,
- R²: für (C₁-C₃)-Alkyl, Cyclopropyl oder Cyclobutyl steht,
wobei (C₁-C₃)-Alkyl mit 1 oder 2 Substituenten unabhängig voneinander ausgewählt aus der Gruppe Fluor, Chlor, Trifluormethyl, Methoxy, Ethoxy, Cyclopropyl und Cyclobutyl substituiert sein kann,
oder
- R¹ und R²: zusammen mit dem Stickstoffatom, an das sie gebunden sind, einen 4- bis 6-gliedrigen Heterocyclus, der ein weiteres Heteroatom aus der Reihe N, O und S enthalten kann, bilden,
wobei der 4- bis 6-gliedrige Heterocyclus mit 1 oder 2 Substituenten unabhängig voneinander ausgewählt aus der Gruppe Fluor, Trifluormethyl, Methyl, Ethyl, Methoxy und Ethoxy substituiert sein kann,
- R³: für Wasserstoff oder eine Gruppe der Formel 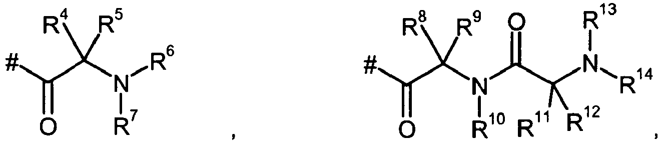 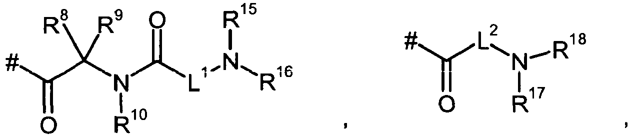 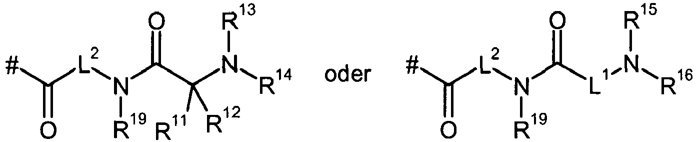 steht, wobei
# für die Anknüpfstelle an das Sauerstoffatom steht,
L¹ für Ethan-1,2-diyl steht,
L² für Ethan-1,2-diyl steht,
R⁴ für Methyl oder 3-Aminopropan-1-yl steht,
R⁵ für Wasserstoff steht,
R⁶ für Wasserstoff steht,
R⁷ für Wasserstoff steht,
R⁸ für Methyl oder 2-Methylpropan-1-yl steht,
R⁹ für Wasserstoff steht,
R¹⁰ für Wasserstoff steht,
R¹¹ für Methyl, 1-Methylpropan-1-yl, Imidazol-4-ylmethyl, 4-Aminobutan-1-yl, 3-Aminopropan-1-yl, 2-Aminoethyl, Aminomethyl oder 3-Guanidinopropan-1-yl steht,
R¹² für Wasserstoff steht,
R¹³ für Wasserstoff steht,
R¹⁴ für Wasserstoff steht, oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
R¹⁷ für Wasserstoff steht,
R¹⁸ für Wasserstoff steht,
R¹⁹ für Wasserstoff steht,
sowie ihre Salze, Solvate und Solvate der Salze.

Besonders bevorzugt sind im Rahmen der vorliegenden Erfindung auch Verbindungen der Formel (I), in welcher
- R¹ und R²: zusammen mit dem Stickstoffatom, an das sie gebunden sind, einen Azetidinyl-, Pyrrolidinyl- oder Piperidinylring bilden,
wobei der Azetidinyl- und Piperidinylring mit einem Substituenten Methoxy substituiert sein können,
- R³: für eine Gruppe der Formel 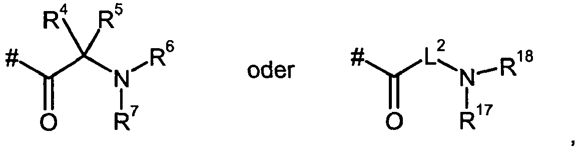 steht, wobei
# für die Anknüpfstelle an das Sauerstoffatom steht,
L² für Ethan-1,2-diyl steht,
R⁴ für Wasserstoff, Methyl, 1-Methylpropan-1-yl, 4-Aminobutan-1-yl oder 3-Guanidinopropan-1-yl steht,
R⁵ für Wasserstoff steht,
R⁶ für Wasserstoff steht,
R⁷ für Wasserstoff steht,
R¹⁷ für Wasserstoff steht, und
R¹⁸ für Wasserstoff steht,
sowie ihre Salze, Solvate und Solvate der Salze.

Besonders bevorzugt sind im Rahmen der vorliegenden Erfindung auch Verbindungen der Formel (I), in welcher
- R¹ und R²: zusammen mit dem Stickstoffatom, an das sie gebunden sind, einen Azetidinyl-, Pyrrolidinyl- oder Piperidinylring bilden,
wobei der Azetidinyl- und Piperidinylring mit einem Substituenten Methoxy substituiert sein können,
- R³: für eine Gruppe der Formel 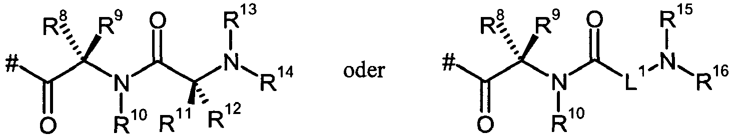 steht, wobei
# für die Anknüpfstelle an das Sauerstoffatom steht,
L¹ für Ethan-1,2-diyl steht,
R⁸ für Methyl oder iso-Butyl steht,
R⁹ für Wasserstoff steht,
R¹⁰ für Wasserstoff steht,
R¹¹ für Wasserstoff, Methyl, 1-Methylpropan-1-yl, Imidazol-4-ylmethyl, 4-Aminobutan-1-yl, 3-Aminopropan-1-yl, 2-Aminoethyl, Aminomethyl, Imidazol-4-ylmethyl oder 3-Guanidinopropan-1-yl steht,
R¹² für Wasserstoff steht,
R¹³ für Wasserstoff steht,
R¹⁴ für Wasserstoff steht, oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
sowie ihre Salze, Solvate und Solvate der Salze.

Besonders bevorzugt sind im Rahmen der vorliegenden Erfindung auch Verbindungen der Formel (I), in welcher
- R¹ und R²: zusammen mit dem Stickstoffatom, an das sie gebunden sind, einen Azetidinyl-, Pyrrolidinyl- oder Piperidinylring bilden,
wobei der Azetidinyl- und Piperidinylring mit einem Substituenten Methoxy substituiert sein können,
- R³: für eine Gruppe der Formel 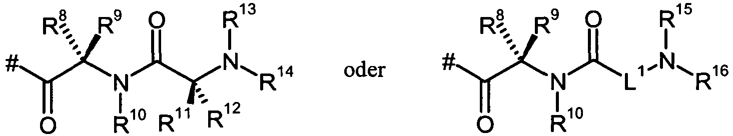 steht, wobei
# für die Anknüpfstelle an das Sauerstoffatom steht,
L¹ für Ethan-1,2-diyl steht,
R⁸ für Methyl oder iso-Butyl steht,
R⁹ für Wasserstoff steht,
R¹⁰ für Wasserstoff steht,
R¹¹ für Wasserstoff, Methyl, 1-Methylpropan-1-yl, 4-Aminobutan-1-yl oder 3-Guanidinopropan-1-yl steht,
R¹² für Wasserstoff steht,
R¹³ für Wasserstoff steht,
R¹⁴ für Wasserstoff steht, oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
sowie ihre Salze, Solvate und Solvate der Salze.

Besonders bevorzugt sind im Rahmen der vorliegenden Erfmdung auch Verbindungen der Formel (I), in welcher
- R¹ und R²: zusammen mit dem Stickstoffatom, an das sie gebunden sind, einen Pyrrolidinylring bilden,
- R³: für eine Gruppe der Formel 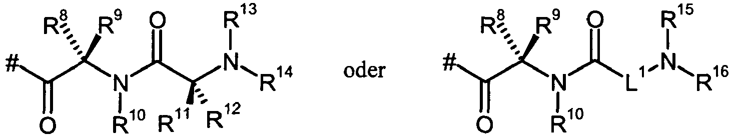 steht, wobei
# für die Anknüpfstelle an das Sauerstoffatom steht,
L¹ für Ethan-1,2-diyl steht,
R⁸ für Methyl oder iso-Butyl steht,
R⁹ für Wasserstoff steht,
R¹⁰ für Wasserstoff steht,
R¹¹ für Wasserstoff, Methyl, 1-Methylpropan-1-yl, 4-Aminobutan-1-yl oder 3-Guanidinopropan-1-yl steht,
R¹² für Wasserstoff steht,
R¹³ für Wasserstoff steht,
R¹⁴ für Wasserstoff steht, oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
sowie ihre Salze, Solvate und Solvate der Salze.

Besonders bevorzugt sind im Rahmen der vorliegenden Erfindung auch Verbindungen der Formel (I), in welcher
- R¹: für Wasserstoff, Methyl oder Ethyl steht,
- R²: für (C₁-C₃)-Alkyl, Cyclopropyl oder Cyclobutyl steht,
wobei (C₁-C₃)-Alkyl mit 1 oder 2 Substituenten unabhängig voneinander ausgewählt aus der Gruppe Fluor, Chlor, Trifluormethyl, Methoxy, Ethoxy, Cyclopropyl und Cyclobutyl substituiert sein kann,
- R³: für Wasserstoff steht
sowie ihre Salze, Solvate und Solvate der Salze.

Besonders bevorzugt sind im Rahmen der vorliegenden Erfindung auch Verbindungen der Formel (I), in welcher
- R¹: für Wasserstoff, Methyl oder Ethyl steht,
- R²: für Methyl, Ethyl oder n-Propyl steht,
wobei Methyl, Ethyl und n-Propyl mit 1 oder 2 Substituenten unabhängig voneinander ausgewählt aus der Gruppe Fluor, Trifluormethyl und Methoxy substituiert sein kann,
oder
- R¹ und R²: zusammen mit dem Stickstoffatom, an das sie gebunden sind, einen Azetidinyl-, Pyrrolidinyl- oder Piperidinylring bilden,
wobei der Azetidinyl- und Piperidinylring mit einem Substituenten Methoxy substituiert sein können,
- R³: für Wasserstoff steht,
sowie ihre Salze, Solvate und Solvate der Salze.

Besonders bevorzugt sind im Rahmen der vorliegenden Erfindung auch Verbindungen der Formel (I), in welcher
- R¹: für Wasserstoff, Methyl oder Ethyl steht,
- R²: für (C₁-C₃)-Alkyl, Cyclopropyl oder Cyclobutyl steht,
wobei (C₁-C₃)-Alkyl mit 1 oder 2 Substituenten unabhängig voneinander ausgewählt aus der Gruppe Fluor, Chlor, Trifluormethyl, Methoxy, Ethoxy, Cyclopropyl und Cyclobutyl substituiert sein kann,
- R³: für eine Gruppe der Formel 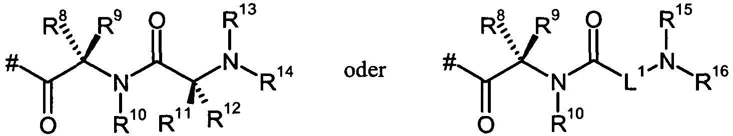 steht, wobei
# für die Anknüpfstelle an das Sauerstoffatom steht,
L¹ für Ethan-1,2-diyl steht,
R⁸ für Methyl oder iso-Butyl steht,
R⁹ für Wasserstoff steht,
R¹⁰ für Wasserstoff steht,
R¹¹ für Wasserstoff, Methyl, 1-Methylpropan-1-yl, 4-Aminobutan-l-yl oder 3-Guanidinopropan-1-yl steht,
R¹² für Wasserstoff steht,
R¹³ für Wasserstoff steht,
R¹⁴ für Wasserstoff steht, oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
sowie ihre Salze, Solvate und Solvate der Salze.

Besonders bevorzugt sind im Rahmen der vorliegenden Erfindung auch Verbindungen der Formel (I), in welcher
- R¹ und R²: zusammen mit dem Stickstoffatom, an das sie gebunden sind, einen Azetidinyl-, Pyrrolidinyl- oder Piperidinylring bilden,
wobei der Azetidinyl-, Pyrrolidinyl- oder Piperidinylring mit 1 oder 2 Substituenten unabhängig voneinander ausgewählt aus der Gruppe Fluor, Trifluormethyl, Methyl, Ethyl, Methoxy und Ethoxy substituiert sein kann,
und
- R³: für Wasserstoff steht
sowie ihre Salze, Solvate und Solvate der Salze.

Besonders bevorzugt sind im Rahmen der vorliegenden Erfindung auch Verbindungen der Formel (I), in welcher
- R¹ und R²: zusammen mit dem Stickstoffatom, an das sie gebunden sind, einen Pyrrolidinylring bilden,
- R³: für eine Gruppe der Formel 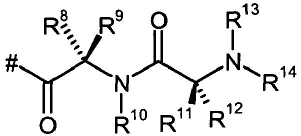 steht, wobei
# für die Anknüpfstelle an das Sauerstoffatom steht,
R⁸ für Methyl steht,
R⁹ für Wasserstoff steht,
R¹⁰ für Wasserstoff steht,
R¹¹ für Methyl oder 1-Methylpropan-1-yl steht,
R¹² für Wasserstoff steht,
R¹³ für Wasserstoff steht,
R¹⁴ für Wasserstoff steht,
sowie ihre Salze, Solvate und Solvate der Salze.

Bevorzugt sind im Rahmen der vorliegenden Erfindung auch Verbindungen der Formel (I), in welcher R³ für Wasserstoff steht, sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in welcher
- R³: für Wasserstoff oder eine Gruppe der Formel 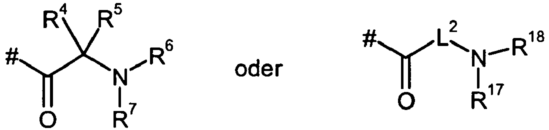 steht, wobei
# für die Anknüpfstelle an das Sauerstoffatom steht,
L² für Ethan-1,2-diyl steht,
R⁴ für Methyl oder 3-Aminopropan-1-yl steht,
R⁵ für Wasserstoff steht,
R⁶ für Wasserstoff steht,
R⁷ für Wasserstoff steht,
R¹⁷ für Wasserstoff steht,
R¹⁸ für Wasserstoff steht,
sowie ihre Salze, Solvate und Solvate der Salze.

Bevorzugt sind im Rahmen der vorliegenden Erfindung auch Verbindungen der Formel (I), in welcher
- R³: für Wasserstoff oder eine Gruppe der Formel 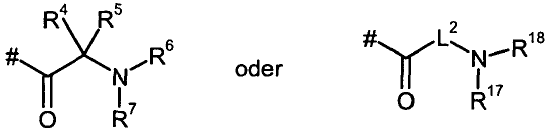 steht, wobei
# für die Anknüpfstelle an das Sauerstoffatom steht,
L² für Ethan-1,2-diyl oder Propan-1,3-diyl steht,
R⁴ für Wasserstoff, Methyl, 2-Methylpropan-1-yl, Hydroxymethyl, 1-Hydroxyethyl, 4-Aminobutan-1-yl oder 3-Aminopropan-1-yl steht,
R⁵ für Wasserstoff steht,
R⁶ für Wasserstoff oder Methyl steht,
R⁷ für Wasserstoff oder Methyl steht, oder
R⁷ zusammen mit R⁴ und den Atomen, an die sie jeweils gebunden sind, einen Pyrrolidinring bildet,
R¹⁷ für Wasserstoff oder Methyl steht,
R¹⁸ für Wasserstoff oder Methyl steht,
sowie ihre Salze, Solvate und Solvate der Salze.

Bevorzugt sind im Rahmen der vorliegenden Erfindung auch Verbindungen der Formel (I), in welcher
- R³: für Wasserstoff oder eine Gruppe der Formel 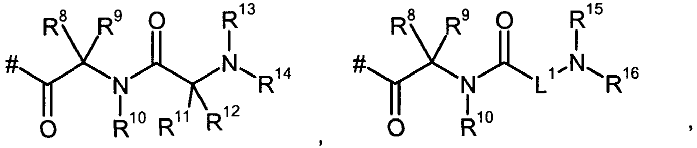 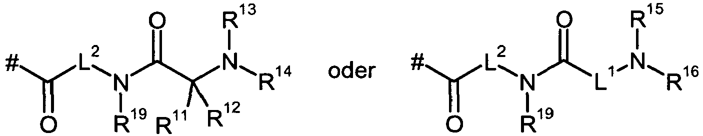 steht, wobei
# für die Anknüpfstelle an das Sauerstoffatom steht,
L¹ für Ethan-1,2-diyl steht,
L² für Ethan-1,2-diyl steht,
R⁸ für Wasserstoff, Methyl, Propan-2-yl, 1-Methylpropan-1-yl, 2-Methylpropan-1-yl oder 1-Hydroxyethyl steht,
R⁹ für Wasserstoff steht,
R¹⁰ für Wasserstoff steht,
R¹¹ für Methyl, 1-Methylpropan-1-yl, Imidazol-4-ylmethyl, 4-Aminobutan-1-yl, 3-Aminopropan-1-yl, 2-Aminoethyl, Aminomethyl oder 3-Guanidinopropan-1-yl steht,
R¹² für Wasserstoff steht,
R¹³ für Wasserstoff oder Methyl steht,
R¹⁴ für Wasserstoff oder Methyl steht, oder
R¹⁴ zusammen mit R¹¹ und den Atomen, an die sie jeweils gebunden sind, einen Pyrrolidinring bildet,
R¹⁵ für Wasserstoff oder Methyl steht,
R¹⁶ für Wasserstoff oder Methyl steht,
R¹⁷ für Wasserstoff oder Methyl steht,
R¹⁸ für Wasserstoff oder Methyl steht,
R¹⁹ für Wasserstoff oder Methyl steht
sowie ihre Salze, Solvate und Solvate der Salze.

Bevorzugt sind im Rahmen der vorliegenden Erfindung auch Verbindungen der Formel (I), in welcher
- R³: für Wasserstoff oder eine Gruppe der Formel 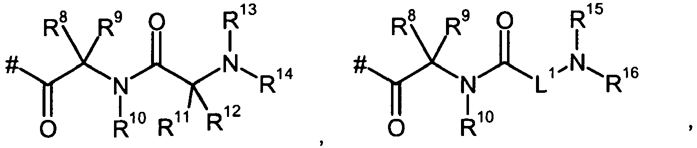 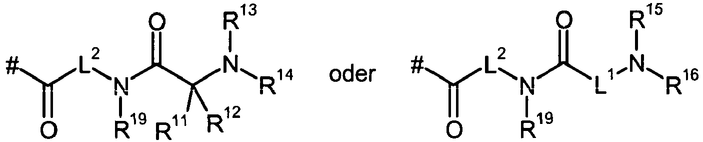 steht, wobei
# für die Anknüpfstelle an das Sauerstoffatom steht,
L¹ für Ethan-1,2-diyl steht,
L² für Ethan-1,2-diyl oder Propan-1,3-diyl steht,
R⁸ für Methyl oder 2-Methylpropan-1-yl steht,
R⁹ für Wasserstoff steht,
R¹⁰ für Wasserstoff steht,
R¹¹ für Methyl, 1-Methylpropan-1-yl, hnidazol-4-ylmethyl, 4-Aminobutan-1-yl, 3-Aminopropan-1-yl, 2-Aminoethyl, Aminomethyl oder 3-Guanidinopropan-1-yl steht,
R¹² für Wasserstoff steht,
R¹³ für Wasserstoff steht,
R¹⁴ für Wasserstoff steht, oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
R¹⁷ für Wasserstoff steht,
R¹⁸ für Wasserstoff steht,
R¹⁹ für Wasserstoff steht,
sowie ihre Salze, Solvate und Solvate der Salze.

Bevorzugt sind im Rahmen der vorliegenden Erfindung auch Verbindungen der Formel (I), in welcher
- R³: für Wasserstoff oder eine Gruppe der Formel 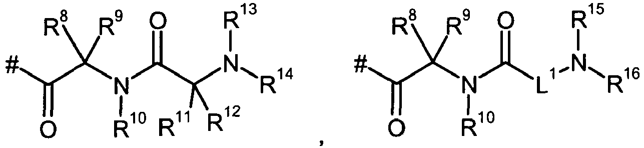 oder 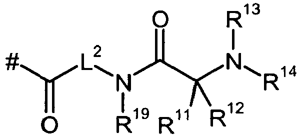 steht, wobei
# für die Anknüpfstelle an das Sauerstoffatom steht,
L¹ für Ethan-1,2-diyl steht,
L² für Ethan-1,2-diyl steht,
R⁸ für Methyl steht,
R⁹ für Wasserstoff steht,
R¹⁰ für Wasserstoff steht,
R¹¹ für Methyl, 1-Methylpropan-1-yl, 4-Aminobutan-1-yl oder 3-Aminopropan-1-yl steht,
R¹² für Wasserstoff steht,
R¹³ für Wasserstoff steht,
R¹⁴ für Wasserstoff steht, oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
R¹⁷ für Wasserstoff steht,
R¹⁸ für Wasserstoff steht,
R¹⁹ für Wasserstoff steht,
sowie ihre Salze, Solvate und Solvate der Salze. Bevorzugt sind im Rahmen der vorliegenden Erfindung auch Verbindungen der Formel (I), in welcher
- R¹: für Wasserstoff, Methyl oder Ethyl steht,
- R²: für (C₁-C₃)-Alkyl steht,
wobei (C₁-C₃)-Alkyl mit 1 oder 2 Substituenten unabhängig voneinander ausgewählt aus der Gruppe Fluor, Chlor, Trifluormethyl, Methoxy, Ethoxy, Cyclopropyl und Cyclobutyl substituiert sein kann,
oder
- R¹ und R²: zusammen mit dem Stickstoffatom, an das sie gebunden sind, einen 4- bis 6-gliedrigen Heterocyclus, der ein weiteres Heteroatom aus der Reihe N, O und S enthalten kann, bilden,
wobei der 4- bis 6-gliedrige Heterocyclus mit 1 oder 2 Substituenten unabhängig voneinander ausgewählt aus der Gruppe Fluor, Trifluormethyl, Methyl, Ethyl, Methoxy und Ethoxy substituiert sein kann,
sowie ihre Salze, Solvate und Solvate der Salze.

Bevorzugt sind im Rahmen der vorliegenden Erfindung auch Verbindungen der Formel (I), in welcher
- R¹: für Ethyl steht,
- R²: für Ethyl steht,
oder
- R¹ und R²: zusammen mit dem Stickstoffatom, an das sie gebunden sind, einen 4- bis 6-gliedrigen Heterocyclus, der ein weiteres Heteroatom aus der Reihe N, O und S enthalten kann, bilden,
sowie ihre Salze, Solvate und Solvate der Salze.

Bevorzugt sind im Rahmen der vorliegenden Erfindung auch Verbindungen der Formel (I), in welcher
- R³: für eine Gruppe der Formel 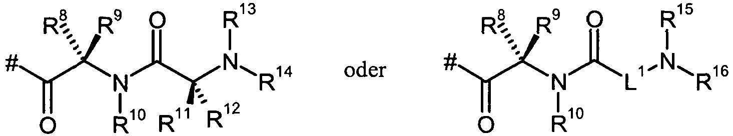 steht, wobei
# für die Anknüpfstelle an das Sauerstoffatom steht,
L¹ für Ethan-1,2-diyl steht,
R⁸ für Methyl oder iso-Butyl steht,
R⁹ für Wasserstoff steht,
R¹⁰ für Wasserstoff steht,
R¹¹ für Wasserstoff, Methyl, 1-Methylpropan-1-yl oder 3-Guanidinopropan-1-yl steht,
R¹² für Wasserstoff steht,
R¹³ für Wasserstoff steht,
R¹⁴ für Wasserstoff steht, oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
sowie ihre Salze, Solvate und Solvate der Salze.

Bevorzugt sind im Rahmen der vorliegenden Erfindung auch Verbindungen der Formel (I), in welcher
- R³: für eine Gruppe der Formel 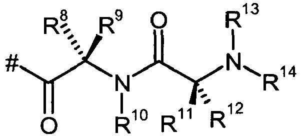 steht, wobei
# für die Anknüpfstelle an das Sauerstoffatom steht,
R⁸ für Methyl steht,
R⁹ für Wasserstoff steht,
R¹⁰ für Wasserstoff steht,
R¹¹ für Methyl oder 1-Methylpropan-1-yl steht,
R¹² für Wasserstoff steht,
R¹³ für Wasserstoff steht,
R¹⁴ für Wasserstoff steht,
sowie ihre Salze, Solvate und Solvate der Salze.

Bevorzugt sind im Rahmen der vorliegenden Erfmdung auch Verbindungen der Formel (I), in welcher
- R¹ und R²: zusammen mit dem Stickstoffatom, an das sie gebunden sind, einen Azetidinyl-, Pyrrolidinyl- oder Piperidinylring bilden,
wobei Azetidinyl-, Pyrrolidinyl- oder Piperidinylring mit 1 oder 2 Substituenten unabhängig voneinander ausgewählt aus der Gruppe Fluor, Trifluormethyl, Methyl, Ethyl, Methoxy und Ethoxy substituiert sein kann,
sowie ihre Salze, Solvate und Solvate der Salze.

Bevorzugt sind im Rahmen der vorliegenden Erfindung auch Verbindungen der Formel (I), in welcher
- R¹ und R²: zusammen mit dem Stickstoffatom, an das sie gebunden sind, einen Azetidinyl-, Pyrrolidinyl- oder Piperidinylring bilden,
sowie ihre Salze, Solvate und Solvate der Salze.

Bevorzugt sind im Rahmen der vorliegenden Erfindung auch Verbindungen der Formel (I), in welcher
- R¹ und R²: zusammen mit dem Stickstoffatom, an das sie gebunden sind, einen Pyrrolidinylring bilden,
wobei der Pyrrolidinylring mit 1 oder 2 Substituenten unabhängig voneinander ausgewählt aus der Gruppe Fluor, Trifluormethyl, Methyl, Ethyl, Methoxy und Ethoxy substituiert sein kann,
sowie ihre Salze, Solvate und Solvate der Salze.

Besonders bevorzugt sind im Rahmen der vorliegenden Erfindung die folgenden Verbindungen:
2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril
2-({[2-(4-Chlorphenyl)-1,3-thiazol-4-yl]methyl}sulfanyl)-4-[4-(2-hydroxyethoxy)phenyl]-6-(methylamino)pyridin-3,5-dicarbonitril
2-({[2-(4-Chlorphenyl)-1,3-thiazol-4-yl]methyl}thio)-6-(ethylamino)-4-[4-(2-hydroxyethoxy)phenyl]pyridin-3,5-dicarbonitril
2-({[2-(4-Chlorphenyl)-1,3-thiazol-4-yl]methyl}thio)-6-(dimethylamino)-4-[4-(2-hydroxyethoxy)phenyl]pyridin-3,5-dicarbonitril
2-({[2-(4-Chlorphenyl)-1,3-thiazol-4-yl]methyl}thio)-6-[ethyl(methyl)amino]-4-[4-(2-hydroxyethoxy)phenyl]pyridin-3,5-dicarbonitril
2-({[2-(4-Chlorphenyl)-1,3-thiazol-4-yl]methyl}thio)-6-(diethylamino)-4-[4-(2-hydroxyethoxy)phenyl]pyridin-3,5-dicarbonitril
2-({[2-(4-Chlorphenyl)-1,3-thiazol-4-yl]methyl}thio)-4-[4-(2-hydroxyethoxy)phenyl]-6-(isopropylamino)pyridin-3,5-dicarbonitril
2-Azetidin-1-yl-6-({[2-(4-chlorphenyl)-1,3-thiazol-4-yl]methyl}thio)-4-[4-(2-hydroxyethoxy)phenyl]pyridin-3,5-dicarbonitril
2-({[2-(4-Chlorphenyl)-1,3-thiazol-4-yl]methyl}sulfanyl)-6-(cyclopropylamino)-4-[4-(2-hydroxyethoxy)phenyl]pyridin-3,5-dicarbonitril
2-({[2-(4-Chlorphenyl)-1,3-thiazol-4-yl]methyl}sulfanyl)-6-(cyclobutylamino)-4-[4-(2-hydroxyethoxy)phenyl]pyridin-3,5-dicarbonitril
2-({[2-(4-Chlorphenyl)-1,3-thiazol-4-yl]methyl}sulfanyl)-4-[4-(2-hydroxyethoxy)phenyl]-6-[(3,3,3-trifluorpropyl)amino]pyridin-3,5-dicarbonitril
2-({[2-(4-Chlorphenyl)-1,3-thiazol-4-yl]methyl}sulfanyl)-4-[4-(2-hydroxyethoxy)phenyl]-6-(propylamino)pyridin-3,5-dicarbonitril
2-({[2-(4-Chlorphenyl)-1,3-thiazol-4-yl]methyl}sulfanyl)-4-[4-(2-hydroxyethoxy)phenyl]-6-(piperidin-1-yl)pyridin-3,5-dicarbonitril
2-({[2-(4-Chlorphenyl)-1,3-thiazol-4-yl]methyl}sulfanyl)-4-[4-(2-hydroxyethoxy)phenyl]-6-[(3-methylbutyl)amino]pyridin-3,5-dicarbonitril
2-((Azepan-1-yl)-6-({[2-(4-chlorphenyl)-1,3-thiazol-4-yl]methyl}sulfanyl)-4-[4-(2-hydroxyethoxy)phenyl]pyridin-3,5-dicarbonitril
2-({[2-(4-Chlorphenyl)-1,3-thiazol-4-yl]methyl}sulfanyl)-4-[4-(2-hydroxyethoxy)phenyl]-6-(4-methylpiperidin-1-yl)pyridin-3,5-dicarbonitril
2-({((2-(4-Chlorphenyl)-1,3-thiazol-4-yl]methyl}sulfanyl)-4-[4-(2-hydroxyethoxy)phenyl]-6-(morpholin-4-yl)pyridin-3,5-dicarbonitril
2-({[2-(4-Chlorphenyl)-1,3-thiazol-4-yl]methyl}sulfanyl)-6-(4,4-dimethylpiperidin-1-yl)-4-[4-(2-hydroxyethoxy)phenyl]pyridin-3,5-dicarbonitril
2-({[2-(4-Chlorphenyl)-1,3-thiazol-4-yl]methyl}sulfanyl)-6-[(2,2-difluorethyl)(methyl)amino]-4-[4-(2-hydroxyethoxy)phenyl]pyridin-3,5-dicarbonitril
2-({[2-(4-Chlorphenyl)-1,3-thiazol-4-yl]methyl}sulfanyl)-4-[4-(2-hydroxyethoxy)phenyl]-6-[methyl(propyl)amino]pyridin-3,5-dicarbonitril
2-({[2-(4-Chlorphenyl)-1,3-thiazol-4-yl]methyl}sulfanyl)-4-[4-(2-hydroxyethoxy)phenyl]-6-[(2-methoxyethyl)(methyl)amino]pyridin-3,5-dicarbonitril
2-({[2-(4-Chlorphenyl)-1,3-thiazol-4-yl]methyl}sulfanyl)-4-[4-(2-hydroxyethoxy)phenyl]-6-[(2-methoxyethyl)amino]pyridin-3,5-dicarbonitril
2-({[2-(4-Chlorphenyl)-1,3-thiazol-4-yl]methyl}sulfanyl)-6-[(2-ethoxyethyl)amino]-4-[4-(2-hydroxyethoxy)phenyl]pyridin-3,5-dicarbonitril
2-({[2-(4-Chlorphenyl)-1,3-thiazol-4-yl]methyl}sulfanyl)-4-[4-(2-hydroxyethoxy)phenyl]-6-(4-methoxypiperidin-1-yl)pyridin-3,5-dicarbonitril
2-({[2-(4-Chlorphenyl)-1,3-thiazol-4-yl]methyl}sulfanyl)-6-[(3R)-3-ethoxypyrrolidin-1-yl]-4-[4-(2-hydroxyethoxy)phenyl]pyridin-3,5-dicarbonitril
2-({[2-(4-Chlorphenyl)-1,3-thiazol-4-yl]methyl}sulfanyl)-6-(3,3-difluorpyrrolidin-1-yl)-4-[4-(2-hydroxyethoxy)phenyl]pyridin-3,5-dicarbonitril
2-({[2-(4-Chlorphenyl)-1,3-thiazol-4-yl]methyl}sulfanyl)-6-(4,4-difluorpipehdin-1-yl)-4-[4-(2-hydroxyethoxy)phenyl]pyridin-3,5-dicarbonitril
2-({[2-(4-Chlorphenyl)-1,3-thiazol-4-yl]methyl}sulfanyl)-4-[4-(2-hydroxyethoxy)phenyl]-6-[4-(trifluormethyl)piperidin-1-yl]pyridin-3,5-dicarbonitril
2-({[2-(4-Chlorphenyl)-1,3-thiazol-4-yl]methyl}sulfanyl)-6-(3,3-difluorazetidin-1-yl)-4-[4-(2-hydroxyethoxy)phenyl]pyridin-3,5-dicarbonitril
2-({[2-(4-Chlorphenyl)-1,3-thiazol-4-yl]methyl}sulfanyl)-4-[4-(2-hydroxyethoxy)phenyl]-6-(3-methoxyazetidin-1-yl)pyridin-3,5-dicarbonitril
2-({[2-(4-Chlorphenyl)-1,3-thiazol-4-yl]methyl}sulfanyl)-6-(3,3-difluorpiperidin-1-yl)-4-[4-(2-hydroxyethoxy)phenyl]pyridin-3,5-dicarbonitril
2-({[2-(4-Chlorphenyl)-1,3-thiazol-4-yl]methyl}sulfanyl)-4-[4-(2-hydroxyethoxy)phenyl]-6-[(2,2,2-trifluorethyl)amino]pyridin-3,5-dicarbonitril
2-({[2-(4-Chlorphenyl)-1,3-thiazol-4-yl]methyl}sulfanyl)-6-[(2-fluorethyl)amino]-4-[4-(2-hydroxyethoxy)phenyl]pyridin-3,5-dicarbonitril
2-({[2-(4-Chlorphenyl)-1,3-thiazol-4-yl]methyl}sulfanyl)-6-[(2,2-difluorethyl)amino]-4-[4-(2-hydroxyethoxy)phenyl]pyridin-3,5-dicarbonitril
2-({[2-(4-Chlorphenyl)-1,3-thiazol-4-yl]methyl)sulfanyl)-4-[4-(2-hydroxyethoxy)phenyl]-6-[methyl(2,2,2-trifluorethyl)amino]pyridin-3,5-dicarbonitril
2-({[2-(4-Chlorphenyl)-1,3-thiazol-4-yl]methyl}sulfanyl)-6-[ethyl(2,2,2-trifluorethyl)amino]-4-[4-(2-hydroxyethoxy)phenyl]pyridin-3,5-dicarbonitril
2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-eta-alanyl-L-alaninat
2-{4-[2-({[2-(4-Chlorphenyl)-1,3-thiazol-4-yl]methyl}sulfanyl)-3,5-dicyan-6-(propylamino)pyridin-4-yl]phenoxy}ethyl-L-alaninat-Trifluoracetat
2-{4-[2-({[2-(4-Chlorphenyl)-1,3-thiazol-4-yl]methyl}sulfanyl)-3,5-dicyan-6-(methylamino)pyridin-4-yl]phenoxy}ethyl-L-alaninat-Trifluoracetat
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat
2- {4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-lysyl-L-alaninat-Dihydrochlorid
2-{4-[2-({[2-(4-Chlorphenyl)-1,3-thiazol-4-yl]methyl}sulfanyl)-3,5-dicyan-6-(methylamino)pyridin-4-yl]phenoxyl}ethyl-L-lysyl-L-alaninat-Dihydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-lysyl-beta-alaninat-Dihydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-alaninat-Hydrochlorid
2-{4-[2-(Azetidin-1-yl)-6-({[2-(4-chlorphenyl)-1,3-thiazol-4-yl]methyl}sulfanyl)-3,5-dicyanopyridin-4-yl]phenoxy}ethyl-L-alaninat-Trifluoracetat
2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-L-lysyl-L-alaninat-Bistrifluoracetat
2-{4-[2-({[2-(4-Chlorphenyl)-1,3-thiazol-yl]methyl}sulfanyl)-3,5-dicyan-6-(propylamino)pyridin-4-yl]phenoxy}ethyl-L-ornithinat-Bistrifluoracetat
2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-L-ornithinat-Bistrifluoracetat
2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-beta-alaninat-Trifluoracetat
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-beta-alaninat-Trifluoracetat
2-{4-[2-({[2-(4-Chlorphenyl)-1,3-thiazol-4-yl]methyl}sulfanyl)-3,5-dicyan-6-(propylamino)pyridin-4-yl]phenoxy}ethyl-L-alanyl-L-alaninat-Trifluoracetat
2-{4-[2-({[2-(4-Chlorphenyl)-1,3-thiazol-4-yl]methyl}sulfanyl)-3,5-dicyan-6-(methylamino)pyridin-4-yl]phenoxy}ethyl-L-ornithinat-Bistrifluoracetat
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-beta-alaninat-Trifluoracetat
2-{4-[2-({[2-(4-Chlorphenyl)-1,3-thiazol-4-yl]methyl}sulfanyl)-3,5-dicyan-6-(propylamino)pyridin-4-yl]phenoxy}ethyl-L-lysyl-L-alaninat-Bistrifluoracetat
2-{4-[2-({[2-(4-Chlorphenyl)-1,3-thiazol-4-yl]methyl}sulfanyl)-3,5-dicyan-6-(propylamino)pyridin-4-yl]phenoxy}ethyl-beta-alanyl-L-alaninat-Trifluoracetat
2-{4-[2-({[2-(4-Chlorphenyl)-1,3-thiazol-4-yl]methyl}sulfanyl)-3,5-dicyan-6-(methyl-amino)pyridin-4-yl]phenoxy}ethyl-beta-alaninat-Trifluoracetat
2-{4-[2-({[2-(4-Chlorphenyl)-1,3-thiazol-4-yl]methyl}sulfanyl)-3,5-dicyan-6-(methylamino)pyridin-4-yl]phenoxy}ethyl-L-alanyl-L-alaninat-Trifluoracetat
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-ornithyl-L-alaninat-Bistrifluoracetat
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-ornithinat-Bistrifluoracetat
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-beta-alanyl-L-alaninat-Trifluoracetat
2-{4-[2-({[2-(4-Chlorphenyl)-1,3-thiazol-4-yl]methyl}sulfanyl)-3,5-dicyan-6-(pyaolidin-1-yl)pyridin-4-yl]phenoxy}ethyl-beta-alanyl-L-alaninat-Hydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-prolyl-L-alaninat-Hydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-isoleucyl-L-alaninat-Hydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-[(2S)-2,4-diaminobutanoyl]-L-alaninat-Dihydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-histidyl-L-alaninat-Dihydrochlorid
2-{4-[2-({[2-(4-Chlorphenyl)-1,3-thiazol-4-yl]methyl}sulfanyl)-3,5-dicyan-6-(pyaolidin-1-yl)pyridin-4-yl]phenoxy}ethyl-L-arginyl-L-alaninat-Dihydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-3-amino-L-alanyl-L-alaninat-Bistrifluoracetat
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-leucinat-Hydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-beta-alanyl-L-leucinat-Hydrochlorid
2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-glycyl-L-leucinat-Hydrochlorid,
sowie ihre N-Oxide, Salze, Solvate, Salze der N-Oxide und Solvate der N-Oxide und Salze.

Weiterer Gegenstand der vorliegenden Erfmdung ist ein Verfahren zur Herstellung der erfindungsgemäßen Verbindungen der Formel (I), in welcher R³ für Wasserstoff steht, dadurch gekennzeichnet, dass man die Verbindung der Formel (II) 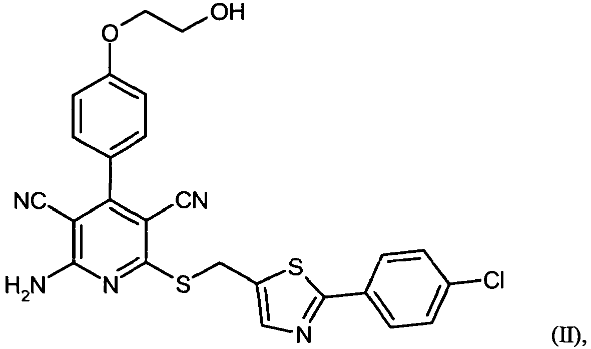 zunächst mit Kupfer(II)chlorid und Isoamylnitrit in einem geeigneten Lösungsmittel in die Verbindung der Formel (III) 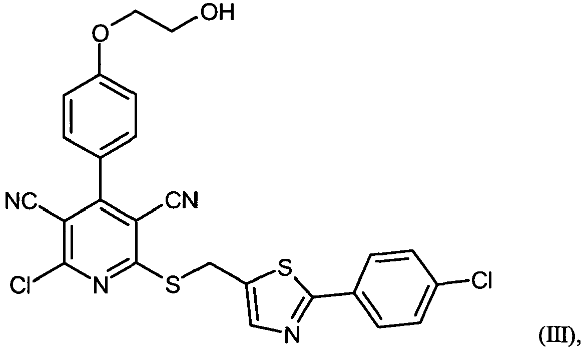 überführt, und diese anschliessend in einem inerten Lösungsmittel gegebenenfalls in Gegenwart einer geeigneten Base mit einer Verbindung der Formel (IV) 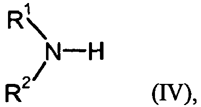 in welcher R¹ und R² jeweils die oben angegebenen Bedeutungen haben,
zu einer Verbindung der Formel (I-A) 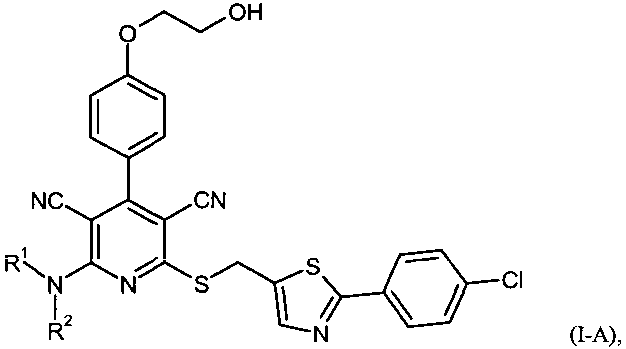 in welcher R¹ und R² jeweils die oben angegebenen Bedeutungen haben,
umsetzt,
anschließend gegebenenfalls vorhandene Schutzgruppen abspaltet und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

Das zuvor beschriebene Verfahren kann durch das folgenden Reaktionsschema 1 beispielhaft erläutert werden:

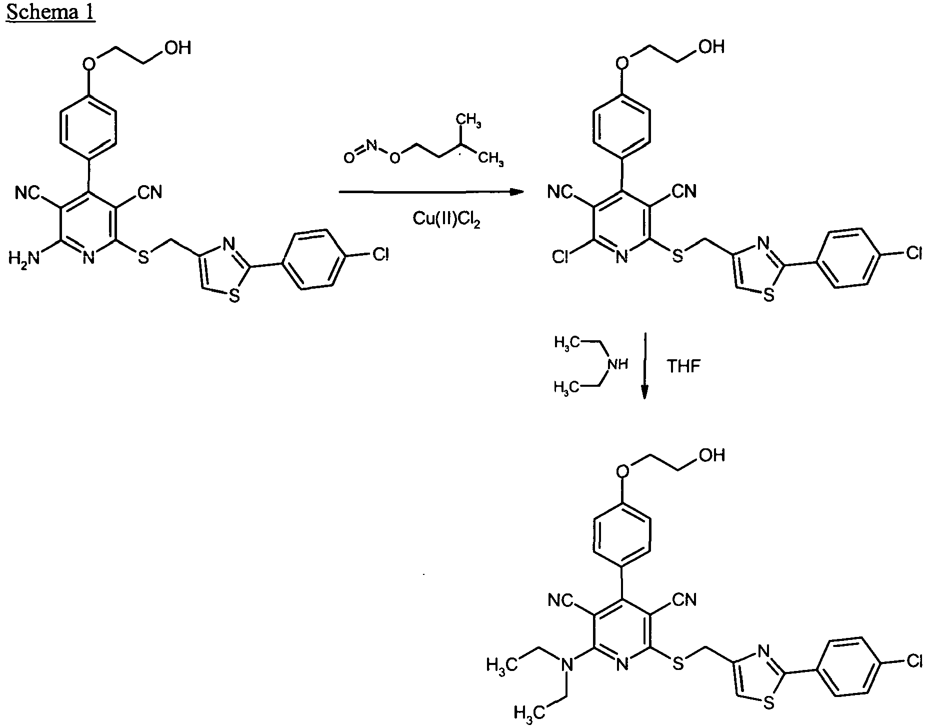

Als Lösungsmittel für die Reaktion (III) + (IV) eignen sich alle organischen Lösungsmittel, die unter den Reaktionsbedingungen inert sind. Hierzu gehören Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), N-Methylpyrrolidinon (NMP), Acetonitril oder Pyridin. Ebenso ist es möglich, Gemische der zuvor genannten Lösungsmittel einzusetzen. Bevorzugt werden Tetrahydrofuran und Dimethylformamid verwendet.

Als Basen für diese Umsetzung eignen sich die üblichen anorganischen oder organischen Basen. Hierzu gehören bevorzugt Alkalicarbonate wie Lithium-, Natrium-, Kalium- oder Cäsiumcarbonat, Alkalihydrogencarbonate wie Natrium- oder Kaliumhydrogencarbonat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN) sowie Phosphazen-Basen (so genannte "Schwesinger-Basen") wie beispielsweise P2-t-Bu oder P4-t-Bu. Bevorzugt sind Cäsiumcarbonat, Triethylamin und Diisopropylethylamin.

Die Reaktion (III) + (IV) erfolgt im Allgemeinen in einem Temperaturbereich von -78°C bis +140°C, bevorzugt im Bereich von -20°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (II) → (III) erfolgt im Allgemeinen mit einem Molverhältnis von 2 bis 12 Mol Kupfer(II)chlorid und 2 bis 12 Mol Isoamylnitrit bezogen auf 1 Mol der Verbindung der Formel (II-A).

Als Lösungsmittel für diesen Verfahrensschritt eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische dieser Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C, bevorzugt im Bereich von +20°C bis +100°C, insbesondere bei +20°C bis +60°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindung der Formel (II) kann wie in WO 03/053441 für Beispiel 6 beschrieben hergestellt werden.

Die Verbindungen der Formel (IV) sind kommerziell erhältlich, literaturbekannt oder nach literaturbekannten Methoden herstellbar.

Weiterer Gegenstand der vorliegenden Erfindung ist ein Verfahren zur Herstellung der erfindungsgemäßen Verbindungen der Formel (I), in welcher
R³ für eine Gruppe der Formel 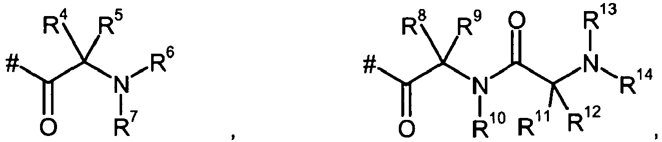 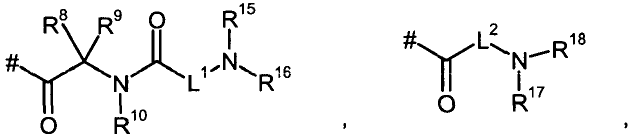 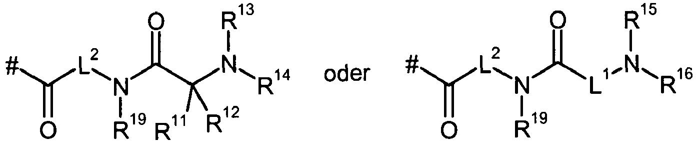 steht, wobei L¹, L², R⁴, R⁵, R⁶, R⁷, R⁸, R⁹, R¹⁰, R¹¹, R¹², R¹³, R¹⁴, R¹⁵, R¹⁶, R¹⁷, R¹⁸ und R¹⁹ jeweils die oben angegebenen Bedeutungen haben,dadurch gekennzeichnet, dass man
[A] eine Verbindung der Formel (I-A) 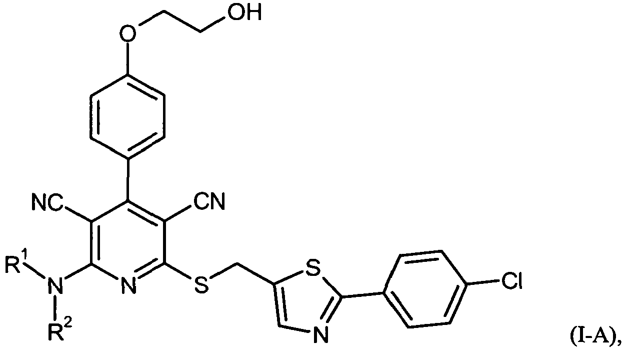 in welcher R¹ und R² jeweils die oben angegebenen Bedeutungen haben,
   in einem inerten Lösungsmittel in Gegenwart eines Kondensationsmittels zunächst mit einer Carbonsäure der Formel (V) oder (VI) 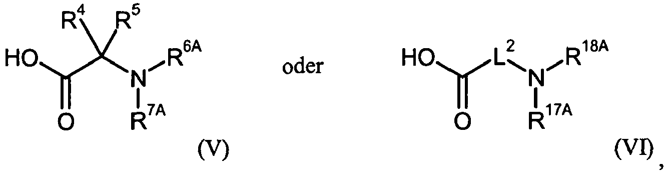 in welcher L², R⁴ und R⁵ jeweils die oben angegebenen Bedeutungen haben
   und
   - R^{6A}, R^{7A}, R^{17A} und R^{18A}: jeweils die für R⁶, R⁷, R¹⁷ bzw. R¹⁸ genannten Bedeutungen haben oder für eine Amino-Schutzgruppe, wie beispielsweise tert.-Butoxycarbonyl, stehen,
   zu einer Verbindungen der Formel (VII) oder (VIII) 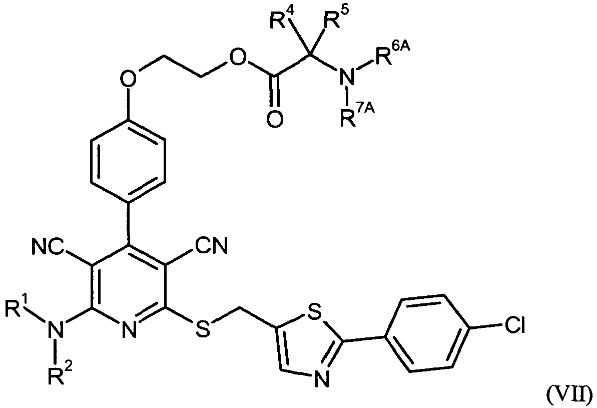 oder 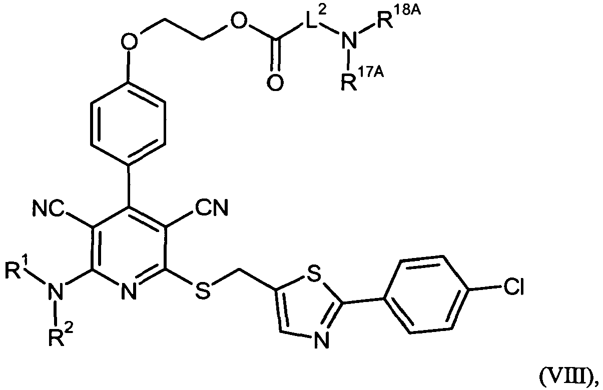 in welchen L², R¹, R², R⁴, R⁵, R^{6A}, R^{7A}, R^{17A} und R^{18A} jeweils die oben angegebenen Bedeutungen haben,
   kuppelt und anschliessend gegebenfalls vorhandene Schutzgruppen zu einer Verbindung der Formel (I-B) oder (I-C) 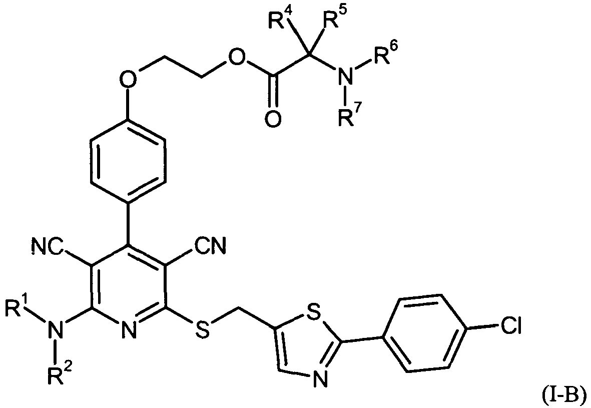 oder 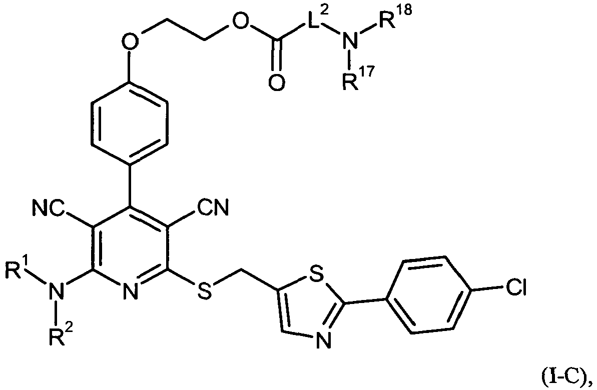 in welchen L², R¹, R², R⁴, R⁵, R⁶, R⁷, R¹⁷ und R¹⁸ jeweils die oben angegebenen Bedeutungen haben,
   abspaltet,
   oder
[B] eine Verbindung der Formel (I-A) in einem inerten Lösungsmittel in Gegenwart eines Kondensationsmittels zunächst mit einer Carbonsäure der Formel (IX), (X), (XI) oder (XII) 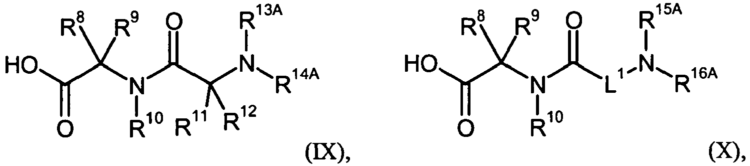 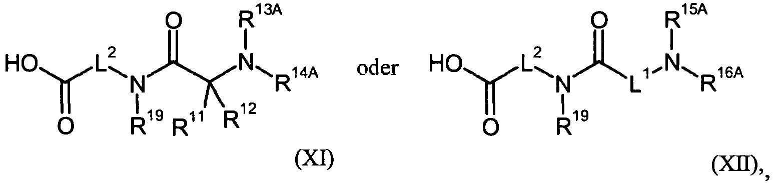 in welchen L¹, L², R⁸, R⁹, R¹⁰, R¹¹, R¹² und R¹⁹ jeweils die oben angegebenen Bedeutungen haben
   und
   - R^{13A}, R^{14A}, R^{15A} und R^{16A}: jeweils die für R¹³, R¹⁴, R¹⁵ bzw. R¹⁶ genannten Bedeutungen haben oder für eine Amino-Schutzgruppe, wie beispielsweise tert.-Butoxycarbonyl, stehen,
   zu Verbindungen der Formel (XIII), (XIV), (XV) oder (XVI) 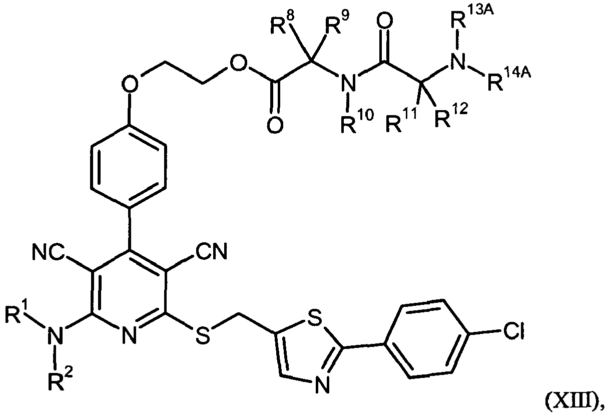 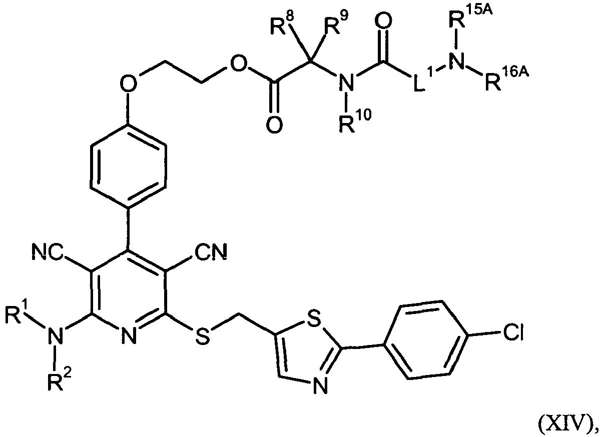 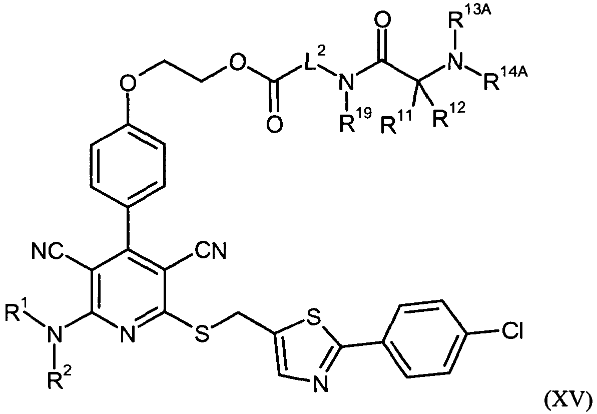 oder 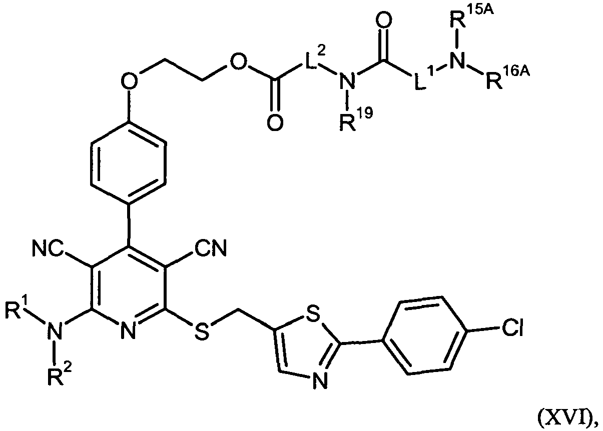 in welchen L¹, L², R¹, R² R⁸, R⁹, R¹⁰, R¹¹, R¹² R^{13A}, R^{14A}, R^{15A}, R^{16A} und R¹⁹ jeweils die oben angegebenen Bedeutungen haben,
   kuppelt und anschliessend gegebenfalls vorhandene Schutzgruppen zu einer Verbindung der Formel (I-D), (I-E), (I-F) oder (I-G) 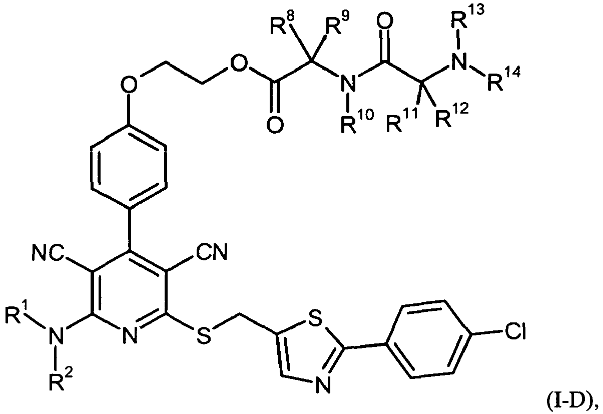 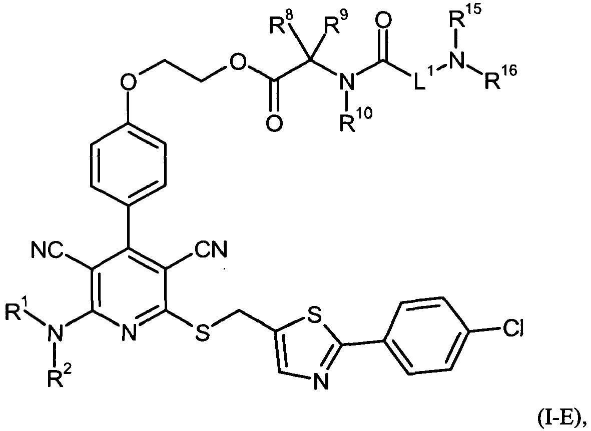 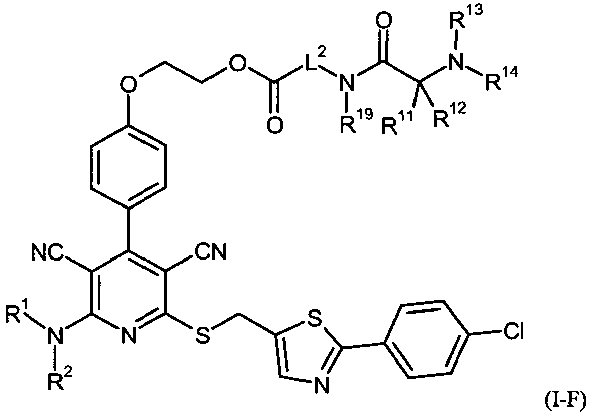 oder 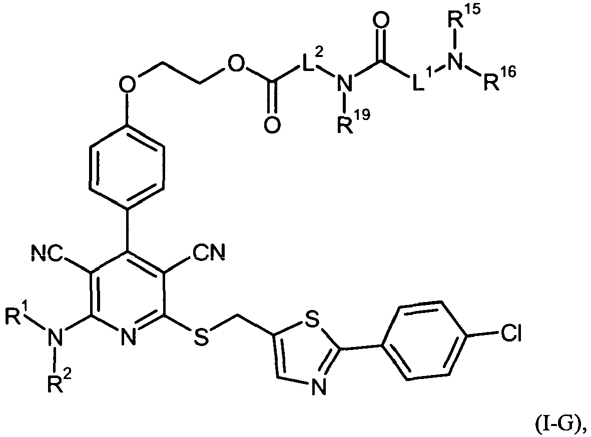 in welchen L¹, L², R¹, R², R⁸, R⁹, R¹⁰, R¹¹, R¹², R¹³, R¹⁴, R¹⁵ R¹⁶ und R¹⁹ jeweils die oben angegebenen Bedeutungen haben,
   abspaltet,
   oder
[C] von einer Verbindung der Formel (VII-1) oder (VIII-1), 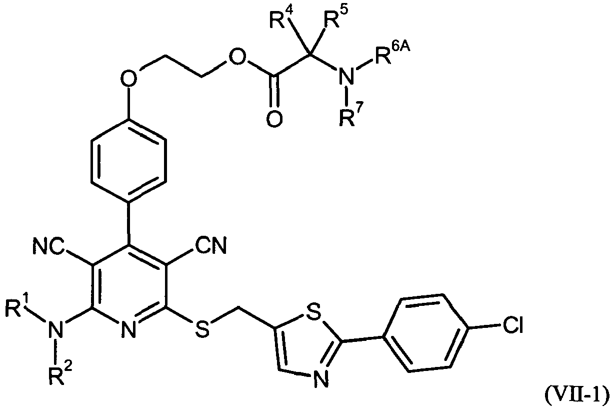 oder 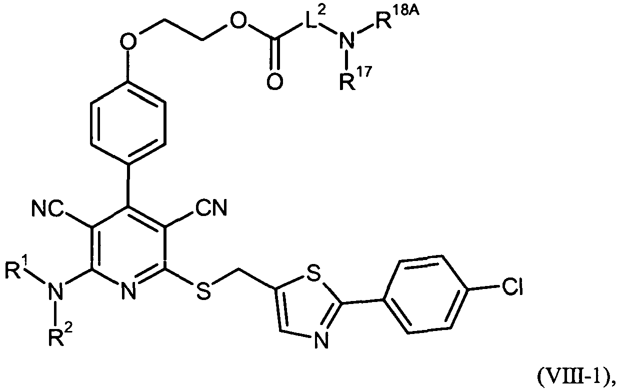 in welchen L², R¹, R², R⁴, R⁵, R⁷ und R¹⁷ jeweils die oben angegebenen Bedeutungen haben,
   und
   R^{6A} und R^{18A} für eine Amino-Schutzgruppe, beispielsweise tert.-Butoxycarbonyl steht,
   die Amino-Schutzgruppe nach Standardmethoden zu einer Verbindung der Formel (I-B-1) oder (I-C-1) 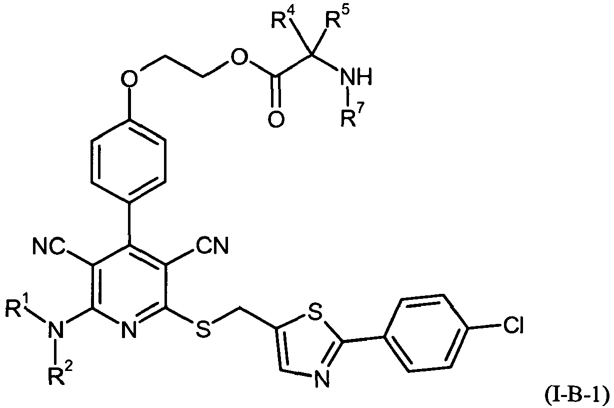 oder 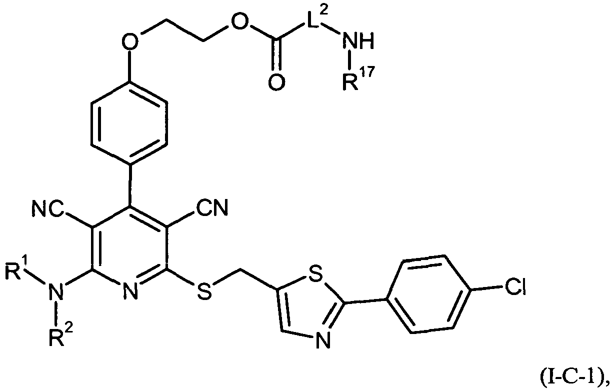 in welcher L², R¹, R², R⁴, R⁵, R⁷ und R¹⁷ jeweils die oben angegebenen Bedeutungen haben,
   abspaltet, und diese in einem inerten Lösungsmittel in Gegenwart eines Kondensationsmittels zunächst mit einer Carbonsäure der Formel (XVII) oder (XVIII) 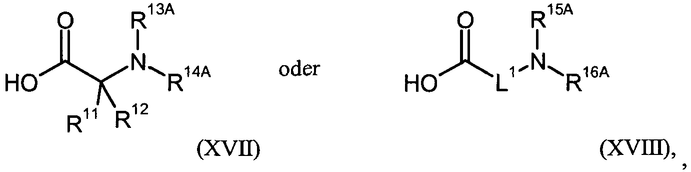 in welchen L¹, R¹¹ und R¹² jeweils die oben angegebenen Bedeutungen haben und
   - R^{13A}, R^{14A}, R^{15A} und R^{16A}: jeweils die für R¹³, R¹⁴, R¹⁵ bzw. R¹⁶ genannten Bedeutungen haben oder für eine Amino-Schutzgruppe, wie beispielsweise tert.-Butoxycarbonyl, stehen,
   zu Verbindungen der Formel (XIII), (XIV), (XV) oder (XVI) kuppelt und anschliessend gegebenfalls vorhandene Schutzgruppen wieder zu den resultierenden Verbindungen (I-D), (I-E), (I-F) oder (I-G) abspaltet,
und die resultierenden Verbindungen der Formel (I-B), (I-C), (I-D), (I-E), (I-F) und (I-G) gegebenenfalls mit den entsprechenden (i) Lösungsmitteln und/oder (ii) Basen oder Säuren in ihre Solvate, Salze und/oder Solvate der Salze überführt.

Die Transformation (I-A) → (I-B), (I-C), (I-D), (I-E), (I-F) bzw. (I-G) erfolgt somit entweder durch direkte Acylierung mit einem geeignet geschützten Dipeptoid-Derivat (Verfahrensvariante [B]) oder durch durch sequentielle Kupplung der einzelnen, gegebenenfalls geeignet geschützten Aminosäure-Komponenten (Verfahrensvariante [C]). Die Kupplungsreaktionen (Ester- bzw. Amid-Bildung) werden hierbei nach bekannten Methoden der Peptidchemie durchgeführt [vgl. z.B. M. Bodanszky, Principles of Peptide Synthesis, Springer-Verlag, Berlin, 1993; H.-D. Jakubke und H. Jeschkeit, Aminosäuren, Peptide, Proteine, Verlag Chemie, Weinheim, 1982].

Inerte Lösungsmittel für die Kupplungsreaktionen sind beispielsweise Ether wie Diethylether, tert.-Butyl-methylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Pyridin, Dimethylsulfoxid, Dimethylformamid, *N,N'*-Dimethylpropylenharnstoff (DMPU), *N*-Methylpyrrolidon (NMP) oder Acetonitril. Ebenso ist es möglich, Gemische der genannten Lösungsmittel zu verwenden. Bevorzugt sind Dichlormethan, Dimethylformamid oder Gemische dieser beiden Lösungsmittel.

Als Kondensationsmittel bei diesen Kupplungsreaktionen eignen sich beispielsweise Carbodiimide wie *N,N'*-Diethyl-, *N,N'*-Dipropyl-, *N,N'*-Diisopropyl-, *N,N'*-Dicyclohexylcarbodiimid (DCC) oder *N*-(3-Dimethylaminoisopropyl)-*N'*-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-*tert*.-Butyl-5-methylisoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris-(pyrrolidino)phosphonium-hexafluorophosphat (PyBOP), *O*-(Benzotriazol-1-yl)-*N,N,N',N'*-tetra-methyluronium-tetrafluoroborat (TBTU), *O*-(Benzotriazol-1-yl)-*N,N,N',N'*-tetramethyluroniumhexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N'*-tetramethyluronium-hexafluorophosphat (HATU) oder *O*-(1H-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder organische Aminbasen wie Triethylamin, *N*-Methylmorpholin, *N*-Methylpiperidin, *N,N*-Diisopropylethylamin oder 4-*N,N*-Dimethylaminopyridin. Zur Ester-Bildung wird bevorzugt *N*-(3-Dimethylaminoisopropyl)-*N*'-ethylcarbodiimid-Hydrochlorid (EDC) in Kombination mit 4-*N,N*-Dimethylaminopyridin eingesetzt. Für die Amid-Bildung wird vorzugsweise *N*-(3-Dimethylaminoisopropyl)-*N'*-ethylcarbodiimid-Hydrochlorid (EDC) in Kombination mit 1-Hydroxybenzotriazol (HOBt) oder *N*-Hydroxysuccinimid (HOSu) und gegebenenfalls einer Base wie *N,N*-Diisopropylethylamin verwendet.

Die Kupplungen werden im Allgemeinen in einem Temperaturbereich von 0°C bis +60°C, bevorzugt bei +10°C bis +30°C durchgeführt. Die Reaktionen können bei normalem, bei erhöhtem oder bei erniedrigtem Druck erfolgen (z.B. von 0.5 bis 5 bar). Im Allgemeinen arbeitet man bei Normaldruck.

Die Verbindungen der Formel (I) können bei der Herstellung nach den oben beschriebenen Verfahren auch direkt in Form von Salzen entstehen. Diese Salze können gegebenenfalls durch Behandlung mit einer Base bzw. Säure in einem inerten Lösungsmittel, über chromatographische Methoden oder mittels Ionenaustauscherharzen in die jeweiligen freien Basen bzw. Säuren überführt werden. Weitere Salze der erfindungsgemäßen Verbindungen können gegebenenfalls auch durch Austausch von Gegenionen mit Hilfe der Ionenaustausch-Chromatographie, beispielsweise mit Amberlite®-Harzen, hergestellt werden.

In den Verbindungen der Formeln (V), (VI), (IX), (X), (XI), (XII), (XVII) und (XVIII) bzw. in den Resten R⁴, R⁶, R⁷, R¹³, R¹⁴, R¹⁵, R¹⁶, R¹⁷ und/oder R¹⁸ gegebenenfalls vorhandene funktionelle Gruppen - wie insbesondere Amino-, Guanidino-, Hydroxy-, Mercapto- und Carboxylgruppen - können bei den zuvor beschriebenen Reaktionssequenzen, falls zweckmäßig oder erforderlich, auch in temporär geschützter Form vorliegen. Die Einführung und Entfernung solcher Schutzgruppen erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984].

Als Amino- und Guanidino-Schutzgruppe wird bevorzugt *tert*.-Butoxycarbonyl (Boc) oder Benzyloxycarbonyl (Z) verwendet. Als Schutzgruppe für eine Hydroxy- oder Carboxyl-Funktion wird vorzugsweise *tert*.-Butyl oder Benzyl eingesetzt. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Diethylether, Dichlormethan oder Essigsäure durchgeführt; gegebenenfalls kann die Abspaltung auch ohne ein zusätzliches inertes Lösungsmittel erfolgen. Im Falle von Benzyl und Benzyloxycarbonyl als Schutzgruppe können diese auch Hydrogenolyse in Gegenwart eines Palladium-Katalysators entfernt werden. Die Abspaltung der genannten Schutzgruppen kann gegebenenfalls simultan in einer Eintopf-Reaktion oder in separaten Reaktionsschritten vorgenommen werden.

Zur Herstellung der definierten Salzstöchiometrien und zur Entfernung von Lösungsmittelresten können die erfindungsgemäßen Verbindungen in organischen Lösungsmitteln als Suspension bei Raumtemperatur gerührt weden. Bevorzugt ist ein mehrtägiges Rühren bei Raumtemperatur in Isopropanol oder Diethylether. Besonders bevorzugt ist Rühren über 7 Tage bei Raumtemperatur in Isopropanol. Die erfindungsgemäßen Verbindungen werden im Anschluss daran abfiltriert und getrocknet.

Die Verbindungen der Formeln (V), (VI), (IX), (X), (XI), (XII), (XVII) und (XVIII) sind kommerziell erhältlich oder literaturbekannt, oder sie können nach literaturüblichen Methoden hergestellt werden.

Die Verbindungen der Formeln (VII), (VII-1), (VIII), (VIII-1), (XIII), (XIV), (XV) und (XVI) sind neu und daher ebenfalls Gegenstand der vorliegenden Erfindung, wobei die Substituenten die oben angegebenen Bedeutungen haben.

Die Herstellung der erfindungsgemäßen Verbindungen kann durch die folgenden Syntheseschemata veranschaulicht werden:

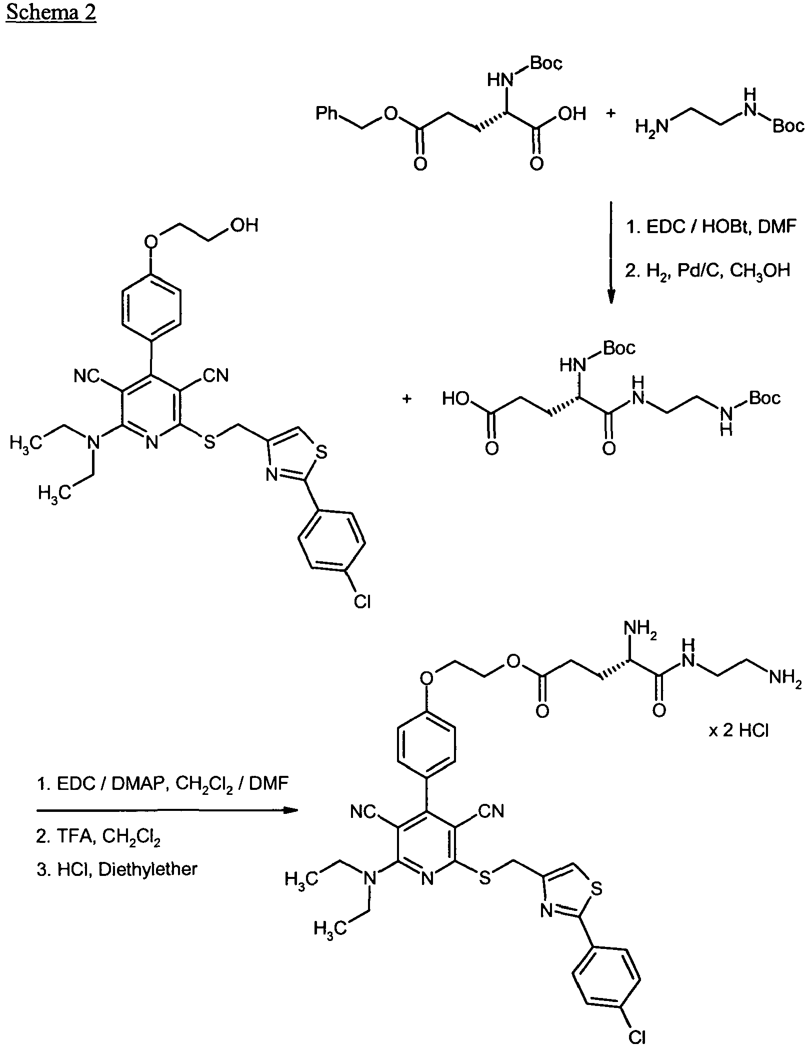

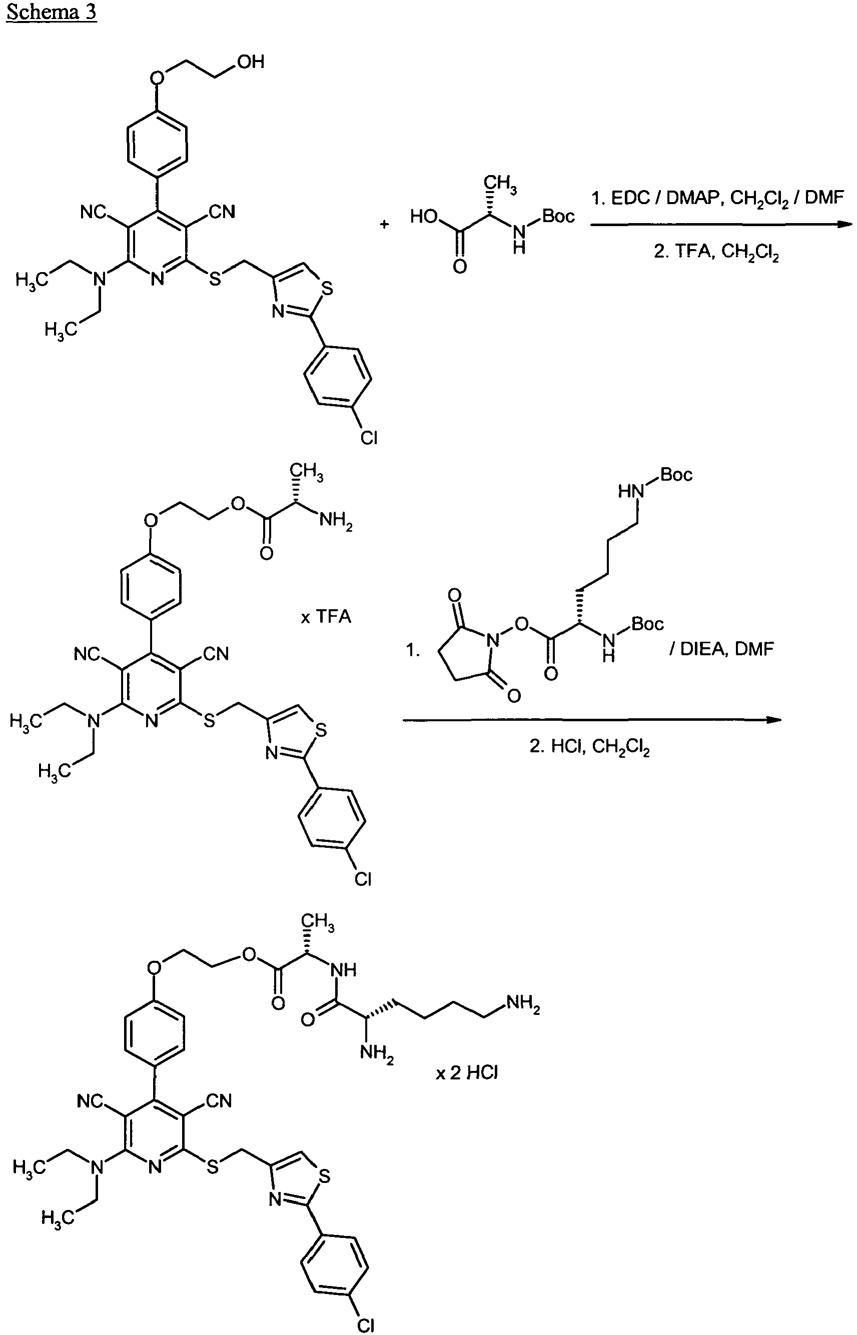

Überraschenderweise zeigen die erfindungsgemäßen Verbindungen ein nicht vorhersehbares, wertvolles pharmakologisches Wirkspektrum und sind daher insbesondere zur Prävention und/oder Behandlung von Erkrankungen geeignet.

Die pharmazeutische Wirksamkeit der erfmdungsgemäßen Verbindungen lässt sich durch ihre Wirkung als potente, selektive Liganden an den Adenosin A1- und/oder A2b-Rezeptoren erklären. Sie wirken hierbei als selektive A1-Agonisten oder selektive duale A1/A2b-Agonisten. Die erfmdungsgemäßen Verbindungen zeigen ein vorteilhaftes therapeutisches und/oder pharmakologisches Wirkprofil.

Als "selektive Liganden an Adenosin A1- und/oder A2b-Rezeptoren" werden im Rahmen der vorliegenden Erfindung solche Adenosin-Rezeptorliganden bezeichnet, bei denen einerseits eine deutliche Wirkung am A1- und/oder A2b-Adenosinrezeptor-Subtypen und andererseits keine oder eine deutliche schwächere Wirkung (Faktor 10 oder höher) an A2a- und A3-Adenosinrezeptor-Subtypen zu beobachten ist, wobei bezüglich der Testmethoden für die Wirk-Selektivität Bezug genommen wird auf die im Abschnitt B-1. beschriebenen Tests.

Die erfindungsgemäßen Verbindungen können in Abhängigkeit von ihrer jeweiligen Struktur als volle oder als partielle Adenosinrezeptor-Agonisten fungieren. Partielle Adenosinrezeptor-Agonisten sind hierbei definiert als Rezeptorliganden, die eine funktionelle Antwort an Adenosinrezeptoren auslösen, welche geringer ist als bei vollen Agonisten (wie beispielsweise Adenosin selbst). Partielle Agonisten weisen infolgedessen eine geringere Wirksamkeit bezüglich der Rezeptoraktivierung auf als volle Agonisten.

Die erfindungsgemäßen Verbindungen und ihre Salze der Formel (I), in denen R³ ungleich Wasserstoff ist, stellen nützliche Prodrugs der Wirkstoff-Verbindungen der Formel (I) dar, in denen R³ für Wasserstoff steht. Sie weisen einerseits eine gute Stabilität bei verschiedenen pH-Werten auf und zeigen andererseits eine effiziente Konversion zur Wirkstoff-Verbindung der Formel (I), in denen R³ für Wasserstoff steht, bei einem physiologischen pH-Wert und insbesondere *in vivo.* Darüber hinaus besitzen die erfindungsgemäßen Prodrugs verbesserte Löslichkeiten in wässrigen oder anderen physiologisch verträglichen Medien, was sie für die therapeutische Anwendung insbesondere bei intravenöser Applikation geeignet macht. Außerdem ist die Bioverfügbarkeit aus Suspension nach oraler Applikation gegenüber der Wirkstoff-Verbindungen der Formel (I), in denen R³ für Wasserstoff steht, verbessert.

Die Verbindungen der Formel (I) sind allein oder in Kombination mit einem oder mehreren anderen Wirkstoffen zur Prävention und/oder Behandlung verschiedener Erkrankungen geeignet, so beispielsweise Erkrankungen des Herzkreislauf-Systems (kardiovaskulären Erkrankungen), zur Kardioprotektion nach Schädigungen des Herzens sowie von Stoffwechsel- und Nierenerkrankungen.

Im Sinne der vorliegenden Erfindung sind unter Erkrankungen des Herzkreislauf-Systems bzw. kardiovaskulären Erkrankungen beispielsweise die folgenden Erkrankungen zu verstehen: Hypertonie, periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, akutes Koronarsyndrom mit ST-Erhebung, akutes Koronarsyndrom ohne ST-Erhebung, stabile und instabile Angina pectoris, Herzmuskelschwäche, Prinzmetal Angina, persistierende ischämische Dysfunktion (hibernating Myokardium), vorübergehende postischämische Dysfunktion (stunned Myokardium), Herzinsuffizienz, Tachykardien, atriale Tachykardie, Arrhythmien, Vorhof- und Kammerflimmern, persistierendes Vorhofflimmern, permanentes Vorhofflimmern, Vorhofflimmern mit normaler linksventrikulärer Funktion, Vorhofflimmern mit eingeschränkter linksventrikulärer Funktion, Wolff-Parkinson-White Syndrom, periphere Durchblutungsstörungen, erhöhte Spiegel von Fibrinogen und von LDL geringer Dichte, sowie erhöhte Konzentrationen von Plasminogenaktivator-Inhibitor 1 (PAI-1), insbesondere koronare Herzerkrankung, akutes Koronarsyndrom, Angina pectoris, Herzinsuffizienz, Myokardinfarkt und Vorhofflimmern

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische sowie systolische Herzinsuffizienz und akute Phasen der Verschlechterung einer bestehenden chronischen Herzinsuffizienz (worsening heart failure).

Weiterhin eignen sich die erfindungsgemäßen Verbindungen auch zur Reduktion des von einem Infarkt betroffenen Myokardbereichs sowie zur Prävention von Sekundärinfarkten.

Des weiteren sind die erfindungsgemäßen Verbindungen zur Prävention und/oder Behandlung von thromboembolischen Erkrankungen, Reperfüsionsschäden nach Ischämie, mikro- und makrovaskulären Schädigungen (Vaskulitis), arteriellen sowie venösen Thrombosen, Ödemen, Ischämien wie Myokardinfarkt, Hirnschlag und transitorischen ischämischen Attacken, zur Kardioprotektion bei Koronararterien Bypass Operationen (CABG), primären PTCAs, PTCAs nach Thrombolyse, Rescue-PTCA, Herztransplantationen und Operationen am offenen Herzen, sowie zur Organprotektion bei Transplantationen, Bypass Operationen, Herzkatheteruntersuchungen und anderen operativen Eingriffen geeignet.

Weitere Indikationsgebiete, für die die erfindungsgemäßen Verbindungen eingesetzt werden können, sind beispielsweise die Prävention und/oder Behandlung von Erkrankungen des Urogenitalbereiches, wie z.B. Reizblase, erektile Dysfunktion und weibliche sexuelle Dysfunktion, daneben aber auch die Prävention und/oder Behandlung von inflammatorischen Erkrankungen, wie z.B. entzündliche Dermatosen (Psoriasis, Akne, Ekzeme, Neurodermitis, Dermatitis, Keratitis, Narbenbildung, Warzenbildung, Frostbeulen), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), von Schmerzzuständen, Krebserkrankungen (Hautkrebs, Liposarcome, Karzinome des Magen-Darm-Traktes, der Leber, Bauchspeicheldrüse, Lunge, Niere, Harnleiter, Prostata und des Genitaltraktes) sowie von Übelkeit und Erbrechen in Verbindung mit Krebstherapien.

Weitere Indikationsgebiete sind beispielsweise die Prävention und/oder Behandlung von Entzündungs- und Immunerkrankungen (Morbus Crohn, Colitis ulcerosa, Lupus erythematodes, rheumatoide Arthritis) und von Erkrankungen der Atemwege, wie beispielsweise chronischobstruktive Atemwegserkrankungen (chronische Bronchitis, COPD), Asthma, Lungenemphysem, Bronchiektasien, zystische Fibrose (Mukoviszidose) und pulmonale Hypertonie, insbesondere pulmonale arterielle Hypertonie.

Schließlich kommen die erfindungsgemäßen Verbindungen auch für die Prävention und/ oder Behandlung von Diabetes, insbesondere Diabetes mellitus, Gestationsdiabetes, Insulin-abhängiger Diabetes und Nicht-Insulin-abhängiger Diabetes, von diabetischen Folgeerkrankungen wie z.B. Retinopathie, Nephropathie und Neuropathie, von metabolischen Erkrankungen (Metabolisches Syndrom, Hyperglykämie, Gestationsdiabetes, Hyperinsulinämie, Insulinresistenz, Glukose-Intoleranz, Fettsucht (Adipositas)) sowie von Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien, in Betracht

Darüber hinaus können die erfindungsgemäßen Verbindungen auch zur Behandlung und/oder Prävention von Schilddrüsenerkrankungen (Hyperthyreose), Erkrankungen der Bauchspeicheldrüse (Pankreatitis), Leberfibrose, viralen Erkrankungen (HPV, HCMV, HIV), Kachexie, Osteoporose, Gicht, Inkontinenz sowie zur Wundheilung und Angiogenese eingesetzt werden.

Weiterer Gegenstand der vorliegenden Erfmdung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung sind die erfindungsgemäßen Verbindungen zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

Weiterer Gegenstand der vorliegenden Erfindung sind die erfindungsgemäßen Verbindungen zur Verfahren zur Behandlung und/oder Prophylaxe von Diabetes, Metabolischem Syndrom und Dyslipidämien.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Als geeignete Kombinationswirkstoffe seien beispielhaft und vorzugsweise genannt: den Fettstoffwechsel verändernde Wirkstoffe Antidiabetika, Blutdruck-Senker, durchblutungsfördernd und/ oder antithrombotisch wirkende Mittel, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten), Analgetika wie beispielsweise Aspirin, Anti-Depressiva und andere Pyschopharmaka.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylhamstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/ oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Rezeptoren-Blocker, alpha-Rezeptoren-Blocker, Aldosteron-Antagonisten, Mineralocorticoid-Rezeptor-Antagonisten, ECE-Inhibitoren, ACE/NEP Inhibitoren sowie der Vasopeptidase-Inhibitoren; und/oder
- antithrombotisch wirkenden Mitteln, beispielhaft und vorzugsweise aus der Gruppe der Thrombozytenaggregationshemmer oder der Antikoagulantien
- Diuretika;
- Vasopressin-Rezeptor-Antagonisten;
- organischen Nitraten und NO-Donatoren;
- positiv-inotrop wirksamen Verbindungen;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Agonisten des Prostacyclin-Rezeptors (IP-Rezeptors), wie beispielsweise Iloprost, Beraprost, Cicaprost;
- Hemmer des I_{f} (funny channel) Kanals, wie beispielsweise Ivabradine;
- Calcium-Sensitizern, wie beispielhaft und vorzugsweise Levosimendan;
- Kalium-Supplements;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussenden Verbindungen, wie beispielweise Etomoxir, Dichloracetat, Ranolazine und Trimetazidine
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren und PPAR-gamma-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfmdungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin und Vildagliptin, verabreicht.

Bei einer bevorzugten Ausführungsform der Erfindung werden die erfindungsgemäßen Verbindungen in Kombination mit einem PPAR-gamma-Agonisten beispielsweise aus der Klasse der Thiazolindione, wie beispielhaft und vorzugsweise Pioglitazon und Rosiglitazon, verabreicht.

Unter den Blutdruck senkenden Mitteln werden vorzugsweise Verbindungen aus der Gruppe der Calcium-Antagonisten, Angiotensin AII-Antagonisten, ACE-Hemmer, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfmdung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfmdung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfmdung werden die erfmdungsgemäßen Verbindungen in Kombination mit einem alpha-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralokortikoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Nitroglycerin, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin, verabreicht.

Bei einer bevorzugten Ausführungsform der Erfmdung werden die erfmdungsgemäßen Verbindungen in Kombination mit Antisympathotonika wie Reserpin, Clonidin oder alpha-Methyl-Dopa, mit Kaliumkanal-Agonisten wie Minoxidil, Diazoxid, Dihydralazin oder Hydralazin, oder mit Stickoxid freisetzenden Stoffen wie Glycerinnitrat oder Nitroprussidnatrium verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfmdungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfmdungsgemäßen Verbindungen in Kombination mit Heparin oder einem low molecular weight (LMW)-Heparin-Derivat verabreicht.

Bei einer bevorzugten Ausführungsform der Erfindung werden die erfmdungsgemäßen Verbindungen in Kombination mit einem Vitamin K-Antagonisten, wie beispielhaft und vorzugsweise Coumarin, verabreicht.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Rezeptoren-Blocker, organische Nitrate und NO-Donatoren, ACE-Inhibitoren, Angiotensin AII-Antagonisten, Aldosteron- und Mineralokortikoid-Rezeptor-Antagonisten, Vasopressin-Rezeptor-Antagonisten, Thrombozytenaggregationshemmer und Antikoagulantien, sowie deren Verwendung zur Behandlung und/oder Prävention der zuvor genannten Erkrankungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfmdungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfmdungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- aq.: wässrig
- Bsp.: Beispiel
- c: Konzentration
- d: Dublett (bei NMR)
- dd: Dublett von Dublett (bei NMR)
- DBU: 1,8-Diazabicyclo[5.4.0]undec-7-en
- DC: Dünnschichtchromatographie
- DCI: direkte chemische Ionisation (bei MS)
- DMF: *N,N*-Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ESI: Elektrospray-Ionisation (bei MS)
- Et: Ethyl
- Fp.: Schmelzpunkt
- h: Stunde(n)
- HPLC: Hochdruck-, Hochleistungsflüssigchromatographie
- kat.: katalytisch
- konz.: konzentriert
- LC-MS: Flüssigchromatographie-gekoppelte Massenspektrometrie
- Lit.: Literatur(stelle)
- Me: Methyl
- MeCN: Acetonitril
- min: Minute(n)
- MS: Massenspektrometrie
- NMM: *N*-Methylmorpholin
- NMR: Kernresonanzspektrometrie
- q: Quartett (bei NMR)
- rac.: racemisch
- RP-HPLC: reverse phase HPLC
- RT: Raumtemperatur
- Rₜ: Retentionszeit (bei HPLC)
- s: Singulett (bei NMR)
- s br: breites Singulett (bei NMR)
- t: Triplett (bei NMR)
- t-Bu: tert.-Butyl
- TFA: Trifluoressigsäure
- THF: Tetrahydrofuran
- verd.: verdünnt

### HPLC-, LC-MS- und GC-MS-Methoden:

### Methode 1 (LC-MS):

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 2 (LC-MS):

Instrument: Waters ACQUITY SQD UPLC System; Säule: Waters Acquity UPLC HSS T3 1,8µ50 x 1mm; Eluent A: 1 1 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 3 (LC-MS):

Gerätetyp MS: Micromass Quattro LCZ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min. 2 ml/min; Ofen: 50°C; UV-Detektion: 210 nm.

### Methode 4 (LC-MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 5 (LC-MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 6 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 7 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 8 (LC-MS):

Gerätetyp MS: Micromass ZQ; Gerätetyp HPLC: Waters Alliance 2795; Säule: Merck Chromolith SpeedROD RP-18e 100 x 4.6mm; Eluent A: 1 1 Wasser + 0.5 ml 50%ige Ameisensäure; Eluent B: 1 1 Acetonitril + 0.5 ml 50%ige Ameisensäure; Gradient: 0.0 min 10% B→ 7.0 min 95% B→ 9.0 min 95% B; Ofen: 35°C; Fluss: 0.0 min 1.0 ml/min→ 7.0 min 2.0 ml/min→ 9.0 min 2.0 ml/min; UV-Detektion: 210 nm

### Ausgangsverbindungen und Intermediate:

### Beispiel 1A

### 2-Amino-6-({[2-(4-chlorphenyl)-1,3-thiazol-4-yl]methyl}sulfanyl)-4-[4-(2-hydroxyethoxy)phenyl]pyridin-3,5-dicarbonitril

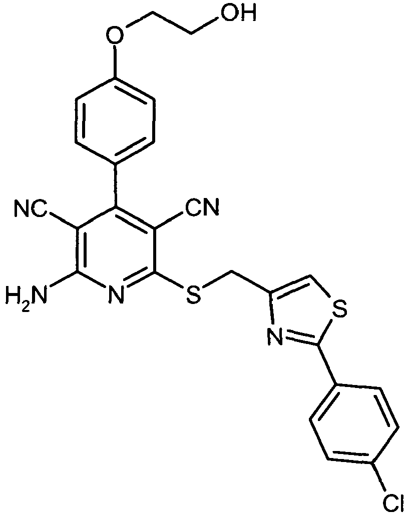

Die Darstellung wurde beschrieben in WO 03/053441, Beispiel 6.
LC-MS (Methode 8): Rₜ = 5.69 min; MS (ESIpos): m/z = 520 [M+H]⁺.

### Beispiel 2A

### 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril

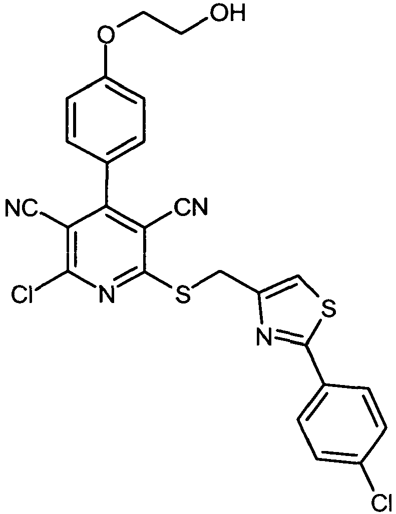

15.00 g (28.84 mmol) 2-Amino-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 1A] wurden in 200 ml Acetonitril vorgelegt und mit 6.76 g (57.69 mmol) Isopentylnitrit und 7.76 g (57.69 mmol) Kupfer(II)-chlorid versetzt. Es wurde 6 h bei 70°C gerührt. Nach Abkühlen auf RT wurden 750 ml 1N Salzsäure zugegeben und 30 min gerührt. Die wässrige Phase mit dreimal mit Essigsäureethylester extrahiert. Die vereinigten organischen Phasen wurden über Natriumsulfat getrocknet. Nach Entfernen des Lösungsmittels wurde das Rohprodukt mittels Säulenchromatographie an Kieselgel gereinigt (Laufmittel: Toluol/Essigsäureethylester 4:1). Es wurden 10.8 g (69% d. Th., Reinheit 90%) der gewünschten Zielverbindung erhalten. Zur weiteren Reinigung kann gegebenenfalls mit Diethylether verrührt werden.
LC-MS (Methode 2): Rₜ = 1.36 min; MS (ESIpos): m/z = 539 [M+H]⁺.
¹H-NMR (400 MHz, DMSO-d₆): δ = 7.95 (d, 2H), 7.75 (s, 1H), 7.61 (d, 2H), 7.57 (d, 2H), 7.18 (d, 2H), 4.77 (s, 2H), 4.10 (t, 2H), 3.75 (t, 2H).

### Beispiel 3A

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-N²,N⁶-bis(tert.-butoxycarbonyl)-L-lysyl-L-alaninat

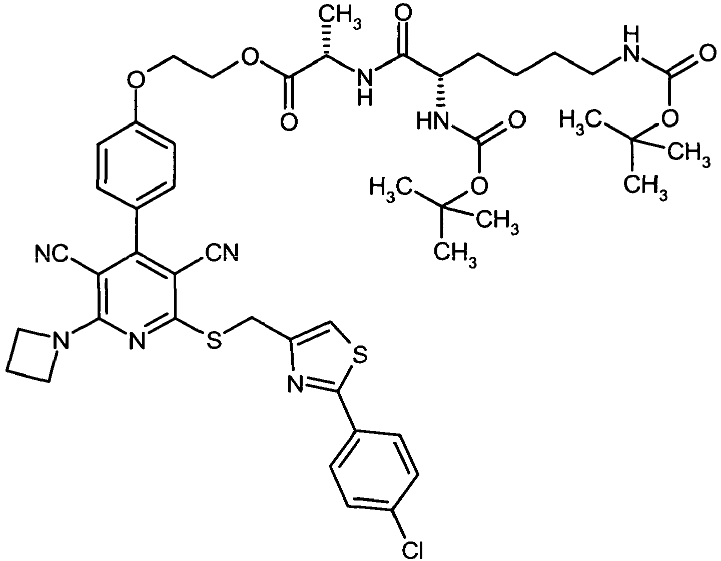

32.73 mg (0.094 mmol) N²,N⁶-Bis(tert.-butoxycarbonyl)-L-lysin wurden in 1.5 ml DMF vorgelegt. Nach Versetzen mit 19.8 mg (0.103 mmol) 1-(3-Dimethylaminopropyl)-3-ethylcarbodiimid-Hydrochlorid, 17.4 mg (0.129 mmol) 1-Hydroxy-1H-benzotriazol-Hydrat und 55.5 mg (0.429 mmol) N,N-Diisopropylethylamin wurde 15 min bei RT gerührt, anschließend mit 64 mg (0.086 mmol) 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-L-alaninat-Trifluoracetat [Beispiel 45] versetzt und über Nacht bei RT gerührt. Das Rohprodukt wurde mittels präparativer HPLC (Acetonitril/Wasser + 0.1% TFA) gereinigt. Es wurden 76 mg (92% d. Th.) der Zielverbindung erhalten.
LC-MS (Methode 6): Rₜ = 3.30 min; MS (ESIpos): m/z = 959 [M+H]⁺.

Die in Tabelle 1 aufgeführten Beispiele wurden analog zu Beispiel 3A aus den entsprechenden Edukten hergestellt.

**Tabelle 1:**

| **Beis piel Nr.** | **Struktur** | **LC-MS: Rₜ [min] (Methode); MS (ESI): m/z [M+H]⁺** |
|---|---|---|
| **4A** | 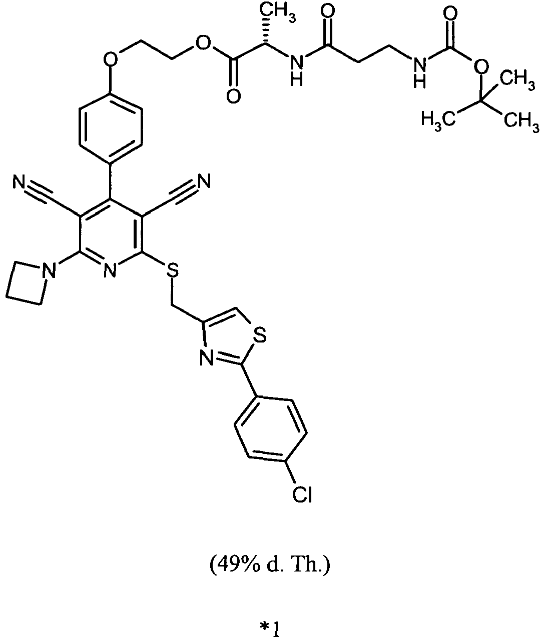 | 1.62 min (Methode 4); m/z = 802 |
| **5A** | 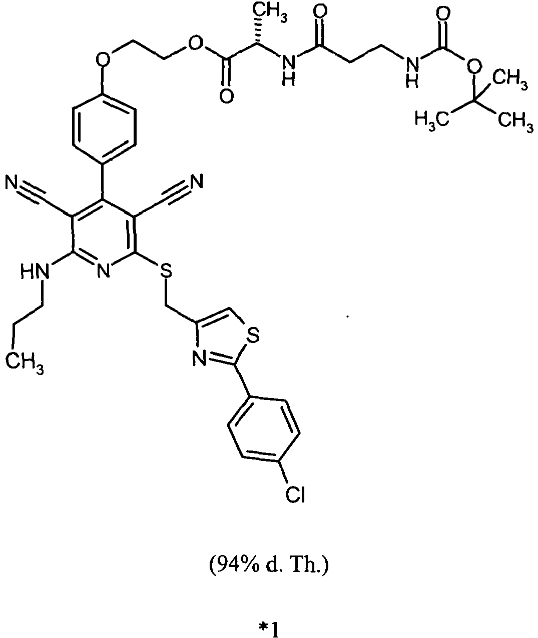 | 1.68 min (Methode 4); m/z = 804 |

| **Beispiel Nr.** | **Struktur** | **LC-MS: Rₜ [min] (Methode); MS (ESI): m/z [M+H]⁺** |
|---|---|---|
| **6A** | 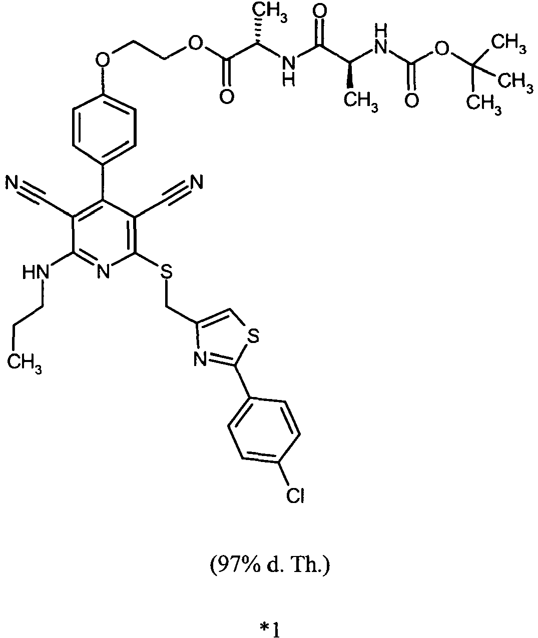 | 1.70 min (Methode 4); m/z = 704 [M+H-Boc]⁺ |

| | | |
|---|---|---|
| * 1 Aufreinigung; bevor die Reaktionslösung auf die präparativen HPLC gegeben wurde, wurde die Reaktionslösung mit etwas Wasser/THF bzw. Wasser/Acetonitril versetzt, so dass eine klare Lösung entstand. | | |

### Beispiel 7A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-N-(tert-butoxycarbonyl)-L-alanyl-L-alaninat

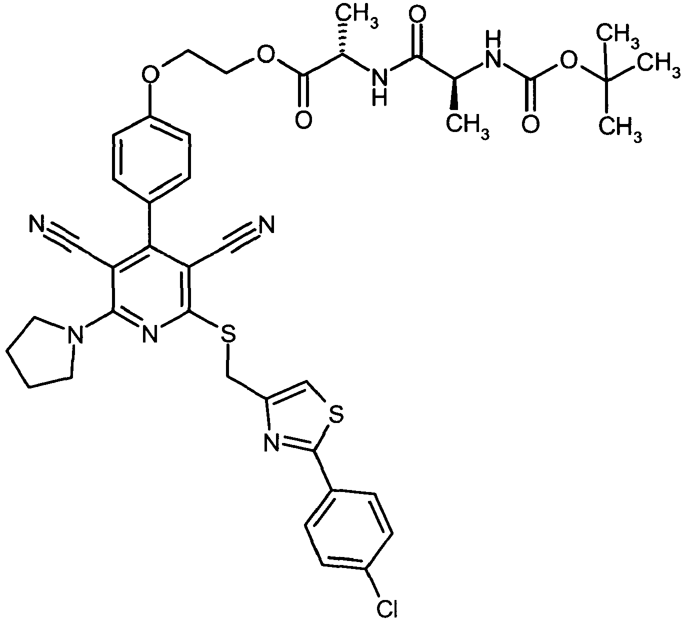

26.756 g (141.406 mmol) N-(tert.-Butoxycarbonyl)-L-alanin wurden gemeinsam mit 29.572 g (154.261 mmol) 1-(3-Dimethylaminopropyl)-3-ethylcarbodiimid-Hydrochlorid, 29.529 g (192.827 mmol) 1-Hydroxy-1H-benzotriazol-Hydrat und 55.979 ml (321.378 mmol) N,N-Diisopropylethylamin in 10 1 DMF gelöst. Anschließend wurden 97.60 g (128.551 mmol) Trifluoressigsäure--2-{4-[2-({[2-(4-chlorphenyl)-1,3-thiazol-4-yl]methyl}sulfanyl)-3,5-dicyan-6-(pyrrolidin-1-yl)pyridin-4-yl]phenoxy}ethyl-L-alaninat(1:1) zugegeben und 3h bei Raumtemperatur gerührt. Das Reaktionsgemisch wurde in Wasser eingerührt und mit Dichlormethan extrahiert. Die organische Phase wurde mit Wasser gewaschen, über Natriumsulfat getrocknet, filtriert und eingeengt. Der Rückstand wurde mit Diethylether verrührt, der Feststoff abgesaugt und an der Luft getrocknet. Es wurden 95 g (91% d. Th.) der gewünschten Zielverbindung erhalten.
LC-MS (Methode 2): Rₜ = 1.49 min; MS (ESIpos): m/z = 816 [M+H]⁺.
¹H-NMR (400 MHz, DMSO-d₆): δ = 8.21 (d, 1H), 7.95 (d, 2H), 7.70 (s, 1H), 7.58 (d, 2H), 7.48 (d, 2H), 7.11 (d, 2H), 6.84 (d, 1H), 4.70 (s, 2H), 4.46-4.32 (m, 2H), 4.31-4.24 (m, 3H), 4.03-3.93 (m, 1H), 3.97-3.79 (m, 4H), 2.01-1.88 (m, 4H) 1.36 (s, 9H), 1.29 (d, 3H), 1.16 (d, 3H).

Die in Tabelle 6 aufgeführten Beispiele wurden analog zu Beispiel 3A aus den entsprechenden Edukten hergestellt.

**Tabelle 6:**

| **Beis piel Nr.** | **Struktur** | **LC-MS: Rₜ [min] (Methode); MS (ESI): m/z [M+H]⁺** |
|---|---|---|
| **8A** | 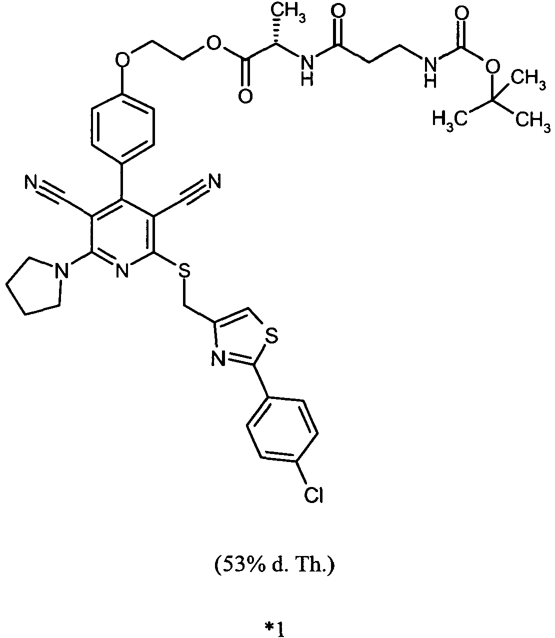 | 1.69 min (Methode 4); m/z = 816 |
| **9A** | 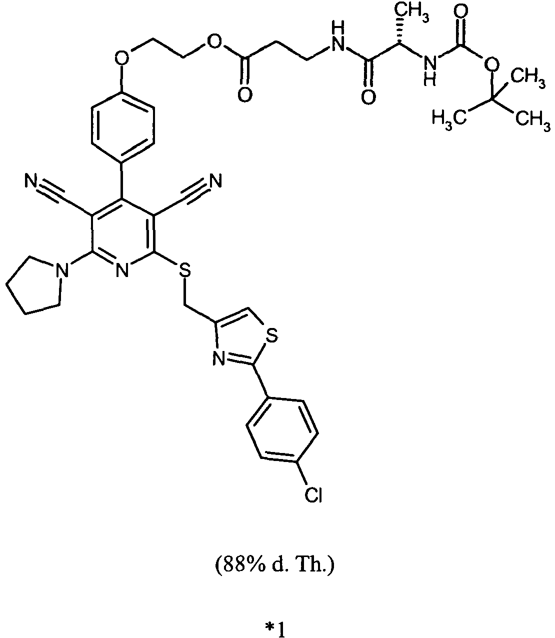 | 1.68 min (Methode 4); m/z = 716 [M+H-Boc]⁺ |

| **Beispiel Nr.** | **Struktur** | **LC-MS: Rₜ [min] (Methode); MS (ESI): m/z [M+H]⁺** |
|---|---|---|
| **10A** | 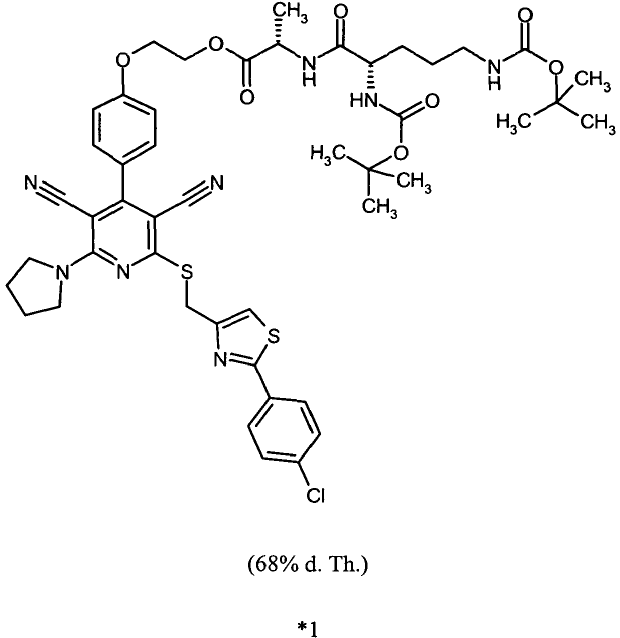 | 1.77 min (Methode 4); m/z = 859 [M+H-Boc]⁺ |

| | | |
|---|---|---|
| *1 Aufreinigung; bevor die Reaktionslösung auf die präparativen HPLC gegeben wurde, wurde die Reaktionslösung mit etwas Wasser/THF bzw. Wasser/Acetonitril versetzt, so dass eine klare Lösung entstand. | | |

### Beispiel 11A

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-N-(tert.-butoxycarbonyl)-beta-alaninat

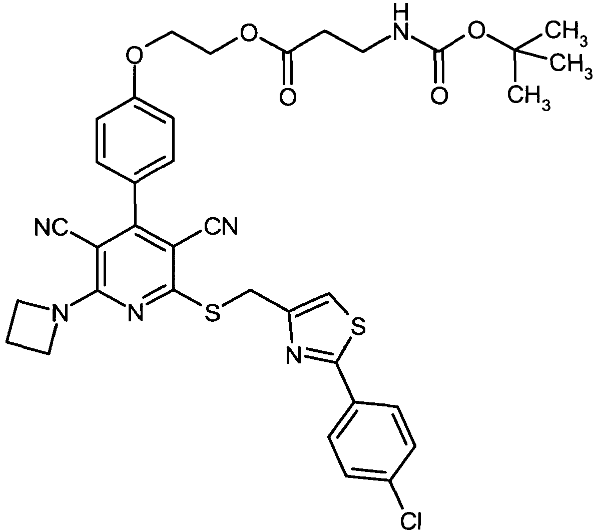

101 mg (0.536 mmol) N-(tert.-Butoxycarbonyl)-beta-alanin wurden in 2 ml DMF/Dichlormethan (1:1) vorgelegt. Nach Versetzen mit 44.5 mg (0.232 mmol) 1-(3-Dimethylaminopropyl)-3-ethylcarbodiimid-Hydrochlorid, 10.9 mg (0.89 mmol) 4-Dimethylaminopyridin und 100 mg (0.179 mmol) 2-(Azetidin-1-yl)-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 8] wurde über Nacht bei RT gerührt. Das Rohprodukt wurde mittels präparativer HPLC (Acetonitril/Wasser + 0.1% TFA) gereinigt. Es wurden 118 mg (90% d. Th.) der Zielverbindung erhalten.
LC-MS (Methode 5): Rₜ = 2.90 min; MS (ESIpos): m/z = 731 [M+H]⁺.

### Beispiel 12A

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-N²,N⁵-bis(tert.-butoxycarbonyl)-L-ormithinat

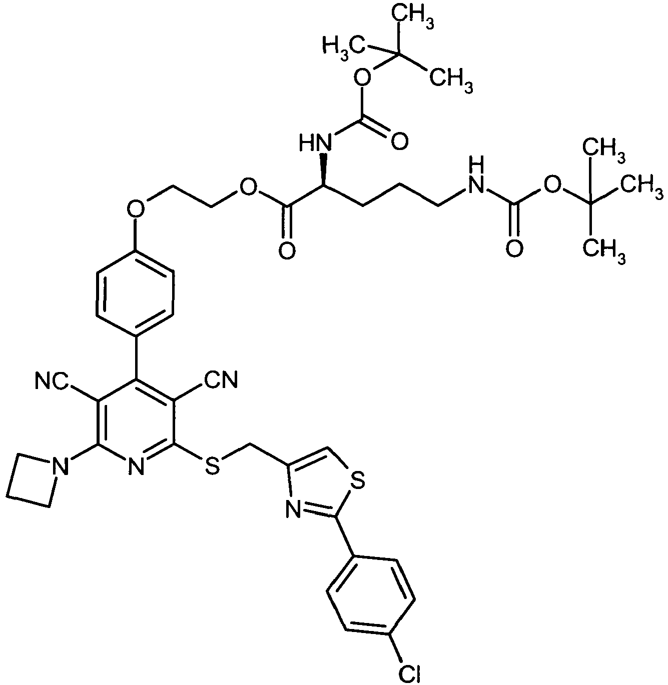

75 mg (0.134 mmol) 2-(Azetidin-1-yl)-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 8], 133.53 mg (0.402 mmol) N²,N⁵-Bis(tert.-butoxycarbonyl)-L-ornithin und 8.18 mg (0.067 mmol) 4-Dimethylaminopyridin wurden in 1 ml DMF vorgelegt. Nach Versetzen mit 1 ml Dichlormethan und 33.37 mg (0.174 mmol) 1-(3-Dimethylaminopropyl)-3-ethylcarbodiimid-Hydrochlorid wurde die Reaktionslösung über Nacht bei 40°C gerührt. Nach dem Abkühlen wurde die Reaktionslösung mit soviel Wasser/THF versetzt, dass eine klare Lösung entstand und mittels präparativer HPLC (Acetonitril/Wasser + 0.1 % TFA) gereinigt. Es wurden 104 mg (89% d. Th.) der Zielverbindung erhalten.
LC-MS (Methode 6): Rₜ = 3.35 min; MS (ESIpos): m/z = 874 [M+H]⁺.

### Beispiel 13A

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-N-(tert.-butoxycarbonyl)-L-alaninat

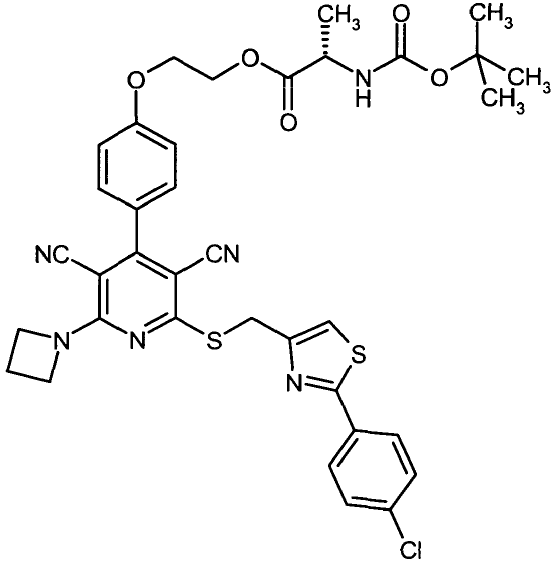

350 mg (0.625 mmol) 2-(Azetidin-1-yl)-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 8], 354.7 mg (1.875 mmol) N-(tert.-Butoxycarbonyl)-L-alanin und 38.17 mg (0.312 mmol) 4-Dimethylaminopyridin wurden in 3.3 ml DMF vorgelegt. Nach Versetzen mit 3.3 ml THF und 155.7 mg (0.812 mmol) 1-(3-Dimethylaminopropyl)-3-ethylcarbodiimid-Hydrochlorid wurde die Reaktionslösung über Nacht bei 40°C gerührt. Nach dem Abkühlen wurde die Reaktionslösung mittels präparativer HPLC (Acetonitril/Wasser + 0.1% TFA) gereinigt. Es wurden 377 mg (83% d. Th.) der Zielverbindung erhalten.
LC-MS (Methode 6): Rₜ = 3.26 min; MS (ESIpos): m/z = 731 [M+H]⁺.

### Beispiel 14A

### 2-{4-[2-({[2-(4-Chlorphenyl)-1,3-thiazol-4-yl]methyl}sulfanyl)-3,5-dicyan-6-(propylamino)pyridin-4-yl]phenoxy}ethyl-N-(tert.-butoxycarbonyl)-L-alaninat

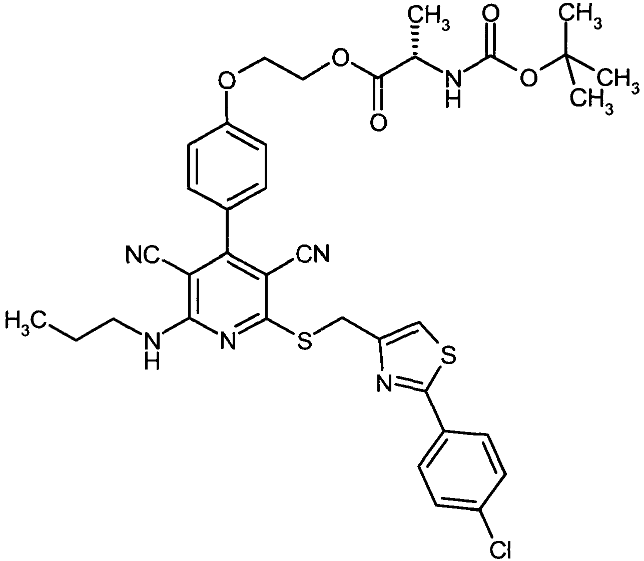

758 mg (1.348 mmol) 2-({[2-(4-Chlorphenyl)-1,3-thiazol-4-yl]methyl}sulfanyl)-4-[4-(2-hydroxyethoxy)phenyl]-6-(propylamino)pyridin-3,5-dicarbonitril [Beispiel 12], 765 mg (4.043 mmol) N-(tert.-Butoxycarbonyl)-L-alanin und 82 mg (0.674 mmol) 4-Dimethylaminopyridin wurden in 10.3 ml DMF/Dichlormethan (1:1) vorgelegt. Nach Versetzen mit 336 mg (1.752 mmol) 1-(3-Dimethylaminopropyl)-3-ethylcarbodiimid-Hydrochlorid wurde die Reaktionslösung über Nacht bei RT gerührt. Die Reaktionslösung wurde vom Dichlormethan befreit und der Rückstand wurde mittels präparativer HPLC (Acetonitril/Wasser + 0.1% TFA) gereinigt. Es wurden 929 mg (94% d. Th.) der Zielverbindung erhalten.
LC-MS (Methode 2): Fₜ = 1.54 min; MS (ESIneg): m/z = 731 [M-H]⁻.

Die in Tabelle 2 aufgeführten Beispiele wurden analog zu Beispiel 14A aus den entsprechenden Edukten hergestellt.

**Tabelle 2:**

| **Beispiel Nr.** | **Struktur** | **LC-MS: Rₜ [min] (Methode); MS (ESI): m/z [M+H]⁺** |
|---|---|---|
| **15A** | 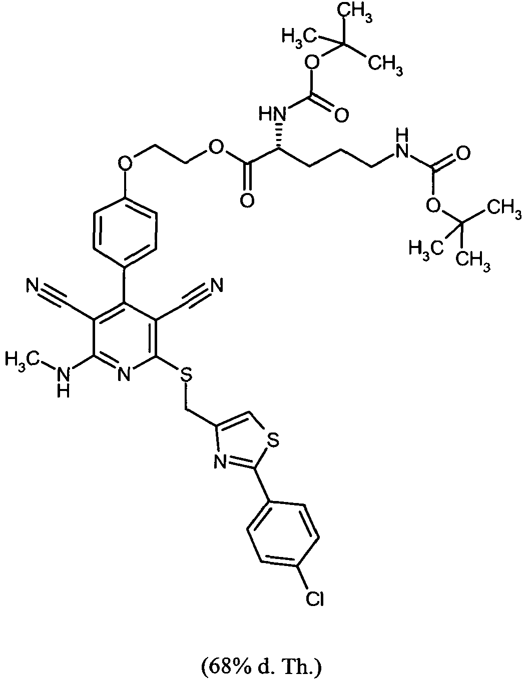 | 1.51 min (Methode 2); m/z = 848 |
| **16A** | 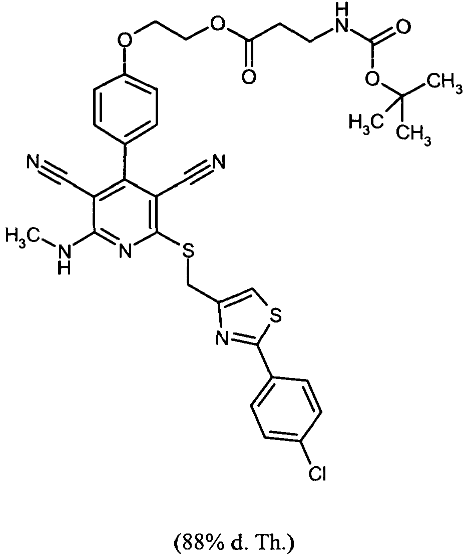 | 1.66 min (Methode 4); m/z = 605 [M+H-BOC]⁺ |
| **17A** | 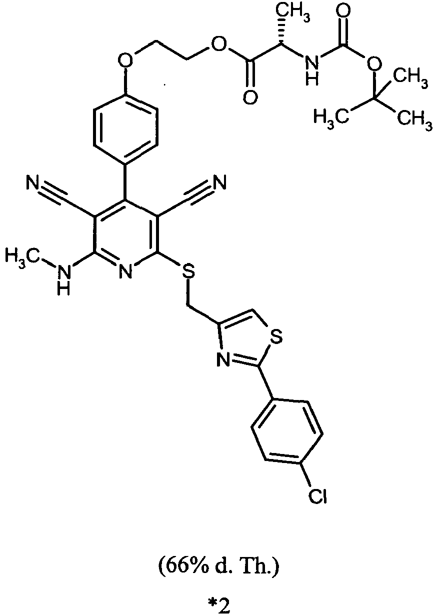 | 1.47 min (Methode 2); m/z = 705 |

| **Beis piel Nr.** | **Struktur** | **LC-MS: Rₜ [min] (Methode); MS (ESI): m/z [M+H]⁺** |
|---|---|---|
| **18A** | 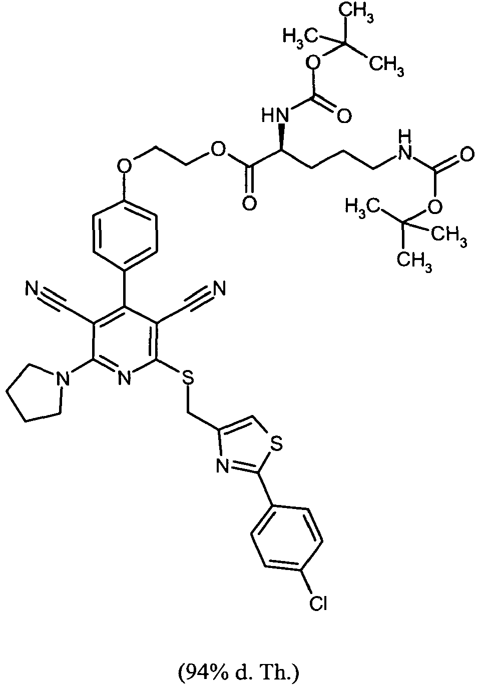 | 3.38 min (Methode 6); m/z = 888 |
| **19A** | 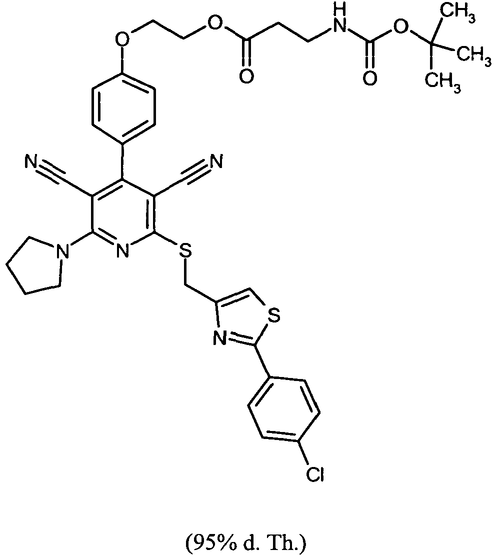 | 1.51 min (Methode 2); m/z = 745 |
| **20A** | 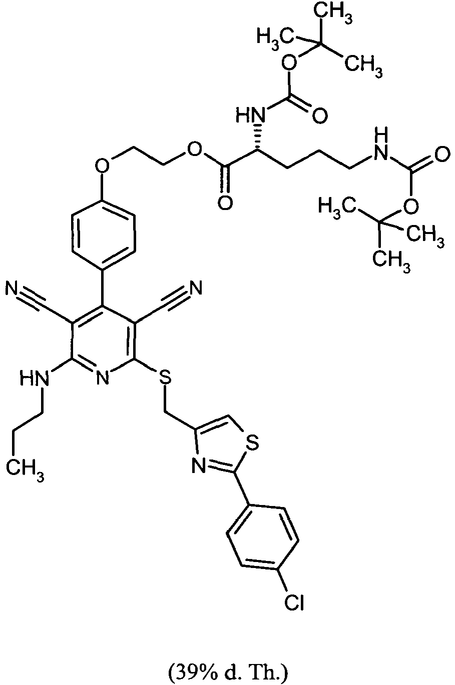 | 1.57 min (Methode 2); m/z = 876 |

| | | |
|---|---|---|
| *2 abweichende Aufreinigung; das Rohprodukt wurde mittels präprativer HPLC (Acetonitril/Wasser + 0.1% TFA) gereinigt. Anschließend wurde das Produkt mittels Säulenchromatographie an Kieselgel 60 gereinigt (Laufmittel: Toluol/Acetonitril 10:1). | | |

### Beispiel 21A

### 2-{4-[2-({[2-(4-Chlorphenyl)-1,3-thiazol-4-yl]methyl}sulfanyl)-3,5-dicyan-6-(propylamino)pyridin-4-yl]phenoxy}ethyl-N²,N⁶-bis(tert.-butoxycarbonyl)-L-lysyl-L-alaninat

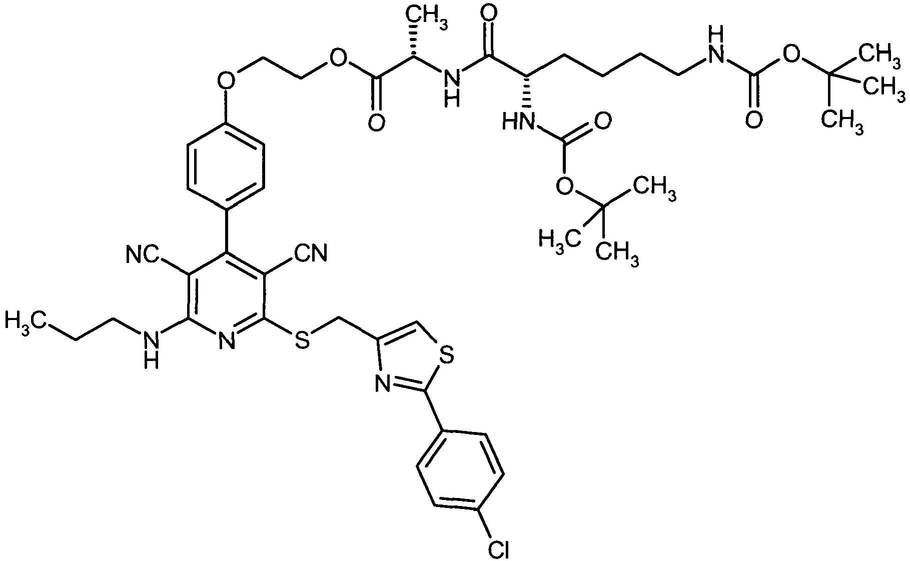

445 mg (1.284 mmol) N²,N⁶-Bis(tert.-butoxycarbonyl)-L-lysin wurden in 12.3 ml DMF vorgelegt. Nach Versetzen mit 268 mg (1.400 mmol) 1-Hydroxy-1H-benzotriazol-Hydrat, 237 mg (1.750 mmol) 1-(3-Dimethylaminopropyl)-3-ethylcarbodiimid-Hydrochlorid und 0.51 ml (2.917 mmol) N,N-Diisopropylethylamin wurde mit 872 mg (1.167 mmol) 2-{4-[2-({[2-(4-Chlorphenyl)-1,3-thiazol-4-yl]methyl} sulfanyl)-3,5-dicyan-6-(propylamino)pyridin-4-yl]phenoxy} ethyl-L-alaninat-Trifluoracetat [Beispiel 38] versetzt und über Nacht bei RT gerührt. Die Reaktionslösung wurde mit Wasser versetzt und dreimal mit Essigsäureethylester extrahiert. Die vereinigten organischen Phasen wurden über Natriumsulfat getrocknet, filtriert und eingedampft. Der Rückstand wurde mittels präparativer HPLC (Acetonitril/Wasser + 0.1% TFA) gereinigt. Das erhaltene Produkt wurde zur weiteren Reinigung mittels Säulenchromatographie an Kieselgel 60 (Laufmittel: Cyclohexan/Essigsäureethylester 1/1) gesäult. Es wurden 698 mg (62% d. Th.) der Zielverbindung erhalten.
LC-MS (Methode 2): Rₜ = 1.55 min; MS (ESIpos): m/z = 961 [M+H]⁺.

Die in Tabelle 3 aufgeführten Beispiele wurden analog zu Beispiel 21A aus den entsprechenden Edukten hergestellt.

**Tabelle 3:**

| **Beispiel Nr.** | **Struktur** | **LC-MS: Rₜ [min] (Methode); MS (ESI): m/z [M+H]⁺** |
|---|---|---|
| **22A** | 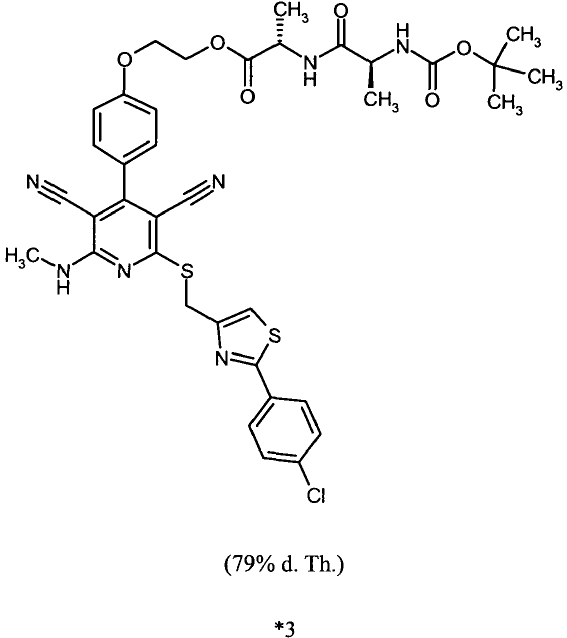 | 1.60 min (Methode 4); m/z = 776 |
| **23A** | 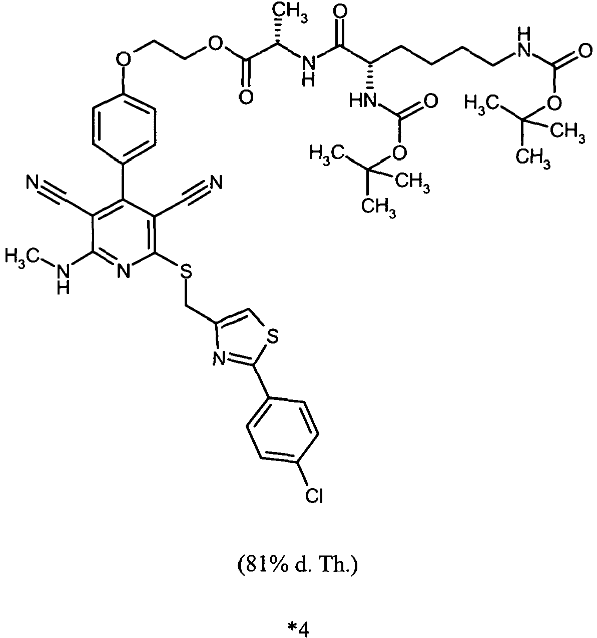 | 1.65 min (Methode 4); m/z = 933 |
| **24A** | 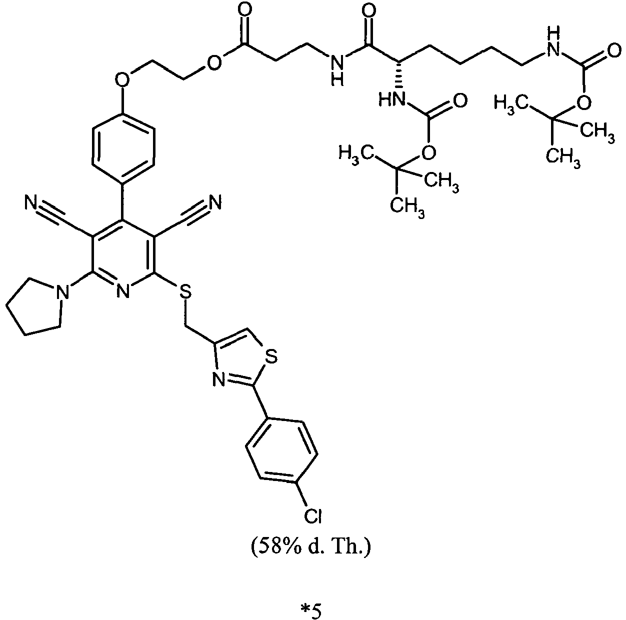 | 1.54 min (Methode 2); m/z = 973 |

| | | |
|---|---|---|
| *3 Abweichende Aufarbeitung; die Reaktionslösung wurde mittels präparativer HPLC (Acetonitril/Wasser + 0.1 % TFA) gereinigt. *4 Abweichende Aufarbeitung; die Reaktionslösung wurde eingedampft. Das Rohprodukt wurde mittels Säulenchromatographie an Kieselgel 60 gereinigt (Laufmittel: Dichlormethan /Methanol 20:1). Das Produkt wurde mittels präparativer HPLC (Acetonitril/Wasser + 0.1% TFA) weiter aufgereinigt. *5 Abweichende Aufarbeitung; die Reaktionslösung mit Wasser/Acetonitril versetzt und dreimal mit Dichlormethan extrahiert. Die vereinigten organischen Phasen wurden einmal mit Wasser gewaschen, über Natriumsulfat getrocknet, filtriert und eingedampft. Der Rückstand wurde mittels Säulenchromatographie (Säule: Waters Sunfire C 18, 5µm, 250 x 30mm, Eluent: Wasser/Methanol/THF = 15/70/15) gereinigt. | | |

### Beispiel 25A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)-L-alaninat

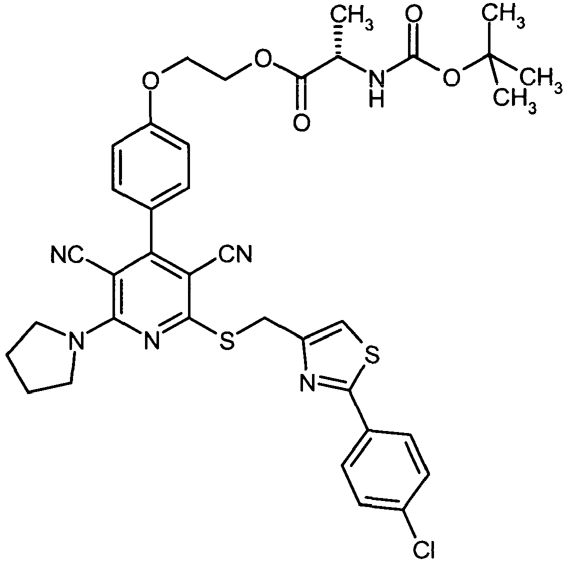

218 mg (1.15 mmol) N-(tert.-Butoxycarbonyl)-L-alanin wurden in 5 ml DMF vorgelegt. Nach Versetzen mit 240 mg (1.254 mmol) 1-(3-Dimethylaminopropyl)-3-ethylcarbodiimid-Hydrochlorid, 240 mg (1.568 mmol) 1-Hydroxy-1H-benzotriazol-Hydrat und 0.455 ml (2.613 mmol) N,N-Diisopropylethylamin, wurde mit 300 mg (0.523 mmol) 2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril [Beispiel 1] versetzt und über Nacht bei RT gerührt. Das Reaktionsgemisch wurde mittels präparativer HPLC (Acetonitril/Wasser + 0.1% TFA) gereinigt. Es wurden 382 mg (98% d. Th.) der Zielverbindung erhalten.
LC-MS (Methode 2): Rₜ = 1.52 min; MS (ESIpos): m/z = 745 [M+H]⁺.
¹H-NMR (400 MHz, DMSO-d₆): δ = 7.95 (d, 2H), 7.70 (s, 1H), 7.58 (d, 2H), 7.48 (d, 2H), 7.31 (d, 1H), 7.11 (d, 2H), 4.70 (s, 2H), 4.48-4.33 (m, 2H), 4.30-4.23 (m, 2H), 4.07-3.99 (m, 1H), 3.89-3.78 (m, 4H), 1.98-1.87 (m, 4H), 1.35 (s, 9H) 1.24 (d, 3H).

### Beispiel 26A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N²,N⁶-bis(tert.-butoxycarbonyl)-L-lysyl-L-alaninat

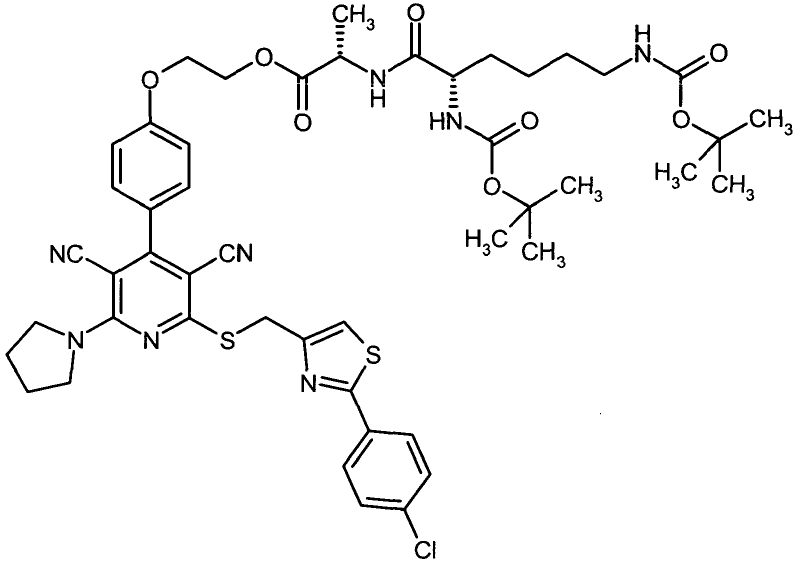

166 mg (0.478 mmol) N²,N⁶-Bis(tert.-butoxycarbonyl)-L-lysin wurden in 6.4 ml DMF vorgelegt. Nach Versetzen mit 88 mg (0.652 mmol) 1-Hydroxy-1H-benzotriazol-Hydrat, 100 mg (0.522 mmol) 1-(3-Dimethylaminopropyl)-3-ethylcarbodiimid-Hydrochlorid und 0.379 ml (2.173 mmol) N,N-Diisopropylethylamin wurde mit 330 mg (0.435 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat [Beispiel 40] versetzt und über Nacht bei RT gerührt. Die Reaktionslösung wurde mit soviel Wasser/Acetonitril versetzt, so dass eine klare Lösung entstand. Diese wurde mittels präparativer HPLC (Acetonitril/Wasser + 0.1 % TFA) gereinigt. Es wurden 216 mg (44% d. Th.) der Zielverbindung erhalten.
LC-MS (Methode 4): Rₜ = 1.79 min; MS (ESIpos): m/z = 973 [M+H]⁺.

### Beispiel 27A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-1-(tert.-butoxycarbonyl)-L-prolyl-L-alaninat

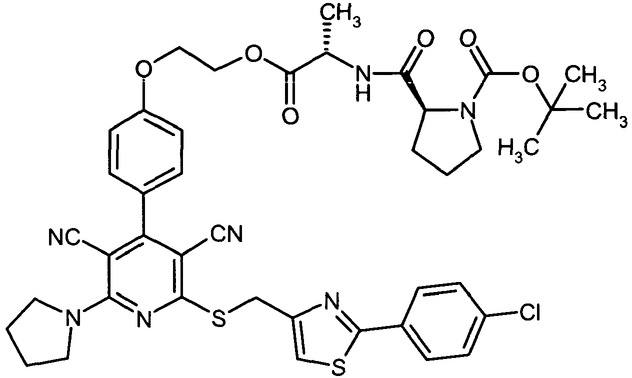

202 mg (0.942 mmol) 1-(tert.-Butoxycarbonyl)-L-prolin wurden gemeinsam mit 246 mg (1.284 mmol) 1-(3-Dimethylaminopropyl)-3-ethylcarbodiimid-Hydrochlorid, 157 mg (1.027 mmol) 1-Hydroxy-1H-benzotriazol-Hydrat und 0.746 ml (4.281 mmol) N,N-Diisopropylethylamin in 7.5 ml DMF gelöst, bevor 650 mg (0.856 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat zugegeben wurden. Nachdem über Nacht bei Raumtemperatur gerührt wurde, wurde das Reaktionsgemisch mittels präparativer HPLC (Acetonitril/Wasser) gereinigt. Man erhielt 495 mg (69% d. Th.) der Zielverbindung.
LC-MS (Methode 6): Rₜ = 3.29 min; MS (ESIpos): m/z = 842 [M+H]⁺.

### Beispiel 28A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)-L-isoleucyl-L-alaninat

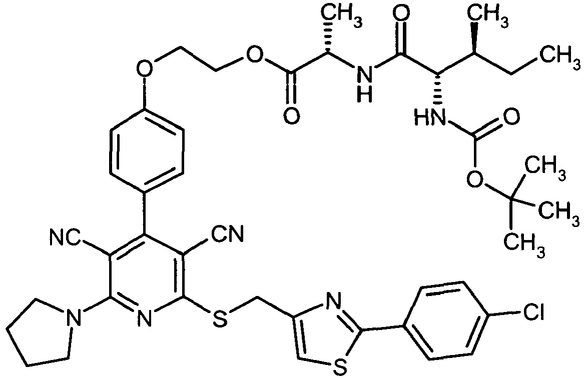

217 mg (0.942 mmol) N-(tert.-Butoxycarbonyl)-L-isoleucin wurden gemeinsam mit 246 mg (1.284 mmol) 1-(3-Dimethylaminopropyl)-3-ethylcarbodiimid-Hydrochlorid, 157 mg (1.027 mmol) 1-Hydroxy-1H-benzotriazol-Hydrat und 0.746 ml (4.281 mmol) N,N-Diisopropylethylamin in 7.5 ml DMF gelöst, bevor 650 mg (0.856 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat zugegeben wurden. Nachdem über Nacht bei Raumtemperatur gerührt wurde, wurde das Reaktionsgemisch mittels präparativer HPLC (Acetonitril/Wasser) gereinigt. Man erhielt 414 mg (56% d. Th.) der Zielverbindung.
LC-MS (Methode 2): Rₜ = 1.54 min; MS (ESIpos): m/z = 858 [M+H]⁺.
¹H-NMR (400 MHz, DMSO-d₆): δ = 8.31 (d, 1H), 7.95 (d, 2H), 7.70 (s, 1H), 7.58 (d, 2H), 7.48 (d, 2H), 7.11 (d, 2H), 6.61 (d, 1H), 4.70 (s, 2H), 4.46-4.23 (m, 5H), 3.89-3.78 (m, 5H), 1.99-1.89 (m, 4H), 1.71-1.59 (m, 1H), 1.46-1.39 (m, 1H) 1.36 (s, 9H), 1.29 (d, 3H), 1.13-1.00 (m, 1H), 0.83-0.76 (m, 6H).

### Beispiel 29A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-{(2S)-2,4-bis[(tert.-butoxycarbonyl)amino]butanoyl}-L-alaninat

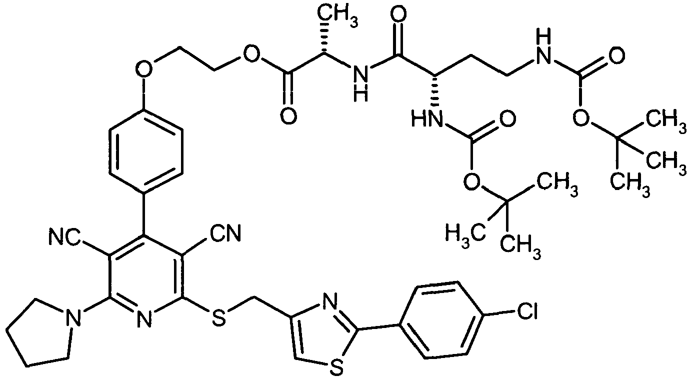

361 mg (0.724 mmol) (2S)-2,4-Bis[(tert.-butoxycarbonyl)amino]butansäure--N,N-Dicyclohexylamin-Salz wurden gemeinsam mit 151 mg (0.790 mmol) 1-(3-Dimethylaminopropyl)-3-ethylcarbodiimid-Hydrochlorid, 151 mg (0.988 mmol) 1-Hydroxy-1H-benzotriazol-Hydrat und 0.574 ml (3.293 mmol) N,N-Diisopropylethylamin in 10 ml DMF gelöst, bevor 500 mg (0.659 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat zugegeben wurden. Nachdem über Nacht bei Raumtemperatur gerührt wurde, wurde das Reaktionsgemisch zweimal mittels präparativer HPLC (Acetonitril/Wasser) gereinigt. Man erhielt 420 mg (67% d. Th.) der Zielverbindung.
LC-MS (Methode 1): Rₜ = 3.19 min; MS (ESIpos): m/z = 945 [M+H]⁺.

### Beispiel 30A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)-3-[(tert.-butoxycarbonyl)amino]-L-alanyl-L-alaninat

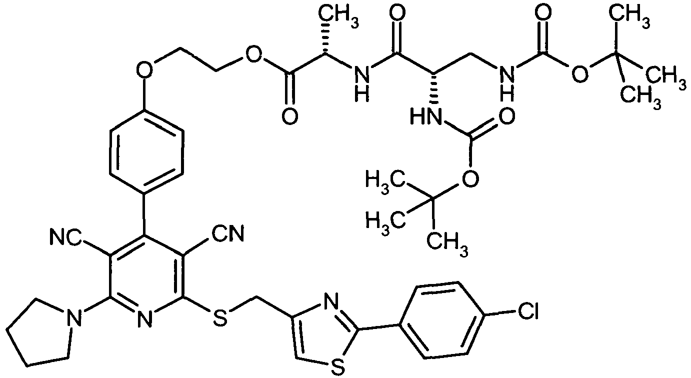

351 mg (0.724 mmol) N-(tert.-Butoxycarbonyl)-3-[(tert.-butoxycarbonyl)amino]-L-alanin-N,N-Dieyclohexylamin-Salz wurden gemeinsam mit 151 mg (0.790 mmol) 1-(3-Dimethylaminopropyl)-3-ethylcarbodiimid-Hydrochlorid, 151 mg (0.988 mmol) 1-Hydroxy-1H-benzotriazol-Hydrat und 0.574 ml (3.293 mmol) N,N-Diisopropylethylamin in 10 ml DMF gelöst, bevor 500 mg (0.659 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat zugegeben wurden. Nachdem über Nacht bei Raumtemperatur gerührt wurde, wurde das Reaktionsgemisch zweimal mittels präparativer HPLC (Acetonitril/Wasser) gereinigt. Man erhielt 290 mg (47% d. Th.) der Zielverbindung.
LC-MS (Methode 1): Rₜ = 3.18 min; MS (ESIpos): m/z = 931 [M+H]⁺.

### Beispiel 31A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)-L-histidyl-L-alaninat

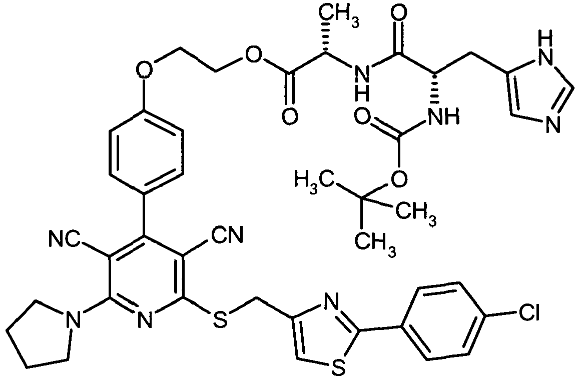

257 mg (0.724 mmol) N-(tert.-Butoxycarbonyl)-L-histidyl-L-alanin wurden gemeinsam mit 151 mg (0.790 mmol) 1-(3-Dimethylaminopropyl)-3-ethylcarbodiimid-Hydrochlorid, 151 mg (0.988 mmol) 1-Hydroxy-1H-benzotriazol-Hydrat und 0.574 ml (3.293 mmol) N,N-Diisopropylethylamin in 10 ml DMF gelöst, bevor 500 mg (0.659 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat zugegeben wurden. Nachdem über Nacht bei Raumtemperatur gerührt wurde, wurde das Reaktionsgemisch zweimal mittels präparativer HPLC (Acetonitril/Wasser) gereinigt. Man erhielt 169 mg (28% d. Th.) der Zielverbindung.
LC-MS (Methode 4): Rₜ = 1.42 min; MS (ESIpos): m/z = 882 [M+H]⁺.

### Beispiel 32A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N⁵-[N,N'-bis(tert.-butoxycarbonyl)carbamimidoyl]-N²-(tert.-butoxycarbonyl)-L-ornithyl-L-alaninat

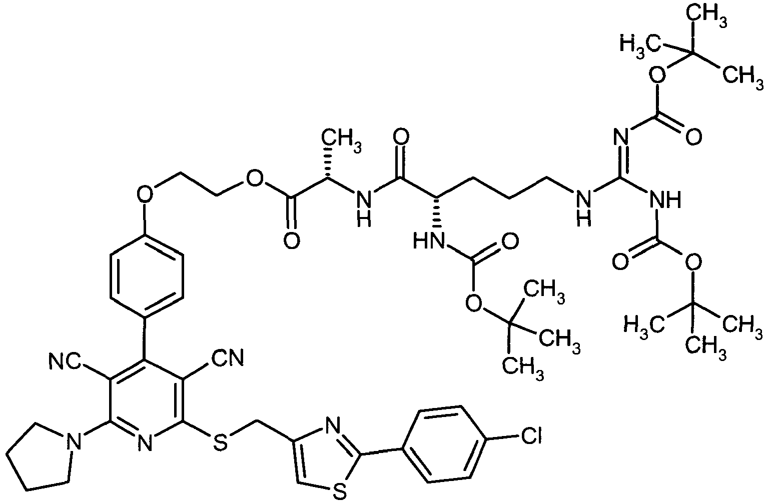

344 mg (0.724 mmol) N⁵-[N,N'-Bis(tert.-butoxycarbonyl)carbamimidoyl]-N²-(tert.-butoxycarbonyl)-L-ornithin wurden gemeinsam mit 151 mg (0.790 mmol) 1-(3-Dimethylaminopropyl)-3-ethylcarbodiimid-Hydrochlorid, 151 mg (0.988 mmol) 1-Hydroxy-1H-benzotriazol-Hydrat und 0.574 ml (3.293 mmol) N,N-Diisopropylethylamin in 10 ml DMF gelöst, bevor 500 mg (0.659 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat zugegeben wurden. Nachdem über Nacht bei Raumtemperatur gerührt wurde, wurde das Reaktionsgemisch zweimal mittels präparativer HPLC (Acetonitril/Wasser) gereinigt. Man erhielt 341 mg (47% d. Th.) der Zielverbindung.
LC-MS (Methode 4): Rₜ = 1.88 min; MS (ESIpos): m/z = 1101 [M+H]⁺.

### Beispiel 33A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)-L-leucinat

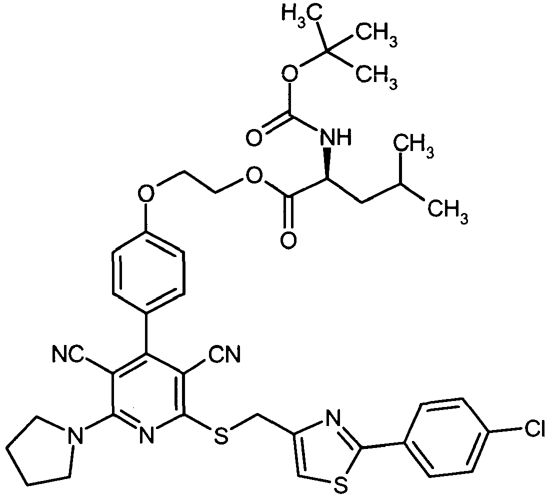

0.89 g (3.82 mmol) *N*-(tert.-Butoxycarbonyl)-L-leucin wurden in 10 ml DMF vorgelegt und mit 0.81 g (4.18 mmol) 1-(3-Dimethylaminopropyl)-3-ethylcarbodiimidhydrochlorid, 0.80 g (5.23 mmol) 1-Hydroxy-1H-benzotriazol Hydrat und 1.13 g (8.71 mmol) *N,N*-Diisopropylethylamin versetzt. Es wurde gerührt bis eine klare Lösung eintrat. Danach wurden 1.00 g (1.74 mmol) 2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril zugegeben und 18 h bei Raumtemperatur gerührt. Es wurde auf 300 ml Wasser gegeben. Der entstandene Rückstand wurde abgesaugt und mit 50 ml Wasser gewaschen. Das Rohprodukt wurde in 50 ml Dichlormethan gelöst. Die wässrige Phase wurde abgetrennt und die organische Phase wurde über Natriumsulfat getrocknet. Nach Entfernen des Lösesmittels.im Vakuum wurden 1.37 g (100% d. Th.) der gewünschten Zielverbindung erhalten.
LC/MS (Methode 2): Rₜ = 1.64 min; MS (ESIpos): m/z = 787 [M+H]⁺.

### Beispiel 34A

### 2-{4-[2-({[2-(4-chlorphenyl)-1,3-thiazol-4-yl]methyl}sulfanyl)-3,5-dicyan-6-(pyrrolidin-1-yl)pyridin-4-yl]phenoxy}ethyl-L-leucinat-Trifluoracetat

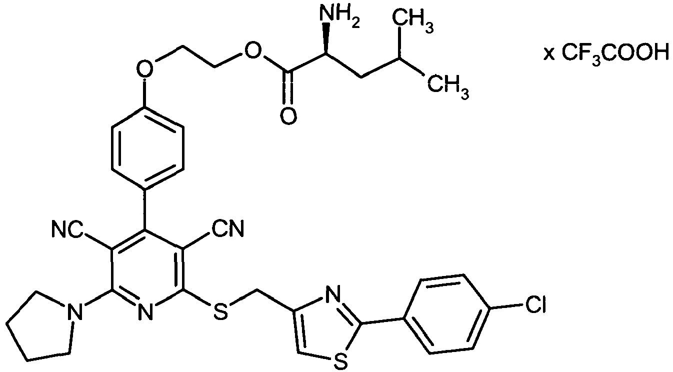

1.37 g (1.74 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-*N*-(tert.-butoxycarbonyl)-L-leucinat wurden in 15 ml Dichlormethan vorgelegt und mit 15.00 ml (194.70 mmol) Trifluoressigsäure versetzt. Es wurde 1h bei Raumtemperatur gerührt. Nach Entfernen des Lösungsmittels im Vakuum wurde der Rückstand in 5.00 ml Dichlormethan gelöst und mit 10.00 ml Diethylether versetzt. Der entstandene Rückstand wurde abgesaugt. Nach dem Trocknen wurde 1.09 g (76.1% d. Th.) der gewünschten Zielverbindung erhalten.
LC/MS (Methode 2): Rₜ = 1.16 min; MS (ESIpos): m/z = 687 [M-TFA+H]⁺.

### Beispiel 35A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)-L-alanyl-L-leucinat

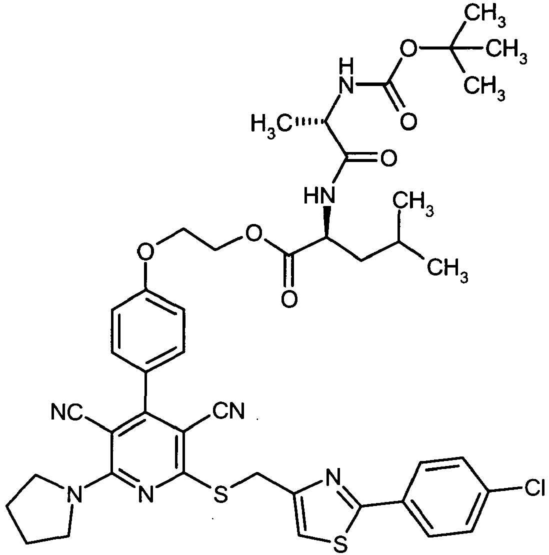

86 mg (0.45 mmol) N-(tert.-Butoxycarbonyl)-L-alanin wurden in 5.0 ml DMF vorgelegt und mit 95 mg (0.49 mmol) 1-(3-Dimethylaminopropyl)-3-ethylcarbodiimidhydrochlorid, 95 mg (0.62 mmol) 1-Hydroxy-1H-benzotriazol Hydrat und 266.13 g (2.06 mmol) *N,N*-Diisopropylethylamin versetzt. Es wurde gerührt bis eine klare Lösung eintrat. Danach wurden 330 mg (0.41 mmol) 2-{4-[2-({[2-(4-chlorphenyl)-1,3-thiazol-4-yl]methyl}sulfanyl)-3,5-dicyan-6-(pyrrolidin-1-yl)pyridin-4-yl]phenoxy}ethyl-L-leucinat-Trifluoracetat zugegeben und 18 h bei Raumtemperatur gerührt. Das Reaktionsgemisch wurde auf 300 ml Wasser gegeben. Der entstandene Rückstand wurde abgesaugt und mit 20 ml Wasser gewaschen. Das Rohprodukt wurde in 15 ml Methanol suspendiert und 5 min im Utraschallbad beschallt. Der Rückstand wurde abgesaugt und mit 10 ml Diethylether gewaschen. Es wurden 0.15 g (43% d. Th.) der gewünschten Zielverbindung erhalten.
LC/MS (Methode 2): Rₜ = 1.58 min; MS (ESIpos): m/z = 858 [M+H]⁺.

### Beispiel 36A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)-beta-alanyl-L-leucinat

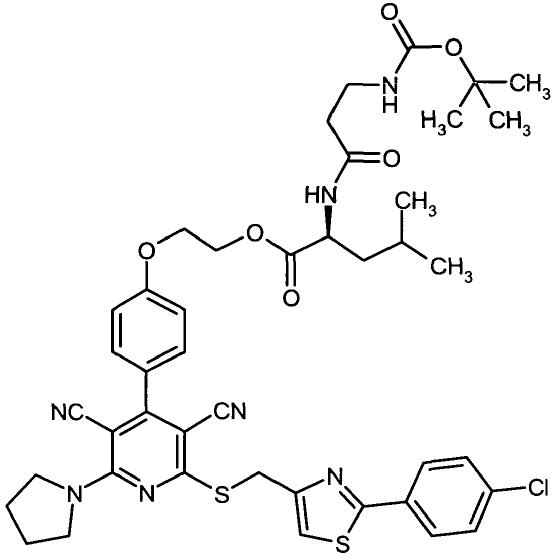

86 mg (0.45 mmol) N-(tert.-Butoxycarbonyl)-beta-alanin wurden in 5.0 ml DMF vorgelegt und mit 95 mg (0.49 mmol) 1-(3-Dimethylaminopropyl)-3-ethylcarbodiimidhydrochlorid, 95 mg (0.62 mmol) 1-Hydroxy-1H-benzotriazol Hydrat und 266.13 g (2.06 mmol) *N,N*-Diisopropylethylamin versetzt. Es wurde gerührt bis eine klare Lösung eintrat. Danach wurden 330 mg (0.41 mmol) 2-{4-[2-({[2-(4-chlorphenyl)-1,3-thiazol-4-yl]methyl}sulfanyl)-3,5-dicyan-6-(pyrrolidin-1-yl)pyridin-4-yl]phenoxy}ethyl-L-leucinat-Trifluoracetat zugegeben und 18 h bei Raumtemperatur gerührt. Das Reaktionsgemisch wurde auf 300 ml Wasser gegeben. Der entstandene Rückstand wurde abgesaugt und mit 20 ml Wasser gewaschen. Das Rohprodukt wurde in 15 ml Methanol suspendiert und 5 min im Utraschallbad beschallt. Der Rückstand wurde abgesaugt und mit 10 ml Diethylether gewaschen. Es wurden 0.13 g (37% d. Th.) der gewünschten Zielverbindung erhalten.
LC/MS (Methode 2): Rₜ = 1.56 min; MS (ESIpos): m/z = 858 [M+H]⁺.

### Beispiel 37A

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)glycyl-L-leucinat

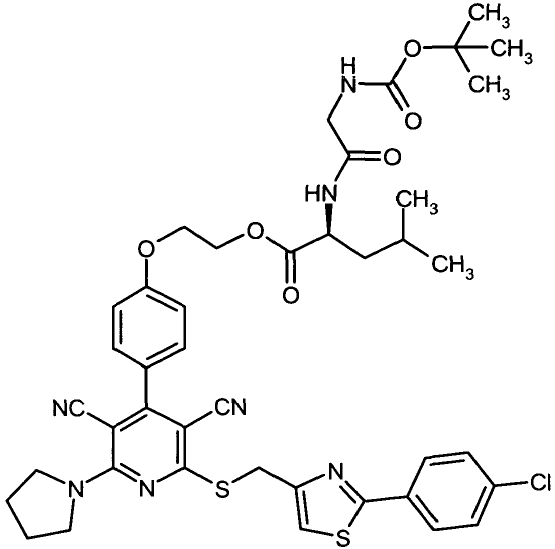

80 mg (0.45 mmol) *N*-(tert.-Butoxycarbonyl)glycin wurden in 5.0 ml DMF vorgelegt und mit 95 mg (0.49 mmol) 1-(3-Dimethylaminopropyl)-3-ethylcarbodiimidhydrochlorid, 95 mg (0.62 mmol) 1-Hydroxy-1H-benzotriazol Hydrat und 267 mg (2.06 mmol) N,N-Diisopropylethylamin versetzt. Es wurde gerührt bis eine klare Lösung eintrat. Danach wurden 330 mg (0.41 mmol) 2-{4-[2-({[2-(4-chlorphenyl)-1,3-thiazol-4-yl]methyl}sulfanyl)-3,5-dicyan-6-(pyrrolidin-1-yl)pyridin-4-yl]phenoxy}ethyl-L-leucinat-Trifluoracetat zugegeben und 18 h bei Raumtemperatur gerührt. Das Reaktionsgemisch wurde auf 300 ml Wasser gegeben. Der entstandene Rückstand wurde abgesaugt und mit 20 ml Wasser gewaschen. Das Rohprodukt wurde in 15 ml Methanol suspendiert und 5 min im Utraschallbad beschallt. Der Rückstand wurde abgesaugt und mit 10 ml Diethylether gewaschen. Es wurden 0.17 g (47% d. Th.) der gewünschten Zielverbindung erhalten.
LC/MS (Methode 2): Rₜ = 1.56 min; MS (ESIpos): m/z = 844 [M+H]⁺.

### Ausführungsbeispiele:

### Beispiel 1

### 2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril

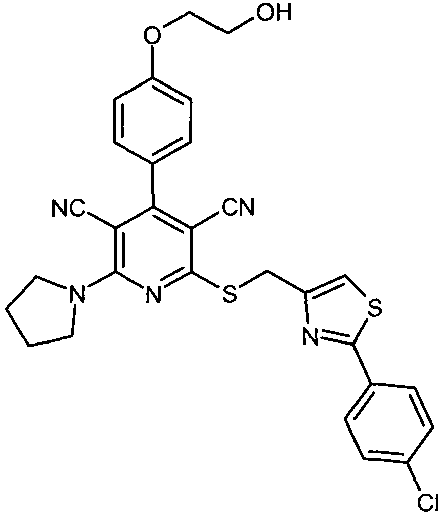

90 mg (0.17 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 30 µl (0.37 mmol) Pyrrolidin wurden in 2.3 ml THF 30 min bei RT gerührt. Das Reaktionsgemisch wurde mit ca. 12 ml Wasser versetzt und die entstandene Suspension wurde am Rotationsverdampfer vom THF befreit und der entstandene Niederschlag wurde abfiltriert und mit Wasser gewaschen und im Hochvakuum getrocknet. Es wurden 78 mg (81% d. Th.) der gewünschten Zielverbindung erhalten.
¹H-NMR (400 MHz, DMSO-d₆): δ = 7.95 (d, 2H), 7.70 (s, 1H), 7.58 (d, 2H), 7.47 (d, 2H), 7.10 (d, 2H), 4.90 (t, 1H), 4.70 (s, 2H), 4.07 (t, 2H), 3.83 (br s, 4H), 3.74 (q, 2H), 1.94 (br s, 4H).
LC-MS (Methode 5): Rₜ = 2.63 min; MS (ESIpos): m/z = 574 [M+H]⁺.

### Beispiel 2

### 2-({[2-(4-Chlorphenyl)-1,3-thiazol-4-yl]methyl}sulfanyl)-4-[4-(2-hydroxyethoxy)phenyl]-6-(methylamino)pyridin-3,5-dicarbonitril

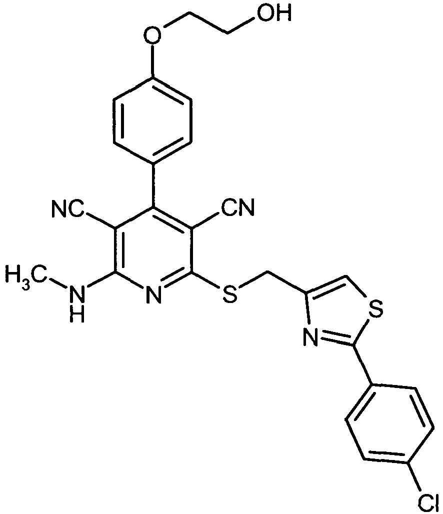

3.0 g (5.56 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 11.12 ml (22.24 mmol) Methylamin wurden in 75 ml THF über Nacht bei RT gerührt. Das Reaktionsgemisch wurde mit ca. 300 ml Wasser versetzt und der entstandene Niederschlag wurde abfiltriert und mit Wasser gewaschen und im Hochvakuum getrocknet. Es wurden 2.69 g (91% d. Th.) der gewünschten Zielverbindung erhalten.
¹H-NMR (400 MHz, DMSO-d₆): δ = 8.12 (q, 1H), 7.96 (d, 2H), 7.69 (s, 1H), 7.59 (d, 2H), 7.48 (d, 2H), 7.11 (d, 2H), 4.91 (t, 1H), 4.72 (s, 2H), 4.09 (t, 2H), 3.74 (q, 2H), 3.01 (d, 3H).
LC-MS (Methode 5): Rₜ = 2.41 min; MS (ESIpos): m/z = 534 [M+H]⁺.

### Beispiel 3

### 2-({[2-(4-Chlorphenyl)-1,3-thiazol-4-yl]methyl}sulfanyl)-6-(ethylamino)-4-[4-(2-hydroxy-ethoxy)phenyl]pyridin-3,5-dicarbonitril

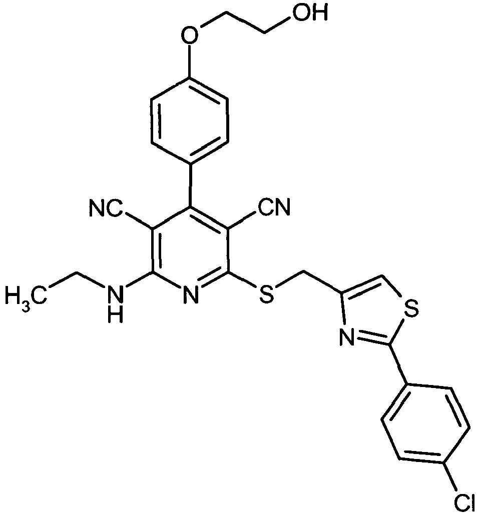

100 mg (0.17 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 0.17 ml (0.33 mmol) Ethylamin (2M Lösung in THF) wurden in 2 ml THF 30 min bei RT gerührt. Anschließend wurden nochmals 0.17 ml (0.33 mmol) Ethylamin (2M Lösung in THF) zugegeben und 2 h bei RT gerührt. Das Reaktionsgemisch wurde mit ca. 15 ml Wasser versetzt und der entstandene Niederschlag wurde abfiltriert, mit Wasser gewaschen und im Hochvakuum getrocknet. Es wurden 81 mg (89% d. Th.) der gewünschten Zielverbindung erhalten.
¹H-NMR (400 MHz, DMSO-d₆): δ = 8.19 (q, 1H), 7.95 (d, 2H), 7.67 (s, 1H), 7.58 (d, 2H), 7.48 (d, 2H), 7.10 (d, 2H), 4.90 (t, 1H), 4.70 (s, 2H), 4.09 (t, 2H), 3.74 (q, 2H), 3.50 (Quintett, 2H), 1.09 (t, 3H).
LC-MS (Methode 7): Rₜ = 2.87 min; MS (ESIpos): m/z = 548 [M+H]⁺.

### Beispiel 4

### 2-({[2-(4-Chlorphenyl)-1,3-thiazol-4-yl]methyl}sulfanyl)-6-(dimethylamino)-4-[4-(2-hydroxyethoxy)phenyl]pyridin-3,5-dicarbonitril

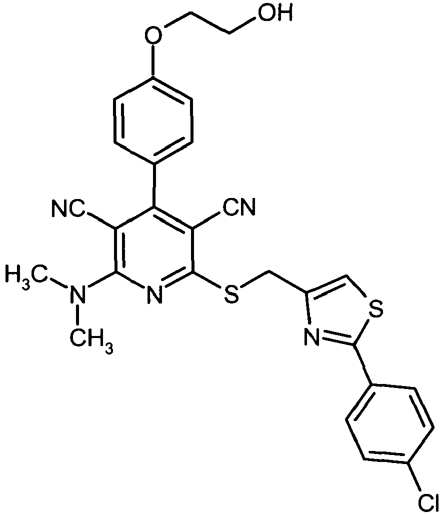

80 mg (0.15 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 28 mg (0.30 mmol) Methansulfonsäureamid wurden in 1.5 ml DMF über Nacht bei 100°C gerührt. Nach Abkühlen wurde das Rohprodukt mittels präparativer HPLC (Acetonitril/Wasser) gereinigt. Es wurden 41 mg (50% d. Th.) der Zielverbindung erhalten.
¹H-NMR (400 MHz, DMSO-d₆): δ = 7.96 (d, 2H), 7.70 (s, 1H), 7.59 (d, 2H), 7.51 (d, 2H), 7.10 (d, 2H), 4.90 (t, 1H), 4.70 (s, 2H), 4.09 (t, 2H), 3.74 (q, 2H), 3.34 (s, 6H).
LC-MS (Methode 7): Rₜ = 2.81 min; MS (ESIpos): m/z = 548 [M+H]⁺.

### Beispiel 5

### 2-({[2-(4-Chlorphenyl)-1,3-thiazol-4-yl]methyl}sulfanyl)-6-[ethyl(methyl)amino]-4-[4-(2-hydroxyethoxy)phenyl]pyridin-3,5-dicarbonitril

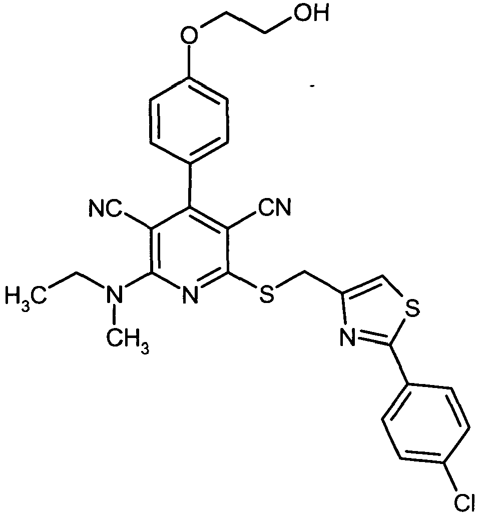

60 mg (0.11 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 0.019 ml (0.22 mmol) N-Ethyl-methylamin wurden in 1.5 ml THF 30 min bei RT gerührt. Das Reaktionsgemisch wurde mit ca. 15 ml Wasser versetzt und die wässrige Phase wurde 3x mit Essigsäureethylester extrahiert. Die vereinten organischen Phasen wurden einmal mit Natriumchlorid-Lsg. gewaschen, über Natriumsulfat getrocknet, eingedampft und im Hochvakuum getrocknet. Es wurden 63 mg (99% d. Th.) der gewünschten Zielverbindung erhalten.
¹H-NMR (400 MHz, DMSO-d₆): δ = 7.95 (d, 2H), 7.69 (s, 1H), 7.58 (d, 2H), 7.51 (d, 2H), 7.10 (d, 2H), 4.90 (t, 1H), 4.70 (s, 2H), 4.09 (t, 2H), 3.80-3.70 (m, 4H), 3.31 (s, 3H), 1.19 (t, 3H).
LC-MS (Methode 3): Rₜ = 3.02 min; MS (ESIpos): m/z = 562 [M+H]⁺.

### Beispiel 6

### 2-({[2-(4-Chlorphenyl)-1,3-thiazol-4-yl]methyl}sulfanyl)-6-(diethylamino)-4-[4-(2-hydroxyethoxy)phenyl]pyridin-3,5-dicarbonitril

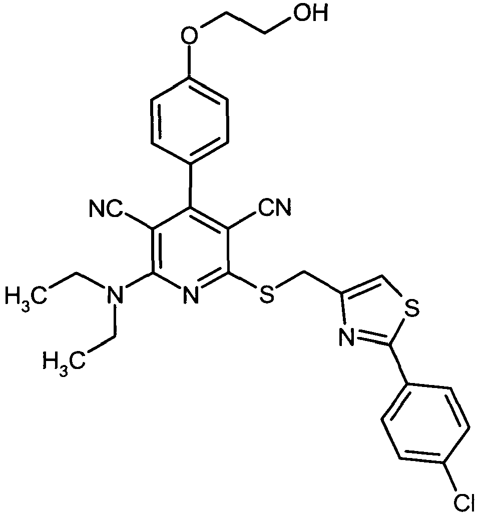

60 mg (0.11 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 0.023 ml (0.22 mmol) Diethylamin wurden in 1.5 ml THF 30 min bei RT gerührt. Das Reaktionsgemisch wurde mit ca. 15 ml Wasser versetzt und die wässrige Phase wurde 3x mit Essigsäureethylester extrahiert. Die vereinten organischen Phasen wurden einmal mit gesättigter wässriger Natriumchlorid-Lösung gewaschen, über Natriumsulfat getrocknet, eingedampft und im Hochvakuum getrocknet. Es wurden 67 mg (99% d. Th., Reinheit 95%) der gewünschten Zielverbindung erhalten.
¹H-NMR (400 MHz, DMSO-d₆): δ = 7.95 (d, 2H), 7.70 (s, 1H), 7.59 (d, 2H), 7.49 (d, 2H), 7.10 (d, 2H), 4.91 (t, 1H), 4.70 (s, 2H), 4.09 (t, 2H), 3.80-3.70 (m, 6H), 3.31 (s, 3H), 1.20 (t, 6H).
LC-MS (Methode 3): Rₜ = 3.11 min; MS (ESIpos): m/z = 576 [M+H]⁺.

### Beispiel 7

### 2-({[2-(4-Chlorphenyl)-1,3-thiazol-4-yl]methyl}sulfanyl)-4-[4-(2-hydroxyethoxy)phenyl]-6-(propan-2-ylamino)pyridin-3,5-dicarbonitril

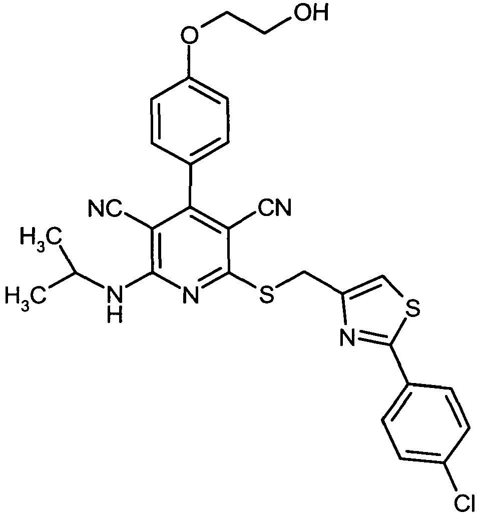

60 mg (0.11 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 0.011 ml (0.13 mmol) Isopropylamin wurden in 1.5 ml THF 60 min bei RT gerührt. Anschließend wurden nochmals 11 µl (0.13 mmol) Isopropylamin zugegeben und nochmals 60 min bei RT gerührt. Das Reaktionsgemisch wurde mit ca. 10 ml Wasser versetzt und der entstandene Niederschlag wurde abfiltriert, mit Wasser gewaschen und im Hochvakuum getrocknet. Es wurden 35 mg (56% d. Th.) der gewünschten Zielverbindung erhalten.
¹H-NMR (400 MHz, DMSO-d₆): δ = 7.96 (d, 2H), 7.80 (d, 1H), 7.67 (s, 1H), 7.58 (d, 2H), 7.48 (d, 2H), 7.10 (d, 2H), 4.90 (t, 1H), 4.68 (s, 2H), 4.50-4.39 (m, 1H), 4.09 (t, 2H), 3.74 (q, 2H), 1.13 (d, 6H).
LC-MS (Methode 7): Rₜ = 2.98 min; MS (ESIpos): m/z = 562 [M+H]⁺.

### Beispiel 8

### 2-(Azetidin-1-yl)-6-({[2-(4-chlorphenyl)-1,3-thiazol-4-yl]methyl}sulfanyl)-4-[4-(2-hydroxyethoxy)phenyl]pyridin-3,5-dicarbonitril

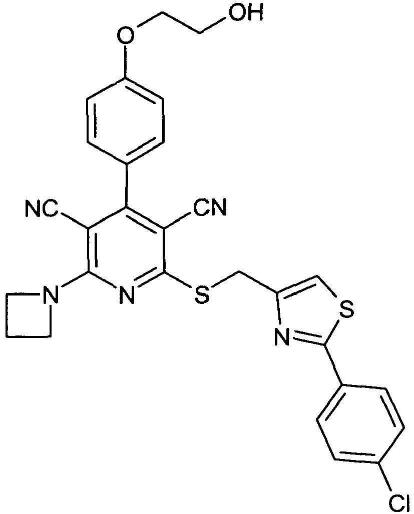

100 mg (0.19 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 0.015 ml (0.22 mmol) Azetidin wurden in 2.5 ml THF über Nacht bei RT gerührt. Anschließend wurden nochmals 0.025 ml (0.36 mmol) Azetidin zugegeben und nochmals über Nacht bei RT gerührt. Das Reaktionsgemisch wurde mit ca. 10 ml Wasser versetzt und der entstandene Niederschlag wurde abfiltriert, mit Wasser gewaschen und im Hochvakuum getrocknet. Es wurden 85 mg (82% d. Th.) der gewünschten Zielverbindung erhalten.
¹H-NMR (400 MHz, DMSO-d₆): δ = 7.95 (d, 2H), 7.68 (s, 1H), 7.59 (d, 2H), 7.47 (d, 2H), 7.10 (d, 2H), 4.90 (t, 1H), 4.68 (s, 2H), 4.48 (br s, 4H), 4.08 (t, 2H), 3.73 (q, 2H), 2.38 (Quintett, 2H).
LC-MS (Methode 4): Rₜ = 1.52 min; MS (ESIpos): m/z = 560 [M+H]⁺.

### Beispiel 9

### 2-({[2-(4-Chlorphenyl)-1,3-thiazol-4-yl]methyl}sulfanyl)-6-(cyclopropylamino)-4-[4-(2-hydroxyethoxy)phenyl]pyridin-3,5-dicarbonitril

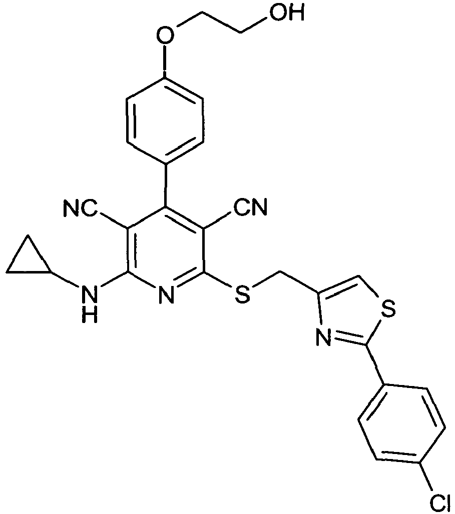

50 mg (0.09 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 0.013 ml (0.19 mmol) Cyclopropanamin wurden in 1.3 ml DMF über Nacht bei RT gerührt. Das Rohprodukt wurde direkt mittels präparativer HPLC (Acetonitril/Wasser) gereinigt. Die gesammelten Produktfraktionen wurden erneut in 2 ml DMF gelöst und mit 0.013 ml (0.19 mmol) Cyclopropanamin versetzt und über Nacht bei RT gerührt. Das Rohprodukt wurde anschließend erneut mittels präparativer HPLC (Acetonitril/Wasser) gereinigt. Es wurden 20 mg (39% d. Th.) der gewünschten Zielverbindung erhalten.
¹H-NMR (400 MHz, DMSO-d₆): δ = 8.30 (br s, 1H), 7.94 (d, 2H), 7.68 (s, 1H), 7.59 (d, 2H), 7.48 (d, 2H), 7.10 (d, 2H), 4.90 (t, 1H), 4.78 (s, 2H), 4.09 (t, 2H), 3.74 (q, 2H), 3.01-2.94 (m, 1H), 0.78-0.65 (m, 4H).
LC-MS (Methode 6): Rₜ = 3.05 min; MS (ESIpos): m/z = 560 [M+H]⁺.

### Beispiel 10

### 2-({[2-(4-Chlorphenyl)-1,3-thiazol-4-yl]methyl}sulfanyl)-6-(cyclobutylamino)-4-[4-(2-hydroxyethoxy)phenyl]pyridin-3,5-dicarbonitril

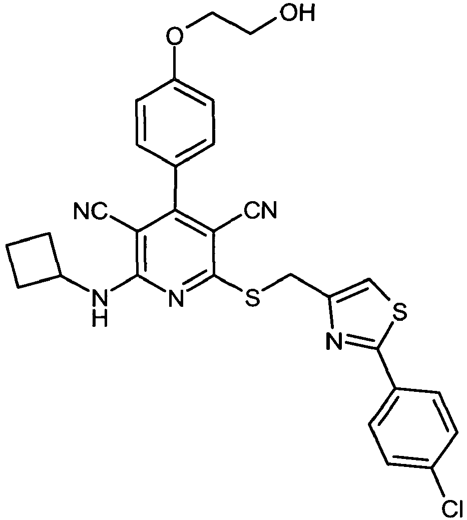

80 mg (0.15 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 26 µl (0.30 mmol) Cyclobutanamin wurden in 1.5 ml DMF über Nacht bei RT gerührt. Anschließend wurde die Reaktionsmischung mittels präparativer HPLC (Acetonitril/Wasser) gereinigt. Es wurden 56 mg (65% d. Th.) der Zielverbindung erhalten.
¹H-NMR (400 MHz, DMSO-d₆): δ = 8.28 (d, 1H), 7.97 (d, 2H), 7.68 (s, 1H), 7.59 (d, 2H), 7.48 (d, 2H), 7.11 (d, 2H), 4.90 (t, 1H), 4.70 (s, 2H), 4.59 (Quintett, 1H), 4.09 (t, 2H), 3.74 (q, 2H), 2.20-2.10 (m, 4H), 1.68-1.49 (m, 2H).
LC-MS (Methode 6): Rₜ = 3.18 min; MS (ESIpos): m/z = 574 [M+H]⁺.

### Beispiel 11

### 2-({[2-(4-Chlorphenyl)-1,3-thiazol-4-yl]methyl}sulfanyl)-4-[4-(2-hydroxyethoxy)phenyl]-6-[(3,3,3-trifluorpropyl)amino]pyridin-3,5-dicarbonitril

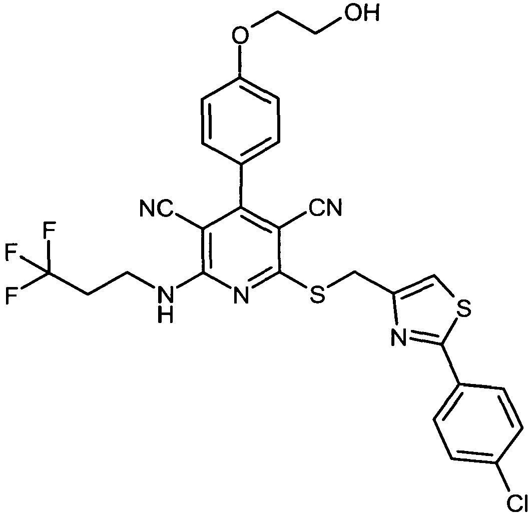

150 mg (0.21 mmol, Reinheit ca. 74%) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 93 mg (0.82 mmol) 3,3,3-Trifluorpropan-1-amin wurden in 2.0 ml DMF 2 h bei 100°C gerührt. Anschließend wurde die Reaktionsmischung mit ca. 1 ml Wasser und ca. 3 ml THF verdünnt und mittels präparativer HPLC (Acetonitril/Wasser + 0.1% TFA) gereinigt. Es wurden 105 mg (83% d. Th.) der Zielverbindung erhalten.
¹H-NMR (400 MHz, DMSO-d₆): δ = 8.32 (t, 1H), 7.93 (d, 2H), 7.68 (s, 1H), 7.58 (d, 2H), 7.49 (d, 2H), 7.12 (d, 2H), 4.91 (t, 1H), 4.73 (s, 2H), 4.09 (t, 2H), 3.78-3.69 (m, 4H), 2.61-2.49 (m, 2H).
LC-MS (Methode 2): Rₜ = 1.34 min; MS (ESIpos): m/z = 616 [M+H]⁺.

### Beispiel 12

### 2-({[2-(4-Chlorphenyl)-1,3-thiazol-4-yl]methyl}sulfanyl)-4-[4-(2-hydroxyethoxy)phenyl]-6-(propylamino)pyridin-3,5-dicarbonitril

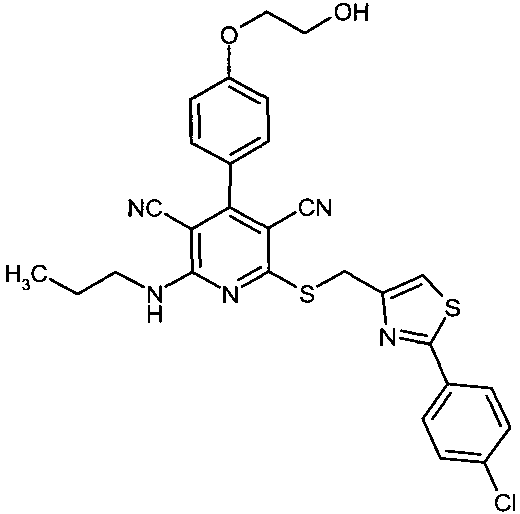

100 mg (0.19 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 30 µl (0.37 mmol) n-Propylamin wurden in 2.5 ml THF 2 h bei RT gerührt. Anschließend wurde die Reaktionsmischung mittels präparativer HPLC (Acetonitril/Wasser + 0.1% TFA) gereinigt. Es wurden 67 mg (64% d. Th.) der Zielverbindung erhalten.
¹H-NMR (400 MHz, DMSO-d₆): δ = 8.20 (t, 1H), 7.94 (d, 2H), 7.63 (s, 1H), 7.58 (d, 2H), 7.49 (d, 2H), 7.11 (d, 2H), 4.90 (t, 1H), 4.70 (s, 2H), 4.09 (t, 2H), 3.74 (q, 2H), 3.43-3.34 (m, 2H), 1.49 (Quintett, 2H), 0.78 (t, 3H).
LC-MS (Methode 2): Rₜ = 1.40 min; MS (ESIpos): m/z = 562 [M+H]⁺.

### Beispiel 13

### 2-({[2-(4-Chlorphenyl)-1,3-thiazol-4-yl]methyl}sulfanyl)-4-[4-(2-hydroxyethoxy)phenyl]-6-(piperidin-1-yl)pyridin-3,5-dicarbonitril

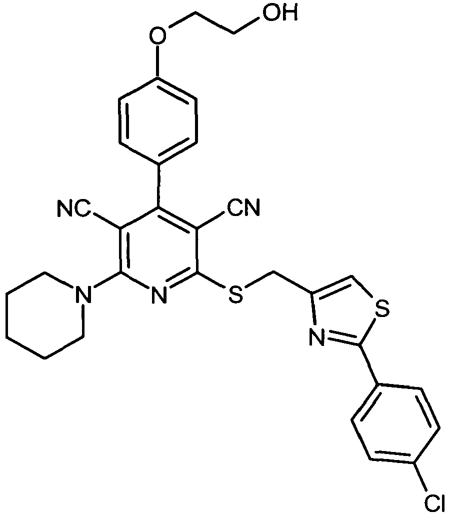

300 mg (0.41 mmol, Reinheit ca. 74%) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 163 µl (1.65 mmol) Piperidin wurden in 5.6 ml THF 2 h bei RT gerührt. Anschließend wurde die Reaktionsmischung mit ca. 1 ml Wasser und ca. 3 ml THF verdünnt und mittels präparativer HPLC (Acetonitril/Wasser + 0.1% TFA) gereinigt. Es wurden 215 mg (89% d. Th.) der Zielverbindung erhalten.
¹H-NMR (400 MHz, DMSO-d₆): δ = 7.96 (d, 2H), 7.69 (s, 1H), 7.58 (d, 2H), 7.54 (d, 2H), 7.11 (d, 2H), 4.90 (br s, 1H), 4.69 (s, 2H), 4.09 (t, 2H), 3.88-3.78 (m, 4H), 3.74 (t, 2H), 1.70-1.54 (m, 6H).
LC-MS (Methode 2): Rₜ = 1.45 min; MS (ESIpos): m/z = 588 [M+H]⁺.

Die in Tabelle 4 aufgeführten Beispiele werden aus den entsprechenden Ausgangsverbindungen analog zu Beispiel 13 hergestellt. Die Menge des eingesetzten Amins beträgt 2.2-4.0 Äquivalente in Bezug auf 2-Chlor-6-({[2-(4-chlorphenyl)-1,3-thiazol-4-yl]methyl}sulfanyl)-4-[4-(2-hydroxy-ethoxy)phenyl]pyridin-3,5-dicarbonitril [Beispiel 2A]:

**Tabelle 4:**

| **Beispiel Nr.** | **Struktur (Ausbeute)** | **LC-MS: Rₜ [min] (Methode); MS (ESI): m/z [M+H]⁺** | **¹H-NMR (DMSO-d₆):** |
|---|---|---|---|
| **14** | 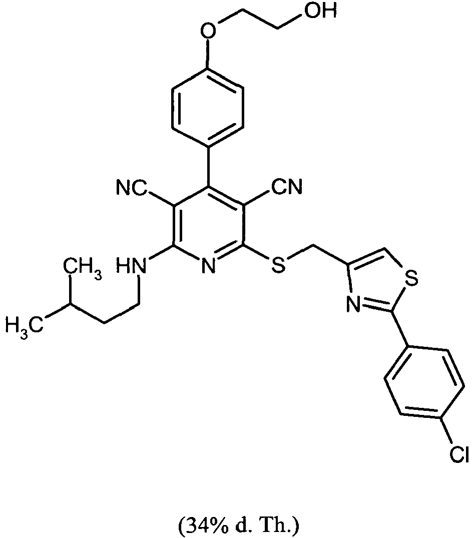 | 1.69 min (Methode 4); m/z = 590 | δ (400 MHz) = 8.20 (t, 1H), 7.95 (d, 2H), 7.64 (s, 1H), 7.58 (d, 2H), 7.50 (d, 2H), 7.11 (d, 2H), 4.90 (t, 1H), 4.71 (s, 2H), 4.08 (t, 2H), 3.74 (q, 2H), 3.48 (q, 2H), 1.51 (Septett, 1H), 1.39 (q, 2H), 0.80 (d, 6H). |
| **15** | 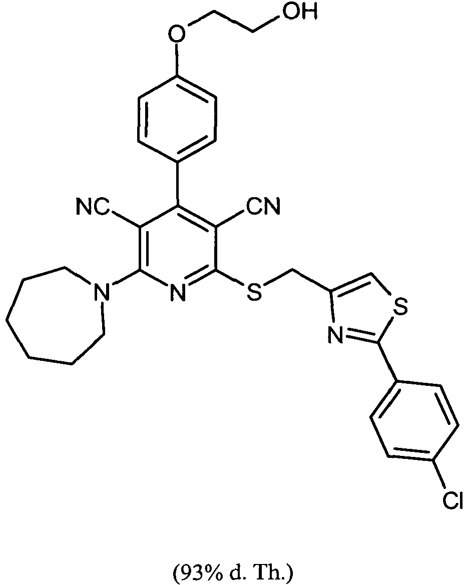 | 1.45 min (Methode 2); m/z = 602 | δ (400 MHz) = 7.94 (d, 2H), 7.69 (s, 1H), 7.58 (d, 2H), 7.50 (d, 2H), 7.10 (d, 2H), 4.69 (s, 2H), 4.09 (t, 2H), 3.92-3.59 (m, 7H), 1.78 (br s, 4H), 1.50 (br s, 4H). |
| **16** | 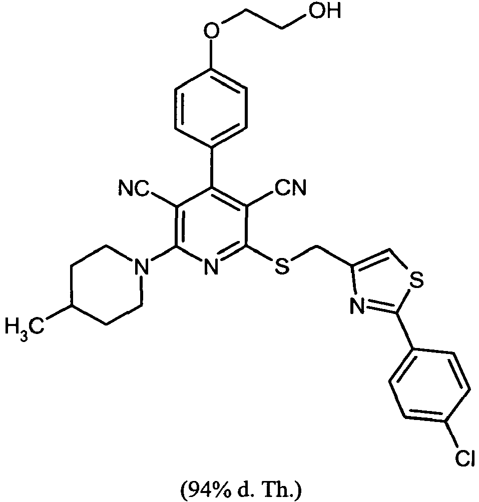 | 1.69 min (Methode 4); m/z = 602 | δ (400 MHz) = 7.94 (d, 2H), 7.68 (s, 1H), 7.58 (d, 2H), 7.52 (d, 2H), 7.10 (d, 2H), 4.69 (s, 2H), 4.55 (d, 2H), 4.09 (t, 2H), 3.76 (t, 3H), 3.18 (t, 2H), 1.72-1.60 (m, 3H), 1.11 (q, 2H), 0.86 (d, 3H). |
| **17** | 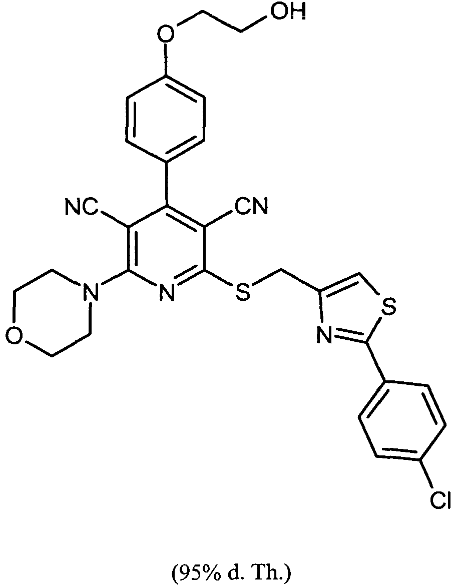 | 1.30 min (Methode 2); m/z = 590 | δ (400 MHz) = 7.95 (d, 2H), 7.70 (s, 1H), 7.59 (d, 2H), 7.54 (d, 2H), 7.11 (d, 2H), 4.69 (s, 2H), 4.09 (t, 2H), 3.93-3.87 (m, 4H), 3.73 (t, 2H), 3.71-3.62 (m, 4H). |
| **18** | 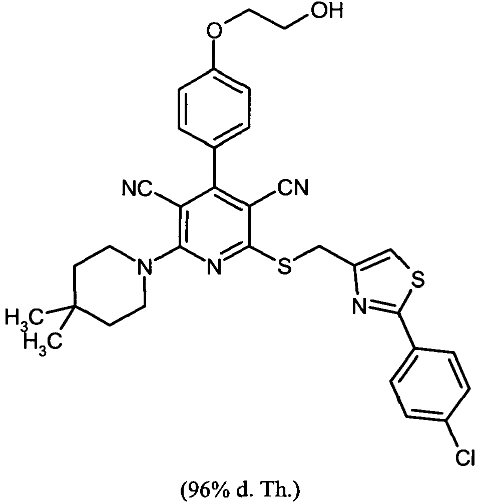 | 1.52 min (Methode 2); m/z = 616 | δ (400 MHz) = 7.95 (d, 2H), 7.67 (s, 1H), 7.59 (d, 2H), 7.54 (d, 2H), 7.11 (d, 2H), 4.68 (s, 2H), 4.09 (t, 2H), 3.86-3.80 (m, 4H), 3.73 (t, 2H), 1.40-1.36 (m, 4H), 0.92 (s, 6H). |
| **19** | 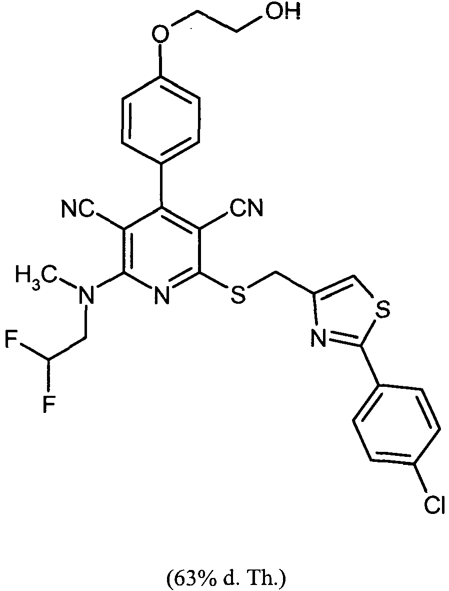 | 1.32 min (Methode 2); m/z = 598 | δ (400 MHz) = 7.94 (d, 2H), 7.70 (s, 1H), 7.58 (d, 2H), 7.54 (d, 2H), 7.11 (d, 2H), 6.50-6.18 (m, 1H), 4.70 (s, 2H), 4.32-4.20 (m, 2H), 4.09 (t, 2H), 3.74 (t, 2H), 3.49 (s, 3H). |
| **20** | 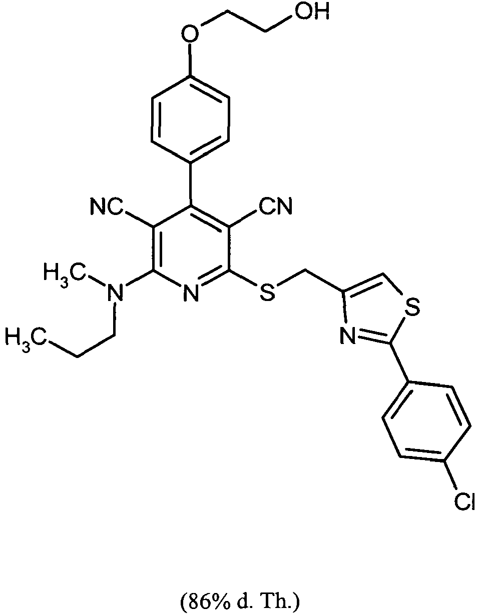 | 1.40 min (Methode 2); m/z = 576 | δ (400 MHz) = 7.95 (d, 2H), 7.68 (s, 1H), 7.58 (d, 2H), 7.53 (d, 2H), 7.11 (d, 2H), 4.69 (s, 2H), 4.09 (t, 2H), 3.76-3.60 (m, 4H), 3.32 (s, 3H), 1.62 (Sextett, 2H), 0.80 (t, 3H). |
| **21** | 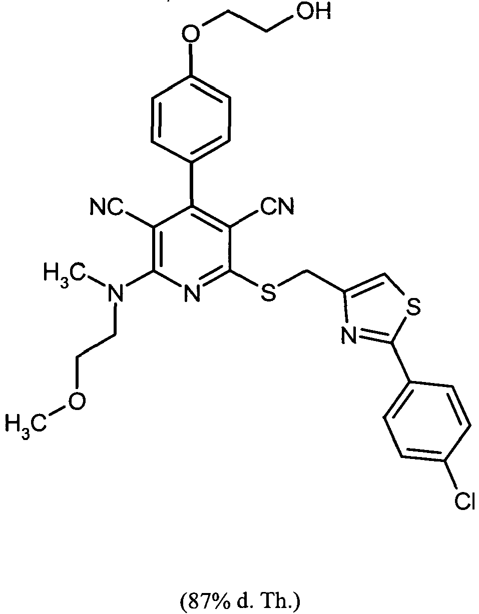 | 1.32 min (Methode 2); m/z = 592 | δ (400 MHz) = 7.96 (d, 2H), 7.69 (s, 1H), 7.59 (d, 2H), 7.52 (d, 2H), 7.11 (d, 2H), 4.69 (s, 2H), 4.09 (t, 2H), 3.96 (t, 2H), 3.74 (t, 2H), 3.54 (t, 2H), 3.40 (s, 3H), 3.20 (s, 3H). |
| **22** | 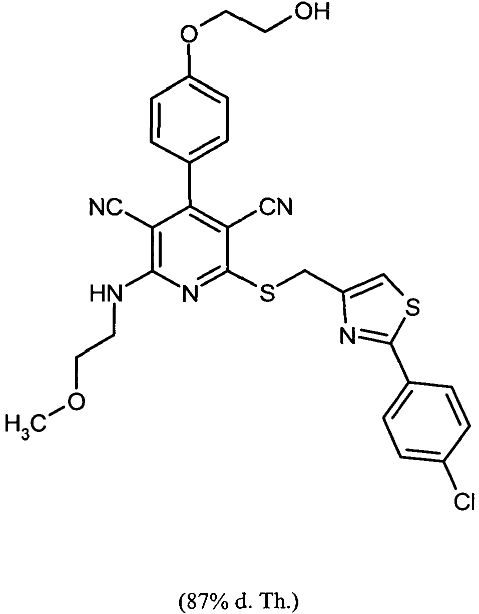 | 1.28 min (Methode 2); m/z = 578 | δ (400 MHz) = 8.12 (t, 1H), 7.94 (d, 2H), 7.69 (s, 1H), 7.59 (d, 2H), 7.49 (d, 2H), 7.11 (d, 2H), 4.90 (t, 1H), 4.70 (s, 2H), 4.09 (t, 2H), 3.74 (q, 2H), 3.68 (q, 2H), 3.42 (t, 2H), 3.19 (s, 3H). |
| **23** | 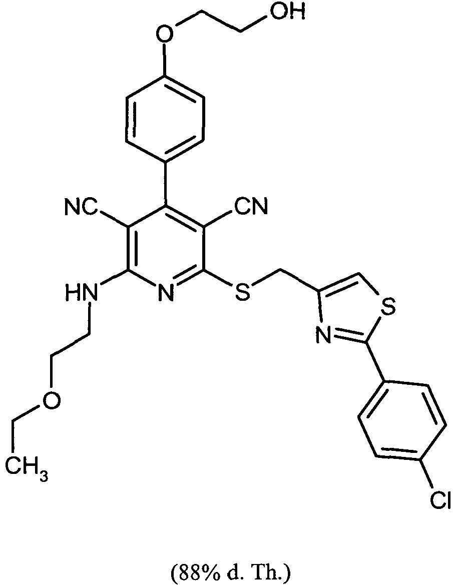 | 1.33 min (Methode 2); m/z = 592 | δ (400 MHz) = 8.11 (t, 1H), 7.95 (d, 2H), 7.68 (s, 1H), 7.59 (d, 2H), 7.49 (d, 2H), 7.11 (d, 2H), 4.70 (s, 2H), 4.09 (t, 2H), 3.74 (t, 2H), 3.65 (q, 2H), 3.48 (t, 2H), 3.38 (q, 2H), 1.03 (t, 3H). |
| **24** | 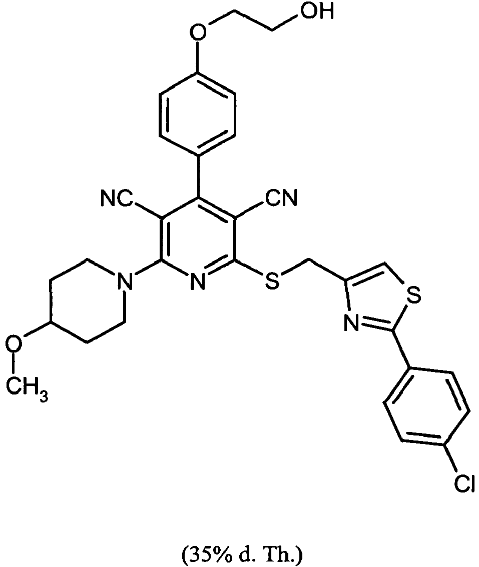 | 1.37 min (Methode 2); m/z = 618 | δ (400 MHz) = 7.96 (d, 2H), 7.68 (s, 1H), 7.59 (d, 2H), 7.55 (d, 2H), 7.10 (d, 2H), 4.68 (s, 2H), 4.13-4.05 (m, 4H), 3.72 (t, 2H), 3.67-3.58 (m, 2H), 3.50-3.38 (m, 1H), 3.23 (s, 3H), 1.96-1.87 (m, 2H), 1.56-1.46 (m, 2H). |
| **25** | 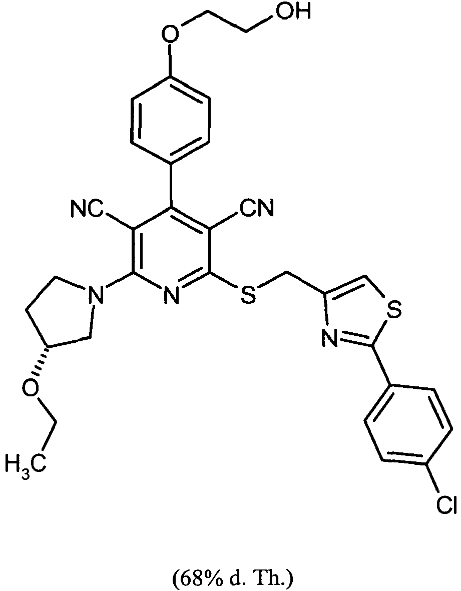 | 1.58 min (Methode 4); m/z = 618 | δ (400 MHz) = 7.95 (d, 2H), 7.69 (s, 1H), 7.59 (d, 2H), 7.49 (d, 2H), 7.10 (d, 2H), 4.70 (s, 2H), 4.14 (br s, 1H), 4.09 (t, 2H), 3.99-3.70 (m, 6H), 3.48-3.34 (m, 2H), 2.12-1.93 (m, 2H), 1.05 (t, 3H). |

### Beispiel 26

### 2-({[2-(4-Chlorphenyl)-1,3-thiazol-4-yl]methyl}sulfanyl)-6-(3,3-difluorpyrrolidin-1-yl)-4-[4-(2-hydroxyethoxy)phenyl]pyridin-3,5-dicarbonitril

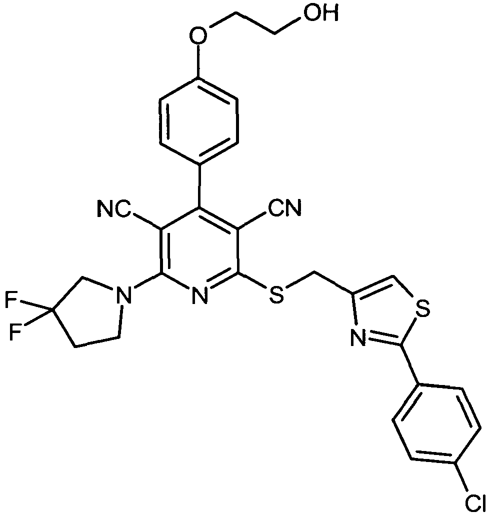

100 mg (0.19 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 53 mg (0.37 mmol) 3,3-Difluorpyrrolidin-Hydrochlorid und 52 µl (0.37 mmol) Triethylamin wurden in 2.5 ml THF 2 h bei RT gerührt. Anschließend wurde die Reaktionsmischung mit ca. 1 ml Wasser und ca. 3 ml THF verdünnt und mittels präparativer HPLC (Acetonitril/Wasser + 0.1 % TFA) gereinigt. Es wurden 62 mg (54% d. Th.) der Zielverbindung erhalten.
¹H-NMR (400 MHz, DMSO-d₆): δ = 7.92 (d, 2H), 7.71 (s, 1H), 7.58 (d, 2H), 7.50 (d, 2H), 7.11 (d, 2H), 4.72 (s, 2H), 4.29 (t, 2H), 4.17-4.07 (m, 4H), 3.73 (t, 2H), 2.62-2.50 (m, 2H).
LC-MS (Methode 2): Rₜ = 1.35 min; MS (ESIpos): m/z = 610 [M+H]⁺.

Die in Tabelle 5 aufgeführten Beispiele werden aus den entsprechenden Ausgangsverbindungen analog zu Beispiel 26 hergestellt. Die Menge des eingesetzten Amins beträgt 1.5-2.0 Äquivalente, die von Triethylamin 2.0-3.0 Äquivalente in Bezug auf 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A]:

**Tabelle 5:**

| **Beispiel Nr.** | **Struktur (Ausbeute)** | **LC-MS: Rₜ [min] (Methode); MS (ESI): m/z [M+H]⁺** | **¹H-NMR (DMSO-d₆):** |
|---|---|---|---|
| **27** | 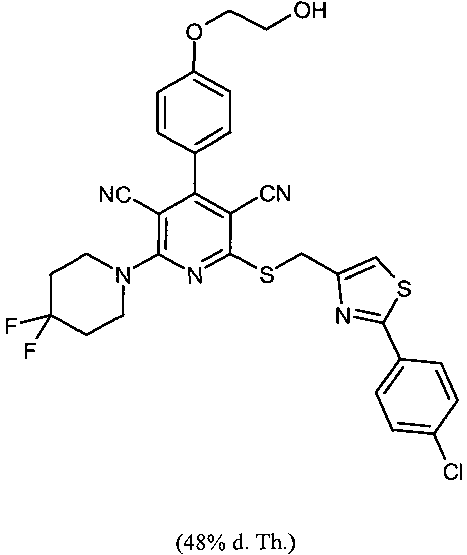 | 1.36 min (Methode 2); m/z = 624 | δ (400 MHz) = 7.95 (d, 2H), 7.70 (s, 1H), 7.59 (d, 2H), 7.55 (d, 2H), 7.12 (d, 2H), 4.90 (t, 1H), 4.71 (s, 2H), 4.09 (t, 2H), 4.02-3.93 (m, 4H), 3.74 (q, 2H), 2.19-2.05 (m, 4H). |
| **28** | 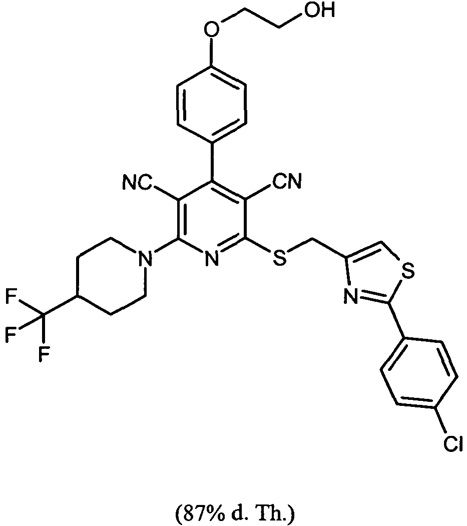 | 1.63 min (Methode 4); m/z = 656 | δ (400 MHz) = 7.95 (d, 2H), 7.69 (s, 1H), 7.58 (d, 2H), 7.56 (d, 2H), 7.11 (d, 2H), 4.90 (br s, 1H), 4.70 (s, 2H), 4.64 (d, 2H), 4.09 (t, 2H), 3.74 (t, 2H), 3.22 (t, 2H), 2.78-2.62 (m, 1H), 1.91-1.82 (m, 2H), 1.50-1.39 (m, 2H). |
| **29** | 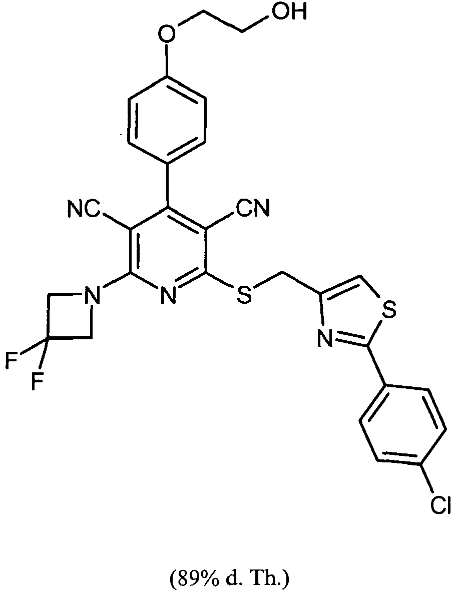 | 1.36 min (Methode 2); m/z = 596 | δ (400 MHz) = 7.96 (d, 2H), 7.71 (s, 1H), 7.58 (d, 2H), 7.49 (d, 2H), 7.12 (d, 2H), 4.89 (t, 4H), 4.71 (s, 2H), 4.09 (t, 2H), 3.74 (t, 2H). |
| **30** | 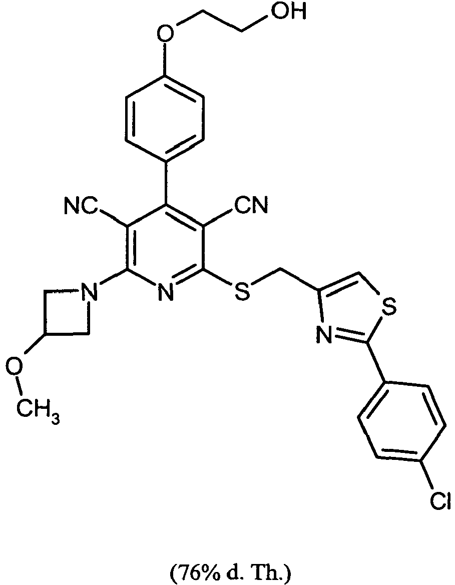 | 1.32 min (Methode 2); m/z = 590 | δ (400 MHz) = 7.96 (d, 2H), 7.69 (s, 1H), 7.58 (d, 2H), 7.47 (d, 2H), 7.10 (d, 2H), 4.85 (br s, 1H), 4.72-4.55 (m, 4H), 4.32-4.28 (m, 1H), 4.27-4.15 (m, 2H), 4.09 (t, 2H), 3.74 (t, 2H), 3.24 (s, 3H). |

### Beispiel 31

### 2-({[2-(4-Chlorphenyl)-1,3-thiazol-4-yl]methyl}sulfanyl)-6-(3,3-difluorpiperidin-1-yl)-4-[4-(2-hydroxyethoxy)phenyl]pyridin-3,5-dicarbonitril

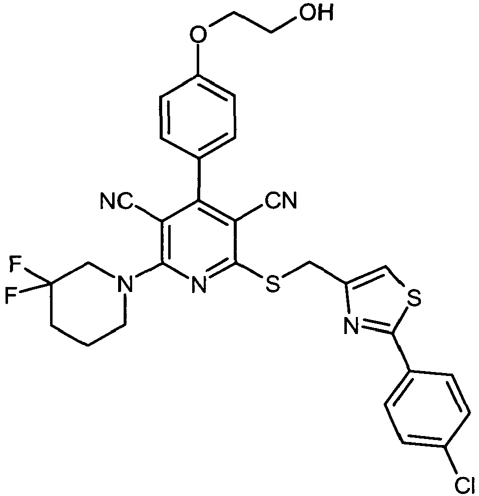

150 mg (0.20 mmol, Reinheit ca. 74%) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A], 64 mg (0.41 mmol) 3,3-Difluorpiperidin-Hydrochlorid und 57 µl (0.41 mmol) Triethylamin wurden in 3 ml THF 2 h bei RT gerührt. Anschließend wurde die Reaktionsmischung mit ca. 1 ml Wasser und ca. 3 ml THF verdünnt und mittels präparativer HPLC (Acetonitril/Wasser + 0.1% TFA) gereinigt. Es wurden 90 mg (71% d. Th.) der Zielverbindung erhalten.
¹H-NMR (400 MHz, DMSO-d₆): δ = 7.95 (d, 2H), 7.69 (s, 1H), 7.56 (d, 4H), 7.12 (d, 2H), 4.90 (br s, 1H), 4.71 (s, 2H), 4.17 (t, 2H), 4.09 (t, 2H), 3.92-3.85 (m, 2H), 3.74 (t, 2H), 2.20-2.07 (m, 2H), 1.86-1.78 (m, 2H).
LC-MS (Methode 2): Rₜ = 1.34 min; MS (ESIpos): m/z = 624 [M+H]⁺.

### Beispiel 32

### 2-({[2-(4-Chlorphenyl)-1,3-thiazol-4-yl]methyl}sulfanyl)-4-[4-(2-hydroxyethoxy)phenyl]-6-[(2,2,2-trifluorethyl)amino]pyridin-3,5-dicarbonitril

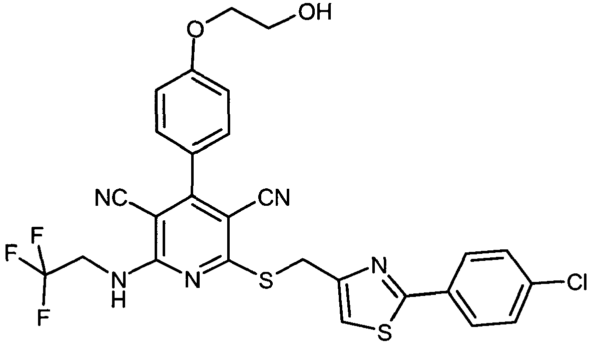

140 mg (0.260 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] und 52 mg (0.519 mmol) 2,2,2-Trifluor-1-aminoethan wurden in 2 ml Tetrahydrofuran gelöst und über Nacht bei Raumtemperatur gerührt. Es wurden weitere 52 mg (0.519 mmol) 2,2,2-Trifluor-1-aminoethan nachgegeben und über 6 Stunden gerührt, bevor das Reaktionsgemisch auf 50°C erhitzt und über Nacht bei dieser Temperatur gerührt wurde. Weitere 52 mg (0.519 mmol) 2,2,2-Trifluor-1-aminoethan wurden nachgegeben. Dann wurde zunächst 2 Stunden bei 50°C und schließlich vier Stunden unter Rückfluss gerührt. Das Reaktionsgemisch wurde ohne weitere Aufarbeitung mittels präparativer HPLC gereinigt. 36 mg (Ausbeute: 23%) der Zielverbindung wurden erhalten.
¹H-NMR (400 MHz, DMSO-d₆): δ = 8.59 (t, 1H), 7.95 (d, 2H), 7.69 (s, 1H), 7.58 (d, 2H), 7.53 (d, 2H), 7.13 (d, 2H), 4.92 (t, 1H), 4.71 (s, 2H), 4.31 (m, 2H), 4.09 (t, 2H), 3.74 (m, 2H).
LC-MS (Methode 1): Rₜ = 2.77 min; MS (ESIpos): m/z = 602 [M+H]⁺.

### Beispiel 33

### 2-({[2-(4-Chlorphenyl)-1,3-thiazol-4-yl]methyl}sulfanyl)-6-[(2-fluorethyl)amino]-4-[4-(2-hydroxyethoxy)phenyl]pyridin-3,5-dicarbonitril

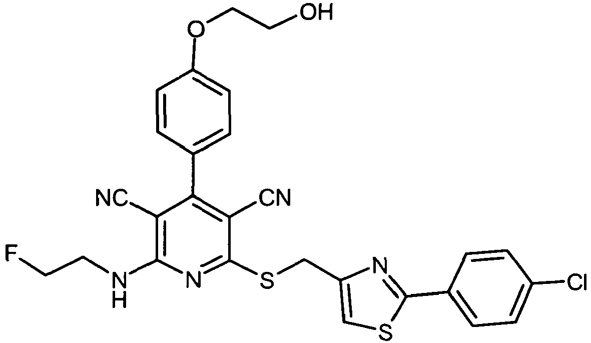

140 mg (0.260 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A], 52 mg (0.519 mmol) 2-Fluorethylamin-Hydrochlorid und 67 mg (0.519 mmol) N,N-Diisopropylethylamin wurden in 2 ml Tetrahydrofuran gelöst und über Nacht bei Raumtemperatur gerührt. Das Reaktionsgemisch wurde ohne weitere Aufarbeitung mittels präparativer HPLC gereinigt. 78 mg (Ausbeute: 53% d. Th.) der Zielverbindung wurden erhalten.
¹H-NMR (400 MHz, DMSO-d₆): δ = 8.29 (t, 1H), 7.95 (d, 2H), 7.68 (s, 1H), 7.57 (d, 2H), 7.50 (d, 2H), 7.12 (d, 2H), 4.91 (t, 1H), 4.69 (s, 2H), 4.59 (t, 1H), 4.47 (t, 1H), 4.08 (t, 2H), 3.84 (m, 1H), 3.80-3.73 (m, 3H).
LC-MS (Methode 2): Rₜ = 1.27 min; MS (ESIpos): m/z = 566 [M+H]⁺.

### Beispiel 34

### 2-({[2-(4-Chlorphenyl)-1,3-thiazol-4-yl]methyl}sulfanyl)-6-[(2,2-difluorethyl)amino]-4-[4-(2-hydroxyethoxy)phenyl]pyridin-3,5-dicarbonitril

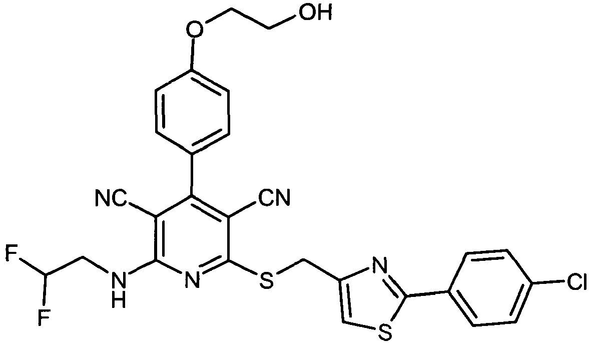

140 mg (0.260 mmol) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A], 61 mg (0.519 mmol) 2,2-Difluorethylamin-Hydrochlorid und 67 mg (0.519 mmol) N,N-Diisopropylethylamin wurden in 2 ml Tetrahydrofuran gelöst und über Nacht bei Raumtemperatur gerührt. Das Reaktionsgemisch wurde ohne weitere Aufarbeitung mittels präparativer HPLC gereinigt. 77 mg (Ausbeute: 51 % d. Th.) der Zielverbindung wurden erhalten.
¹H-NMR (400 MHz, DMSO-d₆): δ = 8.41 (m, 1H), 7.95 (d, 2H), 7.69 (s, 1H), 7.57 (d, 2H), 7.51 (d, 2H), 7.13 (d, 2H), 6. 17 (tt, 1H), 4.92 (t, 1H), 4.72 (s, 2H), 4.09 (t, 2H), 3.90 (m, 2H), 3.75 (m, 2H).
LC-MS (Methode 2): Rₜ = 1.28 min; MS (ESIpos): m/z = 584 [M+H]⁺.

### Beispiel 35

### 2-({[2-(4-Chlorphenyl)-1,3-thiazol-4-yl]methyl}sulfanyl)-4-[4-(2-hydroxyethoxy)phenyl]-6-[methyl(2,2,2-trifluorethyl)amino]pyridin-3,5-dicarbonitril

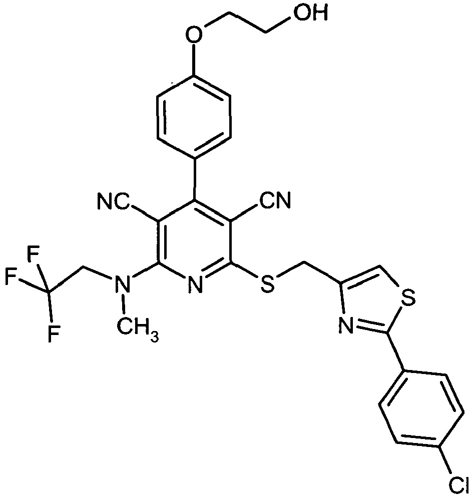

41 mg (0.27 mmol) 2,2,2-Trifluor-N-methylethanamin-Hydrochlorid wurden in 2 ml DMF gelöst und mit 40 mg Amberlyst A-21 versetzt und 30 min bei RT gerührt. Die Mischung wurde abfiltriert und zu 100 mg (0.14 mmol, Reinheit ca. 74%) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] gegeben und die Lösung wurde über Nacht bei RT gerührt. Anschließend wurden in einem separaten Kolben nochmals 82 mg (0.54 mmol) 2,2,2-Trifluor-N-methylethanamin-Hydrochlorid in 0.5 ml DMF gelöst und mit 80 mg Amberlyst A-21 versetzt und 30 min bei RT gerührt. Die Mischung wurde abfiltriert und zur ersten Lösung hinzugegeben. Die resultierende Reaktionsmischung wurde über Nacht bei RT gerührt. Daraufhin wurde auf 60°C erwärmt und der Reaktionsansatz wurde über Nacht bei dieser Temperatur gerührt, bevor auf 100°C erwärmt wurde. Nach Rühren über Nacht bei 100°C wurde mit etwas Wasser/THF verdünnt und mittels präparativer HPLC (Acetonitril/Wasser + 0.1 % TFA) gereinigt. Es wurden 64 mg (74% d. Th.) der Zielverbindung erhalten.
¹H-NMR (400 MHz, DMSO-d₆): δ = 7.94 (d, 2H), 7.70 (s, 1H), 7.58 (d, 2H), 7.56 (d, 2H), 7.11 (d, 2H), 4.90 (br s, 1H), 4.78 (q, 2H), 4.70 (s, 2H), 4.09 (t, 2H), 3.73 (t, 2H), 3.51 (s, 3H).
LC-MS (Methode 2): Rₜ = 1.36 min; MS (ESIpos): m/z = 616 [M+H]⁺.

### Beispiel 36

### 2-({[2-(4-Chlorphenyl)-1,3-thiazol-4-yl]methyl}sulfanyl)-6-[ethyl(2,2,2-trifluorethyl)amino]-4-[4-(2-hydroxyethoxy)phenyl]pyridin-3,5-dicarbonitril

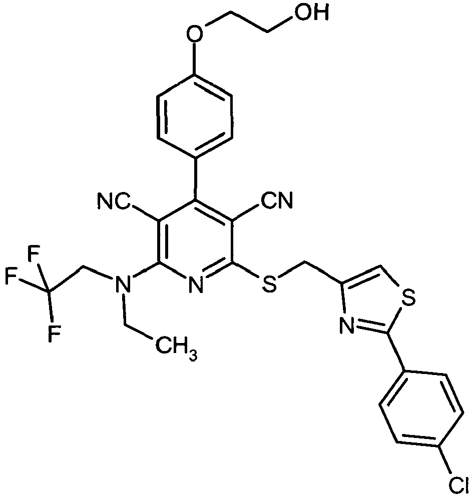

133 mg (0.81 mmol) N-Ethyl-2,2,2-trifluorethanamin-Hydrochlorid wurden in 2 ml DMF gelöst und mit 130 mg Amberlyst A-21 versetzt und 30 min bei RT gerührt. Die Mischung wurde abfiltriert und zu 100 mg (0.14 mmol, Reinheit ca. 74%) 2-Chlor-6-({[2-(4-chlorphenyl)-1,3-thiazol-4-yl]methyl}sulfanyl)-4-[4-(2-hydroxyethoxy)phenyl]pyridin-3,5-dicarbonitril [Beispiel 2A] gegeben und die Lösung wurde über Nacht bei RT gerührt. Daraufhin wurde die Mischung auf 60°C erwärmt und über Nacht bei dieser Temperatur gerührt. Anschließend wurden in einem separaten Kolben nochmals 87 mg (0.54 mmol) 2,2,2-Trifluor-N-methylethanamin-Hydrochlorid in 0.5 ml DMF gelöst und mit 88 mg Amberlyst A-21 versetzt und 30 min bei RT gerührt. Die Mischung wurde abfiltriert und zur ersten Lösung hinzugegeben. Die resultierende Reaktionsmischung wurde 4.5 h bei 100°C gerührt. Daraufhin wurde das Gemisch mit etwas Wasser/THF verdünnt und mittels präparativer HPLC (Acetonitril/Wasser + 0.1 % TFA) gereinigt. Es wurden 37 mg (41% d. Th.) der Zielverbindung erhalten.
¹H-NMR (400 MHz, DMSO-d₆): δ = 7.94 (d, 2H), 7.71 (s, 1H), 7.58 (d, 2H), 7.54 (d, 2H), 7.11 (d, 2H), 4.78 (q, 2H), 4.70 (s, 2H), 4.09 (t, 2H), 3.93 (q, 2H), 3.74 (t, 2H), 1.26 (t, 3H).
LC-MS (Methode 2): Rₜ = 1.40 min; MS (ESIpos): m/z = 630 [M+H]⁺.

### Beispiel 37

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-beta-alanyl-L-alaninat

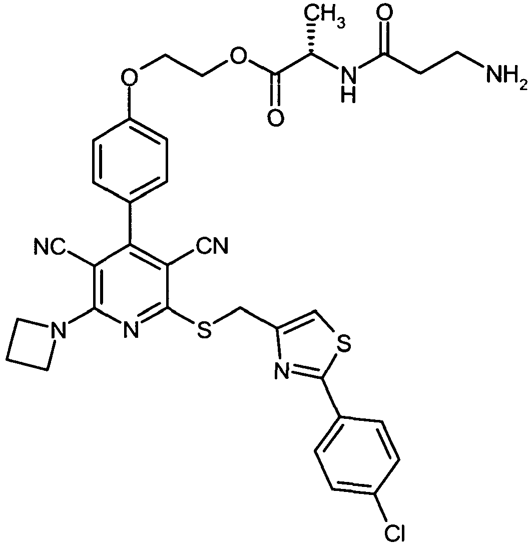

90 mg (0.113 mmol) 2-{4-[2-(Azetidin-1-yl)-6-({[2-(4-chlorphenyl)-1,3-thiazol-4-yl]methyl} sulfanyl)-3,5-dicyanpyridin-4-yl]phenoxy} ethyl-N-(tert.-butoxycarbonyl)-beta-alanyl-L-alaninat [Beispiel 4A] wurden in 3.5 ml Dichlormethan vorgelegt. Nach Versetzen mit 0.347 ml (4.502 mmol) Trifluoressigsäure wurde die Reaktionslösung über Nacht bei RT gerührt. Die Reaktionslösung wurde eingedampft und der Rückstand mittels präparativer HPLC (Aceto-nitril/Wasser + 0.1% TFA) gereinigt. Zur weiteren Reinigung wurde das Produkt erneut mittels präparativer HPLC (Säulenmaterial: XBridge; Eluent: Acetonitril/0.1% aq. Ammoniak = 65/35) gereinigt. Es wurden 51 mg (65% d. Th.) der Zielverbindung erhalten.
¹H-NMR (400 MHz, DMSO-d₆): δ = 8.38 (d, 1H), 7.94 (d, 2H), 7.67 (s, 1H), 7.58 (d, 2H), 7.47 (d, 2H), 7.12 (d, 2H), 4.65 (s, 2H), 4.53-4.32 (m, 6H), 4.30-4.23 (m, 3H), 2.75-2.68 (m, 2H), 2.42-2.32 (m, 2H), 2.18 (t, 2 H) 1.27 (d, 3H).
LC-MS (Methode 1): Rₜ = 2.25 min; MS (ESIpos): m/z = 702 [M+H]⁺.

### Beispiel 38

### 2-{4-[2-({[2-(4-Chlorphenyl)-1,3-thiazol-4-yl]methyl}sulfanyl)-3,5-dicyan-6-(propylamino)pyridin-4-yl]phenoxy}ethyl-L-alaninat-Trifluoracetat

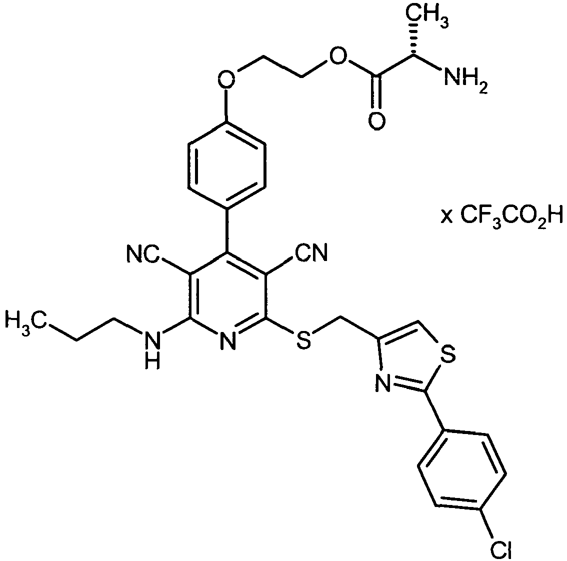

873 mg (1.191 mmol) 2-{4-[2-({[2-(4-Chlorphenyl)-1,3-thiazol-4-yl]methyl}sulfanyl)-3,5-dicyan-6-(propylamino)pyridin-4-yl]phenoxy} ethyl-N-(tert.-butoxycarbonyl)-L-alaninat [Beispiel 14A] wurde in 29 ml Dichlormethan vorgelegt. Nach Versetzen mit 1.84 ml (23.817 mmol) Trifluoressigsäure wurde die Reaktionslösung über Nacht bei RT gerührt. Die Reaktionslösung wurde eingedampft und der Rückstand mit Diethylether verrührt. Der entstandene Feststoff wurde abfiltriert und getrocknet. Es wurden 914 mg (89% d. Th.) der Zielverbindung erhalten.
¹H-NMR (400 MHz, DMSO-d₆): δ = 8.32 (br s, 2H), 8.22 (t, 1H), 7.94 (d, 2H), 7.62 (s, 1H), 7.58 (d, 2H), 7.52 (d, 2H), 7.13 (d, 2H), 4.70 (s, 2H), 4.62-4.49 (m, 2H), 4.38-4.28 (m, 2H), 4.19 (q, 1H), 3.40 (q, 2H), 1.50 (Sextett, 2H), 1.40 (d, 3H), 0.78 (t, 3H).
LC-MS (Methode 4): Rₜ = 1.37 min; MS (ESIpos): m/z = 633 [M+H-TFA]⁺.

### Beispiel 39

### 2-{4-[2-({[2-(4-Chlorphenyl)-1,3-thiazol-4-yl]methyl}sulfanyl)-3,5-dicyan-6-(methylamino)pyridin-4-yl]phenoxy}ethyl-L-alaninat-Trifluoracetat

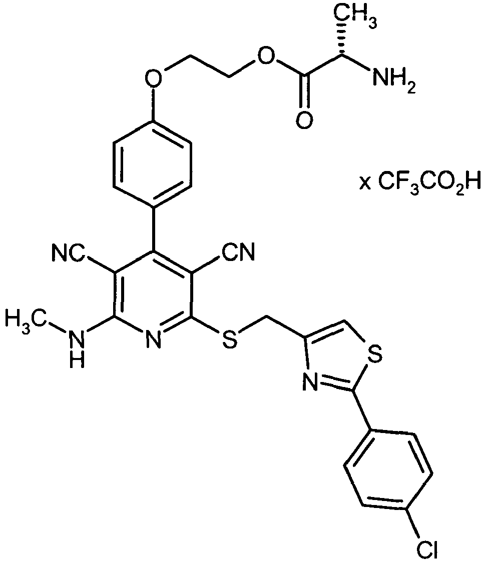

2.18 g (3.091 mmol) 2-{4-[2-({[2-(4-Chlorphenyl)-1,3-thiazol-4-yl]methyl}sulfanyl)-3,5-dicyan-6-(methylamino)pyridin-4-yl]phenoxy}ethyl-N-(tert.-butoxycarbonyl)-L-alaninat [Beispiel 17A] wurde in 45 ml Dichlormethan vorgelegt. Nach Versetzen mit 4.76 ml (61.821 mmol) Trifluoressigsäure wurde die Reaktionslösung über Nacht bei RT gerührt. Die Reaktionslösung wurde einrotiert und der Rückstand wurde mittels präparativer HPLC (Acetonitril/Wasser + 0.1 % TFA) gereinigt. Es wurden 2.04 g (92% d. Th.) der Zielverbindung erhalten.
LC-MS (Methode 2): Rₜ = 1.10 min; MS (ESIpos): m/z = 605 [M+H-TFA]⁺.

### Beispiel 40

### 2-{4-[2-({[2-(4-chlorphenyl)-1,3-thiazol-4-yl]methyl}sulfanyl)-3,5-dicyan-6-(pyrrolidin-1-yl)pyridin-4-yl]phenoxy}ethyl-L-alaninat-Trifluoracetat

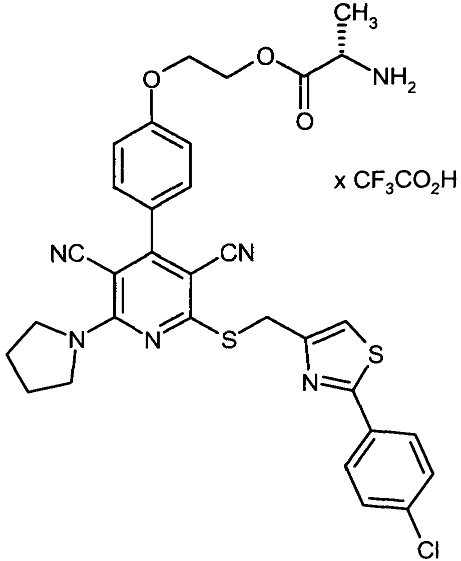

250 mg (0.335 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-N-(tert.-butoxycarbonyl)-L-alaninat [Beispiel 25A] wurden in 3.5 ml Dichlormethan vorgelegt. Nach Versetzen mit 0.258 ml (3.354 mmol) Trifluoressigsäure wurde die Reaktionslösung über Nacht bei RT gerührt. Nach einem Tag wurden erneut 0.125 ml (1.624 mmol) Trifluoressigsäure zu der Reaktion gegeben. Die Reaktionslösung wurde eingedampft und der Rückstand mit Diethylether verrührt. Der entstandene Feststoff wurde abfiltriert. Es wurden 255 mg (98% d. Th.) der Zielverbindung erhalten.
¹H-NMR (400 MHz, DMSO-d₆): δ = 8.41-8.41 (m, 2H), 7.94 (d, 2H), 7.69 (s, 1H), 7.58 (d, 2H), 7.49 (d, 2H), 7.14 (d, 2H), 4.70 (s, 2H), 4.61-4.50 (m, 2H), 4.40-4.29 (m, 2H), 4.23-4.12 (m, 1H), 3.84 (br s, 4H), 1.95 (br s, 4H), 1.40 (d, 3H).
LC-MS (Methode 4): Rₜ = 1.36 min; MS (ESIpos): m/z = 645 [M+H-TFA]⁺.

### Beispiel 41

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-lysyl-L-alaninat-Dihydrochlorid

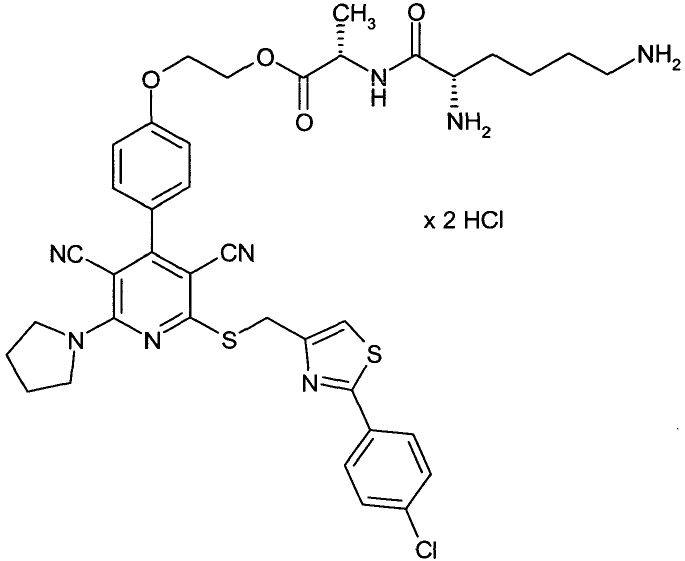

300 mg (0.308 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N²,N⁶-bis(tert.-butoxycarbonyl)-L-lysyl-L-alaninatwerden [Beispiel 26A] wurden in 5.4 ml Dichlormethan vorgelegt. Nach Versetzen mit 3.08 ml (6.163 mmol) einer 1N Lösung von Chlorwasserstoff in Diethylether wurde die Reaktionslösung über Nacht bei RT gerührt. Der entstandene Feststoff wurde abfiltriert, mit 2.5 ml kaltem Dichlormethan verrührt und erneut abfiltriert. Es wurden 250 mg (96% d. Th.) der Zielverbindung erhalten.
¹H-NMR (400 MHz, DMSO-d₆): δ = 8.99 (d, 1H), 8.25 (br s, 3H), 7.95 (d, 2H), 7.89 (br s, 3H), 7.70 (s, 1H). 7.58 (d, 2H), 7.50 (d, 2H), 7.13 (d, 2H), 4.70 (s, 2H), 4.52-4.35 (m, 3H), 4.33-4.26 (m, 2H), 3.85 (br s, 4H), 3.82-3.74 (m, 1H), 2.79-2.71 (m, 2H), 1.94 (br s, 4H), 1.81-1.70 (m, 2H), 1.62-1.51 (m, 2H), 1.46-1.38 (m, 2H), 1.35 (d, 3H).
LC-MS (Methode 2): Rₜ = 0.98 min; MS (ESIpos): m/z = 773 [M+H-2HCl]⁺.

### Beispiel 42

### 2-{4-[2-({[2-(4-Chlorphenyl)-1,3-thiazol-4-yl]methyl}sulfanyl)-3,5-dicyan-6-(methylamino)pyridin-4-yl]phenoxy}ethyl-L-lysyl-L-alaninat-Dihydrochlorid

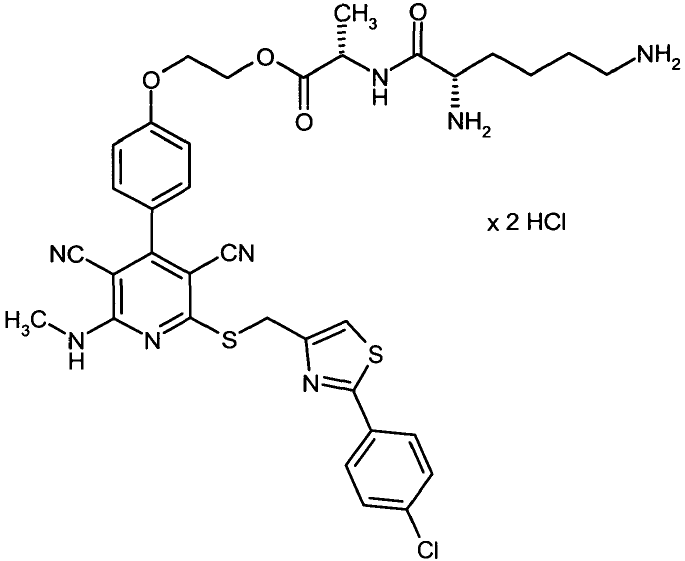

1.00 g (1.071 mmol) 2-{4-[2-({[2-(4-Chlorphenyl)-1,3-thiazol-4-yl]methyl}sulfanyl)-3,5-dicyan-6-(methylamino)pyridin-4-yl]phenoxy}ethyl-N²,N⁶-bis(tert.-butoxycarbonyl)-L-lysyl-L-alaninat [Beispiel 23A] wurden in 18.7 ml Dichlormethan vorgelegt. Nach Versetzen mit 10.71 ml einer 1N Lösung von Chlorwasserstoff in Diethylether wurde die Reaktionslösung für 18 h bei RT gerührt. Im Anschluss wurden nochmals 10.71 ml einer 1N Lösung von Chlorwasserstoff in Diethylether nachgegeben. Nach 18 h Reaktionszeit wurde die Reaktionsmischung für 90 min im Ultraschallbad behandelt. Die Mischung wurde eingedampft. Es wurden 867 mg (100% d. Th.) der Zielverbindung erhalten.
¹H-NMR (400 MHz, DMSO-d₆): δ = 8.98 (d, 1H), 8.29-8.19 (m, 2H), 8.16 (q, 1H), 7.95 (d, 2H), 7.91-7.81 (m, 2H), 7.69 (s, 1H), 7.58 (d, 2H), 7.50 (d, 2H), 7.13 (d, 2H), 4.72 (s, 2H), 4.51-4.34 (m, 3H), 4.32-4.25 (m, 2H), 3.85-3.75 (m, 1H), 3.01 (d, 3H), 2.79-2.69 (m, 2H), 1.79-1.68 (m, 2H), 1.62-1.50 (m, 2H), 1.46-1.37 (m, 2H), 1.35 (d, 3H).
LC-MS (Methode 2): Rₜ = 0.95 min; MS (ESIpos): m/z = 733 [M+H-2HCl]⁺.

### Beispiel 43

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-lysyl-beta-alaninat-Dihydrochlorid

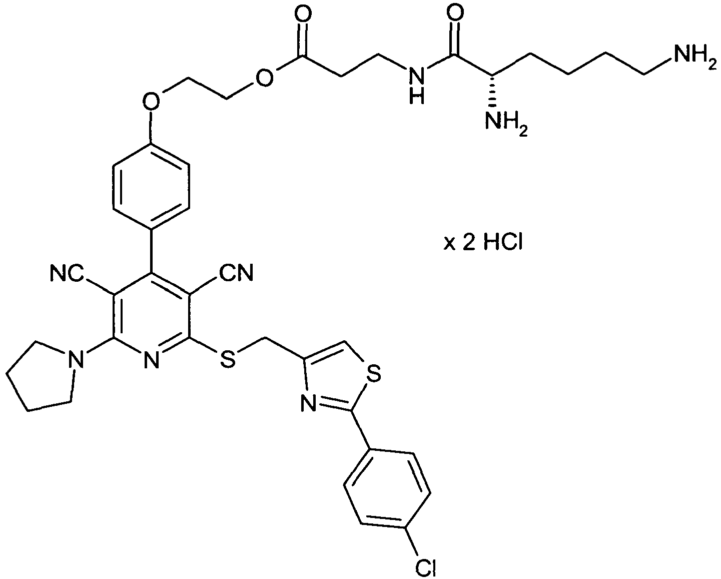

950 mg (0.976 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N²,N⁶-bis(tert.-butoxycarbonyl)-L-lysyl-beta-alaninat [Beispiel 24A] wurden in 25 ml Dichlormethan vorgelegt. Nach Versetzen mit 9.76 ml einer 1N Lösung von Chlorwasserstoff in Diethylether wurde die Reaktionslösung über Nacht bei RT gerührt. Die Reaktionsmischung wurde 15 min mit Argon begast und anschließend wurde die Mischung eingedampft. Es wurden 816 mg (99% d. Th.) der Zielverbindung erhalten.
¹H-NMR (400 MHz, DMSO-d₆): δ = 8.68 (t, 1H), 8.21 (br s, 3H), 7.95 (d, 2H), 7.89 (br s, 3H), 7.70 (s, 1H). 7.58 (d, 2H), 7.49 (d, 2H), 7.12 (d, 2H), 4.70 (s, 2H), 4.44-4.38 (m, 2H), 4.33-4.27 (m, 2H), 3.85 (br s, 4H), 3.79-3.65 (m, 1H), 3.49-3.28 (m, 2H), 2.79-2.71 (m, 2H), 2.60 (t, 2H), 1.95 (br s, 4H), 1.72-1.65 (m, 2H), 1.60-1.51 (m, 2H), 1.38-1.29 (m, 2H).
LC-MS (Methode 2): Rₜ = 1.08 min; MS (ESIpos): m/z = 773 [M+H-2HCl]⁺.

### Beispiel 44

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-alanyl-L-alaninat-Hydrochlorid

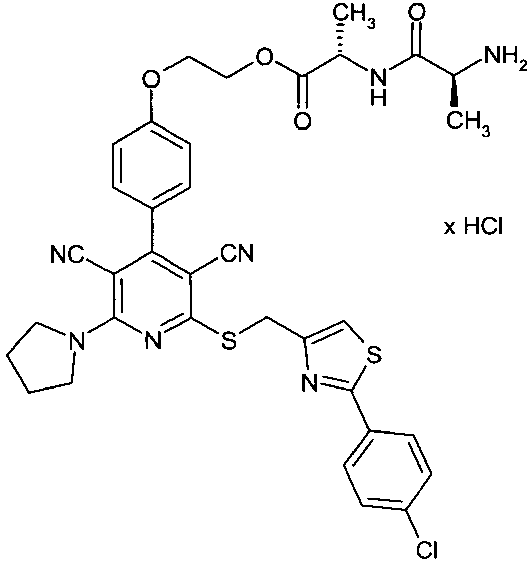

1.5 g (1.84 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxyl ethyl-N-(tert.-butoxycarbonyl)-L-alanyl-L-alaninat [Beispiel 7A] wurden in 24 ml Dichlormethan vorgelegt. Nach Versetzen mit 18.37 ml einer 1N Lösung von Chlorwasserstoff in Diethylether wurde die Reaktionslösung über Nacht bei RT gerührt. Der entstandene Feststoff wurde abfiltriert und mit Diethylether gewaschen. Es wurden 1.44 g (97% d. Th., Reinheit ca. 94%) der Zielverbindung erhalten.
¹H-NMR (400 MHz, DMSO-d₆): δ = 8.82 (d, 1H), 8.19-8.06 (m, 2H), 7.94 (d, 2H), 7.70 (s, 1H), 7.58 (d, 2H), 7.49 (d, 2H), 7.11 (d, 2H), 4.68 (s, 2H), 4.49-4.34 (m, 3H), 4.31-4.25 (m, 2H), 3.88-3.78 (m, 5H), 1.99-1.89 (m, 4H), 1.36-1.27 (m, 6H).
LC-MS (Methode 2): Rₜ = 2.26 min; MS (ESIpos): m/z = 716 [M+H-HCl]⁺.

### Beispiel 45

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-L-alaninat-Trifluoracetat

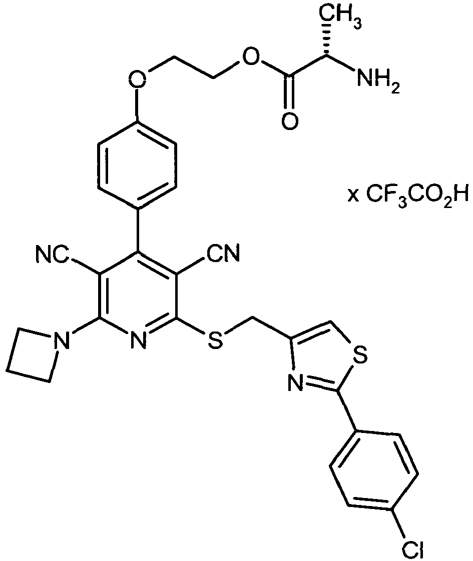

39 mg (0.053 mmol) 2-{4-[2-(Azetidin-1-yl)-6-({[2-(4-chlorphenyl)-1,3-thiazol-4-yl]methyl} sulfanyl)-3,5-dicyanpyridin-4-yl]phenoxy} ethyl-N-(tert.-butoxycarbonyl)-L-alaninat [Beispiel 13A] wurden in 1.5 ml Dichlormethan vorgelegt. Nach Versetzen mit 0.5 ml (6.49 mmol) Trifluoressigsäure wurde die Reaktionslösung für 1.5 h bei RT gerührt. Die Reaktionslösung wurde eingedampft und der Rückstand mittels präparativer HPLC (Acetonitril/Wasser + 0.1% TFA) gereinigt. Es wurden 40 mg (100% d. Th.) der Zielverbindung erhalten.
¹H-NMR (400 MHz, DMSO-d₆): δ = 8.37-8.26 (m, 2H), 7.95 (d, 2H), 7.67 (s, 1H), 7.58 (d, 2H), 7.48 (d, 2H), 7.13 (d, 2H), 4.65 (s, 2H), 4.60-4.42 (m, 6H), 4.37-4.31 (m, 2H), 4.22-4.12 (m, 1H), 2.44-2.31 (m, 2H), 1.39 (d, 3H).
LC-MS (Methode 1): Rₜ = 2.26 min; MS (ESIpos): m/z = 631 [M+H-TFA]⁺.

### Beispiel 46

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-L-lysyl-L-alaninat-Bis(trifluoracetat)

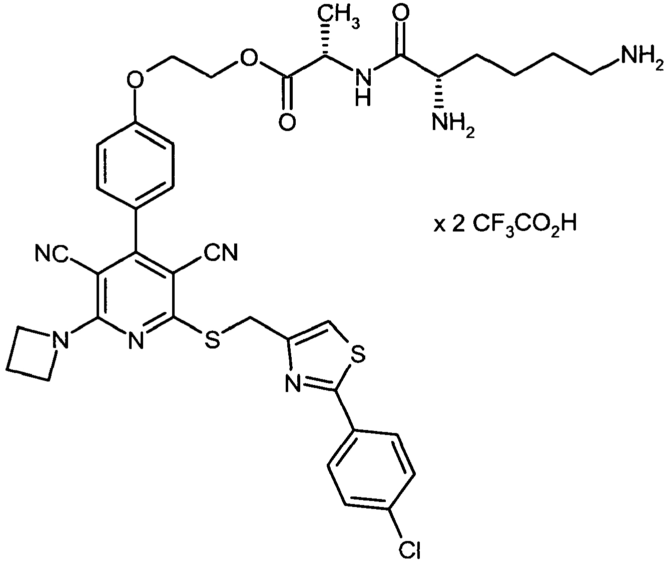

180 mg (0.188 mmol) 2-{4-[2-(Azetidin-1-yl)-6-({[2-(4-chlorphenyl)-1,3-thiazol-4-yl]methyl} sulfanyl)-3,5-dicyanpyridin-4-yl]phenoxy} ethyl-N²,N⁶-bis(tert.-butoxycarbonyl)-L-lysyl-L-alaninat [Beispiel 3A] wurden in 5 ml Dichlormethan vorgelegt. Nach Versetzen mit 1.0 ml (12.98 mmol) Trifluoressigsäure wurde die Reaktionslösung für 30 min bei RT gerührt. Die Reaktionslösung wurde eingedampft und der Rückstand mittels präparativer HPLC (Acetonitril/Wasser + 0.1% TFA) gereinigt. Es wurden 153 mg (83% d. Th.) der Zielverbindung erhalten.
¹H-NMR (400 MHz, DMSO-d₆): δ = 8.88 (d, 1H), 8.27-8.10 (m, 2H), 7.95 (d, 2H), 7.82-7.69 (m, 2H), 7.67 (s, 1H), 7.58 (d, 2H), 7.47 (d, 2H), 7.12 (d, 2H), 4.66 (s, 2H), 4.54-4.35 (m, 7H), 4.32-4.26 (m, 2H), 3.82-3.71 (m, 1H), 2.80-2.69 (m, 2H), 2.43-2.31 (m, 2H), 1.77-1.64 (m, 2H), 1.59-1.47 (m, 2H), 1.43-1.36 (m, 2H), 1.34 (d, 3H).
LC-MS (Methode 6): Rₜ = 1.67 min; MS (ESIpos): m/z = 759 [M+H-2TFA]⁺.

### Beispiel 47

### 2-{4-[2-({[2-(4-Chlorphenyl)-1,3-thiazol-4-yl]methyl}sulfanyl)-3,5-dicyan-6-(propylamino)pyridin-4-yl]phenoxy}ethyl-L-ornithinat-Bis(trilluoracetat)

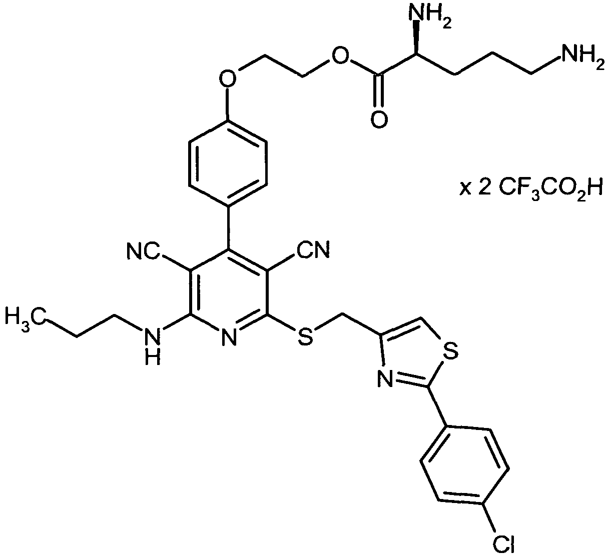

60 mg (0.068 mmol) 2-{4-[2-({[2-(4-Chlorphenyl)-1,3-thiazol-4-yl]methyl}sulfanyl)-3,5-dicyan-6-(propylamino)pyridin-4-yl]phenoxy} ethyl-N²,N⁵-bis(tert.-butoxycarbonyl)-L-ornithinat [Beispiel 20A] wurden in 1.8 ml Dichlormethan vorgelegt. Nach Versetzen mit 0.211 ml (2.738 mmol) Trifluoressigsäure wurde die Reaktionslösung über Nacht bei RT gerührt. Die Reaktionslösung wurde eingedampft und der Rückstand mittels präparativer HPLC (Acetonitril/Wasser + 0.1 % TFA) gereinigt. Es wurden 55 mg (89% d. Th.) der Zielverbindung erhalten.
¹H-NMR (400 MHz, DMSO-d₆): δ = 8.46-8.38 (m, 2H), 8.26-8.20 (t, 1H), 7.94 (d, 2H), 7.78-7.67 (m, 2H), 7.64 (s, 1H), 7.58 (d, 2H), 7.53 (d, 2H), 7.15 (d, 2H), 4.70 (s, 2H), 4.63-4.50 (m, 2H), 4.38-4.32 (m, 2H), 4.21-4.13 (m, 1H), 3.46-3.37 (m, 2H), 2.85-2.77 (m, 2H), 1.96-1.56 (m, 4H), 1.55-1.46 (m, 2H), 0.79 (t, 3H).
LC-MS (Methode 1): Rₜ = 1.98 min; MS (ESIpos): m/z = 676 [M+H-2TFA]⁺.

### Beispiel 48

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-L-ornithinat-Bis(trifluoracetat)

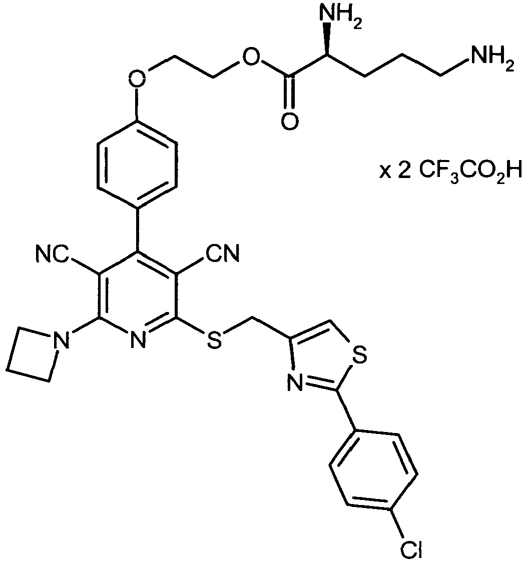

Die Darstellung wurde, wie in Beispiel 47 beschrieben, aus den entsprechenden Edukten durchgeführt.
Ausbeute: 71%d. Th.
¹H-NMR (400 MHz, DMSO-d₆): δ = 8.55-8.38 (m, 2H), 7.94 (d, 2H), 7.84-7.69 (m, 2H), 7.67 (s, 1H), 7.58 (d, 2H), 7.49 (d, 2H), 7.13 (d, 2H), 4.67 (s, 2H), 4.62-4.51 (m, 2H), 4.50-4.41 (m, 4H), 4.37-4.31 (m, 2H), 4.21-4.12 (m, 1H), 2.85-2.75 (m, 2H), 2.44-2.35 (m, 2H), 1.92-1.53 (m, 4H).
LC-MS (Methode 1): Rₜ = 1.93 min; MS (ESIpos): m/z = 674 [M+H-2TFA]⁺.

### Beispiel 49

### 2-{4-[2-(Azetidin-1-yl)-6-({[2-(4-chlorphenyl)-1,3-thiazol-4-yl]methyl}sulfanyl)-3,5-dicyanpyridin-4-yl]phenoxy}ethyl-beta-alaninat-Trifluoracetat

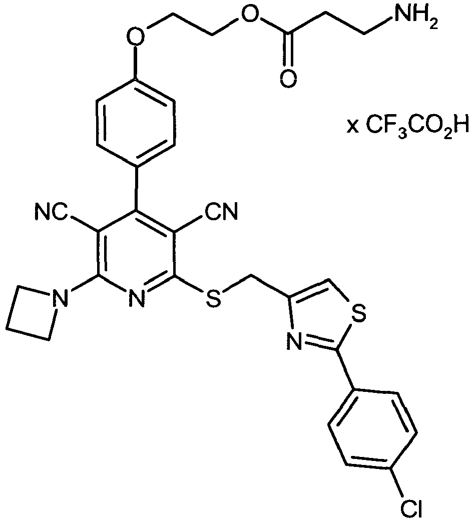

Die Darstellung wurde, wie in Beispiel 47 beschrieben, aus den entsprechenden Edukten durchgeführt.
Ausbeute: 54% d. Th.
¹H-NMR (400 MHz, DMSO-d₆): δ = 7.95 (d, 2H), 7.80-7.58 (m, 2H), 7.67 (s, 1H), 7.58 (d, 2H), 7.47 (d, 2H), 7.13 (d, 2H), 4.66 (s, 2H), 4.53-4.40 (m, 6H), 4.34-4.27 (m, 2H), 3.10-2.99 (m, 2H), 2.74-2.66 (m, 2H), 2.44-2.31 (m, 2H).
LC-MS (Methode 1): Rₜ = 2.23 min; MS (ESIpos): m/z = 631 [M+H-TFA]⁺.

### Beispiel 50

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-beta-alaninat-Trifluoracetat

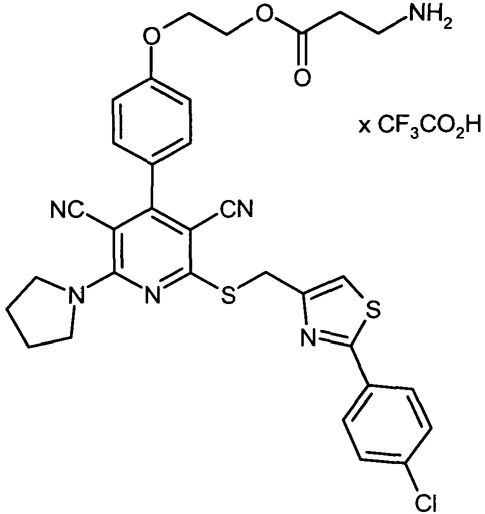

Die Darstellung wurde, wie in Beispiel 47 beschrieben, aus den entsprechenden Edukten durchgeführt.
Ausbeute: 63% d. Th.
¹H-NMR (400 MHz, DMSO-d₆): δ = 7.94 (d, 2H), 7.80-7.68 (br s, 2H), 7.70 (s, 1H), 7.58 (d, 2H), 7.49 (d, 2H), 7.14 (d, 2H), 4.70 (s, 2H), 4.47-4.41 (m, 2H), 4.34-4.28 (m, 2H), 3.87-3.80 (m, 4H), 3.09-3.01 (m, 2H), 2.74-2.67 (m, 2H), 1.99-1.91 (m, 4H).
LC-MS (Methode 1): Rₜ = 2.31 min; MS (ESIpos): m/z = 645 [M+H-TFA]⁺.

### Beispiel 51

### 2-{4-[2-({[2-(4-Chlorphenyl)-1,3-thiazol-4-yl]methyl}sulfanyl)-3,5-dicyan-6-(propylamino)pyridin-4-yl]phenoxy}ethyl-L-alanyl-L-alaninat-Trifluoracetat

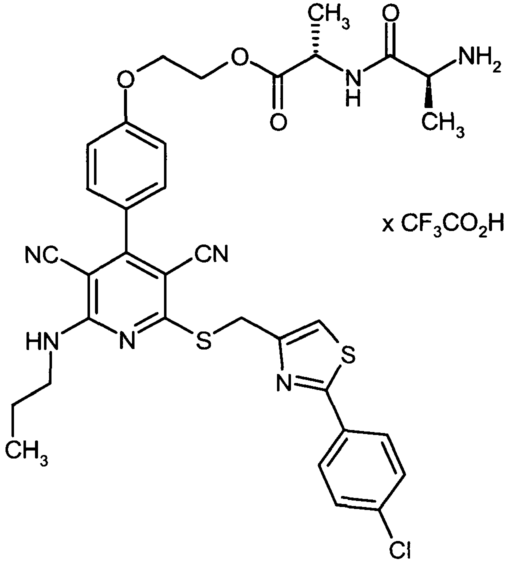

Die Darstellung wurde, wie in Beispiel 47 beschrieben, aus den entsprechenden Edukten durchgeführt. Es wurden 20 Äquivalente Trifluoressigsäure verwendet.
Ausbeute: 44% d. Th.
¹H-NMR (400 MHz, DMSO-d₆): δ = 8.78 (d, 1H), 8.22 (t, 1H), 8.12-8.02 (m, 2H), 7.95 (d, 2H), 7.65 (s, 1H), 7.58 (d, 2H), 7.51 (d, 2H), 7.13 (d, 2H), 4.70 (s, 2H), 4.49-4.35 (m, 3H), 4.31-4.26 (m, 2H), 3.87-3.80 (m, 1H), 3.40 (q, 2H), 1.56-1.45 (m, 2H), 1.34 (dd, 6H), 0.78 (t, 3H).
LC-MS (Methode 1): Rₜ = 2.31 min; MS (ESIpos): m/z = 704 [M+H-TFA]⁺.

### Beispiel 52

### 2-{4-[2-({[2-(4-Chlorphenyl)-1,3-thiazol-4-yl]methyl}sulfanyl)-3,5-dicyan-6-(methylamino)pyridin-4-yl]phenoxy}ethyl-L-ornithinat-Bis(trifluoracetat)

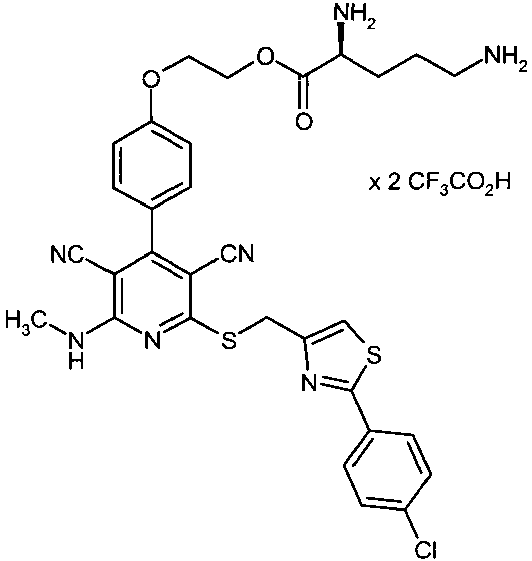

Die Darstellung wurde, wie in Beispiel 47 beschrieben, aus den entsprechenden Edukten durchgeführt.
Ausbeute: 87% d. Th.
¹H-NMR (400 MHz, DMSO-d₆): δ = 8.51-8.39 (m, 2H), 8.17 (q, 1H), 7.94 (d, 2H), 7.84-7.72 (m, 2H), 7.68 (s, 1H), 7.59 (d, 2H), 7.51 (d, 2H), 7.15 (d, 2H), 4.73 (s, 2H), 4.62-4.49 (m, 2H), 4.38-4.31 (m, 2H), 4.21-4.14 (m, 1H), 3.03 (d, 3H), 2.85-2.76 (m, 2H), 1.95-1.55 (m, 4H).
LC-MS (Methode 1): Rₜ = 1.84 min; MS (ESIpos): m/z = 648 [M+H-2TFA]⁺.

### Beispiel 53

### 2-{4-[2-({[2-(4-Chlorphenyl)-1,3-thiazol-4-yl]methyl} sulfanyl)-3,5-dicyan-6-(pyrrolidin-1-yl)pyridin-4-yl]phenoxy}ethyl-L-alanyl-beta-alaninat-Trifluoracetat

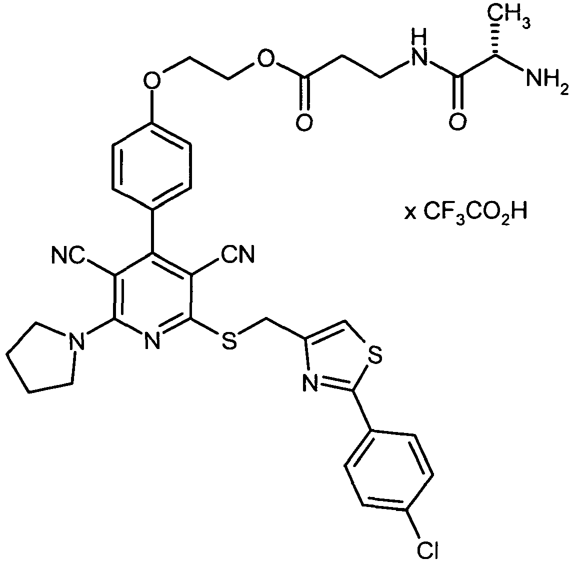

Die Darstellung wurde, wie in Beispiel 47 beschrieben, aus den entsprechenden Edukten durchgeführt.
Ausbeute: 88% d. Th.
¹H-NMR (400 MHz, DMSO-d₆): δ = 8.47 (t, 1H), 8.09-7.99 (m, 2H), 7.95 (d, 2H), 7.69 (s, 1H), 7.58 (d, 2H), 7.50 (d, 2H), 7.13 (d, 2H), 4.70 (s, 2H), 4.44-4.37 (m, 2H), 4.32-4.25 (m, 2H), 4.11-4.04 (m, 1H), 3.85 (br s, 4H) 3.47-3.28 (m, 2H), 2.59-2.54 (m, 2H), 1.95 (br s, 4H), 1.30 (d, 3H).
LC-MS (Methode 1): Rₜ = 2.31 min; MS (ESIpos): m/z = 716 [M+H-TFA]⁺.

### Beispiel 54

### 2-{4-[2-({[2-(4-Chlorphenyl)-1,3-thiazol-4-yl]methyl}sulfanyl)-3,5-dicyan-6-(propylamino)pyridin-4-yl]phenoxy}ethyl-L-lysyl-L-alaninat-Bis(trifluoracetat)

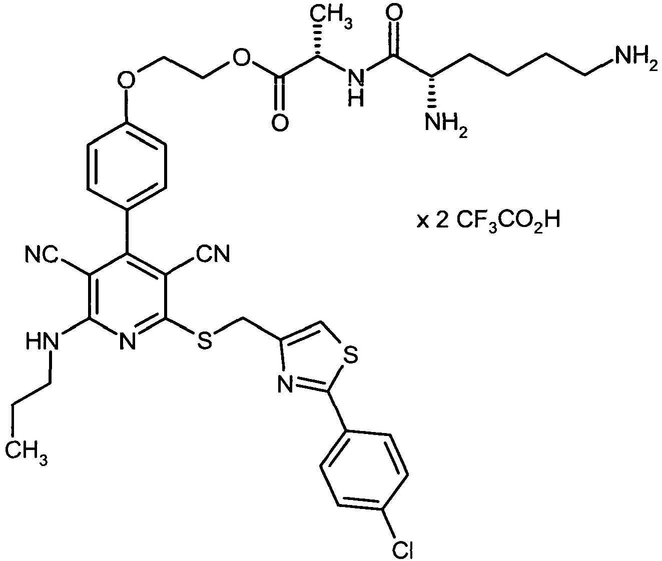

Die Darstellung wurde, wie in Beispiel 47 beschrieben, aus den entsprechenden Edukten durchgeführt. Es wurden 20 Äquivalente Trifluoressigsäure verwendet.
Ausbeute: 26% d. Th.
¹H-NMR (400 MHz, DMSO-d₆): δ = 8.88 (d, 1H), 8.23 (t, 1H), 8.16-8.11 (m, 2H), 7.94 (d, 2H), 7.75-7.60 (m, 2H), 7.64 (s, 1H), 7.58 (d, 2H), 7.51 (d, 2H), 7.10 (d, 2H), 4.69 (s, 2H), 4.54-4.35 (m, 3H), 4.33-4.23 (m, 2H), 3.78-3.75 (m, 1H), 3.41 (q, 2H), 2.80-2.71 (m, 2H), 1.73 (q, 2H), 1.59-1.44 (m, 4H), 1.43-1.31 (m, 2H), 1.35 (d, 3H), 0.79 (t, 3H).
LC-MS (Methode 4): Rₜ = 1.19 min; MS (ESIpos): m/z = 761 [M+H-2TFA]⁺.

### Beispiel 55

### 2-{4-[2-({[2-(4-Chlorphenyl)-1,3-thiazol-4-yl]methyl}sulfanyl)-3,5-dicyan-6-(propylamino)pyridin-4-yl]phenoxy}ethyl-beta-alanyl-L-alaninat-Trifluoracetat

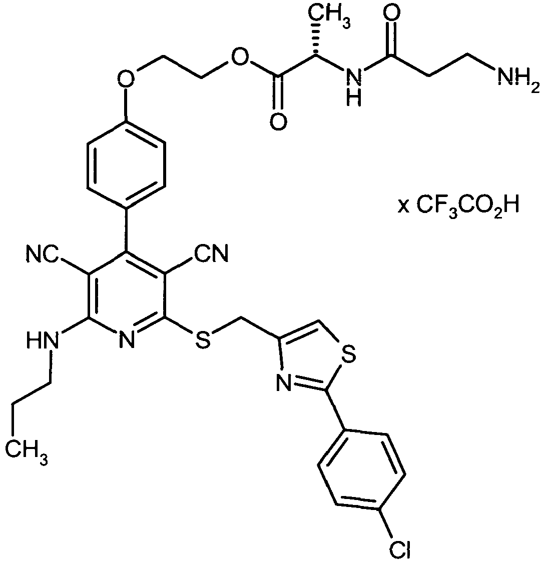

Die Darstellung wurde, wie in Beispiel 47 beschrieben, aus den entsprechenden Edukten durchgeführt. Es wurden 20 Äquivalente Trifluoressigsäure verwendet.
Ausbeute: 87% d. Th.
¹H-NMR (400 MHz, DMSO-d₆): δ = 8.58 (d, 1H), 8.21 (t, 1H), 7.94 (d, 2H), 7.75-7.62 (m, 2H), 7.65 (s, 1H), 7.58 (d, 2H), 7.50 (d, 2H), 7.13 (d, 2H), 4.69 (s, 2H), 4.51-4.23 (m, 5H), 3.40 (q, 2H), 3.02-2.93 (m, 2H), 2.58-2.48 (m, 2H), 1.58-1.42 (m, 2H), 1.29 (d, 3H), 0.79 (t, 3H).
LC-MS (Methode 2): Rₜ = 1.19 min; MS (ESIpos): m/z = 704 [M+H-TFA]⁺.

### Beispiel 56

### 2-{4-[2-({[2-(4-Chlorphenyl)-1,3-thiazol-4-yl]methyl}sulfanyl)-3,5-dicyan-6-(methylamino)pyridin-4-yl]phenoxy}ethyl-beta-alaninat-Trifluoracetat

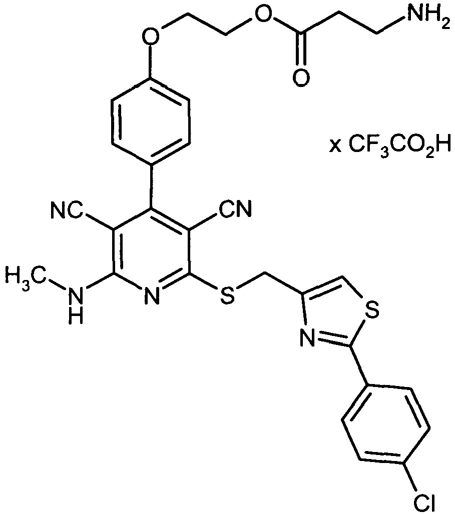

Die Darstellung wurde, wie in Beispiel 47 beschrieben, aus den entsprechenden Edukten durchgeführt.
Ausbeute: 91 % d. Th.
¹H-NMR (400 MHz, DMSO-d₆): δ = 8.16 (q, 1H), 7.94 (d, 2H), 7.79-7.67 (m, 2H), 7.69 (s, 1H), 7.58 (d, 2H), 7.50 (d, 2H), 7.14 (d, 2H), 4.73 (s, 2H), 4.47-4.41 (m, 2H), 4.34-4.28 (m, 2H), 3.11-2.99 (m, 2H), 3.02 (d, 3H), 2.74-2.65 (m, 2H).
LC-MS (Methode 4): Rₜ = 1.28 min; MS (ESIpos): m/z = 605 [M+H-TFA]⁺.

### Beispiel 57

### 2-{4-[2-({[2-(4-chlorphenyl)-1,3-thiazol-4-yl]methyl}sulfanyl)-3,5-dicyan-6-(methylamino)pyridin-4-yl]phenoxy}ethyl-L-alanyl-L-alaninat-Trifluoracetat

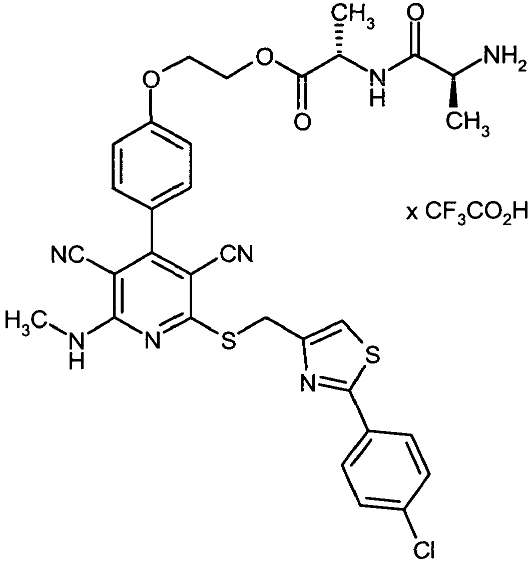

Die Darstellung wurde, wie in Beispiel 47 beschrieben, aus den entsprechenden Edukten durchgeführt. Es wurden 20 Äquivalente Trifluoressigsäure verwendet.
Ausbeute: 90% d. Th.
¹H-NMR (400 MHz, DMSO-d₆): δ = 8.79 (d, 1H), 8.16 (q, 1H), 8.10-8.02 (m, 2H), 7.95 (d, 2H), 7.69 (s, 1H), 7.58 (d, 2H), 7.50 (d, 2H), 7.13 (d, 2H), 4.73 (s, 2H), 4.49-4.35 (m, 3H), 4.31-4.25 (m, 2H), 3.86-3.79 (m, 1H), 3.01 (d, 3H), 1.34 (d, 6H).
LC-MS (Methode 2): Rₜ = 1.12 min; MS (ESIpos): m/z = 676 [M+H-TFA]⁺.

### Beispiel 58

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-ornithyl-L-alaninat-Bis(trifluoracetat)

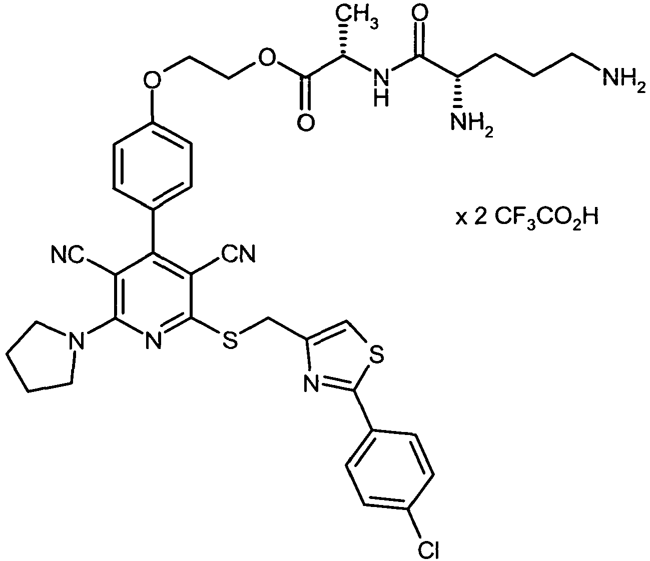

Die Darstellung wurde, wie in Beispiel 47 beschrieben, aus den entsprechenden Edukten durchgeführt. Es wurden 20 Äquivalente Trifluoressigsäure verwendet.
Ausbeute: 60% d. Th.
¹H-NMR (400 MHz, DMSO-d₆): δ = 8.90 (d, 1H), 8.25-8.17 (m, 2H), 7.94 (d, 2H), 7.82-7.71 (m, 2H), 7.70 (s, 1H), 7.58 (d, 2H), 7.50 (d, 2H), 7.12 (d, 2H), 4.70 (s, 2H), 4.53-4.35 (m, 3H), 4.32-4.28 (m, 2H), 3.88-3.76 (m, 5H), 2.85-2.74 (m, 2H), 1.95 (br s, 4H), 1.81-1.69 (m, 2H), 1.67-1.57 (m, 2H), 1.36 (d, 3H).
LC-MS (Methode 2): Rₜ = 1.09 min; MS (ESIpos): m/z = 759 [M+H-2TFA]⁺.

### Beispiel 59

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-ornithinat-Bis(trifluoracetat)

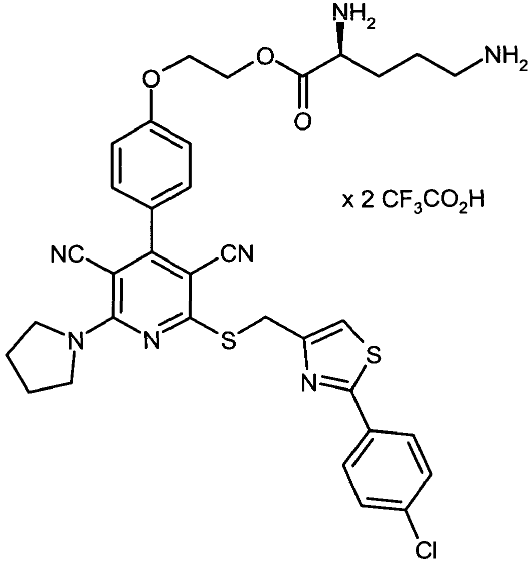

Die Darstellung wurde, wie in Beispiel 47 beschrieben, aus den entsprechenden Edukten durchgeführt. Es wurden 20 Äquivalente Trifluoressigsäure verwendet.
Ausbeute: 72% d. Th.
¹H-NMR (400 MHz, DMSO-d₆): δ = 8.54-8.39 (m, 2H), 7.94 (d, 2H), 7.83-7.71 (m, 2H), 7.70 (s, 1H), 7.59 (d, 2H), 7.51 (d, 2H), 7.14 (d, 2H), 4.70 (s, 2H), 4.64-4.50 (m, 2H), 4.38-4.32 (m, 2H), 4.21-4.13 (m, 1H), 3.84 (br s, 4H), 2.85-2.76 (m, 2H), 1.95 (br s, 4H), 1.90-1.55 (m, 4H).
LC-MS (Methode 1): Rₜ = 1.93 min; MS (ESIpos): m/z = 688 [M+H-2TFA]⁺.

### Beispiel 60

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-beta-alanyl-L-alaninat-Trifluoracetat

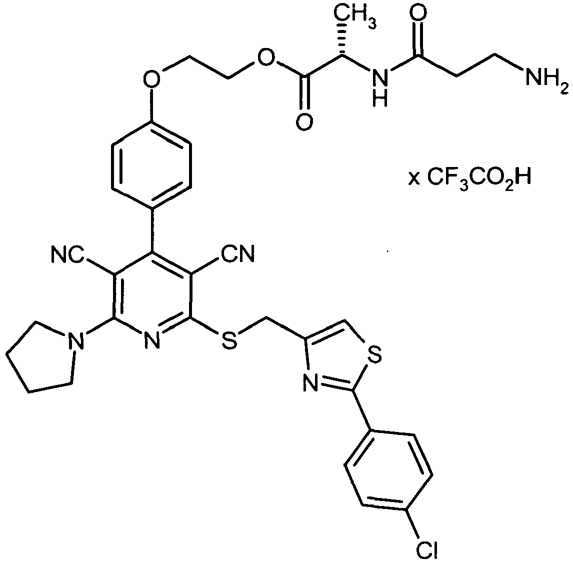

Die Darstellung wurde, wie in Beispiel 47 beschrieben, aus den entsprechenden Edukten durchgeführt.
Ausbeute: 69% d. Th.
¹H-NMR (400 MHz, DMSO-d₆): δ = 8.57 (d, 1H), 7.95 (d, 2H), 7.70 (s, 1H), 7.68-7.61 (m, 2H), 7.58 (d, 2H), 7.49 (d, 2H), 7.12 (d, 2H), 4.70 (s, 2H), 4.50-4.24 (m, 5H), 3.84 (br s, 4H), 3.03-2.92 (m, 2H), 2.51-2.48 (m, 2H), 2.00-1.90 (m, 4H), 1.31 (d, 3H).
LC-MS (Methode 4): Rₜ = 1.38 min; MS (ESIpos): m/z = 716 [M+H-TFA]⁺.

### Beispiel 61

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-beta-alanyl-L-alaninat-Hydrochlorid

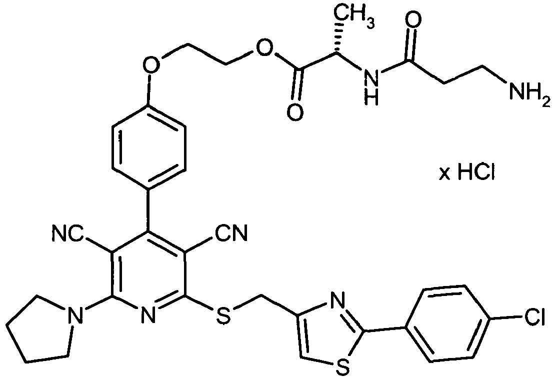

200 mg (0.245 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-N-(tert.-butoxycarbonyl)-beta-alanyl-L-alaninat [Beispiel 8A] wurden in 2 ml Dichlormethan gelöst und mit 2.45 ml 1M HCl in Diethylether versetzt. Nach 3 Stunden wurde 1 ml einer 1N Lösung von Chlorwasserstoff in Diethylether nachgegeben und 2 weitere Stunden bei Raumtemperatur gerührt. Der ausgefallene Feststoff wurde abgesaugt, mit Diethylether gewaschen und im Vakuum getrocknet. 155 mg (Ausbeute: 82% d. Th.) der Zielverbindung wurden erhalten.
¹H-NMR (400 MHz, DMSO-d₆): δ = 8.60 (d, 1H), 7.95 (d, 2H), 7.79 (m br, 3H), 7.70 (s, 1H), 7.58 (d, 2H), 7.49 (d, 2H), 7.13 (d, 2H), 4.70 (s, 2H), 4.48-4.27 (m, 6H), 3.99 (m, 1H), 3.83 (m, 4H), 2.96 (m, 2H), 1.94 (m, 4H), 1.29 (d, 3H).
LC-MS (Methode 1): Rₜ = 2.30 min; MS (ESIpos): m/z = 716 [M+H-HCl]⁺.

### Beispiel 62

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-L-prolyl-L-alaninat-Hydrochlorid

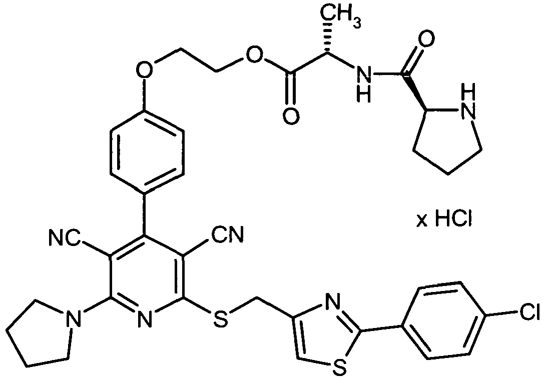

495 mg (0.588 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-1-(tert.-butoxycarbonyl)-L-prolyl-L-alaninat wurden in 3 ml Dichlormethan gelöst und mit 5.876 ml einer 1N Lösung von Chlorwasserstoff in Diethylether versetzt. Nach 6 stündigem Rühren wurde der ausgefallene Feststoff abgesaugt, mit Diethylether gewaschen und im Vakuum getrocknet. 410 mg (90% d. Th.) der Zielverbindung wurden erhalten.
¹H-NMR (400 MHz, DMSO-d₆): δ = 9.56 (m, 1H), 8.97 (d, 1H), 8.53 (m, 1H), 7.94 (d, 2H), 7.70 (s, 1H), 7.58 (d, 2H), 7.49 (d, 2H), 7.12 (d, 2H), 4.70 (s, 2H), 4.46 (m, 1H), 4.40 (m, 2H), 4.27 (m, 2H), 4.16 (m, 1H), 3.91 (m, 4H), 3.14 (m, 2H), 2.28 (m, 1H), 1.94 (m, 4H), 1.86-1.67 (m, 3H), 1.35 (d, 3H).
LC-MS (Methode 4): Rₜ = 1.35 min; MS (ESIpos): m/z = 742 [M+H-HCl]⁺.

### Beispiel 63

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-isoleucyl-L-alaninat-Hydrochlorid

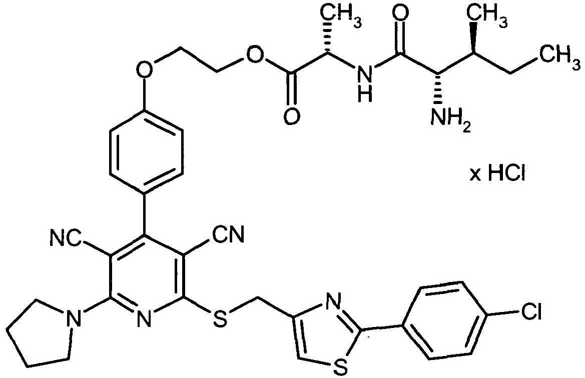

414 mg (0.482 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert-butoxycarbonyl)-L-isoleucyl-L-alaninat wurden in 3 ml Dichlormethan gelöst und mit 4.822 ml einer 1N Lösung von Chlorwasserstoff in Diethylether versetzt. Nach 6 stündigem Rühren wurden 2 ml einer 1N Lösung von Chlorwasserstoff in Diethylether nachgegeben, und es wurde weitere 24 Stunden bei Raumtemperatur gerührt. Der ausgefallene Feststoff wurde abgesaugt, mit Diethylether gewaschen und im Vakuum getrocknet. Da die Reaktion noch nicht vollständig war wurde der Feststoff weitere 24h in 5 ml einer 1N Lösung von Chlorwasserstoff in Diethylether gerührt. Der ausgefallene Feststoff wurde abgesaugt, mit Diethylether gewaschen und im Vakuum getrocknet. 312 mg (81% d. Th.) der Zielverbindung wurden erhalten.
¹H-NMR (400 MHz, DMSO-d₆): δ = 8.83 (d, 1H), 8.20-8.09 (m, 3H), 7.94 (d, 2H), 7.70 (s, 1H), 7.58 (d, 2H), 7.49 (d, 2H), 7.11 (d, 2H), 4.70 (s, 2H), 4.49-4.39 (m, 3H), 4.27 (m, 2H), 3.83 (m, 4H), 3.60 (m, 1H), 1.94 (m, 4H), 1.80 (m, 1H), 1.52 (m, 1H), 1.34 (d, 3H), 1.22-1.07 (m, 1H), 0.91 (d, 3H), 0.83 (t, 3H).
LC-MS (Methode 2): Rₜ = 1.19 min; MS (ESIpos): m/z = 758 [M+H-HCl]⁺.

### Beispiel 64

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-[(2S)-2,4-diaminobutanoyl]-L-alaninat-Dihydrochlorid

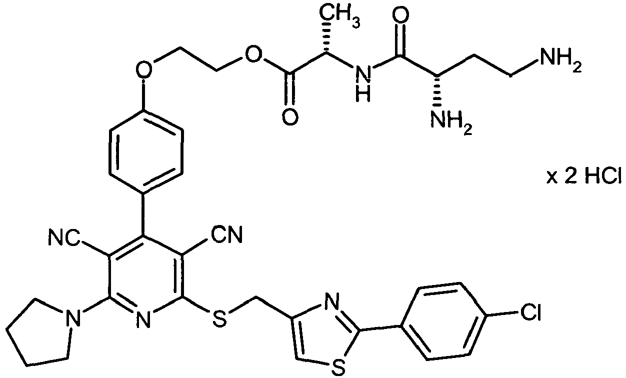

420 mg (0.444 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-{(2S)-2,4-bis[(tert.-butoxycarbonyl)amino]butanoyl}-L-alaninat wurden in 5 ml Dichlormethan gelöst und mit 4.442 ml (einer 1N Lösung von Chlorwasserstoff in Diethylether versetzt. Nach 4 stündigem Rühren wurde der ausgefallene Feststoff abgesaugt, mit Diethylether gewaschen und im Vakuum getrocknet. 322 mg (88% d. Th.) der Zielverbindung wurden erhalten.
¹H-NMR (400 MHz, DMSO-d₆): δ = 9.36 (d, 1H), 8.45 (m, 3H), 8.21 (m, 3H), 7.94 (d, 2H), 7.70 (s, 1H), 7.58 (d, 2H), 7.50 (d, 2H), 7.14 (d, 2H), 4.70 (s, 2H), 4.52-4.34 (m, 5H), 4.05 (m, 1H), 3.83 (m, 4H), 3.01 (m, 2H), 2.16-1.99 (m, 2H), 1.94 (m, 4H), 1.37 (d, 3H).
LC-MS (Methode 4): Rₜ = 1.15 min; MS (ESIpos): m/z = 745 [M+H-2HCl]⁺.

### Beispiel 65

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-L-histidyl-L-alaninat-Dihydrochlorid

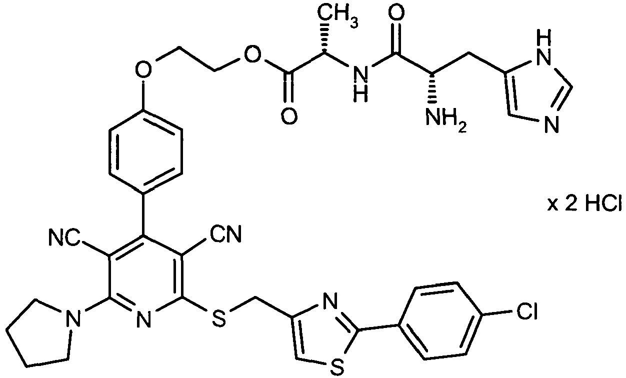

169 mg (0.192 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-N-(tert.-butoxycarbonyl)-L-histidyl-L-alaninat wurden in 3 ml Dichlormethan gelöst und mit 1.915 ml einer 1N Lösung von Chlorwasserstoff in Diethylether versetzt. Nach 6 stündigem Rühren wurde der ausgefallene Feststoff abgesaugt, mit Diethylether gewaschen und im Vakuum getrocknet. 75 mg (44% d. Th.) der Zielverbindung wurden erhalten.
¹H-NMR (400 MHz, DMSO-d₆): δ = 14.73-14.30 (m, 2H), 9.22 (d, 1H), 9.07 (s, 1H), 8.54 (m, 3H), 7.94 (d, 2H), 7.70 (s, 1H), 7.58 (d, 2H), 7.47 (m, 3H), 7.12 (d, 2H), 4.70 (s, 2H), 4.50-4.34 (m, 3H), 4.29 (m, 3H), 3.63 (m, 4H), 3.34-3.14 (m, 2H), 1.94 (m, 4H), 1.35 (d, 3H).
LC-MS (Methode 4): Rₜ = 1.18 min; MS (ESIpos): m/z = 782 [M+H-2HCl]⁺.

### Beispiel 66

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-L-argyl-L-alaninat-Dihydrochlorid

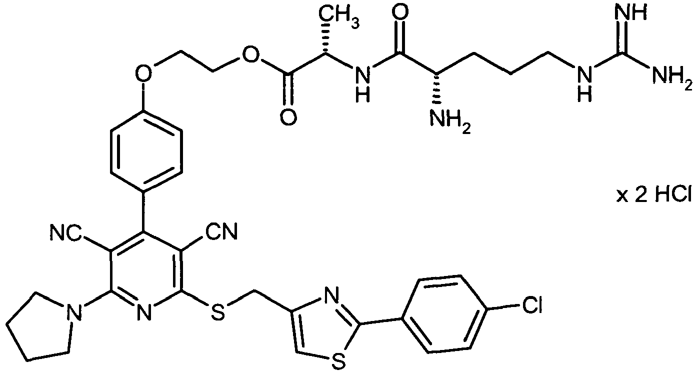

341 mg (0.310 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-N⁵-[N,N'-bis(tert.-butoxycarbonyl)carbamimidoyl]-N²-(tert.-butoxycarbonyl)-L-ornithyl-L-alaninat wurden in 5 ml Dichlormethan gelöst und mit 3.095 ml einer 1N Lösung von Chlorwasserstoff in Diethylether versetzt. Nach 6 stündigem Rühren wurden 5 ml einer 1N Lösung von Chlorwasserstoff in Diethylether nachgegeben, und es wurde über Nacht bei Raumtemperatur weiter gerührt. Es wurden weitere 10 ml einer 1N Lösung von Chlorwasserstoff in Diethylether nachgegeben und weitere 24 Stunden bei Raumtemperatur gerührt. Der ausgefallene Feststoff wurde abgesaugt, mit Diethylether gewaschen und im Vakuum getrocknet. Da die Reaktion noch nicht vollständig war, wurde der Feststoff in 10 ml einer 1N Lösung von Chlorwasserstoff in Diethylether suspendiert und 24 Stunden bei Raumtemperatur gerührt. Es wurden erneut 2 ml einer 1N Lösung von Chlorwasserstoff in Diethylether nachgegeben und weitere 24 Stunden bei Raumtemperatur gerührt. Der ausgefallene Feststoff wurde abgesaugt, mit Diethylether gewaschen und im Vakuum getrocknet. 69 mg (24% d. Th.) der Zielverbindung wurden erhalten.
¹H-NMR (400 MHz, DMSO-d₆): δ = 9.07 (d, 1H), 8.28 (m, 3H), 7.95 (d, 2H), 7.76 (t, 1H), 7.70 (s, 1H), 7.58 (d, 2H), 7.50 (d, 2H), 7.42-7.18 (m br, 2H), 7.13 (d, 2H), 7.07-6.84 (m br, 2H), 4.70 (s, 2H), 4.50-4.33 (m, 3H), 4.29 (m, 2H), 3.83 (m, 5H), 3.15 (m, 2H), 1.94 (m, 4H), 1.75 (m, 2H), 1.57 (m, 2H), 1.36 (d, 3H).
LC-MS (Methode 4): Rₜ = 1.18 min; MS (ESIpos): m/z = 801 [M+H-2HCl]⁺.

### Beispiel 67

### 2-{4-[2-({[2-(4-chlorphenyl)-1,3-thiazol-4-yl]methyl}sulfanyl)-3,5-dicyan-6-(pyrrolidin-1-yl)pyridin-4-yl]phenoxy}ethyl-3-amino-L-alanyl-L-alaninat-Bis(trifluoracetat)

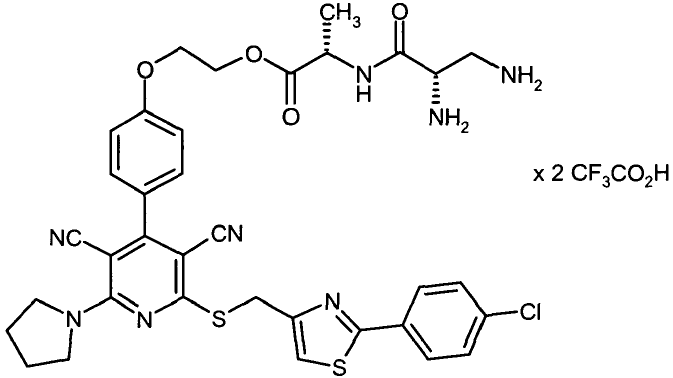

290 mg (0.311 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy} ethyl-N-(tert.-butoxycarbonyl)-3-[(tert.-butoxycarbonyl)amino]-L-alanyl-L-alaninat wurden in 5 ml Dichlormethan gelöst und mit 3.113 ml einer 1N Lösung von Chlorwasserstoff in Diethylether versetzt. Nach 6 stündigem Rühren wurden weitere 3.113 ml einer 1N Lösung von Chlorwasserstoff in Diethylether nachgegeben und über Nacht weiter bei Raumtemperatur gerührt. Es wurden erneut 10 ml einer 1N Lösung von Chlorwasserstoff in Diethylether zugegeben und weitere 24 Stunden bei Raumtemperatur gerührt. Anschließend wurde der ausgefallene Feststoff abgesaugt, mit Diethylether gewaschen und im Vakuum getrocknet. Das Rohprodukt wurde in 2 ml Dichlormethan gelöst und mit 0.126 ml (1.632 mmol) Trifluoressigsäure versetzt. Nach 6 stündigem Rühren wurde das Reaktionsgemisch eingeengt und der Rückstand mittels präparativer HPLC (Acetonitril/Wasser + 0.1% TFA) gereinigt. 81 mg (27% d. Th.) der Zielverbindung wurden erhalten.
¹H-NMR (400 MHz, DMSO-d₆): δ = 9.04 (d, 1H), 8.54-8.01 (m br, 6H), 7.94 (d, 2H), 7.70 (s, 1H), 7.58 (d, 2H), 7.50 (d, 2H), 7.13 (d, 2H), 4.70 (s, 2H), 4.53-4.39 (m, 3H), 4.32 (m, 2H), 4.17 (m, 1H), 3.83 (m, 4H), 3.34-3.17 (m, 2H), 1.94 (m, 4H), 1.37 (d, 3H).
LC-MS (Methode 4): Rₜ = 1.32 min; MS (ESIpos): m/z = 731 [M+H-2TFA]⁺.

### Beispiel 68

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxyl ethyl-N-(tert.-butoxycarbonyl)-L-alanyl-L-leucinat-Hydrochlorid

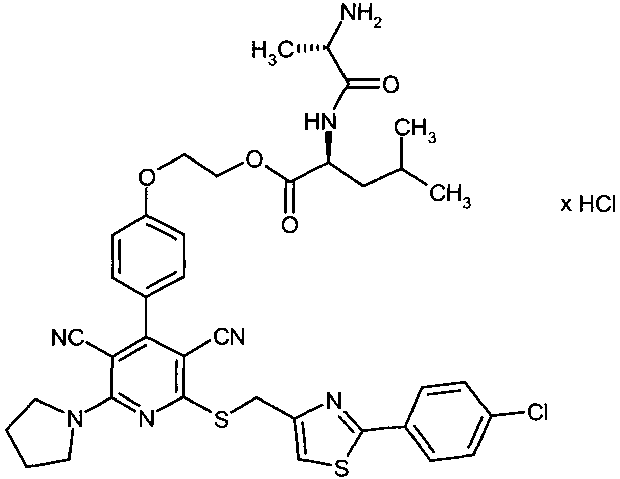

153 mg (0.18 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert-butoxycarbonyl)-L-alanyl-L-leucinat] wurden in 5 ml Dichlormethan und 5 ml Diethylether gelöst. Es wurden 4.5 ml (17.8 mmol) 4M Chlorwasserstoff in Dioxan zugesetzt und 3h bei Raumtemperatur gerührt. Der Niederschlag wurde abfiltriert, mit Diethylether gewaschen und im Hochvakuum getrocknet. Es wurden 68 mg (55.% d. Th.) der gewünschten Zielverbindung erhalten.
¹H-NMR (400 MHz, DMSO-d₆): δ = 8.76 (d, 1H), 8.14 (m, 3H), 7.94 (d, 2H), 7.70 (s, 1H), 7.58 (d, 2H), 7.49 (d, 2H), 7.11 (d, 2H), 4.70 (s, 2H), 4.40 - 4.27 (m, 4H), 4.01-3.52 (m, 6H), 1.94 (s br, 4H), 1.67 (m, 1H), 1.58 (m, 2H), 1.35 (d, 3H), 0.90 (d, 3H), 0.85 (d, 3H).
LC/MS (Methode 2): Rₜ = 1,17 min; MS (ESIpos): m/z = 758 [M-HCl+H]⁺.

### Beispiel 69

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-beta-alanyl-L-leucinat-Hydrochlorid

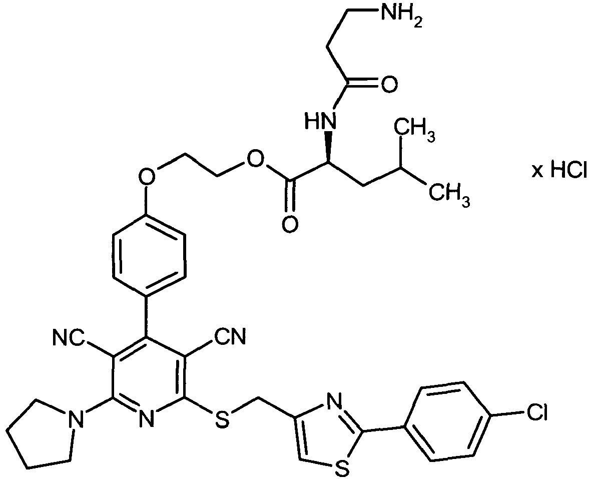

131 mg (0.15 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)-L-alanyl-L-leucinat] wurden in 1.5 ml Dichlormethan und 1.5 ml Diethylether gelöst. Es wurden 3.8 ml (15.2 mmol) 4M Chlorwasserstoff in Dioxan zugesetzt und 3h bei Raumtemperatur gerührt. Der Niederschlag wurde abfiltriert, mit Diethylether gewaschen und im Hochvakuum getrocknet. Es wurden 68 mg (55.% d. Th.) der gewünschten Zielverbindung erhalten.
¹H-NMR (400 MHz, DMSO-d₆): δ = 8.55 (d, 1H), 7.94 (d, 2H), 7.86 (s br, 3H), 7.40 (s, 1H), 7.58 (d, 2H), 7.49 (d, 2H), 7.12 (d, 2H), 4.87 (br s, 2H), 4.70 (s, 2H), 4.42 (m, 2H), 4.30 (m, 3H), 3.85 (s br, 4H), 2.95 (m, 2H), 1.94 (s br, 4H), 1.64 (m, 1H), 1.54 (m, 2H), 0.88 (d, 3H), 0.83 (d, 3H).
LC/MS (Methode 2): Rₜ = 1,16 min; MS (ESIpos): m/z = 758 [M-HCl+H]⁺.

### Beispiel 70

### 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-glycyl-L-leucinat-Hydrochlorid

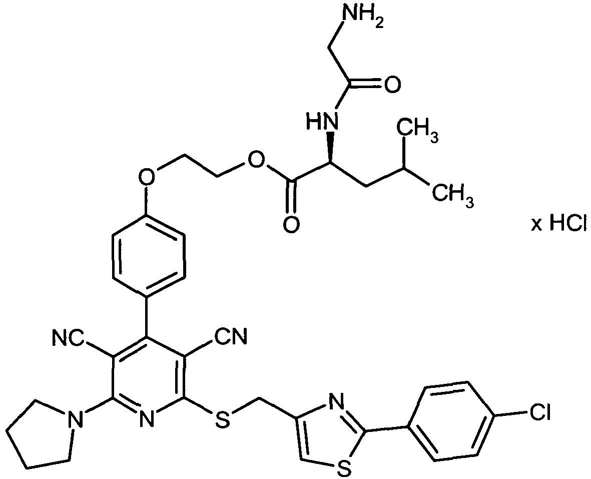

168 mg (0.20 mmol) 2-{4-[2-({[2-(4-Chlorphenyl)-1,3-thiazol-4-yl]methyl}sulfanyl)-3,5-dicyan-6-(pyrrolidin-1-yl)pyridin-4-yl]phenoxy}ethyl-N-(tert.-butoxycarbonyl)glycyl-L-leucinat wurden in 1.5 ml Dichlormethan und 1.5 ml Diethylether gelöst. Es wurden 3.8 ml (15.2 mmol) 4M Chlorwasserstoff in Dioxan zugesetzt und 3h bei Raumtemperatur gerührt. Der Niederschlag wurde abfiltriert, mit Diethylether gewaschen und im Hochvakuum getrocknet. Es wurden 97 mg (60.% d. Th.) der gewünschten Zielverbindung erhalten.
¹H-NMR (400 MHz, DMSO-d₆): δ = 8.84 (d, 1H), 8.10 (m, 3H), 7.94 (d, 2H), 7.70 (s, 1H), 7.58 (d, 2H), 7.49 (d, 2H), 7.12 (d, 2H), 4.70 (s, 2H), 4.41 - 4.30 (m, 5H), 3.85 (s br, 4H), 3.59 (m, 2H), 1.94 (s br, 4H), 1.75 - 1.49 (m, 3H), 0.87 (d, 3H), 0.85 (d, 3H).
LC/MS (Methode 2): Rₜ = 1,16 min; MS (ESIpos): m/z = 744 [M-HCl+H]⁺.

### B. Bewertungder pharmakologischen und physiologischen Wirksamkeit

Die pharmakologische und physiologische Wirkung der erfindungsgemäßen Verbindungen kann in folgenden Assays gezeigt werden:

### B-1. Indirekte Bestimmung des Adenosin-Agonismus über Genexpression

Zellen der permanenten Linie CHO (Chinese Hamster Ovary) werden stabil mit der DNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylat-cyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:

Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 5 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den Al-Zellen zugegeben und anschließend werden alle Kulturen für vier Stunden bei 37°C inkubiert. Danach wird zu den Testkulturen 35 µl einer Lösung, bestehend zu 50% aus Lyse-Reagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der Al-Zelle 50% der Luciferase-Antwort inhibiert bzw. bei den A2b- und A2a-Zellen 50% der maximalen Stimulierbarkeit mit der entsprechenden Substanz erreicht sind. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol. 357, 1-9 (1998)].

In der folgenden Tabelle 1 sind die EC₅₀-Werte repräsentativer Ausführungsbeispiele für die Rezeptorstimulation an Adenosin A1-, A2a- und A2b-Rezeptor-Subtypen aufgeführt:

**Tabelle 1**

| **Beispiel Nr.** | **EC₅₀ A1 [nM] (1 µM Forskolin)** | **EC₅₀ A2a [nM]** | **EC₅₀ A2b [nM]** |
|---|---|---|---|
| **1** | 0.1 | 673 | 80 |
| **3** | 0.3 | 282 | 138 |
| **5** | 0.5 | 315 | 150 |
| **9** | 0.7 | 281 | 231 |
| **17** | 0.3 | 3000 | 55 |
| **21** | 0.9 | 743 | 3000 |
| **24** | 0.6 | 287 | 675 |
| **26** | 0.5 | 3000 | 1000 |
| **33** | 0.05 | 27 | 900 |

### B-2. Untersuchung an isolierten Gefäßen

Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.

### B-3. Blutdruck- und Herzfrequenz-Messungen an wachen Krallenaffen

Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend werden über 6-24 Stunden Blutdruck und Herzfrequenz und deren Veränderungen aufgezeichnet.

### B-4. Hämodynamische Messungen an narkotisierten Ratten:

Wistar-Ratten (250-300 g Körpergewicht; Fa. Harlan-Winkelmann) werden mit 5% Isofluran^{®} narkotisiert. Die Narkose wird mit 2% Isofluran^{®} und Druckluft in einer Narkosemaske aufrechterhalten. Die *A. carotis* wird frei präpariert, und ein Tip-Katheter (Millar Micro-Tip-Transducer, 2 French; Fa. HSE) wird eingeführt und bis in den linken Ventrikel vorgeschoben. Anschließend wird ein zweiter Katheter in die *V. jugularis* eingeführt. Über diesen Katheder werden Placebo-Lösung und Testsubstanz-Lösungen in aufsteigender Konzentration in die Tiere infundiert. Gleichzeitig erfolgt die Messung der Herzfunktion (wie Herzfrequenz, linksventrikulärer Druck, Kontraktilität (dp/dt), linksventrikulärer enddiastolischer Druck) durch den linksventrikulären Katheter. Durch Zurückziehen des Katheders aus dem linken Ventrikel in die Aorta kann auch noch der systemische Blutdruck gemessen werden.

### B-5. Blutdruck- und Herzfrequenz-Messungen

### a) an wachen Ratten:

Wachen spontan-hypertensiven Ratten (SH-Ratten), die einen internen Sender tragen, der dauerhaft sowohl Blutdruck als auch Herzfrequenz messen kann (telemetrische Erfassung von hämodynamischen Parametern), und die in einem Käfig sitzen, der mit Bewegungssensoren ausgestattet ist, werden Testsubstanzen in verschiedenen Dosierungen oral verabreicht. Anschließend werden über 24 Stunden Blutdruck und Herzfrequenz und deren Veränderungen, sowie die Bewegungen und Aktivität der Tiere aufgezeichnet und ausgewertet.

In Tabelle 2 ist die maximale Herzfrequenz-Senkung nach einer p.o.-Gabe von 3 mg/kg der Verbindung aus Beispiel 1 bzw. Beispiel 2 bzw. Beispiel 41 wiedergegeben:

**Tabelle 2**

| **Beispiel Nr.** | **Dosierung** | **Herzfrequenz-Senkung** |
|---|---|---|
| **1** | 3 mg/kg | -20% |
| **2** | 3 mg/kg | -45 % |
| **41** | 3 mg/kg | -5 % |
| **44** | 3 mg/kg | -20 % |
| **63** | 3 mg/kg | -20 % |

### b) an wachen Hunden:

Wachen männlichen Beagle Hunden, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral oder intra duodenal verabreicht. Anschließend werden über 24 Stunden Blutdruck und Herzfrequenz und deren Veränderungen aufgezeichnet und ausgewertet. Gleichzeitig wird das Verhalten der Tiere in Bezug auf ihre Aktivität (Art des Ganges, Seitenlage, Ruhephasen etc.) hin beobachtet, um Hinweise auf eine mögliche CNS Wirkung der Substanzen zu erhalten.

### B-6. GTP shift Versuch

### Preparation der Hirnmembran

Männlichen Wistar Ratten wird das Hirn entnommen und dieses sofort in eine eisgekühlte 0.32 mol/l Sucroselösung überführt. Das Gewebe wird mit einem Glass-Teflon Homogenisator zerkleinert und anschliessend zentrifugiert (1.000 x g für 10 Minuten). Der Überstand wird dann bei 30.000 g für 30 Minuten ultrazentrifugiert. Das so erhaltene Pellet wird in 10 ml Wasser resuspendiert und 30 Minuten auf Eis stehen gelassen. Nach einem finalen Zentrifugationsschritt bei 48.000 g für 10 min werden die Membranen in 50 mmol/l Tris-HCl Puffer, pH 7.4 resuspendiert und mit 2U/ml Adenosin Deaminase bei 37°C für 30 min inkubiert. Danach erfolgt eine Proteinbestimmung nach Bradford. Die Membranen werden in kleinen Aliquots eingefroren und bei - 80°C bis zum Einsatz im Bindungsassay gelagert.

### Rezeptorbindungsstudie

Der A1 Rezeptor GTP Shift Bindungsassay wird mit Ratten Himmembranen und 0,4 nM [³H] DPCPX (K_{d} = 0.28 nM) als Radioligand durchgeführt. 10 µg Membranprotein werden bei 37°C für 20 min mit 0,4 nM [³H]DPCPX und Adenosine A1 Agonisten in verschiedenen Konzentrationen in Puffer (50 mM Tris-HCl, pH 7.4, 2U/ml ADA) in der Gegenwart und Abwesenheit von 1 mM Guanosintriphosphat (GTP) inkubiert. Die Inkubation wird mit einer Filtration durch GF/B Glassfaser Filterplatten beendet. Die Filter werden dann dreimal mit eiskaltem Tris-HCl Puffer 50 mM, pH 7.4 gewaschen. Die Radioaktivität auf dem Filter wird unter Zusatz von 100 µl Szintillationscocktail in einem Microbeta TriLux beta counter (PerkinElmer, Massachusetts, USA) vermessen.

### B-7. Prüfung von Adenosin A1 Rezeptor Agonisten auf motorische Wirkung im Laufradversuch

Zur Bestimmung der Wirkung von Adenosin A1 Rezeptor Agonisten auf die motorischen Funktion, wird das Laufverhalten von Mäusen (Stamm: CD1) in Laufrädern (M. Weber et al., Psychopharmacology 2008, in print) untersucht. Um die Mäuse an die freiwillige Benutzung des Laufrades zu gewöhnen, werden 2-3 Wochen vor Beginn des Versuches die Tiere in Käfigen mit Laufrad vereinzelt und trainiert. 2 Wochen vor Start des Experimentes werden die Bewegungen der Mäuse im Laufrad durch eine Photozelle mittels Computer aufgezeichnet und verschiedene Laufparameter wie z.B. die tägliche Laufstrecke, die zurückgelegten Einzelstrecken, aber auch Ihre zeitliche Verteilung über den Tag bestimmt. Die Tiere werden nach ihrem natürlichen Laufverhalten in Gruppen (8-12 Tiere) randomisiert (Kontrollgruppe und 1- mehrere Substanzgruppen). Nach der 2-wöchigen Einlaufphase werden die Tiere mit den zu prüfenden Substanzen oral, behandelt. Dabei werden Einzeldosen oder auch aufsteigende Dosierungen (z.B. 0.3-1-3-10-30 mg/kg) verabreicht. Substanzen werden in zwei voneinander unabhängigen Experimenten getestet. Zwischen 2 Experimenten liegen mindestens 3 Tage, in denen die Tiere keine Substanzen erhalten. Das Laufverhalten der Tiere wird über 24 Std. nach der Applikation beobachtet und aufgezeichnet. Die Auswertung der Laufintervalle und der Gesamtlaufstrecke erfolgt über einen Zeitraum von mehreren Stunden während der Hauptaktivitätszeit der Mäuse. Effekte werden als Prozent vom Kontrollwert angegeben.

| **Beispiel Nr.** | **Reduktion der Gesamtlaufstrecke bei 1 mg/kg** |
|---|---|
| **1** | 0% |
| **13** | 0% |
| **21** | 7.5 % |
| **33** | 24 % |

### B-8. Bestimmung von Löslichkeit, Stabilität und Freisetzunesverhalten

### a) Bestimmung der Löslichkeit:

Die Prüfsubstanz wird in 5%-iger wässriger Dextrose-Lösung suspendiert. Diese Suspension wird 24 h bei Raumtemperatur geschüttelt. Nach Ultra-Zentrifugation bei 224000g für 30 min wird der Überstand mit DMSO verdünnt und per HPLC analysiert. Quantifiziert wird über eine Zwei-Punkt-Kalibrationskurve der Testverbindung in DMSO.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 5 µm, 50 mm x 2 mm; Temperatur: 40°C; Eluent A: Wasser/Phosphorsäure pH 2, Eluent B: Acetonitril; Flussrate: 0.7 ml/min; Gradient: 0-0.5 min 85% A, 15% B; Rampe 0.5-3 min 10% A, 90% B; 3-3.5 min 10% A, 90% B; Rampe 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 3.5 µm, 60 mm x 2.1 mm; Temperatur: 30°C; Eluent A: Wasser + 5 ml Perchlorsäure/Liter, Eluent B: Acetonitril; Flussrate: 0.75 ml/min; Gradient: 0-0.5 min 98% A, 2% B; Rampe 0.5-4.5 min 10% A, 90% B; 4.5-6 min 10% A, 90% B; Rampe 6.5-6.7 min 98% A, 2% B; 6.7-7.5 min 98% A, 2% B.

In Tabelle 1 sind die Löslichkeitswerte repräsentativer Ausführungsbeispiele in 5%-iger wässriger Dextrose-Lösung dargestellt:

**Tabelle 1**

| **Beispiel Nr.** | **Löslichkeit [mg/Liter]** |
|---|---|
| **41** | 650 |
| **44** | 410 |
| **51** | 540 |
| **52** | 280 |
| **54** | 640 |
| **58** | 470 |

Eine Zersetzung der Beispielverbindungen in diesen Lösungen wurde nicht beobachtet.

Die Löslichkeit der Wirksubstanz aus Beispiel 1, Beispiel 2 und Beispiel 12 liegt unterhalb der Nachweisgrenze.

### b) Stabilität in Puffer bei verschiedenen pH-Werten:

0.3 mg der Prüfsubstanz werden in einem 2 ml-HPLC-Vial eingewogen und mit 0.5 ml Acetonitril bzw. Acetonitril/DMSO (9:1) versetzt. Zum Lösen der Substanz wird das Probengefäß für ca. 10 Sekunden ins Ultraschallbad gegeben. Anschließend werden 0.5 ml der jeweiligen (Puffer-) Lösung zugefügt und die Probe erneut im Ultraschallbad behandelt.

Eingesetzte (Puffer-)Lösungen:
- pH 2:: 0.03 Mol Zitronensäure, 0.061 Mol Natriumchlorid und 0.0082 Mol Salzsäure *ad* 1 Liter Wasser;
- pH 4:: 1 Liter Millipore-Wasser wird mit 1 N Salzsäure auf pH 4.0 eingestellt;
- pH 5:: 0.096 Mol Zitronensäure und 0.2 Mol Natriumhydroxid *ad* 1 Liter Wasser;
- pH 6:: 0.06 Mol Zitronensäure und 0.16 Mol Natriumhydroxid *ad* 1 Liter Wasser;
- pH 7.4:: 90.0 g Natriumchlorid, 13.61 g Kaliumdihydrogenphosphat und 83.35 g 1 N Natronlauge *ad* 1 Liter Wasser; diese Lösung wird dann noch 1:10 mit Millipore-Wasser weiter verdünnt;
- pH 8:: 0.013 Mol Borax und 0.021 Mol Salzsäure *ad* 1 Liter Wasser.

Über einen Zeitraum von 24 Stunden bei 37°C werden stündlich jeweils 5 µl der Probenlösung per HPLC auf ihren Gehalt an unveränderter Testsubstanz bzw. an gebildeter Wirksubstanz (A) analysiert. Quantifiziert wird über die Flächenprozente der entsprechenden Peaks.

### HPLC-Methode für Beispiel 41 und 44:

Agilent 1100 mit DAD (G1315B), binärer Pumpe (G1312A), Autosampler (G1329A), Säulenofen (G1316A), Thermostat (G1330B); Säule: Kromasil 100 C18, 250 mm x 4 mm, 5 µm; Säulentemperatur: 30°C; Eluent A: Wasser + 5 ml Perchlorsäure/Liter, Eluent B: Acetonitril; Gradient: 0 min 90% A → 5.0 min 40% A → 18.0 min 10% A → 19.0 min 10% A → 21.0 min 90% A → 23.0 min 90% A; Flussrate: 2.0 ml/min; UV-Detektion: 288 nm.

### HPLC-Methode für Beispiel 50 und 59:

Agilent 1100 mit DAD (G1315B), binärer Pumpe (G1312A), Autosampler (G1329A), Säulenofen (G1316A), Thermostat (G1330B); Säule: Kromasil 100 C18, 125 mm x 4 mm, 5 µm; Säulentemperatur: 30°C; Eluent A: Wasser + 5 ml Perchlorsäure/Liter, Eluent B: Acetonitril; Gradient: 0 min 90% A → 5.0 min 60% A → 7.0 min 60% A → 10.0 min 10% A → 12.0 min 10% A → 14.0 min 90% A → 16.0 min 90% A; Flussrate: 2.0 ml/min; UV-Detektion: 294 nm.

### HPLC-Methode für Beispiel 54:

Agilent 1100 mit DAD (G1314A), binärer Pumpe (G1312A), Autosampler (G1329A), Säulenofen (G1316A), Thermostat (G1330A); Säule: Kromasil 100 C18, 250 mm x 4 mm, 5 µm; Säulentemperatur: 30°C; Eluent A: Wasser + 5 ml Perchlorsäure/Liter, Eluent B: Acetonitril; Gradient: 0 min 90% A → 5.0 min 40% A → 18.0 min 10% A → 19.0 min 10% A → 21.0 min 90% A → 23.0 min 90% A; Flussrate: 2.0 ml/min; UV-Detektion: 288 nm.

In Tabelle 2 sind zu repräsentativen Ausführungsbeispielen die Verhältnisse der Peakflächen (F) zu den jeweiligen Zeitpunkten in Relation zu den Peakflächen beim Startzeitpunkt dargestellt:

**Tabelle 2**

| **Beispiel Nr.** | **pH-Wert** | **% Testsubstanz nach 4 h [F(t=4h) x 100 / F(t=0h)]** | **% Testsubstanz nach 24 h [F(t=24h) x 100 / F(t=0h)]** |
|---|---|---|---|
| 41 | 4 | 97 | 99 |
| 41 | 7.4 | 37 | 0 |
| 44 | 4 | 99 | 99 |
| 44 | 7.4 | 78 | 26 |
| 50 | 4 | 99 | 99 |
| 50 | 7.4 | 94 | 78 |
| 54 | 4 | 98 | 97 |
| 54 | 7.4 | 29 | 0 |
| 59 | 4 | 99 | 95 |
| 59 | 7.4 | 0 | 0 |
| 63 | 4 | 98 | 98 |
| 63 | 7.4 | 93 | 67 |

In diesem Test wurde gleichzeitig mit einer Abnahme des Gehalts an Testsubstanz eine Zunahme der betreffenden Wirkstoff-Verbindung aus Beispiel 1 bzw. 12 festgestellt.

### c) Stabilität in Suspension und als Feststoff:

4.7-4.8 g der Prüfsubstanz werden in 200 ml Isopropanol suspendiert und 7 Tage bei Raumtemperatur gerührt. Der Feststoff wird abfiltriert und 3 Tage an der Luft bei Raumtemperatur getrocknet. Mittels LC/MS (Methode 2) wird dann gemessen, ob es zu einem Abbau der Prüfsubstanz gekommen ist. Bei Beispiel 63 und 44 ist kein Abbau eingetreten.

Zur Beurteilung der Feststoffstabilität werden 20 mg der so erhaltenen Feststoffe in einem verschlossenen Head-Space Vial 7 Tage bei 90 °C in einem Trockenschrank ohne Vakuum eingelagert. Mittels HPLC wird dann gemessen, ob es zu einem Abbau der Prüfsubstanz gekommen ist.

HPLC-Methode zur Feststoffstabilität für Beispiel 63:
Gerät: Agilent 1100 oder vergleichbares Gerät, UV variable Wellenlänge (z.B. Dioden-Array); Messwellenlänge: 215 nm, Bandbreite 6 nm; Referenzwellenlänge: ausgeschaltet; Ofentemperatur: 40 °C; Säule: Nucleodur Gravity C18, Länge 150 mm, Innendurchmesser 2.0 mm, Komgrösse 3 µm; Mobile Phase: A saurer Ammoniumphosphatpuffer (pH 2.4), B Acetonitril; Analysenprogramm: Fluss 0.25 ml/min, Start 0 min 85% A -> 35 min 20% A -> Stop 45 min 20% A; Equilibrierung: 12 min; Prüflösung: ca. 25 mg der Probe in 50 ml Messkolben genau einwiegen, in 25 ml Isopropanol lösen und mit Wasser bis zur Eichmarke auffüllen; Kalibrierlösung: ca. 25 mg des Standards in 50 ml Messkolben genau einwiegen, in 25 ml Isopropanol lösen und mit Wasser bis zur Eichmarke auffüllen; Injektionsvolumen: 3 µl.

HPLC-Methode zur Feststoffstabilität für Beispiel 44:
Gerät: Agilent 1100 oder vergleichbares Gerät, UV variable Wellenlänge (z.B. Dioden-Array); Messwellenlänge: 220 nm, Bandbreite 6 nm; Referenzwellenlänge: ausgeschaltet; Ofentemperatur: 45 °C; Säule: Zorbax SB-CN, Länge 150 mm, Innendurchmesser 3.0 mm, Korngrösse 3.5 µm; Mobile Phase: neutraler Ammoniumphosphatpuffer (pH 7.2), B Acetonitril; Analysenprogramm: Fluss 0.5 ml/min, Start 0 min 80% A -> 25 min 20% A -> Stop 35 min 20% A; Equilibrierung: 10 min; Prüflösung: ca. 22 mg der Probe in 50 ml Messkolben genau einwiegen, in 25 ml Acetonitril lösen und mit Wasser bis zur Eichmarke auffüllen; Kalibrierlösung: ca. 25 mg des Standards in 50 ml Messkolben genau einwiegen, in 25 ml Acetonitril lösen und mit Wasser bis zur Eichmarke auffüllen; Injektionsvolumen: 3 µl.

Bei Beispiel 63 und 44 ist, im Rahmen der Messgenauigkeit, kein Abbau eingetreten.

### d) In vitro-Stabilität in Ratten- und Humanplasma:

1 mg der Prüfsubstanz wird in einem 2 ml-HPLC-Vial eingewogen und mit 1.5 ml DMSO und 1 ml Wasser versetzt. Zum Lösen der Substanz wird das Probengefäß für ca. 10 Sekunden ins Ultraschallbad gestellt. Zu 0.5 ml dieser Lösung werden 0.5 ml 37°C warmes Ratten- bzw. Humanplasma gegeben. Die Probe wird geschüttelt und für eine erste Analyse ca. 10 µl entnommen (Zeitpunkt t₀). Im Zeitraum bis zu 2 Stunden nach Inkubationsbeginn werden 4-6 weitere Aliquote zur Quantifizierung entnommen. Die Probe wird über die Prüfzeit bei 37°C gehalten. Charakterisierung und Quantifizierung erfolgen per HPLC.

### HPLC-Methode:

Agilent 1100 mit DAD (G1314A), binärer Pumpe (G1312A), Autosampler (G1329A), Säulenofen (G1316A), Thermostat (G1330A); Säule: Kromasil 100 C18, 250 mm x 4 mm, 5 µm; Säulentemperatur: 30°C; Eluent A: Wasser + 5 ml Perchlorsäure/Liter, Eluent B: Acetonitril; Gradient: 0-8.0 min 53% A, 47% B; 8.0-18.0 min 53% A, 47% B; 18.0-20.0 min 90% A, 10% B; 20.0-21.0 min 90% A, 10% B; 21.0-22.5 min 98% A, 2% B; 22.5-25.0 min 98% A, 2% B; Flussrate: 2 ml/min; UV-Detektion: 294 nm.

### e) i.v.-Pharmakokinetik in Wistar-Ratten:

Am Tag vor der Substanzgabe wird den Versuchstieren (männliche Wistar-Ratten, Körpergewicht 200-250 g) unter Isofluran^{®}-Narkose ein Katheter für die Blutgewinnung in die *Vena jugularis* implantiert.

Am Versuchstag wird eine definierte Dosis der Prüfsubstanz als Lösung mit einer Hamilton^{®}-Glasspritze in die Schwanzvene appliziert (Bolusgabe, Applikationsdauer < 10 s). Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, Cₘₐₓ, T_{1/2} (Halbwertszeit) und CL (Clearance) erfolgt aus den gemessenen Plasmakonzentrationen.

Nach i.v.-Applikation der Verbindungen aus Beispiel 63, 44 oder 41 konnten diese Substanzen bereits zum ersten Messpunkt nicht mehr im Plasma detektiert werden. Lediglich der Wirkstoff (Beispiel 1) war auch bis zum Zeitpunkt von 24 Stunden detektierbar.

### f) p.o.-Pharmakokinetik in Wistar-Ratten:

Am Tag vor der Substanzgabe wird den Versuchstieren (männliche Wistar-Ratten, Körpergewicht 200-250 g) unter Isofluran^{®}-Narkose ein Katheter für die Blutgewinnung in die *Vena jugularis* implantiert.

Am Versuchstag wird eine definierte Dosis der Prüfsubstanz als Lösung mit einer Schlundsonde in den Magen appliziert. Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, Cₘₐₓ und T_{1/2} (Halbwertszeit) erfolgt aus den gemessenen Plasmakonzentrationen.

Nach p.o.-Applikation der Verbindungen aus Beispiel 63, 44 oder 41 konnten diese Substanzen bereits zum ersten Messpunkt nicht mehr im Plasma detektiert werden. Lediglich der Wirkstoff (Beispiel 1) war auch bis zum Zeitpunkt von 24 Stunden detektierbar.

### B-9. Bestimmung der metabolischen Stabilität

Zur Bestimmung der metabolischen Stabilität von Testverbindungen werden diese *in vitro* mit Lebermikrosomen oder bevorzugt mit primären frischen Hepatozyten verschiedener Tierspezies (z.B. von Ratte und Hund) als auch humanen Ursprungs inkubiert, um Metabolitenprofile eines möglichst kompletten hepatischen Phase I- und Phase II-Metabolismus zu erhalten und zu vergleichen.

Die Testverbindungen werden mit einer Konzentration von 10-20 µM inkubiert. Dazu werden Stammlösungen der Substanzen mit einer Konzentration von 1-2 mM in Acetonitril hergestellt und dann mit einer 1:100-Verdünnung in den Inkubationsansatz pipettiert. Die Lebermikrosomen werden in 50 mM Kaliumphosphat-Puffer (pH 7.4) mit und ohne NADPH-generierendem System, bestehend aus 1 mM NADP⁺, 10 mM Glucose-6-phosphat und 1 Einheit Glucose-6-phosphat-Dehydrogenase, bei 37°C inkubiert. Primäre Hepatozyten werden in Suspension in Williams E-Medium ebenfalls bei 37°C inkubiert. Nach einer Inkubationszeit von 0-4 Stunden werden die Inkubationsansätze mit Acetonitril abgestoppt (Endkonzentration ca. 30%) und das Protein bei ca. 15000 x g abzentrifugiert. Die so abgestoppten Proben werden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung chromatographiert. Die UV-Chromatogramme in Verbindung mit massenspektrometrischen MS/MS-Daten dienen zur Identifizierung und Strukturaufklärung der Metabolite.

### B-10. CYP-Inhibitionstest

Die Fähigkeit von Substanzen, CYP1A2, CYP 2C8, CYP2C9, CYP2D6 und CYP3A4 im Menschen inhibieren zu können, wird untersucht mit gepoolten Human-Lebermikrosomen als Enzymquelle in Gegenwart von Standardsubstraten (s.u.), die CYP-Isoform-spezifische Metaboliten bilden. Die Inhibitionseffekte werden bei sechs verschiedenen Konzentrationen der Testverbindungen untersucht (0.6, 1.3, 2.5, 5, 10 sowie 20 µM oder 1.5, 3.1, 6.3, 12.5, 25 sowie 50 µM), mit dem Ausmaß der CYP-Isoform-spezifischen Metabolitenbildung der Standardsubstrate in Abwesenheit der Testverbindungen verglichen und die entsprechenden IC₅₀-Werte berechnet. Ein Standard-Inhibitor, der eine einzelne CYP-Isoform spezifisch inhibiert, dient als Kontrolle der erhaltenen Ergebnisse.

### Durchführung:

Die Inkubation von Phenacetin, Amodiaquin, Diclofenac, Dextromethorphan oder Midazolam mit Human-Lebermikrosomen in Gegenwart von jeweils sechs verschiedenen Konzentrationen einer Testverbindung (als potentiellem Inhibitor) wird auf einer Workstation durchgeführt (Tecan, Genesis, Crailsheim, Deutschland). Standard-Inkubationsgemische enthalten 1.0 mM NADP, 1.0 mM EDTA, 5.0 mM Glukose-6-phosphat, Glukose-6-phosphat-Dehydrogenase (1.5 U/ml) und 50 mM Phosphat-Puffer (pH 7.4) in einem Gesamtvolumen von 200 µl. Testverbindungen werden bevorzugt in Acetonitril gelöst. 96-Lochplatten werden eine definierte Zeit bei 37°C mit gepoolten Human-Lebermikrosomen inkubiert. Die Reaktionen werden durch Zugabe von 100 µl Acetonitril, worin sich ein geeigneter interner Standard befindet, abgestoppt. Gefällte Proteine werden durch Zentrifugation abgetrennt, die Überstände werden vereinigt und mittels LC-MS/MS analysiert.

Die Verbindungen aus Beispiel 1, 13, 19, 6, 27, 10, 26, 8, 14 und 29 zeigen auf den CYP-Isoenzymen 1A2, 2C8, 2C9, 2D6, 3A4 und 3A4 mit 30 minütiger Vorinkubation einen IC₅₀/Kᵢ-Wert von > 20 µM.

### B-11. Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen. Dieses wird heparini-5 siert, anschließend wird daraus durch Zentrifugation Plasma gewonnen. Die Substanz wird im Plasma über LC/MS-MS analytisch quantifiziert. Aus den so ermittelten Plasmakonzentration-Zeit-Verläufen werden die pharmakokinetischen Kenngrößen wie AUC (Fläche unter der Konzentration-Zeit-Kurve), Cmax (maximale Plasmakonzentration), T1/2 (Halbwertszeit) und CL (Clearance) mittels eines validierten pharmakokinetischen Rechenprogramms berechnet.

### B-12. Bestimmung der freien Fraktion in Plasma mittels Transil

Die Verteilung einer Verbindung zwischen Wasser und oberflächenunterstützten Ei-Lecithin-Membranen (Transil) einerseits (MA_{puffer}) und zwischen Plasma und oberflächenunterstützten EiLecithin-Membranen (Transil) andererseits (MAₚₗₐₛₘₐ) werden gemessen.

Die gelöste Test-Substanz wird zu Suspensionen aus Transil/Puffer und Transil/Plasma pipetiert. Nach diesen Inkubationen, wird das Transil durch Zentrifugation bei 1800g von der jeweiligen Phase getrennt. Die Substanzkonzentrationen vor der Zentrifugation und im Überstand nach der Zentrifugation werden bestimmt. Die freie Fraktion wird als das Verhältnis der Membranaffinität in Plasma (MAₚₗₐₛₘₐ) und in Puffer (MA_{puffer}) berechnet.

### B-13. ZNS-Wirkung von Substanzen

Mögliche Effekte einer einzelnen oralen Gabe einer Testsubstanz auf Verhaltensparameter, Bewegungsaktivität ("open field test") und Körpertemperatur werden in Ratten untersucht. Die Testsubstanzen werden oral in steigender Dosierung verabreicht. Kontrolltiere erhalten nur das Vehikel (Ethanol/Solutol/Wasser (10:40:50, V/V/V). Jede Behandlungsgruppe besteht aus 6 männlichen Ratten. Die Tiere werden auf Verhaltensänderungen und Änderungen in der Körpertemperatur hin nach 0.5, 1, 2, und 7 Stunden untersucht. Nach ca. 0.5 und 7 Stunden werden die Tiere außerdem auf mögliche Substanz-abhängige Veränderungen in ihrer Bewegungsaktivität im "open field test" (freie Bewegung im Käfig) untersucht. Plasmakonzentrationen der Testsubstanzen werden in Satellitengruppen bestimmt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfmdungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfmdungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfmdungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindungen der Formel (I) 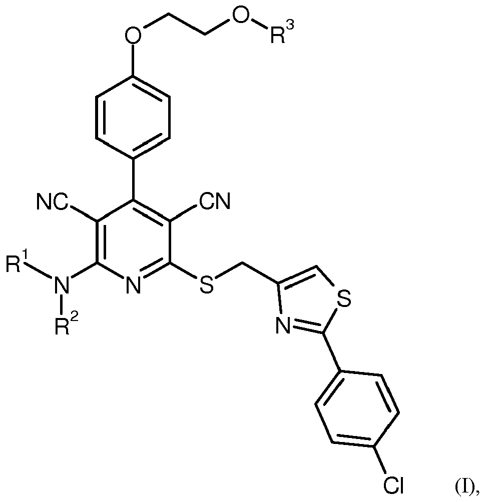 in welcher
R¹ für (C₁-C₄)-Alkyl steht,
R² für (C₁-C₆)-Alkyl, (C₂-C₄)-Alkenyl, (C₂-C₄)-Alkinyl oder (C₃-C₇)-Cycloalkyl steht,
wobei (C₁-C₆)-Alkyl mit 1 bis 3 Substituenten unabhängig voneinander ausgewählt aus der Gruppe Fluor, Chlor, Trifluormethyl, Trifluormethoxy, (C₁-C₄)-Alkoxy, (C₃-C₇)-Cycloalkyl, (C₃-C₇)-Cycloalkoxy, (C₁-C₄)-Alkylsulfanyl oder (C₁-C₄)-Alkylsulfonyl substituiert sein kann,
und
wobei (C₂-C₄)-Alkenyl und (C₂-C₄)-Alkinyl mit 1 oder 2 Substituenten unabhängig voneinander ausgewählt aus der Gruppe Fluor, Trifluormethyl, (C₁-C₄)-Alkyl, Trifluormethoxy und (C₁-C₄)-Alkoxy substituiert sein können,
und
wobei (C₃-C₇)-Cycloalkyl mit 1 oder 2 Substituenten unabhängig voneinander ausgewählt aus der Gruppe Fluor, Chlor, Trifluormethyl, (C₁-C₄)-Alkyl, Trifluormethoxy und (C₁-C₄)-Alkoxy substituiert sein kann,
oder
R¹ und R² zusammen mit dem Stickstoffatom, an das sie gebunden sind, einen 4- bis 7-gliedrigen Heterocyclus, der ein weiteres Heteroatom aus der Reihe N, O und S enthalten kann, bilden,
wobei der 4- bis 7-gliedrige Heterocyclus mit 1 oder 2 Substituenten unabhängig voneinander ausgewählt aus der Gruppe Fluor, Chlor, Oxo, Trifluormethyl, (C₁-C₄)-Alkyl, Trifluormethoxy und (C₁-C₄)-Alkoxy substituiert sein kann,
R³ für Wasserstoff oder eine Gruppe der Formel 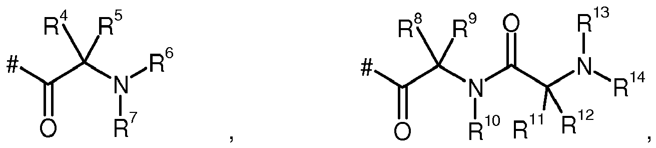 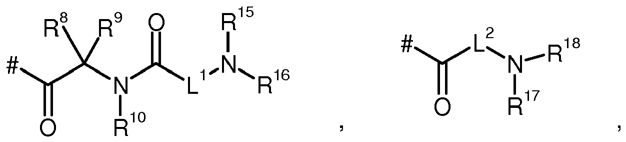 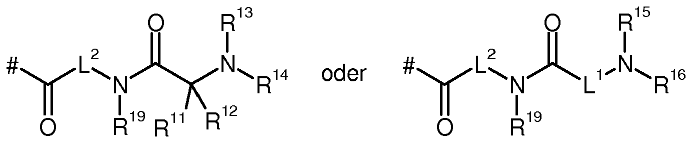 steht, wobei
# für die Anknüpfstelle an das Sauerstoffatom steht,
L¹ für (C₂-C₆)-Alkandiyl steht,
L² für (C₂-C₆)-Alkandiyl steht,
R⁴ für Wasserstoff oder die Seitengruppe einer natürlichen α-Aminosäure oder ihrer Homologe oder Isomere steht,
R⁵ für Wasserstoff oder Methyl steht,
R⁶ für Wasserstoff oder (C₁-C₄)-Alkyl steht,
R⁷ für Wasserstoff oder (C₁-C₄)-Alkyl steht,
oder
R⁶ und R⁷ zusammen mit dem Stickstoffatom, an das sie gebunden sind, einen 5- oder 6-gliedrigen Heterocyclus bilden,
wobei der 5- oder 6-gliedrige Heterocyclus mit 1 oder 2 Substituenten unäbhangig voneinander ausgewählt aus der Gruppe (C₁-C₄)-Alkyl, Amino, Hydroxy und (C₁-C₄)-Alkoxy substituiert sein kann,
oder
R⁷ zusammen mit R⁴ und den Atomen, an die sie jeweils gebunden sind, einen Pyrrolidin- oder Piperidinring bildet,
R⁸ für Wasserstoff oder die Seitengruppe einer natürlichen α-Aminosäure oder ihrer Homologe oder Isomere steht,
R⁹ für Wasserstoff oder Methyl steht,
R¹⁰ für Wasserstoff oder Methyl steht,
R¹¹ für Wasserstoff oder die Seitengruppe einer natürlichen α-Aminosäure oder ihrer Homologe oder Isomere steht,
R¹² für Wasserstoff oder Methyl steht,
R¹³ für Wasserstoff oder (C₁-C₄)-Alkyl steht,
R¹⁴ für Wasserstoff oder (C₁-C₄)-Alkyl steht,
oder
R¹³ und R¹⁴ zusammen mit dem Stickstoffatom, an das sie gebunden sind, einen 5- oder 6-gliedrigen Heterocyclus bilden,
wobei der 5- oder 6-gliedrige Heterocyclus mit 1 oder 2 Substituenten unäbhangig voneinander ausgewählt aus der Gruppe (C₁-C₄)-Alkyl, Amino, Hydroxy und (C₁-C₄)-Alkoxy substituiert sein kann,
oder
R¹⁴ zusammen mit R¹¹ und den Atomen, an die sie jeweils gebunden sind, einen Pyrrolidin- oder Piperidinring bildet,
R¹⁵ für Wasserstoff oder (C₁-C₄)-Alkyl steht,
R¹⁶ für Wasserstoff oder (C₁-C₄)-Alkyl steht,
oder
R¹⁵ und R¹⁶ zusammen mit dem Stickstoffatom, an das sie gebunden sind, einen 5- oder 6-gliedrigen Heterocyclus bilden,
wobei der 5- oder 6-gliedrige Heterocyclus mit 1 oder 2 Substituenten unäbhangig voneinander ausgewählt aus der Gruppe (C₁-C₄)-Alkyl, Amino, Hydroxy und (C₁-C₄)-Alkoxy substituiert sein kann,
R¹⁷ für Wasserstoff oder (C₁-C₄)-Alkyl steht,
R¹⁸ für Wasserstoff oder (C₁-C₄)-Alkyl steht,
oder
R¹⁷ und R¹⁸ zusammen mit dem Stickstoffatom, an das sie gebunden sind, einen 5- oder 6-gliedrigen Heterocyclus bilden,
wobei der 5- oder 6-gliedrige Heterocyclus mit 1 oder 2 Substituenten unäbhangig voneinander ausgewählt aus der Gruppe (C₁-C₄)-Alkyl, Amino, Hydroxy und (C₁-C₄)-Alkoxy substituiert sein kann,
R¹⁹ für Wasserstoff oder Methyl steht,
sowie ihre Salze und Solvate.

2. Verbindungen der Formel (I) nach Anspruch 1, in welcher
R¹ für Methyl oder Ethyl steht,
R² für (C₁-C₃)-Alkyl, Cyclopropyl oder Cyclobutyl steht,
wobei (C₁-C₃)-Alkyl mit 1 oder 2 Substituenten unabhängig voneinander ausgewählt aus der Gruppe Fluor, Chlor, Trifluormethyl, Methoxy, Ethoxy, Cyclopropyl und Cyclobutyl substituiert sein kann,
oder
R¹ und R² zusammen mit dem Stickstoffatom, an das sie gebunden sind, einen 4- bis 6-gliedrigen Heterocyclus, der ein weiteres Heteroatom aus der Reihe N, O und S enthalten kann, bilden,
wobei der 4- bis 6-gliedrige Heterocyclus mit 1 oder 2 Substituenten unabhängig voneinander ausgewählt aus der Gruppe Fluor, Trifluormethyl, Methyl, Ethyl, Methoxy und Ethoxy substituiert sein kann,
R³ für Wasserstoff oder eine Gruppe der Formel 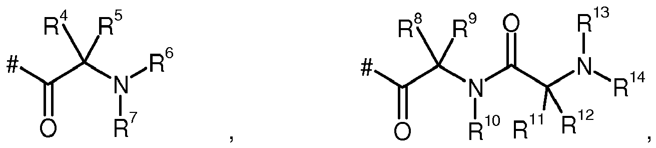 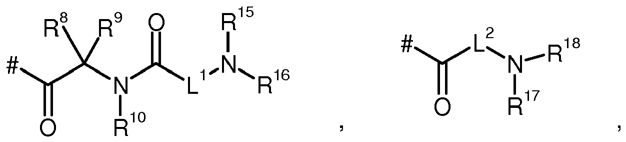 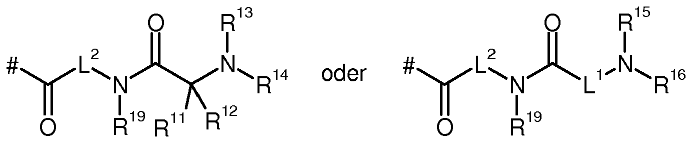 steht, wobei
# für die Anknüpfstelle an das Sauerstoffatom steht,
L¹ für Ethan-1,2-diyl steht,
L² für Ethan-1,2-diyl steht,
R⁴ für Methyl oder 3-Aminopropan-1-yl steht,
R⁵ für Wasserstoff steht,
R⁶ für Wasserstoff steht,
R⁷ für Wasserstoff steht,
R⁸ für Methyl oder 2-Methylpropan-1-yl steht,
R⁹ für Wasserstoff steht,
R¹⁰ für Wasserstoff steht,
R¹¹ für Methyl, 1-Methylpropan-1-yl, Imidazol-4-ylmethyl, 4-Aminobutan-1-yl, 3-Aminopropan-1-yl, 2-Aminoethyl, Aminomethyl oder 3-Guanidinopropan-1-yl steht,
R¹² für Wasserstoff steht,
R¹³ für Wasserstoff steht,
R¹⁴ für Wasserstoff steht,
oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
R¹⁷ für Wasserstoff steht,
R¹⁸ für Wasserstoff steht,
R¹⁹ für Wasserstoff steht,
sowie ihre Salze, Solvate und Solvate der Salze.

3. Verbindungen der Formel (I) nach Anspruch 1 oder 2, in welcher
R¹ für Methyl oder Ethyl steht,
R² für Methyl, Ethyl oder n-Propyl steht,
wobei Methyl, Ethyl und n-Propyl mit 1 oder 2 Substituenten unabhängig voneinander ausgewählt aus der Gruppe Fluor, Trifluormethyl und Methoxy substituiert sein kann,
oder
R¹ und R² zusammen mit dem Stickstoffatom, an das sie gebunden sind, einen Azetidinyl-, Pyrrolidinyl- oder Piperidinylring bilden,
wobei der Azetidinyl- und Piperidinylring mit einem Substituenten Methoxy substituiert sein können,
R³ für Wasserstoff steht,
sowie ihre Salze, Solvate und Solvate der Salze.

4. Verbindungender Formel (I) nach Anspruch 1 oder 2, in welcher
R¹ und R² zusammen mit dem Stickstoffatom, an das sie gebunden sind, einen Azetidinyl-, Pyrrolidinyl- oder Piperidinylring bilden,
wobei der Azetidinyl- und Piperidinylring mit einem Substituenten Methoxy substituiert sein können,
R³ für eine Gruppe der Formel 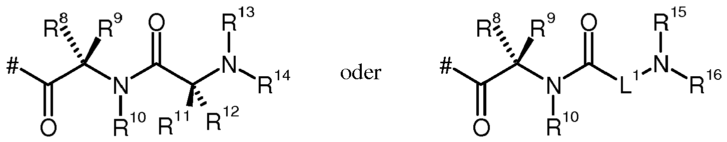 steht, wobei
# für die Anknüpfstelle an das Sauerstoffatom steht,
L¹ für Ethan-1,2-diyl steht,
R⁸ für Methyl oder iso-Butyl steht,
R⁹ für Wasserstoff steht,
R¹⁰ für Wasserstoff steht,
R¹¹ für Wasserstoff, Methyl, 1-Methylpropan-1-yl, 4-Aminobutan-1-yl oder 3-Guanidinopropan-1-yl steht,
R¹² für Wasserstoff steht,
R¹³ für Wasserstoff steht,
R¹⁴ für Wasserstoff steht,
oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
sowie ihre Salze, Solvate und Solvate der Salze.

5. Verbindungender Formel (I) nach Anspruch 1, in welcher
R¹ und R² zusammen mit dem Stickstoffatom, an das sie gebunden sind, einen Pyrrolidinylring bilden,
R³ für eine Gruppe der Formel 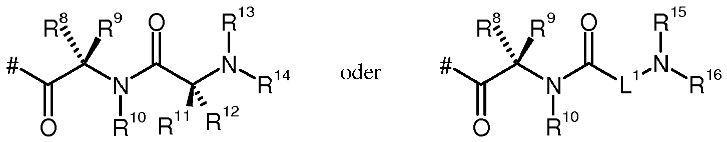 steht, wobei
# für die Anknüpfstelle an das Sauerstoffatom steht,
L¹ für Ethan-1,2-diyl steht,
R⁸ für Methyl oder iso-Butyl steht,
R⁹ für Wasserstoff steht,
R¹⁰ für Wasserstoff steht,
R¹¹ für Wasserstoff, Methyl, 1-Methylpropan-1-yl, 4-Aminobutan-1-yl oder 3-Guanidinopropan-1-yl steht,
R¹² für Wasserstoff steht,
R¹³ für Wasserstoff steht,
R¹⁴ für Wasserstoff steht,
oder
R¹⁴ zusammen mit R¹¹ und den Atomen, an die sie jeweils gebunden sind, einen Pyrrolidinring bildet,
R¹⁵ für Wasserstoff steht,
R¹⁶ für Wasserstoff steht,
sowie ihre Salze, Solvate und Solvate der Salze.

6. Verbindungender Formel (I) nach Anspruche 1, in welcher
R¹ und R² zusammen mit dem Stickstoffatom, an das sie gebunden sind, einen Azetidinyl-, Pyrrolidinyl- oder Piperidinylring bilden,
sowie ihre Salze, Solvate und Solvate der Salze.

7. Verbindungender Formel (I) nach Anspruch 1, in welcher
R¹ und R² zusammen mit dem Stickstoffatom, an das sie gebunden sind, einen Pyrrolidinyl- ring bilden,
R³ für eine Gruppe der Formel 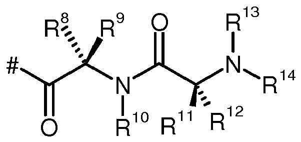 steht, wobei
# für die Anknüpfstelle an das Sauerstoffatom steht,
R⁸ für Methyl steht,
R⁹ für Wasserstoff steht,
R¹⁰ für Wasserstoff steht,
R¹¹ für Methyl oder 1-Methylpropan-1-yl steht,
R¹² für Wasserstoff steht,
R¹³ für Wasserstoff steht,
R¹⁴ für Wasserstoff steht,
sowie ihre Salze, Solvate und Solvate der Salze.

8. Verbindung nach Anspruch 1, wobei die Verbindung folgende Struktur hat 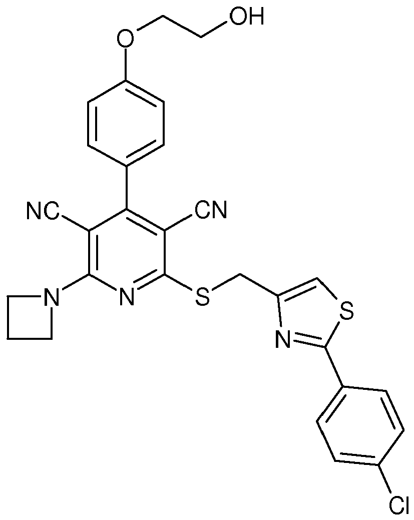 2-(Azetidin-1-yl)-6-({[2-(4-chlorphenyl)-1,3-thiazol-4-yl]methyl}sulfanyl)-4-[4-(2-hydroxyethoxy)phenyl]pyridin-3,5-dicarbonitril sowie Salze und/oder Solvate davon.

9. Verbindung nach Anspruch 1, wobei die Verbindung folgende Struktur hat 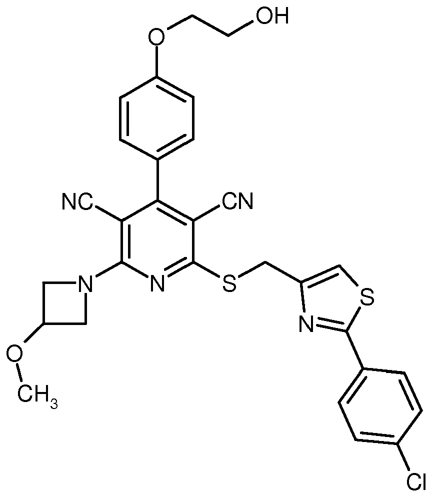 2-((3-Methoxy)-Azetidin-1-yl)-6-({[2-(4-chlorphenyl)-1,3-thiazol-4-yl]methyl}sulfanyl)-4-[4-(2-hydroxyethoxy)phenyl]pyridin-3,5-dicarbonitril
sowie Salze und/oder Solvate davon.

10. Verbindung nach Anspruch 1, wobei die Verbindung folgende Struktur hat 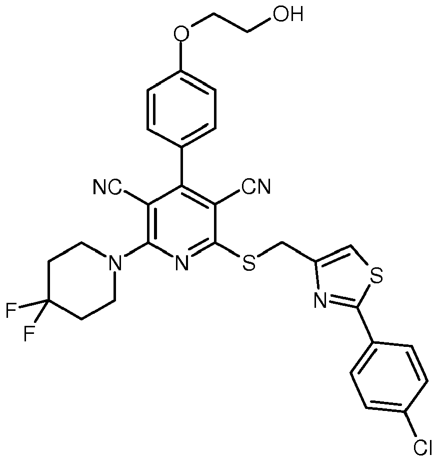 2-({[2-(4-Chlorphenyl)-1,3-thiazol-4-yl]methyl}sulfanyl)-6-(4,4-difluoropiperidin-1-yl)-4-[4-(2-hydroxyethoxy)phenyl]pyridin-3,5-dicarbonitril
sowie Salze und/oder Solvate davon.

11. Verbindung nach Anspruch 1, wobei die Verbindung folgende Struktur hat 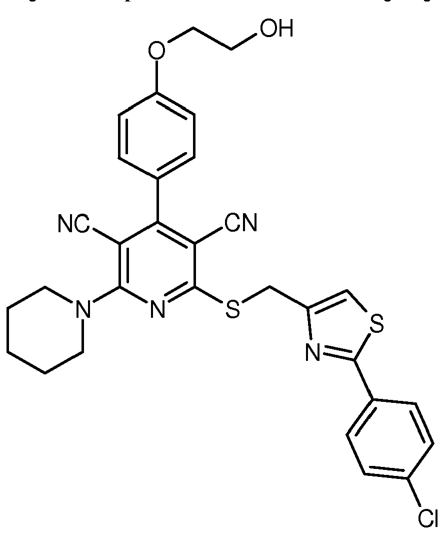 2-({[2-(4-Chlorphenyl)-1,3-thiazol-4-yl]methyl}sulfanyl)-4-[4-(2-hydroxyethoxy)phenyl]-6-(piperidin-1-yl)pyridin-3,5-dicarbonitril
sowie Salze und/oder Solvate davon.

12. Verbindung nach Anspruch 1, wobei die Verbindung folgende Struktur hat 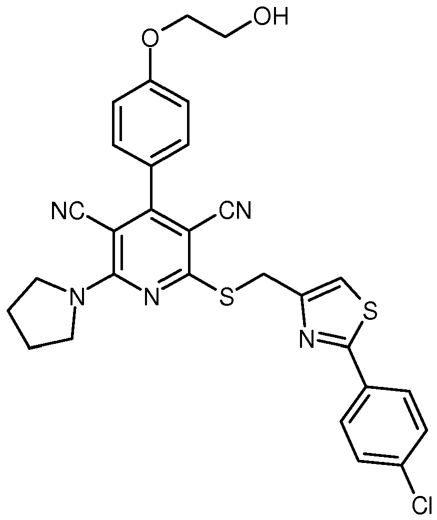 2-({[2-(4-Chlorphenyl)-1,3-thiazol-4-yl]methyl}sulfanyl)-4-[4-(2-hydroxyethoxy)phenyl]-6-(pyrrolidin-1-yl)pyridin-3,5-dicarbonitril
sowie Salze und/oder Solvate davon.

13. Verbindung nach Anspruch 1, wobei die Verbindung folgende Struktur hat 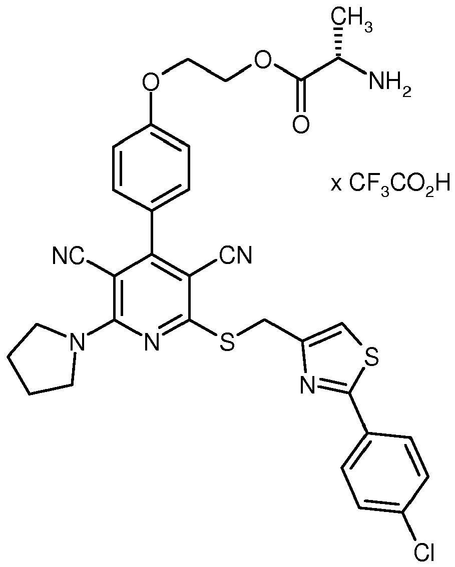 2-{4-[2-({ [2-(4-Chlorphenyl)-1,3-thiazol-4-yl]methyl}sulfanyl)-3,5-dicyano-6-(pyrrolidin-1-yl)pyridin-4-yl]phenoxy}ethyl L-alaninat trifluoroacetat
sowie Solvate davon.

14. Verbindung nach Anspruch 1, wobei die Verbindung folgende Struktur hat 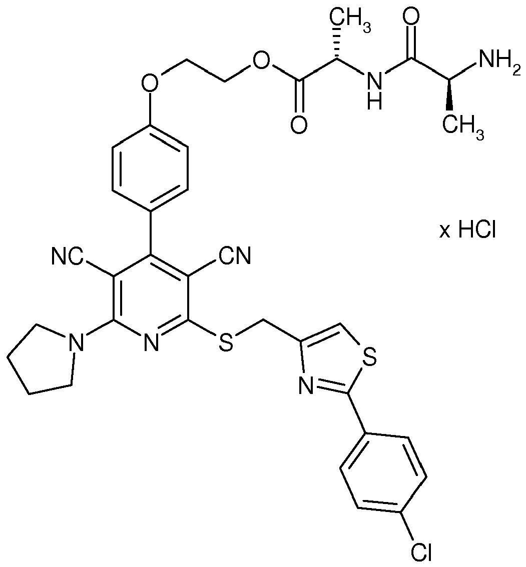 2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl L-alanyl-L-alaninate Hydrochlorid
sowie Solvate davon.

15. Verbindung nach Anspruch 1, wobei die Verbindung folgende Struktur hat 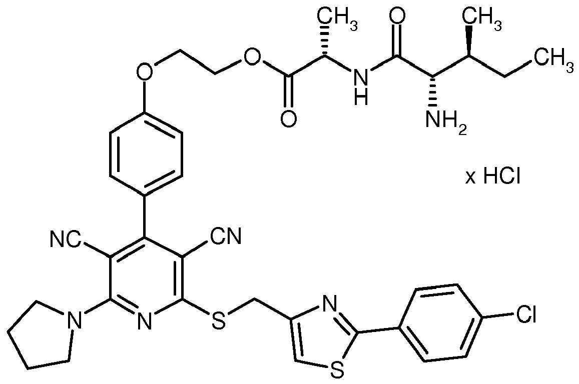 2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl L-isoleucyl-L-alaninat Hydrochlorid
sowie Solvate davon.

16. Verbindung nach Anspruch 1, wobei die Verbindung folgende Struktur hat 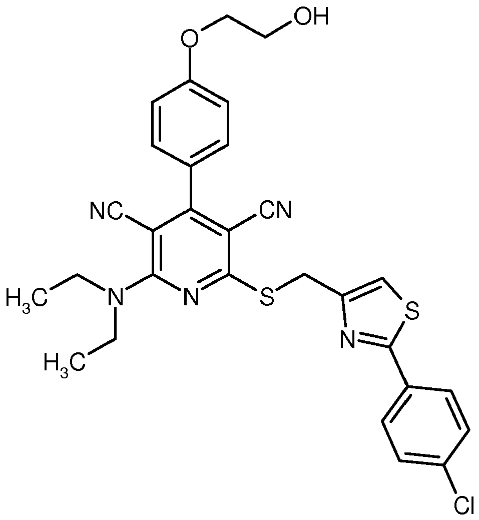 2-({[2-(4-Chlorophenyl)-1,3-thiazol-4-yl]methyl}sulfanyl)-6-(diethylamino)-4-[4-(2-hydroxyethoxy)phenyl]pyridin-3,5-dicarbonitril
sowie Salze und/oder Solvate davon.

17. Verbindung nach Anspruch 1, wobei die Verbindung folgende Struktur hat 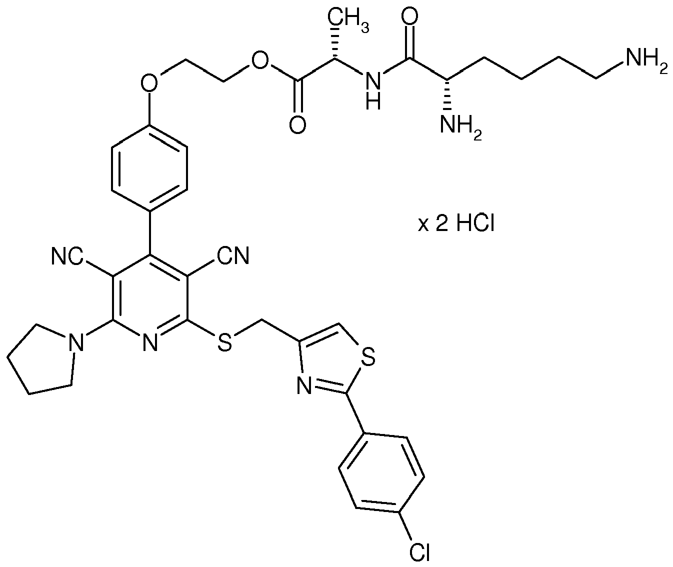 2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl L-lysyl-beta-alaninat Dihydrochlorid
sowie Salze und/oder Solvate davon.

18. Verbindung nach Anspruch 1, wobei die Verbindung folgende Struktur hat 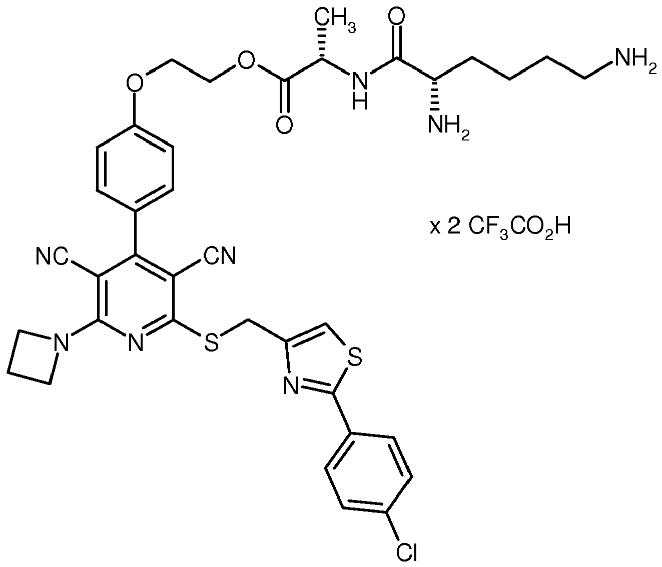 2-{4-[2-(Azetidin-1-yl)-6-({[2-(4-chlorphenyl)-1,3-thiazol-4-yl]methyl}sulfanyl)-3,5-dicyanopyridin-4-yl]phenoxy}ethyl L-lysyl-L-alaninat bis(Trifluoroacetat)
sowie Solvate davon.

19. Verbindung nach Anspruch 1, wobei die Verbindung folgende Struktur hat 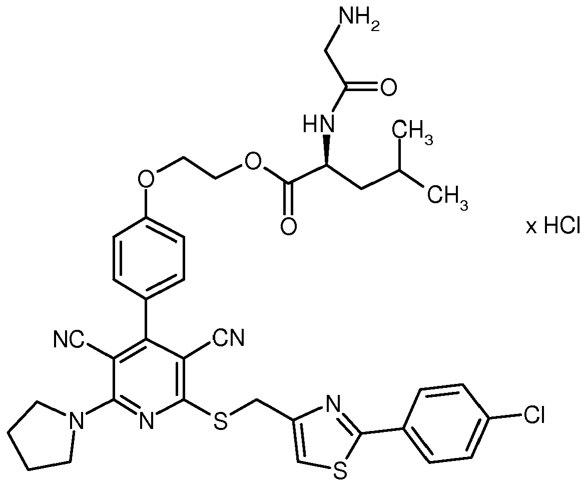 2-{4-[2-({[2-(4-Chlorphenyl)-1,3-thiazol-4-yl]methyl}sulfanyl)-3,5-dicyano-6-(pyrrolidin-1-yl)pyridin-4-yl]phenoxy}ethyl Glycyl-L-leucinat Hydrochlorid
sowie Solvate davon.

20. Verbindung nach Anspruch 1, wobei die Verbindung folgende Struktur hat 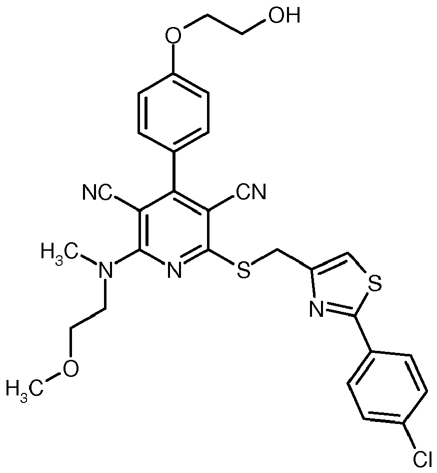 2-({[2-(4-Chlorphenyl)-1,3-thiazol-4-yl]methyl}sulfanyl)-4-[4-(2-hydroxyethoxy)phenyl]-6-[(2-methoxyethyl)(methyl)amino]pyridin-3,5-dicarbonitril
sowie Salze und/oder Solvate davon.

21. Verfahren zur Herstellung von Verbindungen der Formel (I), wie in den Ansprüchen 1 bis 3 definiert, in welcher R³ für Wasserstoff steht, **dadurch gekennzeichnet, dass** man die Verbindung der Formel (II) 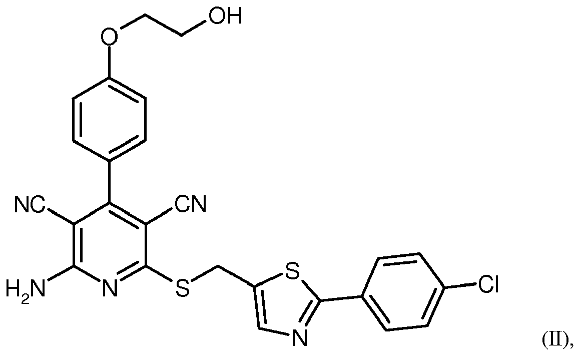 zunächst mit Kupfer(II)chlorid und Isoamylnitrit in einem geeigneten Lösungsmittel in die Verbindung der Formel (III) 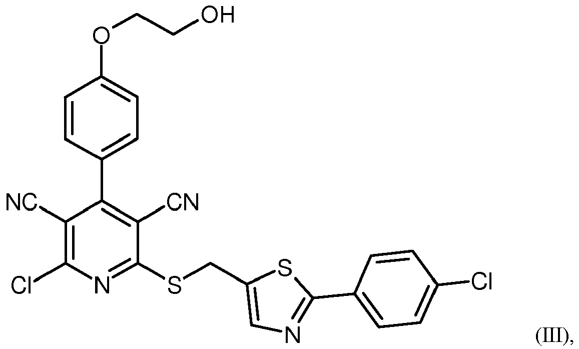 überführt, und diese anschliessend in einem inerten Lösungsmittel gegebenenfalls in Gegenwart einer geeigneten Base mit einer Verbindung der Formel (IV) 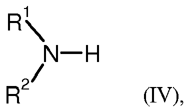 in welcher R¹ und R² jeweils die in den Ansprüchen 1 bis 3 angegebenen Bedeutungen haben, zu einer Verbindung der Formel (I-A) 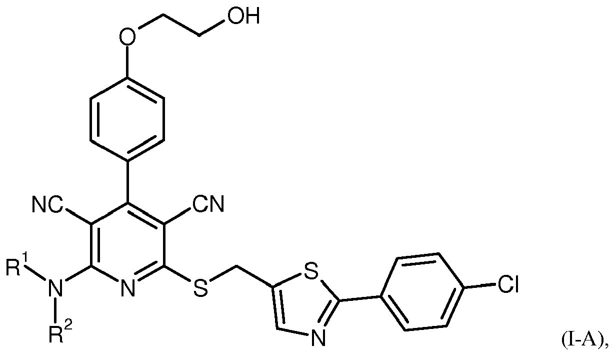 in welcher R¹ und R² jeweils die in den Ansprüchen 1 bis 3 angegebenen Bedeutungen haben, umsetzt, anschließend gegebenenfalls vorhandene Schutzgruppen abspaltet und die resultierenden Verbindungen der Formel (I) gegebenenfalls mit den entsprechenden (i) Lösungsmitteln und/oder (*ii*) Basen oder Säuren in ihre Solvate, Salze und/oder Solvate der Salze überführt.

22. Verfahren zur Herstellung von Verbindungen der Formel (I), wie in den Ansprüchen 1, 2, 4, 5, 6 und 7 definiert, in welcher R³ für eine Gruppe der Formel 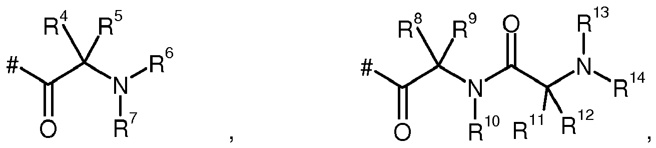 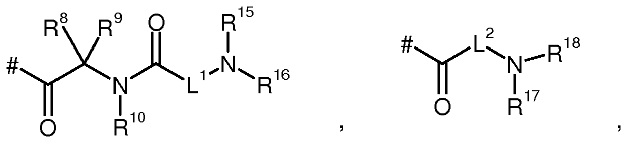 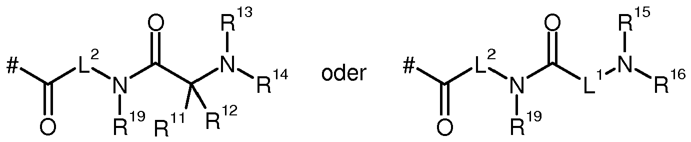 steht, wobei L¹, L², R⁴, R⁵, R⁶, R⁷, R⁸, R⁹, R¹⁰, R¹¹, R¹², R¹³, R¹⁴, R¹⁵, R¹⁶, R¹⁷, R¹⁸ und R¹⁹ jeweils die in den Ansprüchen 1, 2, 4, 5, 6 und 7 angegebenen Bedeutungen haben, **dadurch gekennzeichnet, dass** man
[A] eine Verbindung der Formel (I-A) 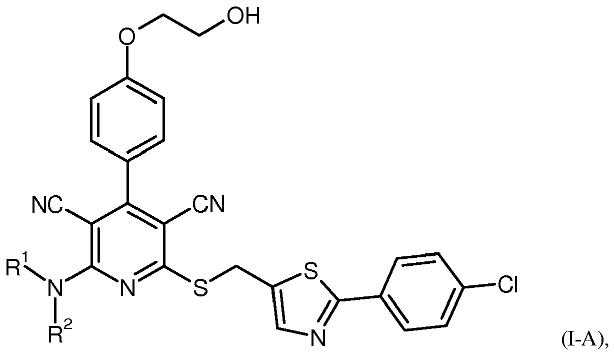 in welcher R¹ und R² jeweils die in den Ansprüchen 1, 2, 4, 5, 6 und 7 angegebenen Bedeutungen haben,
in einem inerten Lösungsmittel in Gegenwart eines Kondensationsmittels zunächst mit einer Carbonsäure der Formel (V) oder (VI) 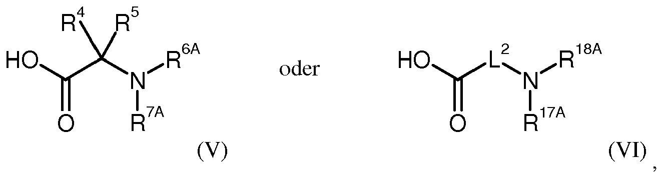 in welchen L², R⁴ und R⁵ jeweils die in den Ansprüchen 1, 2, 4, 5, 6 und 7 angegebenen Bedeutungen haben und
R^{6A}, R^{7A}, R^{17A} und R^{18A} jeweils die für R⁶, R⁷, R¹⁷ bzw. R¹⁸ genannten Bedeutungen haben oder für eine Amino-Schutzgruppe, wie beispielsweise tert.-Butoxycarbonyl, stehen,
zu einer Verbindungen der Formel (VII) oder (VIII) 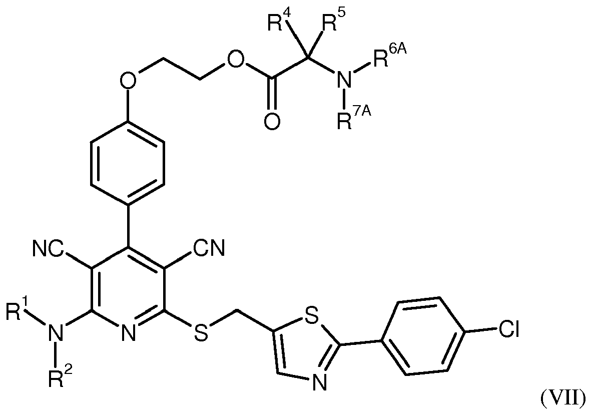 oder 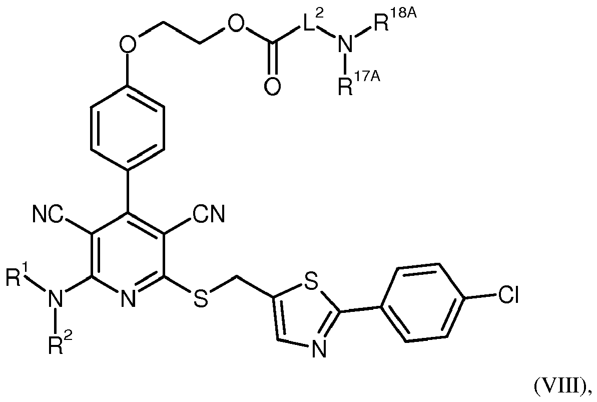 in welchen L², R¹, R², R⁴, R⁵, R^{6A}, R^{7A}, R^{17A} und R^{18A} jeweils die zuvor angegebenen Bedeutungen haben,
kuppelt und anschliessend gegebenfalls vorhandene Schutzgruppen zu einer Verbindung der Formel (I-B) oder (I-C) 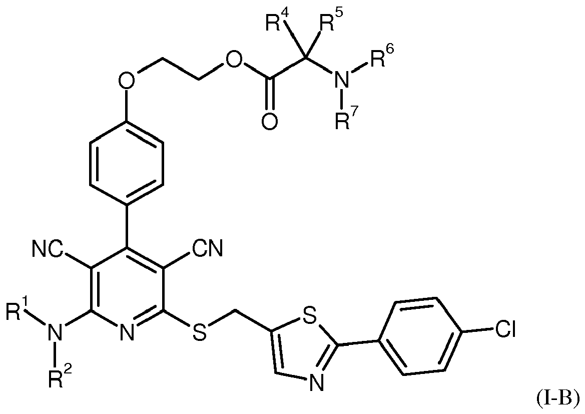 oder 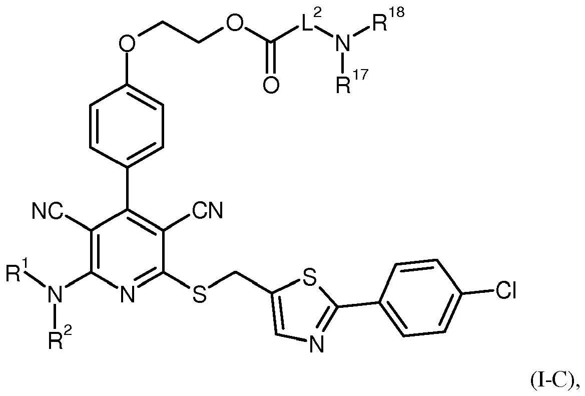 in welchen L², R¹, R², R⁴, R⁵, R⁶, R⁷, R¹⁷ und R¹⁸ jeweils die in den Ansprüchen 1, 2, 4, 5, 6 und 7 angegebenen Bedeutungen haben,
abspaltet,
oder
[B] eine Verbindung der Formel (I-A) in einem inerten Lösungsmittel in Gegenwart eines Kondensationsmittels zunächst mit einer Carbonsäure der Formel (IX), (X), (XI) oder (XII) 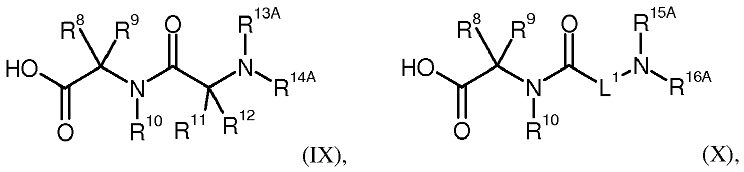 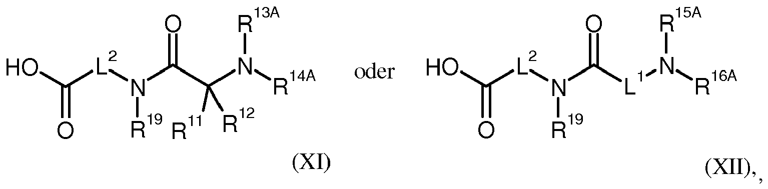 in welchen L¹, L², R⁸, R⁹, R¹⁰, R¹¹, R¹² und R¹⁹ jeweils die in den Ansprüchen 1, 2, 4, 5, 6 und 7 angegebenen Bedeutungen haben
und
R^{13A}, R^{14A}, R^{15A} und R^{16A} jeweils die für R¹³, R¹⁴, R¹⁵ bzw. R¹⁶ genannten Bedeutungen haben oder für eine Amino-Schutzgruppe, wie beispielsweise tert.-Butoxycarbonyl, stehen,
zu Verbindungen der Formel (XIII), (XIV), (XV) oder (XVI) 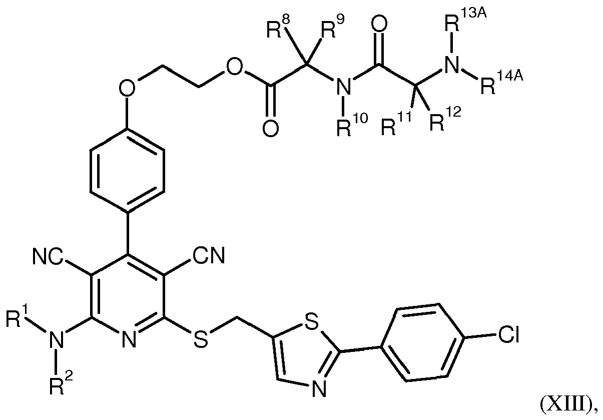 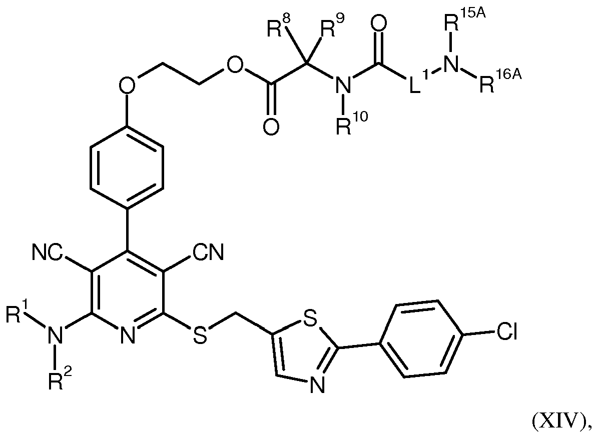 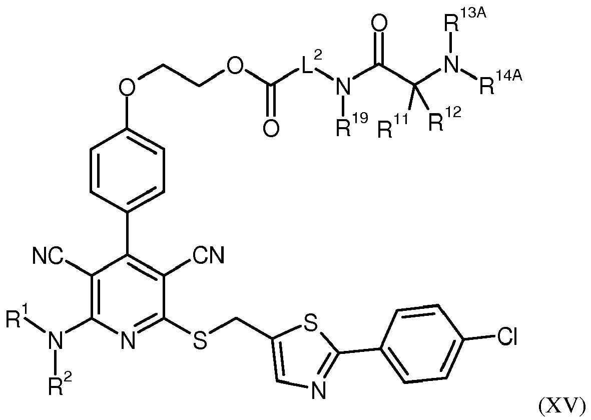 oder 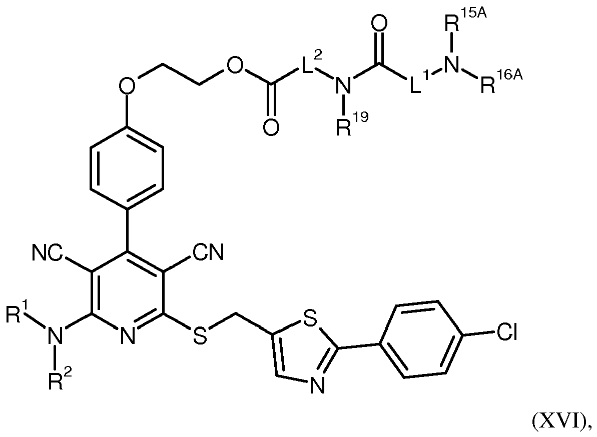 in welchen L¹, L², R¹, R², R⁸, R⁹, R¹⁰, R¹¹, R¹², R^{13A}, R^{14A}, R^{15A}, R^{16A} und R¹⁹ jeweils die zuvor angegebenen Bedeutungen haben,
kuppelt und anschliessend gegebenfalls vorhandene Schutzgruppen zu einer Verbindung der Formel (I-D), (I-E), (I-F) oder (I-G) 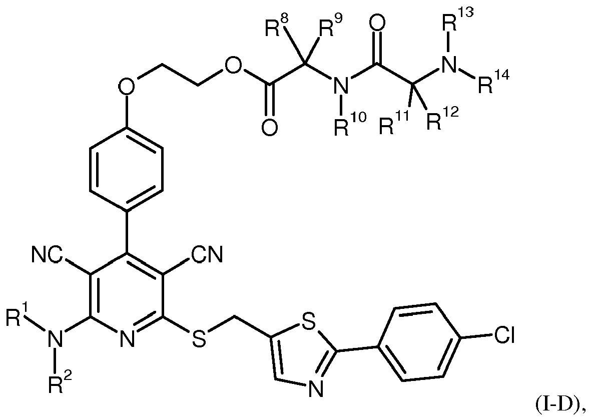 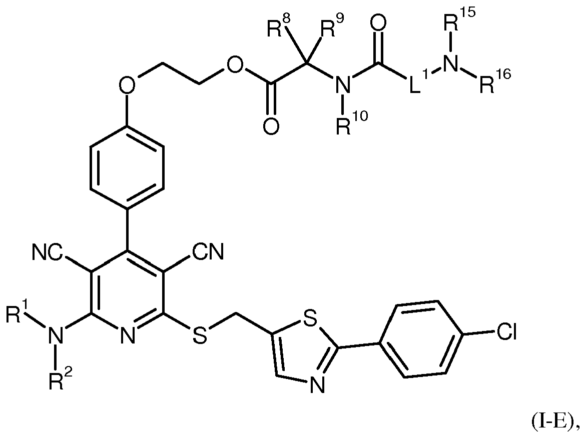 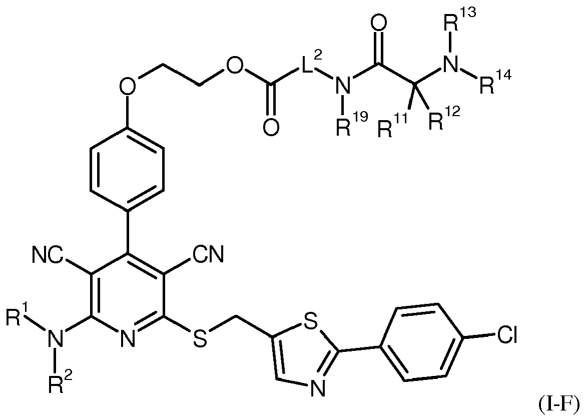 oder 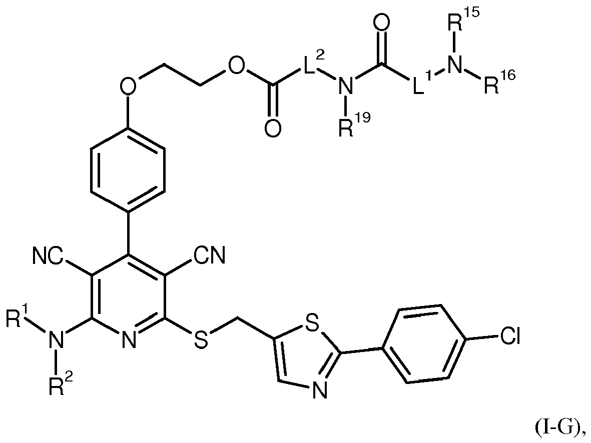 in welchen L¹, L², R¹, R², R⁸, R⁹, R¹⁰, R¹¹, R¹², R¹³, R¹⁴, R¹⁵, R¹⁶ und R¹⁹ jeweils die in den Ansprüchen 1, 2, 4, 5, 6 und 7 angegebenen Bedeutungen haben,
abspaltet,
oder
[C] von einer Verbindung der Formel (VII-1) oder (VIII-1), 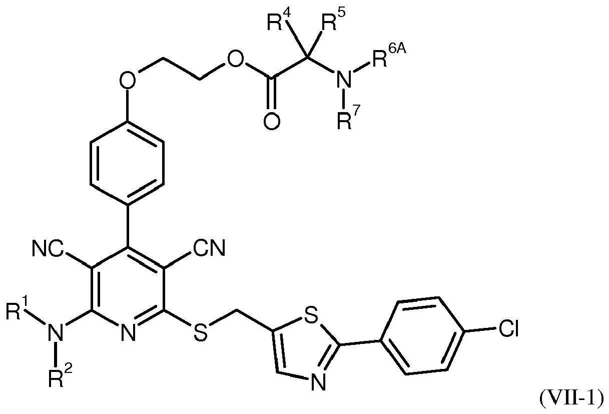 oder 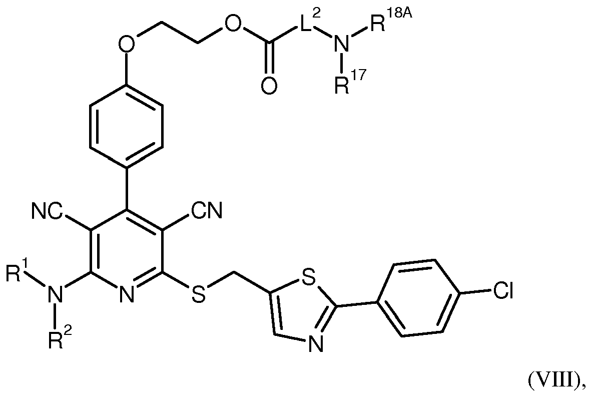 in welchen L², R¹, R², R⁴, R⁵, R⁷ und R¹⁷ jeweils die in den Ansprüchen 1, 2, 4, 5, 6 und 7 angegebenen Bedeutungen haben,
und
R^{6A} und R^{18A} für eine Amino-Schutzgruppe, beispielsweise tert.-Butoxycarbonyl steht,
die Amino-Schutzgruppe nach Standardmethoden zu einer Verbindung der Formel (I-B-1) oder (I-C-1) 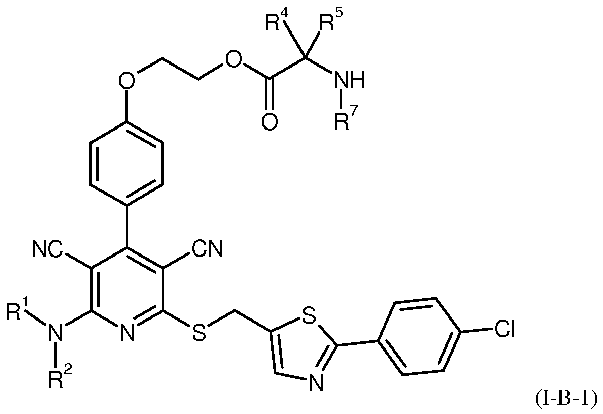 oder 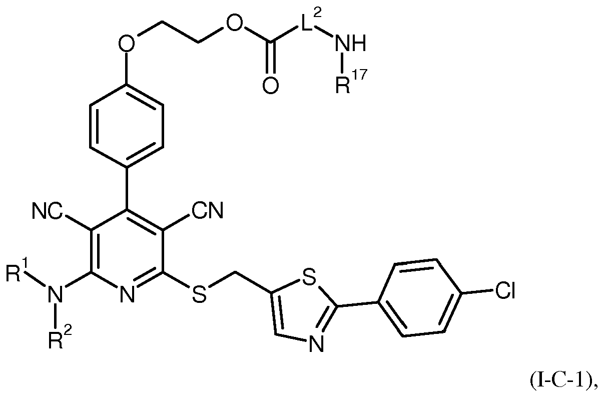 in welchen L², R¹, R², R⁴, R⁵, R⁷ und R¹⁷ jeweils die in den Ansprüchen 1, 2, 4, 5, 6 und 7 angegebenen Bedeutungen haben,
abspaltet, und diese in einem inerten Lösungsmittel in Gegenwart eines Kondensationsmittels zunächst mit einer Carbonsäure der Formel (XVII) oder (XVIII) 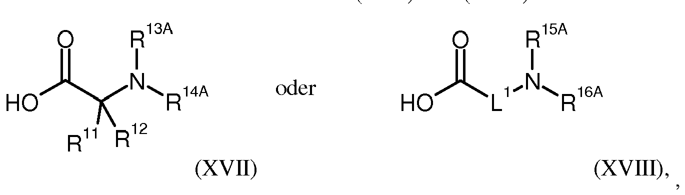 in welchen L¹, R¹¹ und R¹² jeweils die in den Ansprüchen 1, 2, 4, 5, 6 und 7 angegebenen Bedeutungen haben
und
R^{13A}, R^{14A}, R^{15A} und R^{16A} jeweils die für R¹³, R¹⁴, R¹⁵ bzw. R¹⁶ genannten Bedeutungen haben oder für eine Amino-Schutzgruppe, wie beispielsweise tert.-Butoxycarbonyl, stehen,
zu Verbindungen der Formel (XIII), (XIV), (XV) oder (XVI) kuppelt und anschliessend gegebenfalls vorhandene Schutzgruppen wieder zu den resultierenden Verbindungen (I-D), (I-E), (I-F) oder (I-G) abspaltet,
und die resultierenden Verbindungen der Formel (I-B), (I-C), (I-D), (I-E), (I-F) und (I-G) gegebenenfalls mit den entsprechenden (i) Lösungsmitteln und/oder (*ii*) Basen oder Säuren in ihre Solvate, Salze und/oder Solvate der Salze überführt.

23. Agonisten des Adenosin A1-Rezeptors der Formel (I), wie in einem der Ansprüche 1 bis 20 definiert, zur Verwendung in der Behandlung und/oder Prävention von Krankheiten.

24. Agonisten des Adenosin A1-Rezeptors der Formel (I), wie in einem der Ansprüche 1 bis 20 definiert, zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

25. Agonisten des Adenosin A1-Rezeptors der Formel (I), wie in einem der Ansprüche 1 bis 20 definiert, zur Verwendung in einem Verfahren zur Behandlung und/oder Prophylaxe von Diabetes, Metabolischem Syndrom und Dyslipidämien.

26. Agonisten des Adenosin A1-Rezeptors der Formel (I), gemäss Anspruch 1, zur Verwendung in einem Verfahren zur Behandlung und/oder Prophylaxe von Nierenkrankheiten.

27. Verwendung eines Agonisten des Adenosin A1-Rezeptors der Formel (I), wie in einem der Ansprüche 1 bis 20 definiert, zur Herstellung eines Arzneimittels zur Behandlung und/oder Prävention von koronarer Herzerkrankung, akutem Koronarsyndrom, Angina pectoris, Herzinsuffizienz, Myokardinfarkt und Vorhofflimmern.

28. Verwendung eines Agonisten des Adenosin A1-Rezeptors der Formel (I), wie in einem der Ansprüche 1 bis 20 definiert, zur Herstellung eines Arzneimittels zur Behandlung und/oder Prävention von akuten als auch chronischen Erscheinungsformen der Herzinsuffizienz, dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappen-insuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische sowie systolische Herzinsuffizienz und akute Phasen der Verschlechterung einer bestehenden chronischen Herzinsuffizienz (worsening heart failure).

29. Verwendung eines Agonisten des Adenosin A1-Rezeptors der Formel (I), wie in Anspruch 1 definiert, zur Herstellung eines Arzneimittels zur Behandlung und/oder Prävention von Nierenkrankheiten.

30. Verwendung eines Agonisten des Adenosin A1-Rezeptors der Formel (I), wie in einem der Ansprüche 1 bis 20 definiert, zur Herstellung eines Arzneimittels zur Behandlung und/oder Prävention von Diabetes, Metabolischem Syndrom und Dyslipidämien.

31. Arzneimittel enthaltend einen Agonisten des Adenosin A1-Rezeptors der Formel (I), wie in einem der Ansprüche 1 bis 20 definiert, in Kombination mit einem inerten, nicht-toxischen, pharmazeutisch geeigneten Hilfsstoff.

32. Arzneimittel enthaltend einen Agonisten des Adenosin A1-Rezeptors der Formel (I), wie in einem der Ansprüche 1 bis 20 definiert, in Kombination mit einem oder mehreren weiteren Wirkstoffen ausgewählt aus der Gruppe bestehend aus den Fettstoffwechsel verändernden Wirkstoffen, Antidiabetika, blutdrucksenkenden Wirkstoffen und antithrombotisch wirkenden Mitteln.

33. Arzneimittel nach Anspruch 31 oder 32 zur Verwendung in der Behandlung und/oder Prävention von koronarer Herzerkrankung, akutem Koronarsyndrom, Angina pectoris, Herzinsuffizienz, Myokardinfarkt und Vorhofflimmern.

34. Arzneimittel nach Anspruch 31 oder 32 zur Verwendung in der Behandlung und/oder Prävention von akuten als auch chronischen Erscheinungsformen der Herzinsuffizienz, dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappen-insuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische sowie systolische Herzinsuffizienz und akute Phasen der Verschlechterung einer bestehenden chronischen Herzinsuffizienz (worsening heart failure).

35. Arzneimittel nach Anspruch 31 oder 32 zur Verwendung in der Behandlung und/oder Prävention von Diabetes, Metabolischem Syndrom und Dyslipidämien.

36. Arzneimittel nach Anspruch 31 oder 32 zur Verwendung in der Behandlung und/oder Prävention von Nierenkrankheiten.

## Claims

1. Compounds of the formula (I) 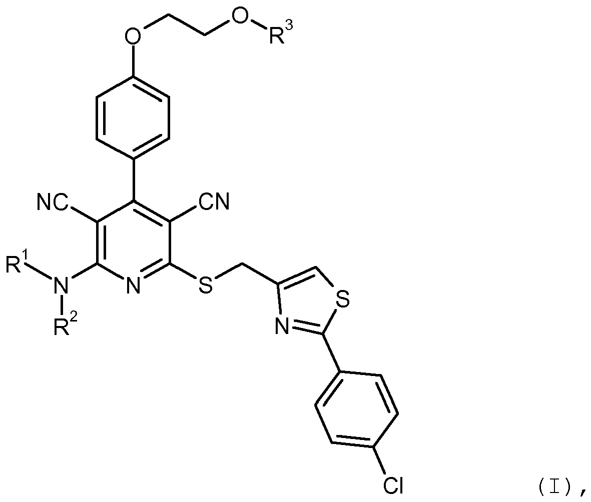 in which
R¹ represents (C₁-C₄) -alkyl,
R² represents (C₁-C₆) -alkyl, (C₂-C₄)-alkenyl, (C₂-C₄) -alkynyl or (C₃-C₇)-cycloalkyl,
where (C₁-C₆) -alkyl may be substituted by 1 to 3 substituents independently of one another selected from the group consisting of fluorine, chlorine, trifluoromethyl, trifluoromethoxy, (C₁-C₄) -alkoxy, (C₃-C₇)-cycloalkyl, (C₃-C₇)-cycloalkoxy, (C₁-C₄) - alkylsulphanyl and (C₁-C₄)-alkylsulphonyl,
and
where (C₂-C₄) -alkenyl and (C₂-C₄) -alkynyl may be substituted by 1 or 2 substituents independently of one another selected from the group consisting of fluorine, trifluoromethyl, (C₁-C₄) -alkyl, trifluoromethoxy and (C₁-C₄) -alkoxy,
and
where (C₃-C₇)-cycloalkyl may be substituted by 1 or 2 substituents independently of one another selected from the group consisting of fluorine, chlorine, trifluoromethyl, (C₁-C₄) -alkyl, trifluoromethoxy and (C₁-C₄) -alkoxy,
or
R¹ and R² together with the nitrogen atom to which they are attached form a 4- to 7-membered heterocycle which may contain a further heteroatom from the group consisting of N, O and S,
where the 4- to 7-membered heterocycle may be substituted by 1 or 2 substituents independently of one another selected from the group consisting of fluorine, chlorine, oxo, trifluoromethyl, (C₁-C₄) -alkyl, trifluoromethoxy and (C₁-C₄) -alkoxy, R³ represents hydrogen or a group of the formula 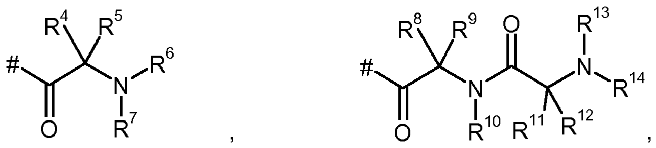 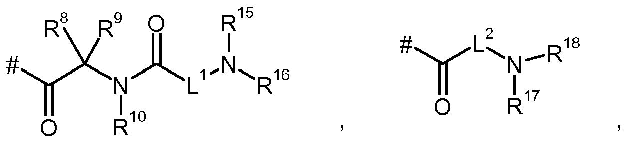 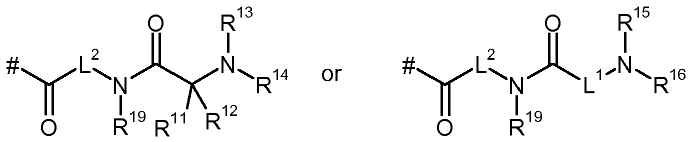 where
# represents the point of attachment to the oxygen atom,
L¹ represents (C₂-C₆) -alkanediyl,
L² represents (C₂-C₆) -alkanediyl,
R⁴ represents hydrogen or the side group of a natural α-amino acid or its homologs or isomers,
R⁵ represents hydrogen or methyl,
R⁶ represents hydrogen or (C₁-C₄)-alkyl,
R⁷ represents hydrogen or (C₁-C₄)-alkyl,
or
R⁶ and R⁷ together with the nitrogen atom to which they are attached form a 5- or 6-membered heterocycle,
where the 5- or 6-membered heterocycle may be substituted by 1 or 2 substituents independently selected from the group consisting of (C₁-C₄) - alkyl, amino, hydroxyl and (C₁-C₄)-alkoxy,
or
R⁷ together with R⁴ and the atoms, to which they are attached, forms a pyrrolidine or piperidine ring,
R⁸ represents hydrogen or the side group of a natural α-amino acid or its homologs or isomers,
R⁹ represents hydrogen or methyl,
R¹⁰ represents hydrogen or methyl,
R¹¹ represents hydrogen or the side group of a natural α-amino acid or its homologs or isomers, R¹² represents hydrogen or methyl,
R¹³ represents hydrogen or (C₁-C₄)-alkyl,
R¹⁴ represents hydrogen or (C₁-C₄)-alkyl,
or
R¹³ and R¹⁴ together with the nitrogen atom to which they are attached form a 5- or 6-membered heterocycle,
where the 5- or 6-membered heterocycle may be substituted by 1 or 2 substituents independently selected from the group consisting of (C₁-C₄) - alkyl, amino, hydroxyl and (C₁-C₄)-alkoxy,
or
R¹⁴ together with R¹¹ and the atoms, to which they are attached, forms a pyrrolidine or piperidine ring,
R¹⁵ represents hydrogen or (C₁-C₄) -alkyl,
R¹⁶ represents hydrogen or (C₁-C₄) -alkyl,
or
R¹⁵ and R¹⁶ together with the nitrogen atom to which they are attached form a 5- or 6-membered heterocycle,
where the 5- or 6-membered heterocycle may be substituted by 1 or 2 substituents independently selected from the group consisting of (C₁-C₄) - alkyl, amino, hydroxyl and (C₁-C₄)-alkoxy,
R¹⁷ represents hydrogen or (C₁-C₄)-alkyl,
R¹⁸ represents hydrogen or (C₁-C₄)-alkyl,
or
R¹⁷ and R¹⁸ together with the nitrogen atom to which they are attached form a 5- or 6-membered heterocycle,
where the 5- or 6-membered heterocycle may be substituted by 1 or 2 substituents independently selected from the group consisting of (C₁-C₄) - alkyl, amino, hydroxyl and (C₁-C₄)-alkoxy,
R¹⁹ represents hydrogen or methyl,
and the salts and solvates thereof.

2. Compounds of the formula (I) according to Claim 1 in which
R¹ represents methyl or ethyl,
R² represents (C₁-C₃)-alkyl, cyclopropyl or cyclobutyl,
where (C₁-C₃)-alkyl may be substituted by 1 or 2 substituents independently of one another selected from the group consisting of fluorine, chlorine, trifluoromethyl, methoxy, ethoxy, cyclopropyl and cyclobutyl,
or
R¹ and R² together with the nitrogen atom to which they are attached form a 4- to 6-membered heterocycle which may contain a further heteroatom from the group consisting of N, O and S,
where the 4- to 6-membered heterocycle may be substituted by 1 or 2 substituents independently of one another selected from the group consisting of fluorine, trifluoromethyl, methyl, ethyl, methoxy and ethoxy,
R³ represents hydrogen or a group of the formula 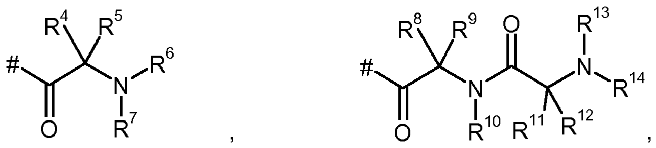 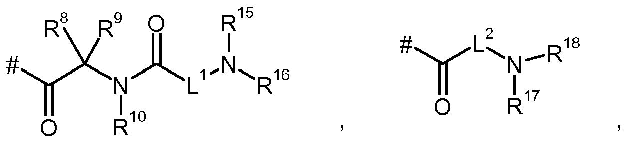 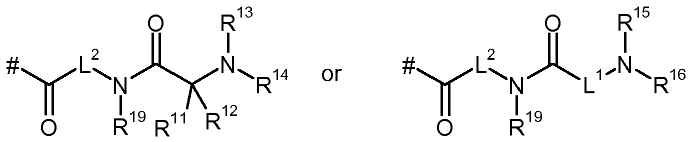 where
# represents the point of attachment to the oxygen atom,
L¹ represents ethane-1,2-diyl,
L² represents ethane-1,2-diyl,
R⁴ represents methyl or 3-aminopropan-1-yl,
R⁵ represents hydrogen,
R⁶ represents hydrogen,
R⁷ represents hydrogen,
R⁸ represents methyl or 2-methylpropan-1-yl,
R⁹ represents hydrogen,
R¹⁰ represents hydrogen,
R¹¹ represents methyl, 1-methylpropan-1-yl, imidazol-4-ylmethyl, 4-aminobutan-1-yl, 3-amino-propan-1-yl, 2-aminoethyl, aminomethyl or 3-guanidinopropan-1-yl,
R¹² represents hydrogen,
R¹³ represents hydrogen,
R¹⁴ represents hydrogen,
or
R¹⁴ together with R¹¹ and the atoms, to which they are attached, forms a pyrrolidine ring,
R¹⁵ represents hydrogen,
R¹⁶ represents hydrogen,
R¹⁷ represents hydrogen,
R¹⁸ represents hydrogen,
R¹⁹ represents hydrogen,
and the salts, solvates and solvates of the salts thereof.

3. Compounds of the formula (I) according to Claim 1 or 2 in which
R¹ represents methyl or ethyl,
R² represents methyl, ethyl or n-propyl,
where methyl, ethyl and n-propyl may be substituted by 1 or 2 substituents independently of one another selected from the group consisting of fluorine, trifluoromethyl and methoxy,
or
R¹ and R² together with the nitrogen atom to which they are attached form an azetidinyl, pyrrolidinyl or piperidinyl ring,
where the azetidinyl and piperidinyl ring may be substituted by a methoxy substituent,
R³ represents hydrogen,
and the salts, solvates and solvates of the salts thereof.

4. Compounds of the formula (I) according to Claim 1 or 2 in which
R¹ and R² together with the nitrogen atom to which they are attached form an azetidinyl, pyrrolidinyl or piperidinyl ring,
where the azetidinyl and piperidinyl ring may be substituted by a methoxy substituent,
R³ represents a group of the formula 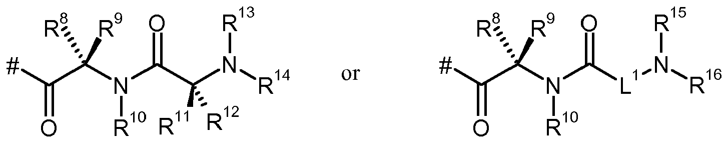 where
# represents the point of attachment to the oxygen atom,
L¹ represents ethane-1,2-diyl,
R⁸ represents methyl or isobutyl,
R⁹ represents hydrogen,
R¹⁰ represents hydrogen,
R¹¹ represents hydrogen, methyl, 1-methylpropan-1-yl, 4-aminobutan-l-yl or 3-guanidinopropan-1-yl,
R¹² represents hydrogen,
R¹³ represents hydrogen,
R¹⁴ represents hydrogen,
or
R¹⁴ together with R¹¹ and the atoms, to which they are attached, forms a pyrrolidine ring,
R¹⁵ represents hydrogen,
R¹⁶ represents hydrogen,
and the salts, solvates and solvates of the salts thereof.

5. Compounds of the formula (I) according to Claim 1 in which
R¹ and R² together with the nitrogen atom to which they are attached form a pyrrolidinyl ring,
R³ represents a group of the formula 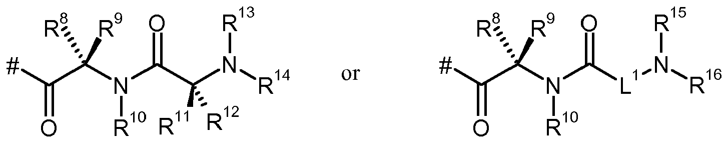 where
# represents the point of attachment to the oxygen atom,
L¹ represents ethane-1,2-diyl,
R⁸ represents methyl or isobutyl,
R⁹ represents hydrogen,
R¹⁰ represents hydrogen,
R¹¹ represents hydrogen, methyl, 1-methylpropan-1-yl, 4-aminobutan-1-yl or 3-guanidinopropan-1-yl,
R¹² represents hydrogen,
R¹³ represents hydrogen,
R¹⁴ represents hydrogen,
or
R¹⁴ together with R¹¹ and the atoms, to which they are attached, forms a pyrrolidine ring,
R¹⁵ represents hydrogen,
R¹⁶ represents hydrogen,
and the salts, solvates and solvates of the salts thereof.

6. Compounds of the formula (I) according to Claim 1 in which
R¹ and R² together with the nitrogen atom to which they are attached form an azetidinyl, pyrrolidinyl or piperidinyl ring,
and the salts, solvates and solvates of the salts thereof.

7. Compounds of the formula (I) according to Claim 1 in which
R¹ and R² together with the nitrogen atom to which they are attached form a pyrrolidinyl ring,
R³ represents a group of the formula 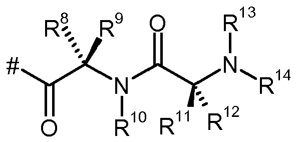 where
# represents the point of attachment to the oxygen atom,
R⁸ represents methyl,
R⁹ represents hydrogen,
R¹⁰ represents hydrogen,
R¹¹ represents methyl or 1-methylpropan-1-yl,
R¹² represents hydrogen,
R¹³ represents hydrogen,
R¹⁴ represents hydrogen,
and the salts, solvates and solvates of the salts thereof.

8. Compound according to Claim 1, where the compound has the structure below 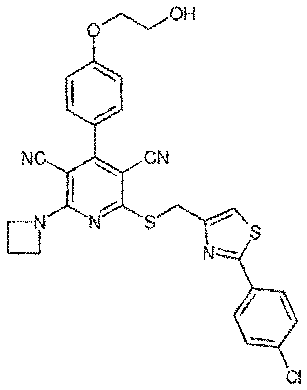 2-(azetidin-1-yl)-6-({[2-(4-chlorophenyl)-1,3-thiazol-4-yl]methyl}sulphanyl)-4-[4-(2-hydroxyethoxy)phenyl]pyridin-3,5-dicarbonitrile and salts and/or solvates thereof.

9. Compound according to Claim 1, where the compound has the structure below 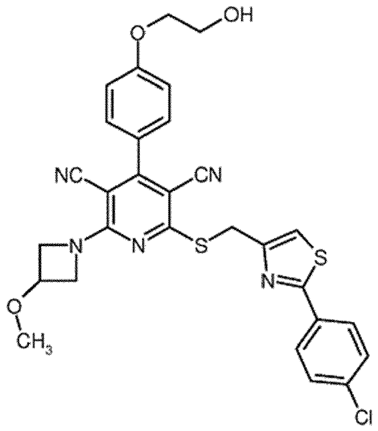 2-((3-methoxy)-azetidin-1-yl)-6-({[2-(4-chlorophenyl)-1,3-thiazol-4-yl]methyl}sulphanyl)-4-[4-(2-hydroxyethoxy)phenyl]pyridin-3,5-dicarbonitrile
and salts and/or solvates thereof.

10. Compound according to Claim 1, where the compound has the structure below 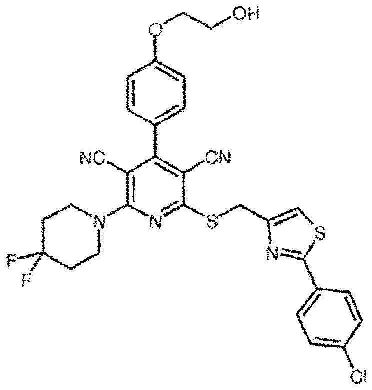 2-({[2-(4-chlorophenyl)-1,3-thiazol-4-yl]methyl}sulphanyl)-6-(4,4-difluoropiperidin-1-yl)-4-[4-(2-hydroxyethoxy)phenyl]pyridin-3,5-dicarbonitrile
and salts and/or solvates thereof.

11. Compound according to Claim 1, where the compound has the structure below 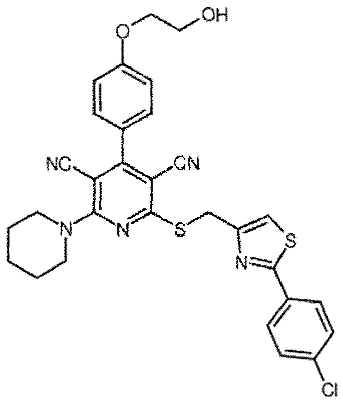 2-({[2-(4-chlorophenyl)-1,3-thiazol-4-yl]methyl}sulphanyl)-4-[4-(2-hydroxy-ethoxy)phenyl]-6-(pyridin-1-yl)pyridin-3,5-dicarbonitrile
and salts and/or solvates thereof.

12. Compound according to Claim 1, where the compound has the structure below 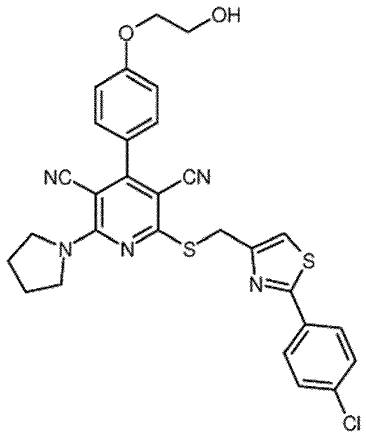 2-({[2-(4-chlorophenyl)-1,3-thiazol-4-yl]methyl}sulphanyl)-4-[4-(2-hydroxy-ethoxy)phenyl]-6-(pyrrolidin-1-yl)pyridin-3,5-dicarbonitrile
and salts and/or solvates thereof.

13. Compound according to Claim 1, where the compound has the structure below 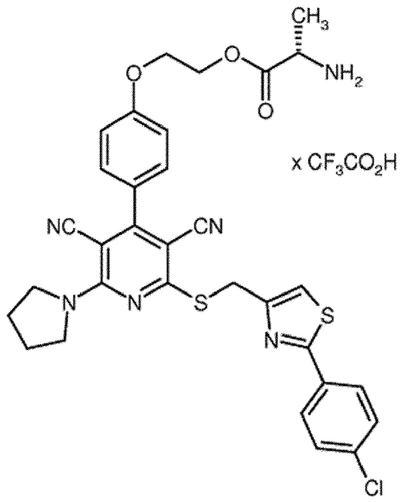 2-{4-[2-({[2-(4-chlorophenyl)-1,3-thiazol-4-yl]methyl}sulphanyl)-3,5-dicyano-6-(pyrrolidin-1-yl)pyridin-4-yl]phenoxy}ethyl L-alaninate trifluoroacetate
and solvates thereof.

14. Compound according to Claim 1, where the compound has the structure below 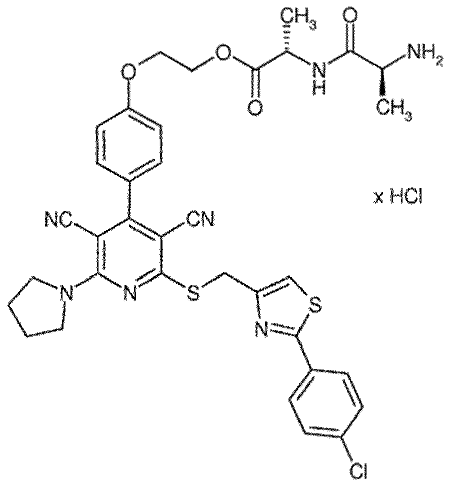 2-{4-[2-({[2-(4-chlorophenyl)-1,3-thiazol-4-yl]methyl}sulphanyl)-3,5-dicyano-6-(pyrrolidin-1-yl)pyridin-4-yl]phenoxy}ethyl L-alanyl L-alaninate hydrochloride
and solvates thereof.

15. Compound according to Claim 1, where the compound has the structure below 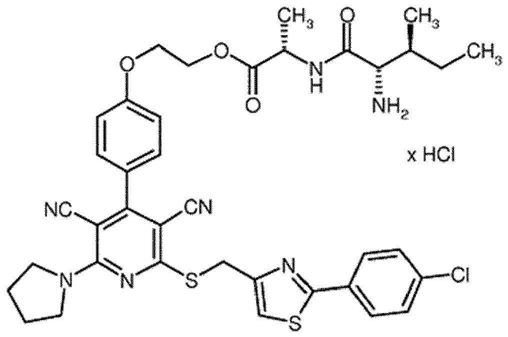 2-{4-[2-({[2-(4-chlorophenyl)-1,3-thiazol-4-yl]methyl}sulphanyl)-3,5-dicyano-6-(pyrrolidin-1-yl)pyridin-4-yl]phenoxy}ethyl L-isoleucyl L-alaninate hydrochloride
and solvates thereof.

16. Compound according to Claim 1, where the compound has the structure below 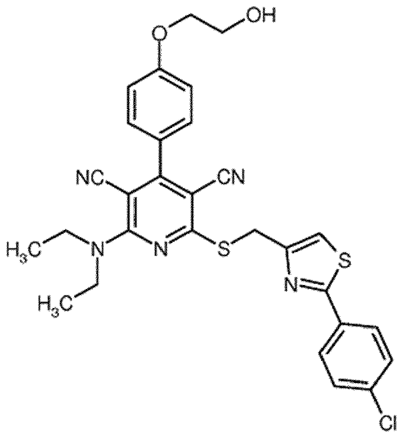 2-({[2-(4-chlorophenyl)-1,3-thiazol-4-yl]methyl}sulphanyl)-6-(diethylamino)-4-[4-(2-hydroxyethoxy)phenyl]pyridin-3,5-dicarbonitrile and salts and/or solvates thereof.

17. Compound according to Claim 1, where the compound has the structure below 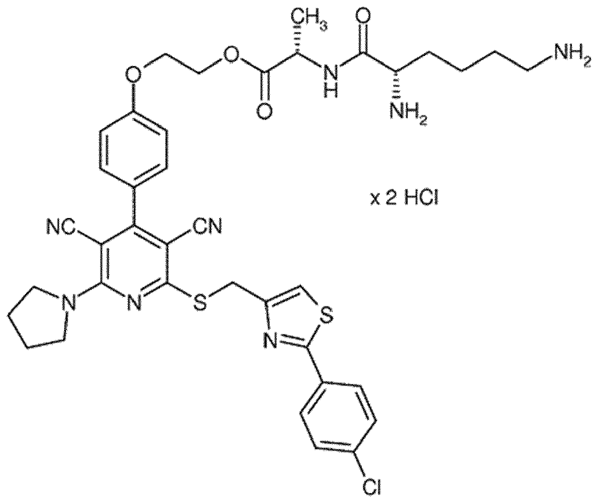 2-{4-[2-({[2-(4-chlorophenyl)-1,3-thiazol-4-yl]methyl}sulphanyl)-3,5-dicyano-6-(pyrrolidin-1-yl)pyridin-4-yl]phenoxy}ethyl L-lysyl-beta-alaninate dihydrochloride
and salts and/or solvates thereof.

18. Compound according to Claim 1, where the compound has the structure below 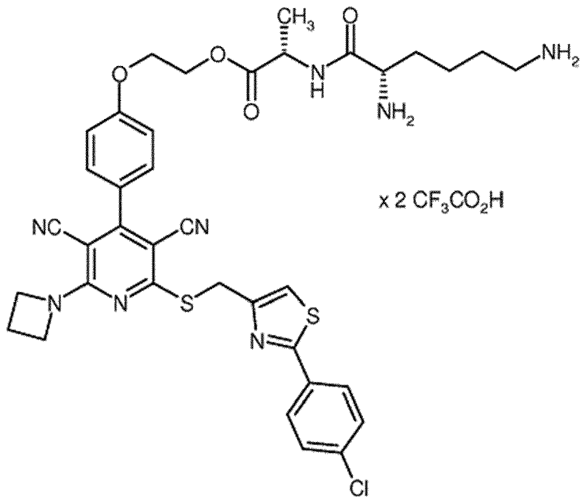 2-{4-[2-(azetidin-1-yl)-6-({[2-(4-chlorophenyl)-1,3-thiazol-4-yl]methyl}sulphanyl)-3,5-dicyanopyridin-4-yl]phenoxy}ethyl L-lysyl-L-alaninate bis(trifluoroacetate)
and solvates thereof.

19. Compound according to Claim 1, where the compound has the structure below 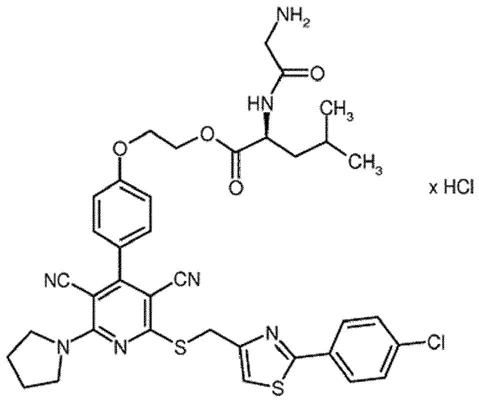 2-{4-[2-({[2-(4-chlorophenyl)-1,3-thiazol-4-yl]methyl}sulphanyl)-3,5-dicyano-6-(pyrrolidin-1-yl)pyridin-4-yl]phenoxy}ethyl glycyl-L-leucinate hydrochloride
and solvates thereof.

20. Compound according to Claim 1, where the compound has the structure below 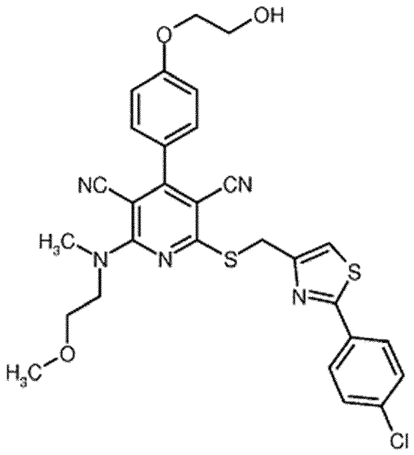 2-({[2-(4-chlorophenyl)-1,3-thiazol-4-yl]methyl}sulphanyl)-4-[4-(2-hydroxy-ethoxy)phenyl]-6-[(2-methoxyethyl)(methyl)amino]pyridin-3,5-dicarbonitrile
and salts and/or solvates thereof.

21. Process for preparing compounds of the formula (I) as defined in any of Claims 1 to 3 in which R³ represents hydrogen, **characterized in that** the compound of the formula (II) 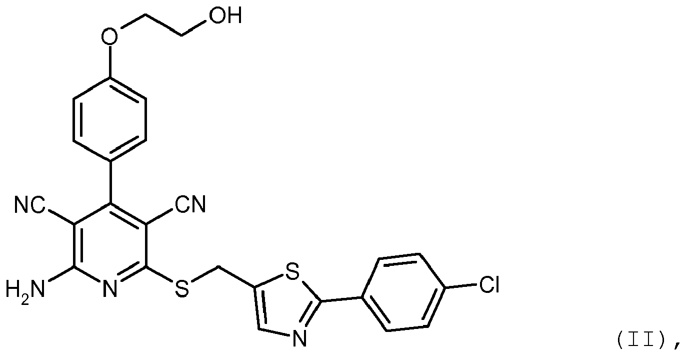 is initially converted with copper(II) chloride and isoamyl nitrite in a suitable solvent into the compound of the formula (III) 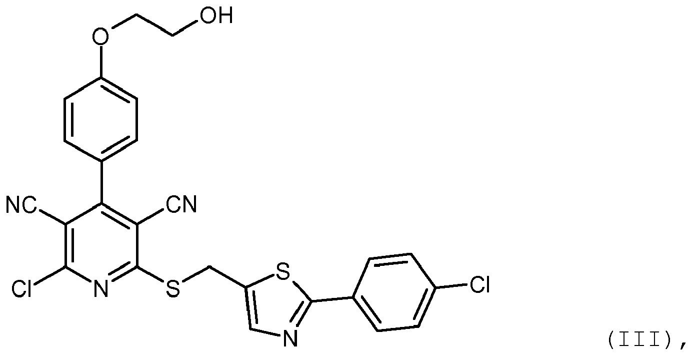 and this is then, in an inert solvent, if appropriate in the presence of a suitable base, reacted with a compound of the formula (IV) 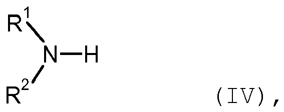 in which R¹ and R² each have the meanings given in any of Claims 1 to 3,
to give a compound of the formula (I-A) 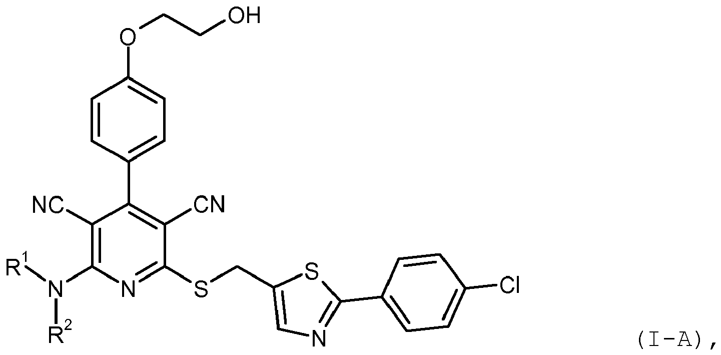 in which R¹ and R² each have the meanings given in any of Claims 1 to 3,
any protective groups present are then removed and the resulting compounds of the formula (I) are, if appropriate, converted with the appropriate (*i*) solvents and/or (*ii*) bases or acids into their solvates, salts and/or solvates of the salts.

22. Process for preparing compounds of the formula (I) as defined in any of Claims 1, 2, 4, 5, 6 and 7 in which R³ represents a group of the formula 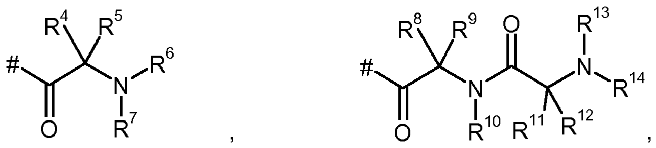 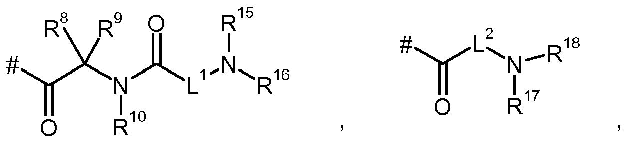 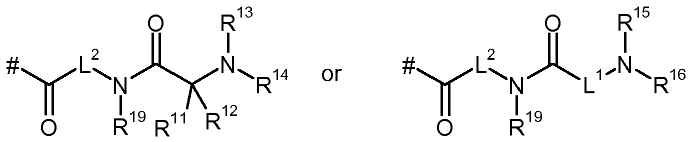 where L¹, L², R⁴, R⁵, R⁶, R⁷, R⁸, R⁹, R¹⁰, R¹¹, R¹², R¹³, R¹⁴, R¹⁵, R¹⁶, R¹⁷, R¹⁸ and R¹⁹ each have the meanings given in any of Claims 1, 2, 4, 5, 6 and 7, **characterized in that**
[A] a compound of the formula (I-A) 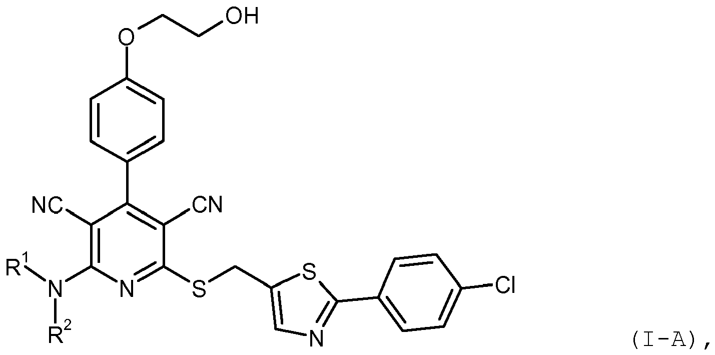 in which R¹ and R² each have the meanings given in any of Claims 1, 2, 4, 5, 6 and 7,
is initially coupled in an inert solvent in the presence of a condensing agent with a carboxylic acid of the formula (V) or (VI) 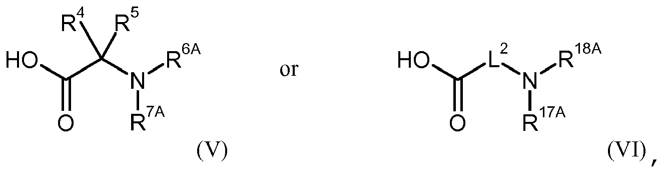 in which L², R⁴ and R⁵ each have the meanings given in any of Claims 1, 2, 4, 5, 6 and 7
and
R^{6A}, R^{7A}, R^{17A} and R^{18A} each have the meanings mentioned for R⁶, R⁷, R¹⁷ and R¹⁸, respectively, or represent an amino protective group, such as, for example, tert-butoxycarbonyl,
to give a compound of the formula (VII) or (VIII) 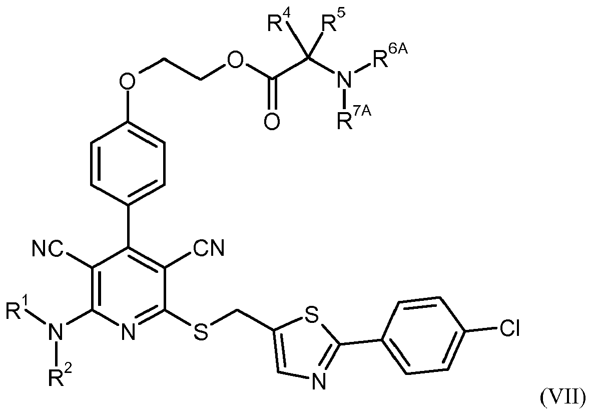 or 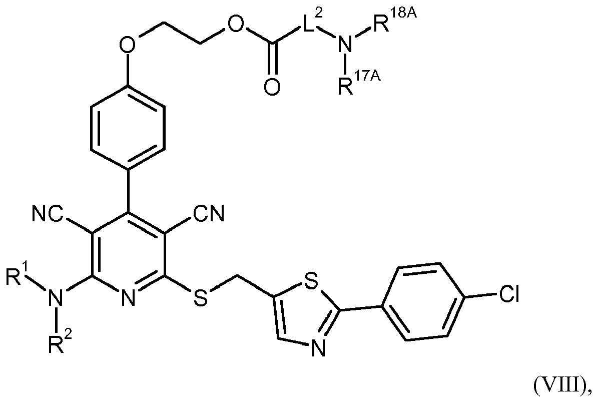 in which L², R¹, R², R⁴, R⁵, R^{6A}, R^{7A}, R^{17A} and R^{18A} each have the meanings given above,
and any protective groups present are then removed to give a compound of the formula (I-B) or (I-C) 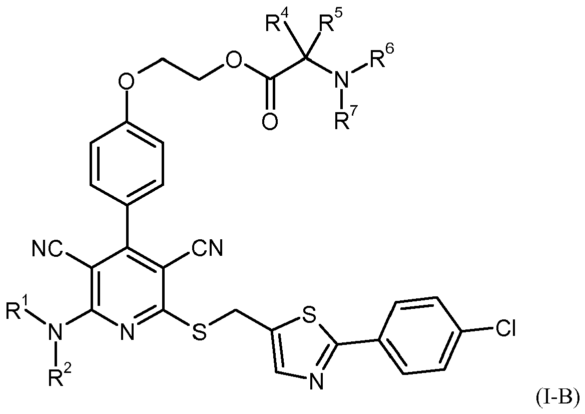 or 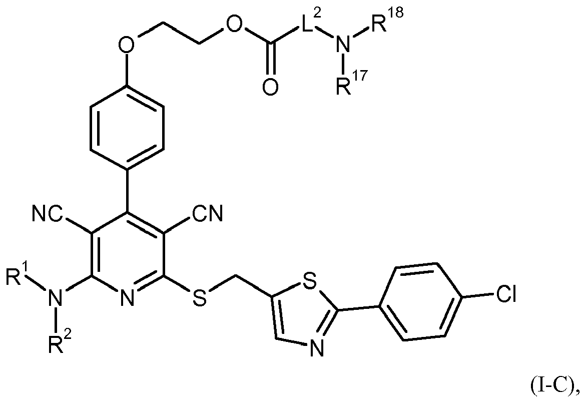 in which L², R¹, R², R⁴, R⁵, R⁶, R⁷, R¹⁷ and R¹⁸ each have the meanings given in any of Claims 1, 2, 4, 5, 6 and 7,
or
[B] a compound of the formula (I-A) is initially coupled in an inert solvent in the presence of a condensing agent with a carboxylic acid of the formula (IX), (X), (XI) or (XII) 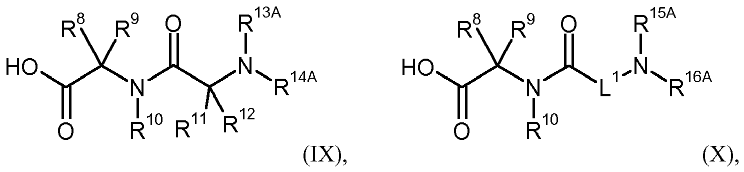 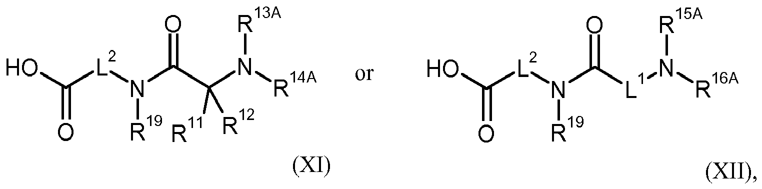 in which L¹, L², R⁸, R⁹, R¹⁰, R¹¹, R¹² and R¹⁹ each have the meanings given in any of Claims 1, 2, 4, 5, 6 and 7
and
R^{13A}, R^{14A}, R^{15A} and R^{16A} each have the meanings mentioned for R¹³, R¹⁴, R¹⁵ and R¹⁶, respectively, or represent an amino protective group, such as, for example, tert-butoxycarbonyl,
to give compounds of the formula (XIII), (XIV), (XV) or (XVI) 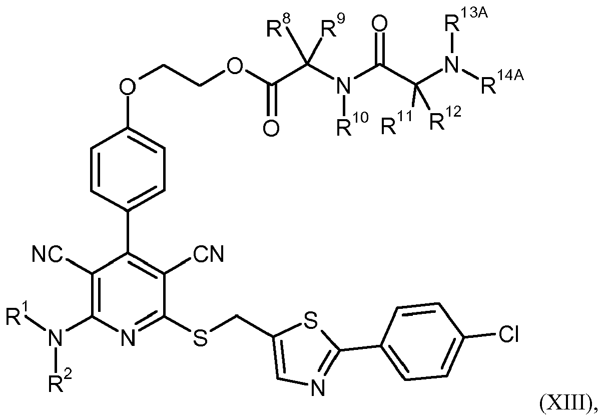 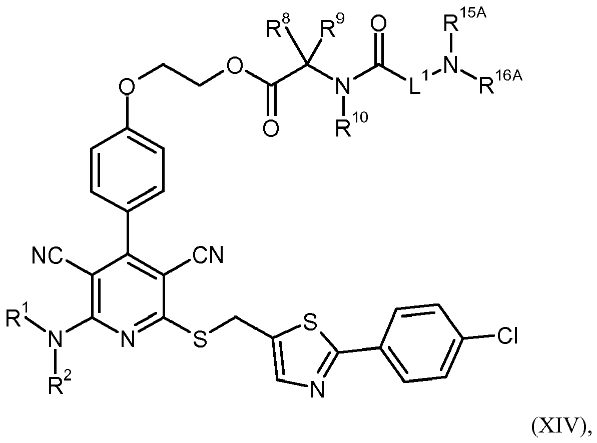 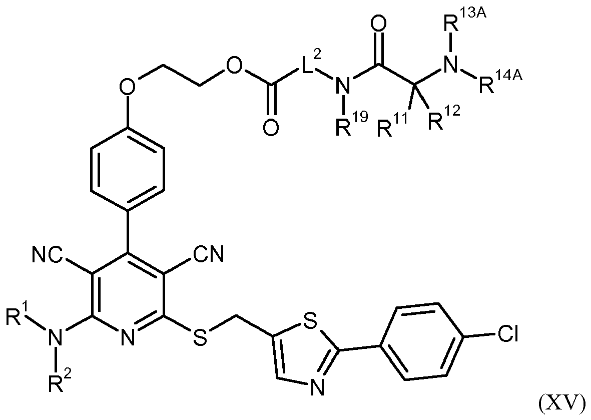 or 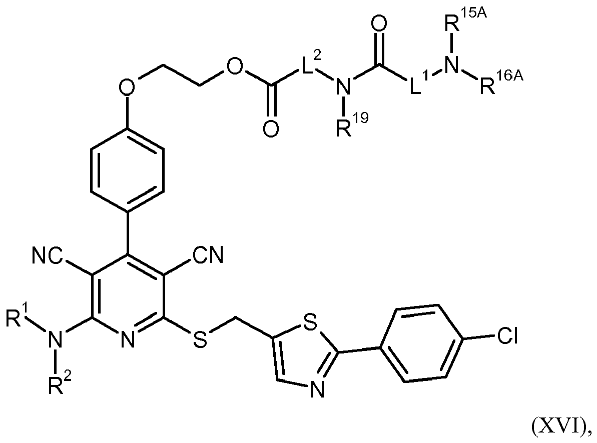 in which L¹, L², R¹, R², R⁸, R⁹, R¹⁰, R¹¹, R¹², R^{13A}, R^{14A}, R^{15A}, R^{16A} and R¹⁹ each have the meanings given above,
and any protectve groups present are then removed to give a compound of the formula (I-D), (I-E), (I-F) or (I-G) 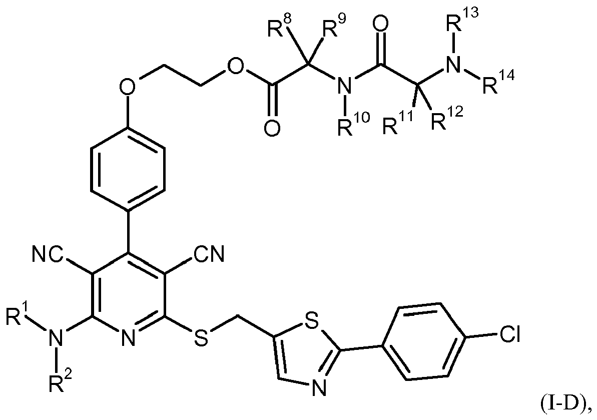 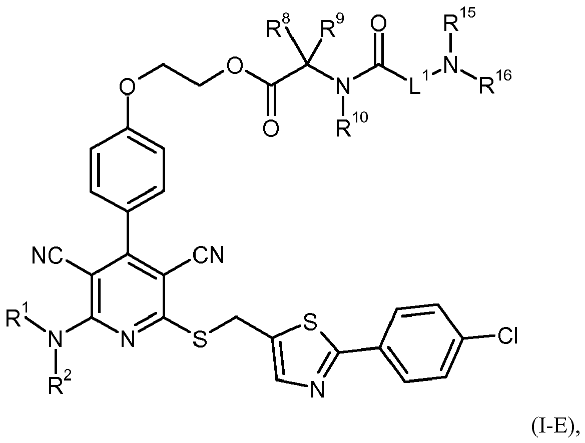 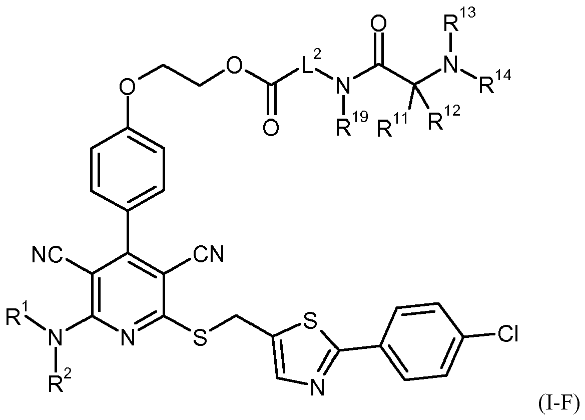 or 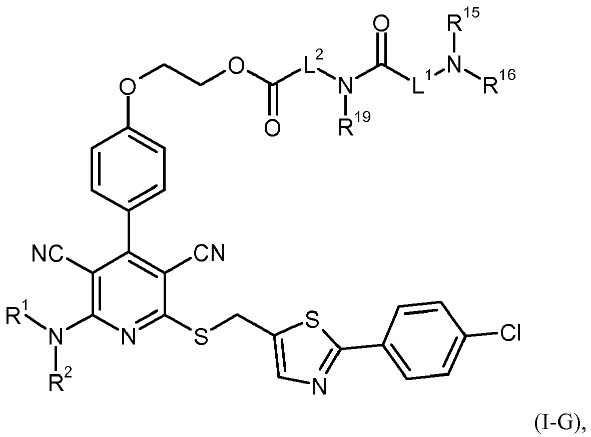 in which L¹, L² R¹, R², R⁸, R⁹, R¹⁰, R¹¹, R¹², R¹³, R¹⁴, R¹⁵, R¹⁶ and R¹⁹ each have the meanings given in any of Claims 1, 2, 4, 5, 6 and 7,
or
[C] the amino protective group is removed from a compound of the formula (VII-1) or (VIII-1) 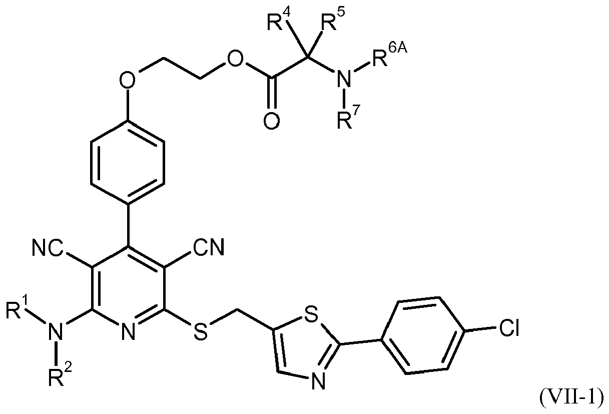 or 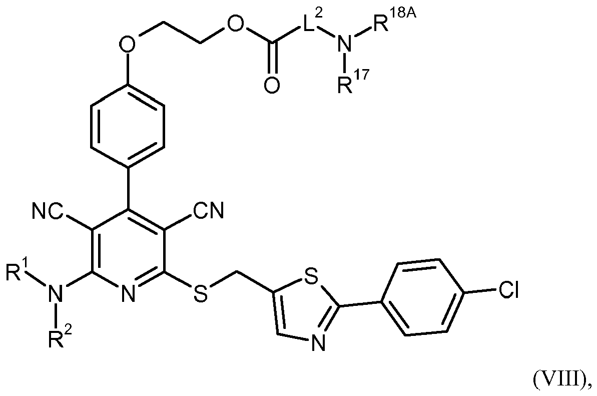 in which L², R¹, R², R⁴, R⁵, R⁷ and R¹⁷ each have the meanings given in any of Claims 1, 2, 4, 5, 6 and 7,
and
R^{6A} and R^{18A} represent an amino protective group, for example tert-butoxycarbonyl,
by standard methods to give a compound of the formula (I-B-1) or (I-C-1) 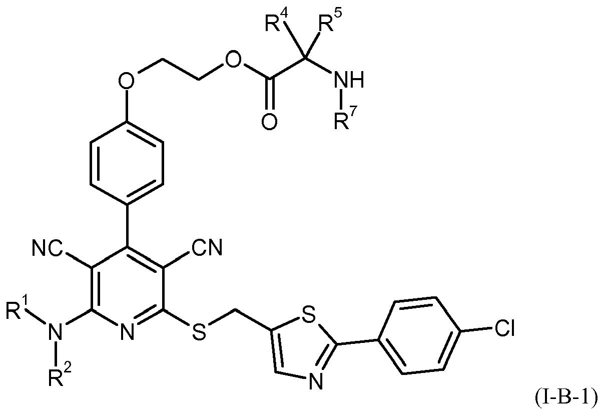 or 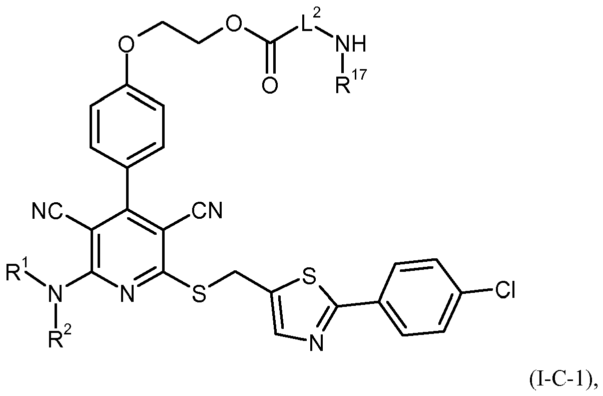 in which L², R¹, R², R⁴, R⁵, R⁷ and R¹⁷ each have the meanings given in any of Claims 1, 2, 4, 5, 6 and 7,
and these are initially coupled in an inert solvent in the presence of a condensing agent with a carboxylic acid of the formula (XVII) or (XVIII) 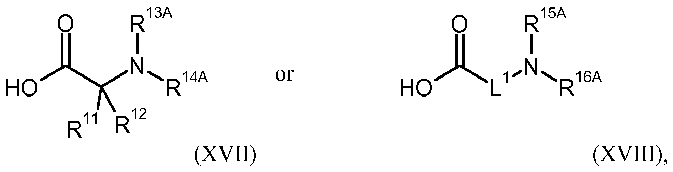 in which L¹, R¹¹ and R¹² each have the meanings given in any of Claims 1, 2, 4, 5, 6 and 7
and
R^{13A}, R^{14A}, R^{15A} and R^{16A} each have the meanings mentioned for R¹³, R¹⁴, R¹⁵ and R¹⁶, respectively, or represent an amino protective group, such as, for example, tert-butoxycarbonyl,
to give compounds of the formula (XIII), (XIV), (XV) or (XVI), and any protective groups present are then removed again to give the resulting compounds (I-D), (I-E), (I-F) or (I-G),
and the resulting compounds of the formula (I-B), (I-C), (I-D), (I-E), (I-F) and (I-G) are, if appropriate, converted with the appropriate (i) solvents and/or (ii) bases or acids into their solvates, salts and/or solvates of the salts.

23. Agonists of the adenosine A1 receptor of the formula (I) as defined in any of Claims 1 to 20 for use in the treatment and/or prophylaxis of diseases.

24. Agonists of the adenosine A1 receptor of the formula (I) as defined in any of Claims 1 to 20 for use in a method for the treatment and/or prophylaxis of coronary heart disease, acute coronary syndrome, angina pectoris, heart failure, myocardial infarction and atrial fibrillation.

25. Agonists of the adenosine A1 receptor of the formula (I) as defined in any of Claims 1 to 20 for use in a method for the treatment and/or prophylaxis of diabetes, metabolic syndrome and dyslipidaemias.

26. Agonists of the adenosine A1 receptor of the formula (I) according to Claim 1 for use in a method for the treatment and/or prophylaxis of kidney diseases.

27. Use of an agonist of the adenosine A1 receptor of the formula (I) as defined in any of Claims 1 to 20 for preparing a medicament for the treatment and/or prophylaxis of coronary heart disease, acute coronary syndrome, angina pectoris, heart failure, myocardial infarction and atrial fibrillation.

28. Use of an agonist of the adenosine A1 receptor of the formula (I) as defined in any of Claims 1 to 20 for preparing a medicament for the treatment and/or prophylaxis of both acute and chronic manifestations of heart failure, decompensated heart failure, right heart failure, left heart failure, global failure, ischaemic cardiomyopathy, dilated cardiomyopathy, congenital heart defects, heart valve defects, heart failure associated with heart valve defects, mitral stenosis, mitral insufficiency, aortic stenosis, aortic insufficiency, tricuspid stenosis, tricuspid insufficiency, pulmonary stenosis, pulmonary valve insufficiency, combined heart valve defects, myocardial inflammation (myocarditis), chronic myocarditis, acute myocarditis, viral myocarditis, diabetic heart failure, alcoholic cardiomyopathy, cardiac storage disorders, and diastolic and systolic heart failure and acute phases of worsening heart failure.

29. Use of an agonist of the adenosine A1 receptor of the formula (I) as defined in Claim 1 for preparing a medicament for the treatment and/or prophylaxis of kidney diseases.

30. Use of an agonist of the adenosine A1 receptor of the formula (I) as defined in any of Claims 1 to 20 for preparing a medicament for the treatment and/or prophylaxis of diabetes, metabolic syndrome and dyslipidaemias.

31. Medicament, comprising an agonist of the adenosine A1 receptor of the formula (I) as defined in any of Claims 1 to 20 in combination with an inert nontoxic pharmaceutically suitable auxiliary.

32. Medicament, comprising an agonist of the adenosine A1 receptor of the formula (I) as defined in any of Claims 1 to 20 in combination with one or more further active ingredients selected from the group consisting of lipid metabolism-modifying active ingredients, antidiabetics, antihypertensive drugs and antithrombotic drugs.

33. Medicament according to Claim 31 or 32 for use in the treatment and/or prophylaxis of coronary heart disease, acute coronary syndrome, angina pectoris, heart failure, myocardial infarction and atrial fibrillation.

34. Medicament according to Claim 31 or 32 for use in the treatment and/or prophylaxis of both acute and chronic manifestations of heart failure, decompensated heart failure, right heart failure, left heart failure, global failure, ischaemic cardiomyopathy, dilated cardiomyopathy, congenital heart defects, heart valve defects, heart failure associated with heart valve defects, mitral stenosis, mitral insufficiency, aortic stenosis, aortic insufficiency, tricuspid stenosis, tricuspid insufficiency, pulmonary stenosis, pulmonary valve insufficiency, combined heart valve defects, myocardial inflammation (myocarditis), chronic myocarditis, acute myocarditis, viral myocarditis, diabetic heart failure, alcoholic cardiomyopathy, cardiac storage disorders, and diastolic and systolic heart failure and acute phases of worsening heart failure.

35. Medicament according to Claim 31 or 32 for use in the treatment and/or prophylaxis of diabetes, metabolic syndrome and dyslipidaemias.

36. Medicament according to Claim 31 or 32 for use in the treatment and/or prophylaxis of kidney diseases.

## Revendications

1. Composés de formule (I) 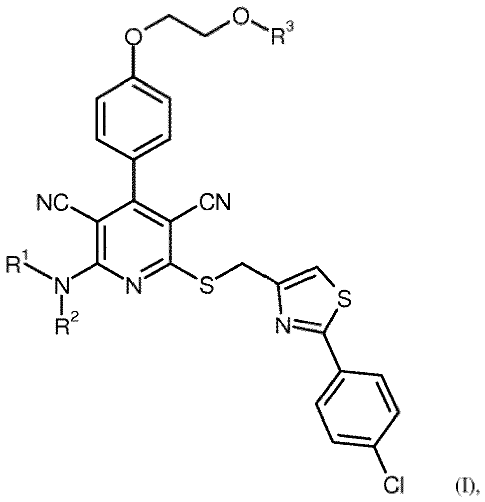 dans laquelle
R¹ représente alkyle en (C₁-C₄),
R² représente alkyle en (C₁-C₆), alcényle en (C₂-C₄), alcynyle en (C₂-C₄) ou cycloalkyle en (C₃-C₇),
alkyle en (C₁-C₆) pouvant être substitué avec 1 à 3 substituants choisis indépendamment les uns des autres dans le groupe constitué par fluor, chlore, trifluorométhyle, trifluorométhoxy, alcoxy en (C₁-C₄), cycloalkyle en (C₃-C₇), cycloalcoxy en (C₃-C₇), alkylsulfanyle en (C₁-C₄) ou alkylsulfonyle en (C₁-C₄),
et
alcényle en (C₂-C₄) et alcynyle en (C₂-C₄) pouvant être substitués avec 1 ou 2 substituants choisis indépendamment l'un de l'autre dans le groupe constitué par fluor, trifluorométhyle, alkyle en (C₁-C₄), trifluorométhoxy et alcoxy en (C_{$1}-C₄),
et
cycloalkyle en (C₃-C₇) pouvant être substitué avec 1 ou 2 substituants choisis indépendamment l'un de l'autre dans le groupe constitué par fluor, chlore, trifluorométhyle, alkyle en (C₁-C₄), trifluorométhoxy et alcoxy en (C₁-C₄),
ou
R¹ et R² forment ensemble avec l'atome d'azote auquel ils sont reliés un hétérocycle de 4 à 7 éléments, qui peut contenir un hétéroatome supplémentaire de la série N, O et S,
l'hétérocycle de 4 à 7 éléments pouvant être substitué avec 1 ou 2 substituants choisis indépendamment l'un de l'autre dans le groupe constitué par fluor, chlore, oxo, trifluorométhyle, alkyle en (C₁-C₄), trifluorométhoxy et alcoxy en (C₁-C₄),
R³ représente hydrogène ou un groupe de formule 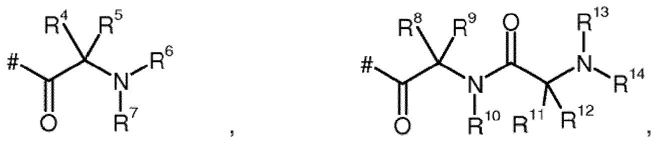 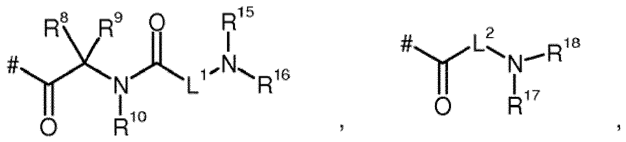 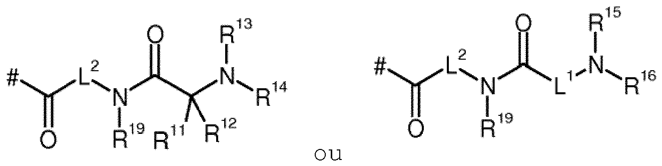 dans lesquelles
# représente l'emplacement de liaison à l'atome d'oxygène,
L¹ représente alcanediyle en (C₂-C₆),
L² représente alcanediyle en (C₂-C₆),
R⁴ représente hydrogène ou le groupe latéral d'un acide aminé α naturel ou ses homologues ou isomères,
R⁵ représente hydrogène ou méthyle,
R⁶ représente hydrogène ou alkyle en (C₁-C₄),
R⁷ représente hydrogène ou alkyle en (C₁-C₄),
ou
R⁶ et Reforment ensemble avec l'atome d'azote auquel ils sont reliés un hétérocycle de 5 ou 6 éléments,
l'hétérocycle de 5 ou 6 éléments pouvant être substitué avec 1 ou 2 substituants choisis indépendamment l'un de l'autre dans le groupe constitué par alkyle en (C₁-C₄), amino, hydroxy et alcoxy en (C₁-C₄),
ou
R⁷ forme avec R⁴ et les atomes auxquels ils sont chacun reliés un cycle pyrrolidine ou pipéridine,
R⁸ représente hydrogène ou le groupe latéral d'un acide aminé α naturel ou ses homologues ou isomères,
R⁹ représente hydrogène ou méthyle,
R¹⁰ représente hydrogène ou méthyle,
R¹¹ représente hydrogène ou le groupe latéral d'un acide aminé α naturel ou ses homologues ou isomères,
R¹² représente hydrogène ou méthyle,
R¹³ représente hydrogène ou alkyle en (C₁-C₄),
R¹⁴ représente hydrogène ou alkyle en (C₁-C₄),
ou
R¹³ et R¹⁴ forment ensemble avec l'atome d'azote auquel ils sont reliés un hétérocycle de 5 ou 6 éléments,
l'hétérocycle de 5 ou 6 éléments pouvant être substitué avec 1 ou 2 substituants choisis indépendamment l'un de l'autre dans le groupe constitué par alkyle en (C₁-C₄), amino, hydroxy et alcoxy en (C₁-C₄),
ou
R¹⁴ forme avec R¹¹ et les atomes auxquels ils sont chacun reliés un cycle pyrrolidine ou pipéridine,
R¹⁵ représente hydrogène ou alkyle en (C₁-C₄),
R¹⁶ représente hydrogène ou alkyle en (C₁-C₄),
ou
R¹⁵ et R¹⁶ forment ensemble avec l'atome d'azote auquel ils sont reliés un hétérocycle de 5 ou 6 éléments, l'hétérocycle de 5 ou 6 éléments pouvant être substitué avec 1 ou 2 substituants choisis indépendamment l'un de l'autre dans le groupe constitué par alkyle en (C₁-C₄), amino, hydroxy et alcoxy en (C₁-C₄),
R¹⁷ représente hydrogène ou alkyle en (C₁-C₄),
R¹⁸ représente hydrogène ou alkyle en (C₁-C₄),
ou
R¹⁷ et R¹⁸ forment ensemble avec l'atome d'azote auquel ils sont reliés un hétérocycle de 5 ou 6 éléments, l'hétérocycle de 5 ou 6 éléments pouvant être substitué avec 1 ou 2 substituants choisis indépendamment l'un de l'autre dans le groupe constitué par alkyle en (C₁-C₄), amino, hydroxy et alcoxy en (C₁-C₄),
R¹⁹ représente hydrogène ou méthyle,
ainsi que leurs sels et solvates.

2. Composés de formule (I) selon la revendication 1, dans lesquels
R¹ représente méthyle ou éthyle,
R² représente alkyle en (C₁-C₃), cyclopropyle ou cyclobutyle,
alkyle en (C₁-C₃) pouvant être substitué avec 1 ou 2 substituants choisis indépendamment les uns des autres dans le groupe constitué par fluor, chlore, trifluorométhyle, méthoxy, éthoxy, cyclopropyle et cyclobutyle,
ou
R¹ et R² forment ensemble avec l'atome d'azote auquel ils sont reliés un hétérocycle de 4 à 6 éléments, qui peut contenir un hétéroatome supplémentaire de la série N, O et S,
l'hétérocycle de 4 à 6 éléments pouvant être substitué avec 1 ou 2 substituants choisis indépendamment l'un de l'autre dans le groupe constitué par fluor, trifluorométhyle, méthyle, éthyle, méthoxy et éthoxy,
R³ représente hydrogène ou un groupe de formule 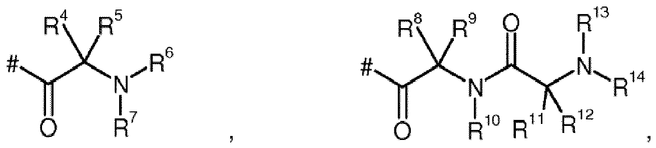 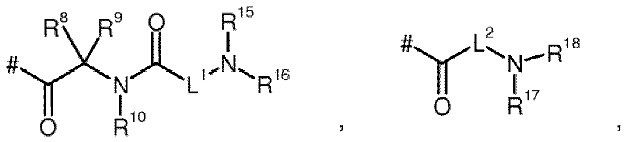 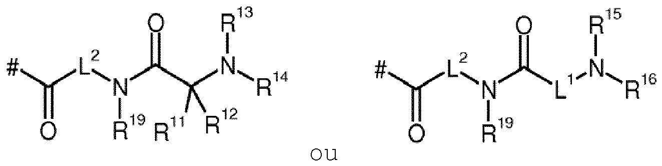 dans lesquelles
# représente l'emplacement de liaison à l'atome d'oxygène,
L¹ représente éthane-1,2-diyle,
L² représente éthane-1,2-diyle,
R⁴ représente méthyle ou 3-aminopropan-1-yle,
R⁵ représente hydrogène,
R⁶ représente hydrogène,
R⁷ représente hydrogène,
R⁸ représente hydrogène ou 2-méthylpropan-1-yle,
R⁹ représente hydrogène,
R¹⁰ représente hydrogène,
R¹¹ représente méthyle, 1-méthylpropan-1-yle, imidazol-4-ylméthyle, 4-aminobutan-1-yle, 3-aminopropan-1-yle, 2-aminoéthyle, aminométhyle ou 3-guanidinopropan-1-yle,
R¹² représente hydrogène,
R¹³ représente hydrogène,
R¹⁴ représente hydrogène,
ou
R¹⁴ forme avec R¹¹ et les atomes auxquels ils sont chacun reliés un cycle pyrrolidine,
R¹⁵ représente hydrogène,
R¹⁶ représente hydrogène,
R¹⁷ représente hydrogène,
R¹⁸ représente hydrogène,
R¹⁹ représente hydrogène,
ainsi que leurs sels, solvates et solvates des sels.

3. Composés de formule (I) selon la revendication 1 ou 2, dans lesquels
R¹ représente méthyle ou éthyle,
R² représente méthyle, éthyle ou n-propyle, méthyle, éthyle et n-propyle pouvant être substitués avec 1 ou 2 substituants choisis indépendamment l'un de l'autre dans le groupe constitué par fluor, trifluorométhyle et méthoxy,
ou
R¹ et R² forment ensemble avec l'atome d'azote auquel ils sont reliés un cycle azétidinyle, pyrrolidinyle ou pipéridinyle,
les cycles azétidinyle et pipéridinyle pouvant être substitués avec un substituant méthoxy,
R³ représente hydrogène,
ainsi que leurs sels, solvates et solvates des sels.

4. Composés de formule (I) selon la revendication 1 ou 2, dans lesquels
R¹ et R² forment ensemble avec l'atome d'azote auquel ils sont reliés un cycle azétidinyle, pyrrolidinyle ou pipéridinyle,
les cycles azétidinyle et pipéridinyle pouvant être substitués avec un substituant méthoxy,
R³ représente un groupe de formule 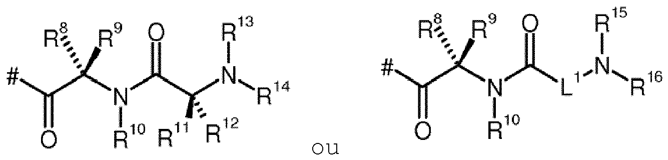 dans lesquelles
# représente l'emplacement de liaison à l'atome d'oxygène,
L¹ représente éthane-1,2-diyle,
R⁸ représente méthyle ou isobutyle,
R⁹ représente hydrogène,
R¹⁰ représente hydrogène,
R¹¹ représente hydrogène, méthyle, 1-méthylpropan-1-yle, 4-aminobutan-1-yle ou 3-guanidinopropan-1-yle,
R¹² représente hydrogène,
R¹³ représente hydrogène,
R¹⁴ représente hydrogène,
ou
R¹⁴ forme avec R¹¹ et les atomes auxquels ils sont chacun reliés un cycle pyrrolidine,
R¹⁵ représente hydrogène,
R¹⁶ représente hydrogène,
ainsi que leurs sels, solvates et solvates des sels.

5. Composés de formule (I) selon la revendication 1, dans lesquels
R¹ et R² forment ensemble avec l'atome d'azote auquel ils sont reliés un cycle pyrrolidinyle,
R³ représente un groupe de formule 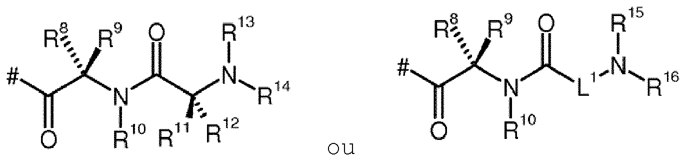 dans lesquelles
# représente l'emplacement de liaison à l'atome d'oxygène,
L¹ représente éthane-1,2-diyle,
R⁸ représente méthyle ou isobutyle,
R⁹ représente hydrogène,
R¹⁰ représente hydrogène,
R¹¹ représente hydrogène, méthyle, 1-méthylpropan-1-yle, 4-aminobutan-1-yle ou 3-guanidinopropan-1-yle,
R¹² représente hydrogène,
R¹³ représente hydrogène,
R¹⁴ représente hydrogène,
ou
R¹⁴ forme avec R¹¹ et les atomes auxquels ils sont chacun reliés un cycle pyrrolidine,
R¹⁵ représente hydrogène,
R¹⁶ représente hydrogène,
ainsi que leurs sels, solvates et solvates des sels.

6. Composés de formule (I) selon la revendication 1, dans lesquels
R¹ et R² forment ensemble avec l'atome d'azote auquel ils sont reliés un cycle azétidinyle, pyrrolidinyle ou pipéridinyle,
ainsi que leurs sels, solvates et solvates des sels.

7. Composés de formule (I) selon la revendication 1, dans lesquels
R¹ et R² forment ensemble avec l'atome d'azote auquel ils sont reliés un cycle pyrrolidinyle,
R³ représente un groupe de formule 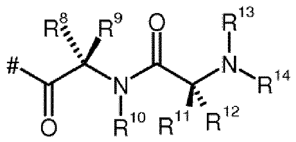 dans laquelle
# représente l'emplacement de liaison à l'atome d'oxygène,
R⁸ représente méthyle,
R⁹ représente hydrogène,
R¹⁰ représente hydrogène,
R¹¹ représente méthyle ou 1-méthylpropan-1-yle,
R¹² représente hydrogène,
R¹³ représente hydrogène,
R¹⁴ représente hydrogène,
ainsi que leurs sels, solvates et solvates des sels.

8. Composé selon la revendication 1, dans lequel le composé a la structure suivante : 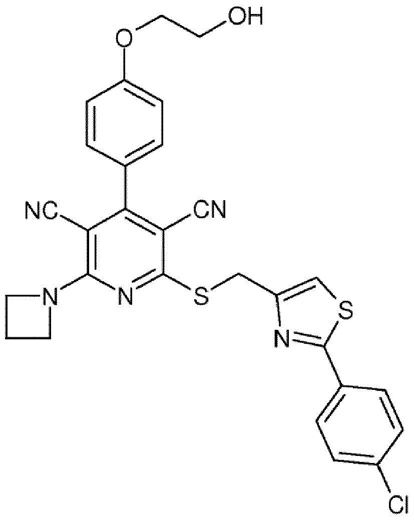 2-(azétidin-1-yl)-6-({[2-(4-chlorophényl)-1,3-thiazol-4-yl]méthyl}sulfanyl)-4-[4-(2-hydroxyéthoxy)phényl]pyridine-3,5-dicarbonitrile,
ainsi que ses sels et/ou solvates.

9. Composé selon la revendication 1, dans lequel le composé a la structure suivante : 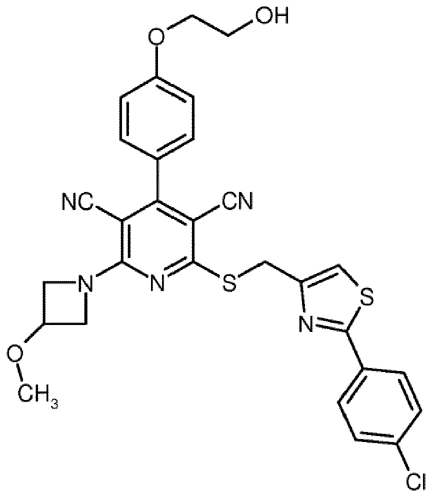 2-((3-méthoxy)-azétidin-1-yl)-6-({[2-(4-chlorophényl)-1,3-thiazol-4-yl]méthyl}sulfanyl)-4-[4-(2-hydroxyéthoxy)phényl]pyridine-3,5-dicarbonitrile,
ainsi que ses sels et/ou solvates.

10. Composé selon la revendication 1, dans lequel le composé a la structure suivante : 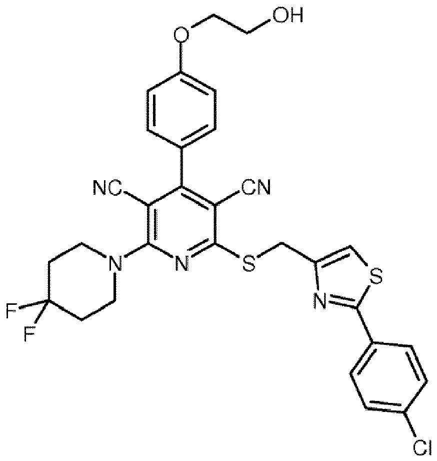 2-({[2-(4-chlorophényl)-1,3-thiazol-4-yl]méthyl}sulfanyl)-6-(4,4-difluoropipéridin-1-yl)-4-[4-(2-hydroxyéthoxy)phényl]pyridine-3,5-dicarbonitrile, ainsi que ses sels et/ou solvates.

11. Composé selon la revendication 1, dans lequel le composé a la structure suivante : 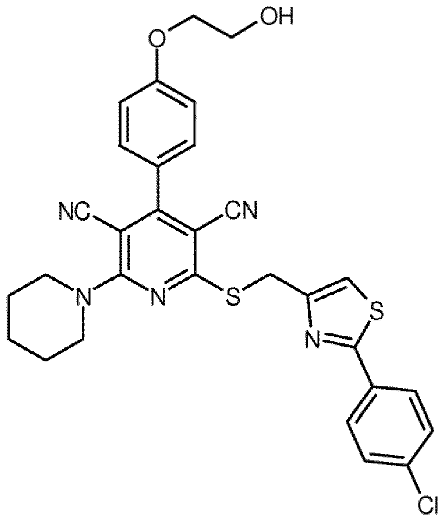 2-({[2-(4-chlorophényl)-1,3-thiazol-4-yl]méthyl}sulfanyl)-4-[4-(2-hydroxyéthoxy)phényl]-6-(pipéridin-1-yl)pyridine-3,5-dicarbonitrile,
ainsi que ses sels et/ou solvates.

12. Composé selon la revendication 1, dans lequel le composé a la structure suivante : 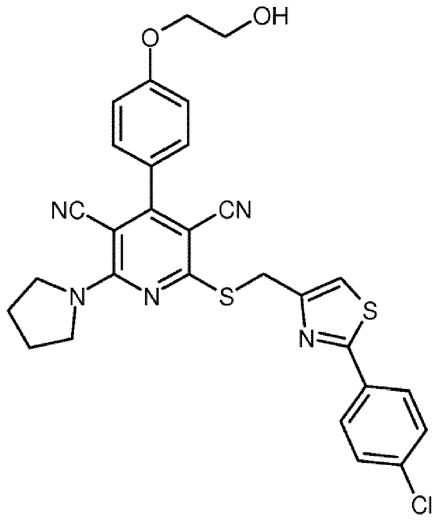 2-({[2-(4-chlorophényl)-1,3-thiazol-4-yl]méthyl}sulfanyl)-4-[4-(2-hydroxyéthoxy)phényl]-6-(pyrrolidin-1-yl)pyridine-3,5-dicarbonitrile,
ainsi que ses sels et/ou solvates.

13. Composé selon la revendication 1, dans lequel le composé a la structure suivante : 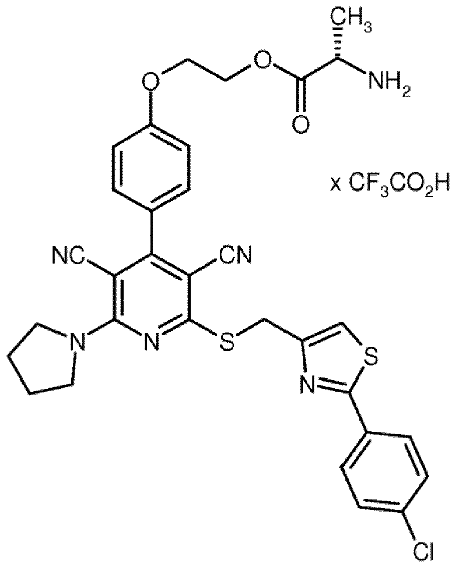 trifluoroacétate de L-alaninate de 2-{4-[2-({[2-(4-chlorophényl)-1,3-thiazol-4-yl]méthyl}sulfanyl)-3,5-dicyano-6-(pyrrolidin-1-yl)pyridin-4-yl]phénoxy}éthyle, ainsi que ses solvates.

14. Composé selon la revendication 1, dans lequel le composé a la structure suivante : 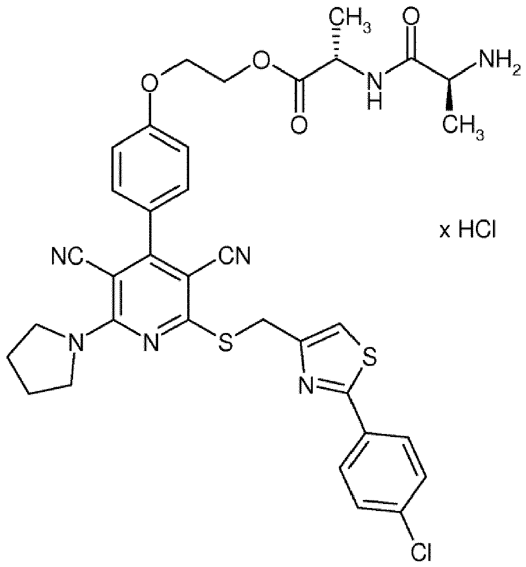 chlorhydrate de L-alanyl-L-alaninate de 2-{4-[2-({[2-(4-chlorophényl)-1,3-thiazol-4-yl]méthyl}sulfanyl)-3,5-dicyano-6-(pyrrolidin-1-yl)pyridin-4-yl]phénoxy}éthyle, ainsi que ses solvates.

15. Composé selon la revendication 1, dans lequel le composé a la structure suivante : 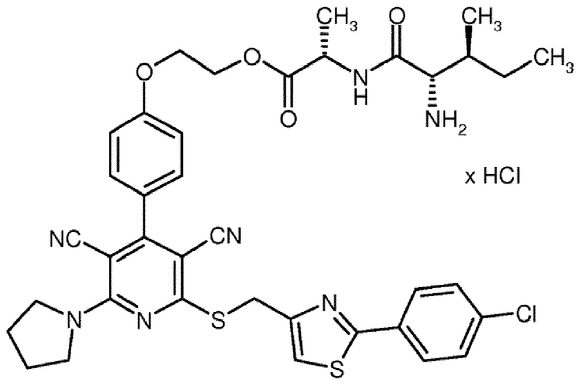 chlorhydrate de L-isoleucyl-L-alaninate de 2-{4-[2-({[2-(4-chlorophényl)-1,3-thiazol-4-yl]méthyl}sulfanyl)-3,5-dicyano-6-(pyrrolidin-1-yl)pyridin-4-yl]phénoxy}éthyle,
ainsi que ses solvates.

16. Composé selon la revendication 1, dans lequel le composé a la structure suivante : 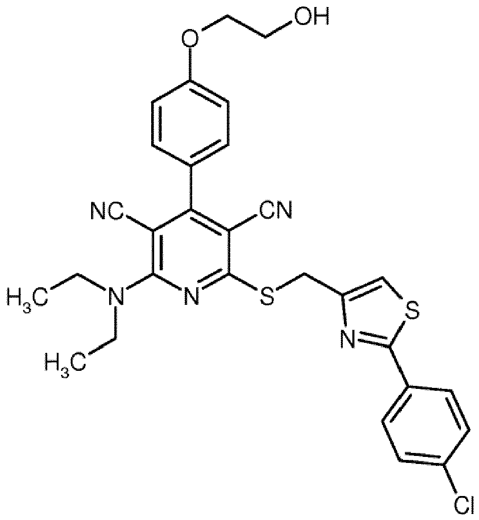 2-({[2-(4-chlorophényl)-1,3-thiazol-4-yl]méthyl}sulfanyl)-6-(diéthylamino)-4-[4-(2-hydroxyéthoxy)phényl]pyridine-3,5-dicarbonitrile, ainsi que ses sels et/ou solvates.

17. Composé selon la revendication 1, dans lequel le composé a la structure suivante : 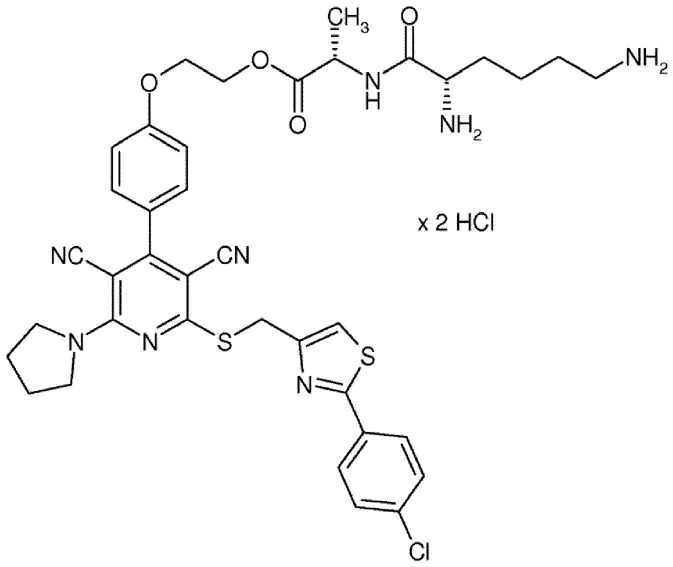 dichlorhydrate de L-lysyl-bêta-alaninate de 2-{4-[2-({[2-(4-chlorophényl)-1,3-thiazol-4-yl]méthyl}sulfanyl)-3,5-dicyano-6-(pyrrolidin-1-yl)pyridin-4-yl]phénoxy}éthyle,
ainsi que ses sels et/ou solvates.

18. Composé selon la revendication 1, dans lequel le composé a la structure suivante : 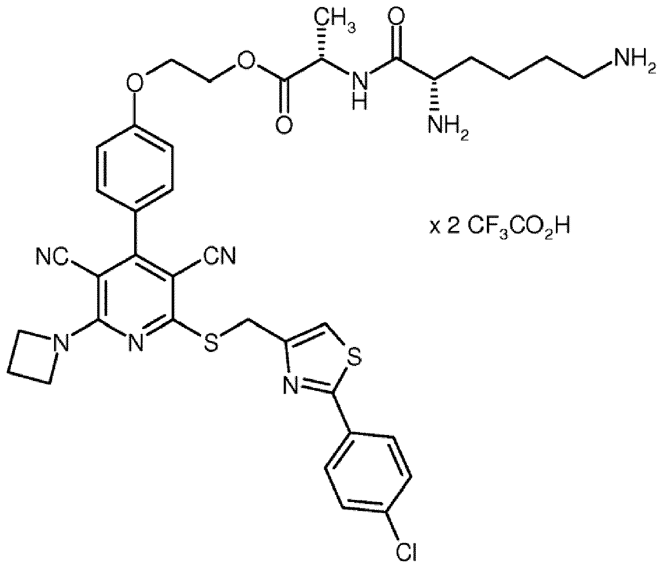 bis(trifluoroacétate) de L-lysyl-L-alaninate de 2-{4-[2-(azétidin-1-yl)-6-({[2-(4-chlorophényl)-1,3-thiazol-4-yl]méthyl}sulfanyl)-3,5-dicyanopyridin-4-yl]phénoxy}éthyle,
ainsi que ses solvates.

19. Composé selon la revendication 1, dans lequel le composé a la structure suivante : 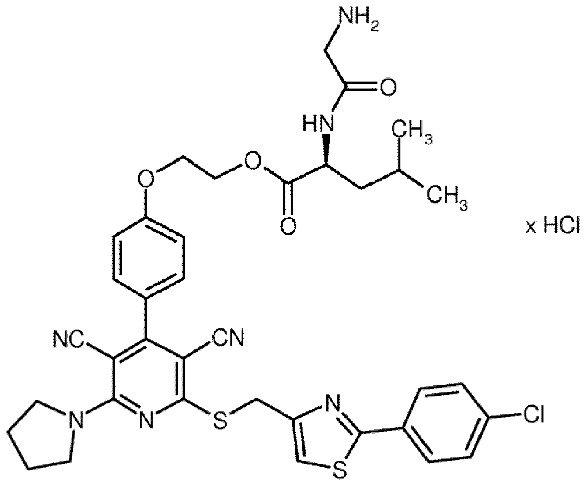 chlorhydrate de glycyl-L-leucinate de 2-{4-[2-({[2-(4-chlorophényl)-1,3-thiazol-4-yl]méthyl}sulfanyl)-3,5-dicyano-6-(pyrrolidin-1-yl)pyridin-4-yl]phénoxy}éthyle, ainsi que ses solvates.

20. Composé selon la revendication 1, dans lequel le composé a la structure suivante : 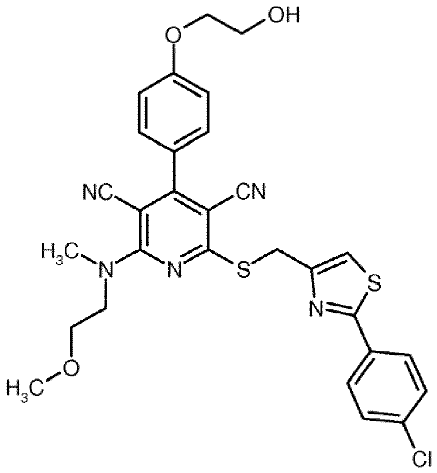 2-({[2-(4-chlorophényl)-1,3-thiazol-4-yl]méthyl}sulfanyl)-4-[4-(2-hydroxyéthoxy)phényl]-6-[(2-méthoxyéthyl)(méthyl)amino]pyridine-3,5-dicarbonitrile,
ainsi que ses sels et/ou solvates.

21. Procédé de fabrication de composés de formule (I), tels que définis dans les revendications 1 à 3, dans lesquels R³ représente l'hydrogène, **caractérisé en ce que** le composé de formule (II) 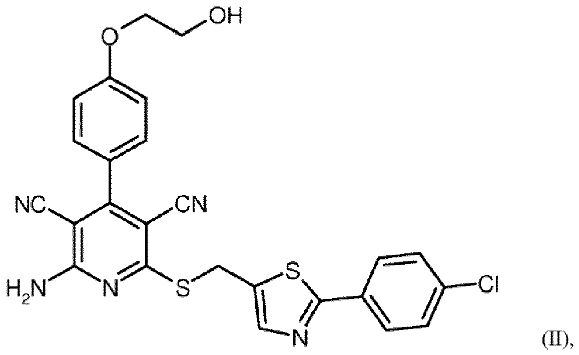 est tout d'abord transformé avec du chlorure de cuivre (II) et de l'isoamylnitrite dans un solvant approprié en le composé de formule (III) 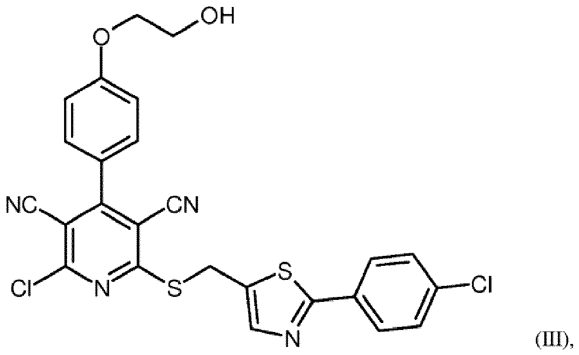 puis celui-ci est transformé dans un solvant inerte éventuellement en présence d'une base appropriée avec un composé de formule (IV) 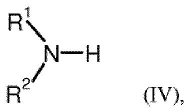 dans laquelle R¹ et R² ont chacun les significations indiquées dans les revendications 1 à 3,
en un composé de formule (I-A) 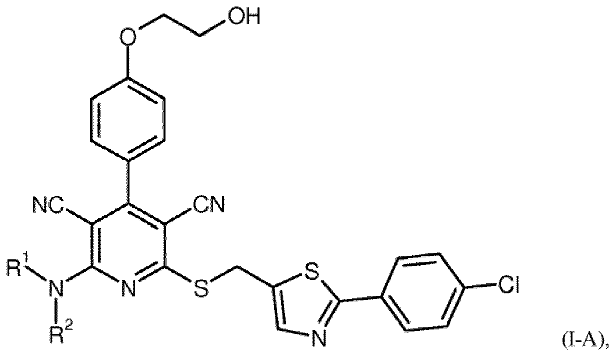 dans laquelle R¹ et R² ont chacun les significations indiquées dans les revendications 1 à 3,
puis les groupes protecteurs éventuellement présents sont clivés et les composés de formule (I) résultants sont éventuellement transformés avec les (i) solvants et/ou (ii) bases ou acides appropriés en leurs solvates, sels et/ou solvates des sels.

22. Procédé de fabrication de composés de formule (I), tels que définis dans les revendications 1, 2, 4, 5, 6 et 7, dans lesquels R³ représente un groupe de formule 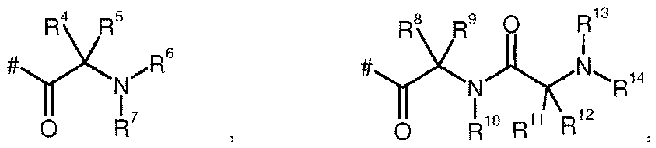 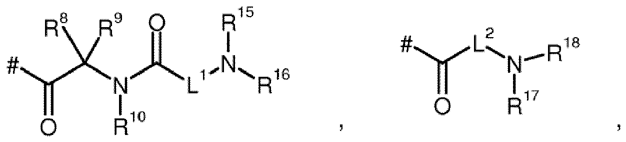 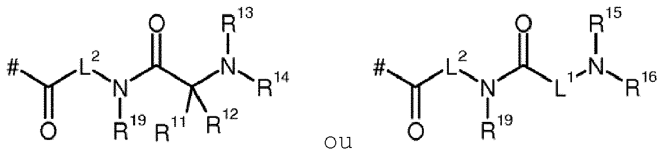 dans lesquelles L¹, L², R⁴, R⁵, R⁶, R⁷, R⁸, R⁹, R¹⁰, R¹¹, R¹², R¹³, R¹⁴, R¹⁵, R¹⁶, R¹⁷, R¹⁸ et R¹⁹ ont chacun les significations indiquées dans les revendications 1, 2, 4, 5, 6 et 7, **caractérisé en ce que**
[A] un composé de formule (I-A) 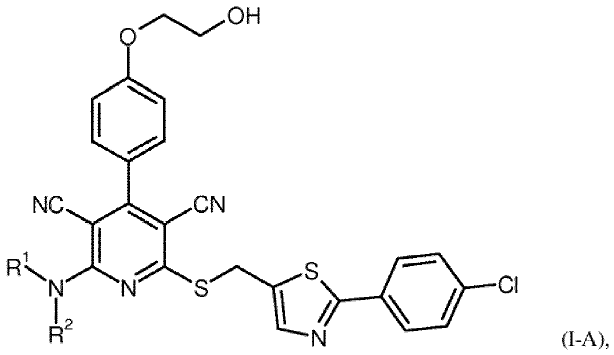 dans laquelle R¹ et R² ont chacun les significations indiquées dans les revendications 1, 2, 4, 5, 6 et 7, est tout d'abord couplé dans un solvant inerte en présence d'un agent de condensation avec un acide carboxylique de formule (V) ou (VI) 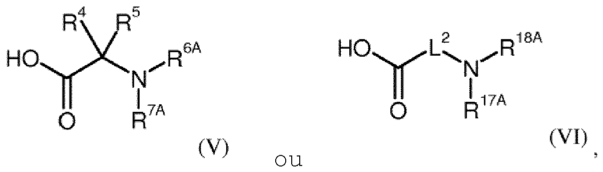 dans lesquelles
L², R⁴ et R⁵ ont chacun les significations indiquées dans les revendications 1, 2, 4, 5, 6 et 7,
et
R^{6A}, R^{7A}, R^{17A} et R^{18A} ont respectivement les significations indiquées pour R⁶, R⁷, R¹⁷ et R¹⁸ ou représentent un groupe protecteur amino, tel que par exemple tert.-butoxycarbonyle,
en un composé de formule (VII) ou (VIII) 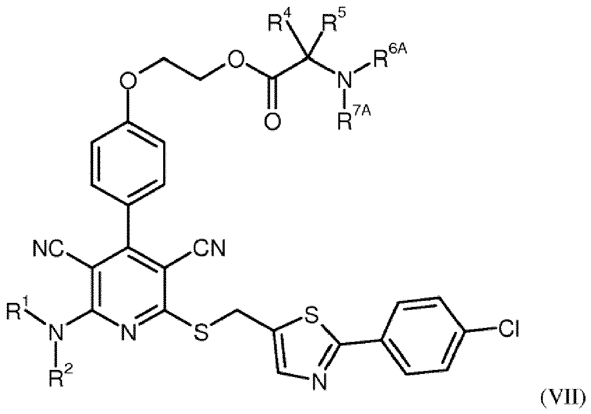 ou 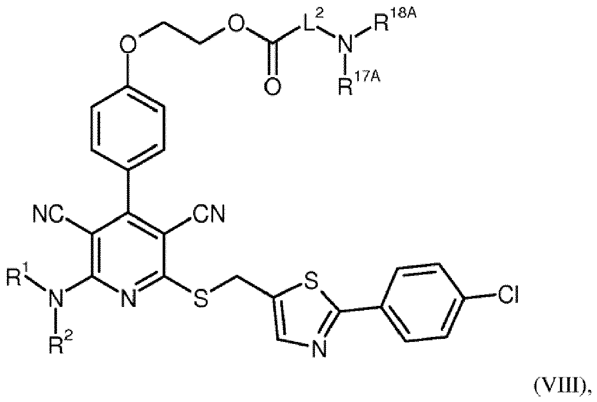 dans lesquelles L², R¹, R², R⁴, R⁵, R^{6A}, R^{7A}, R^{17A} et R^{18A} ont chacun les significations indiquées précédemment, puis les groupes protecteurs éventuellement présents sont clivés pour obtenir un composé de formule (I-B) ou (I-C) 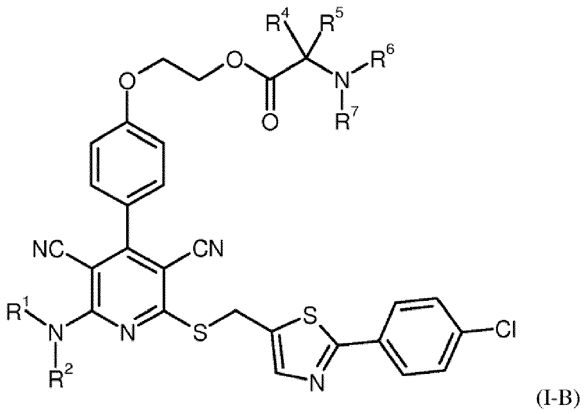 ou 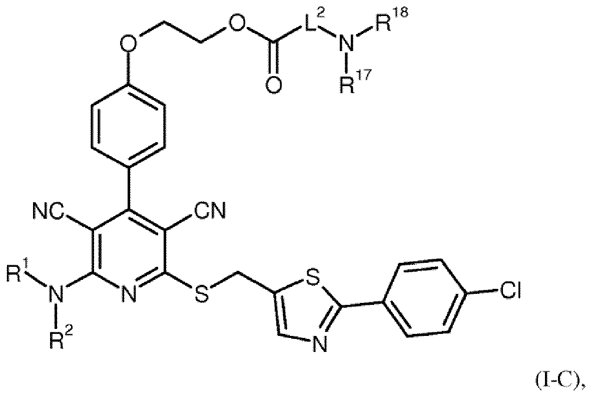 dans lesquelles L², R¹, R², R⁴, R⁵, R⁶, R⁷, R¹⁷ et R¹⁸ ont chacun les significations indiquées dans les revendications 1, 2, 4, 5, 6 et 7,
ou
[B] un composé de formule (I-A) est tout d'abord couplé dans un solvant inerte en présence d'un agent de condensation avec un acide carboxylique de formule (IX), (X), (XI) ou (XII) 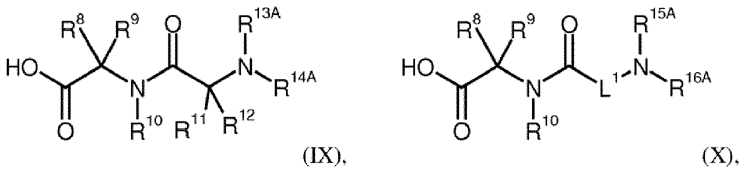 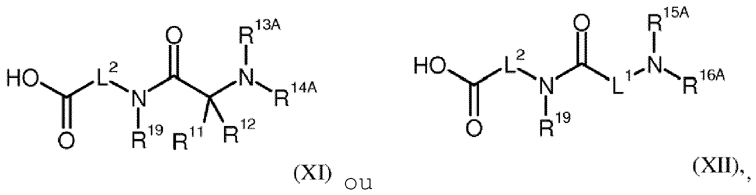 dans lesquelles L¹, L², R⁸, R⁹, R¹⁰, R¹¹, R¹² et R¹⁹ ont chacun les significations indiquées dans les revendications 1, 2, 4, 5, 6 et 7,
et
R^{13A}, R^{14A}, R^{15A} et R^{16A} ont respectivement les significations indiquées pour R¹³, R¹⁴, R¹⁵ et R¹⁶ ou représentent un groupe protecteur amino, tel que par exemple tert.-butoxycarbonyle,
en composés de formule (XIII), (XIV), (XV) ou (XVI) 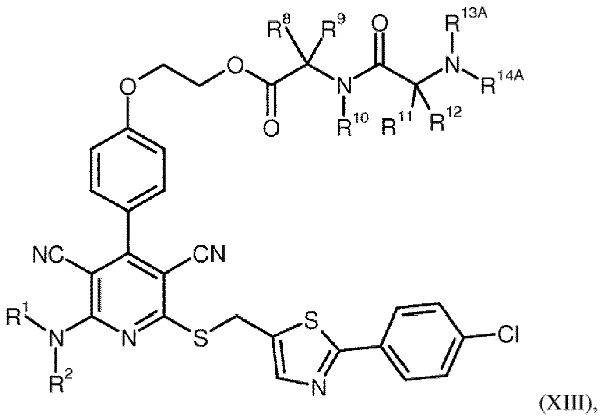 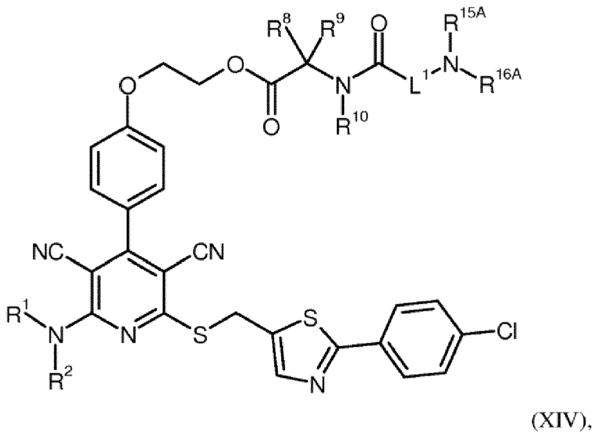 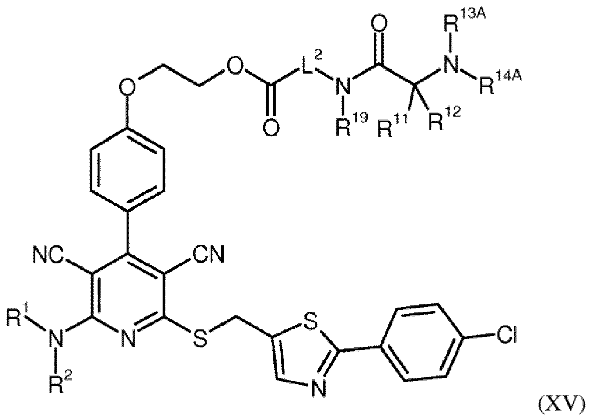 ou 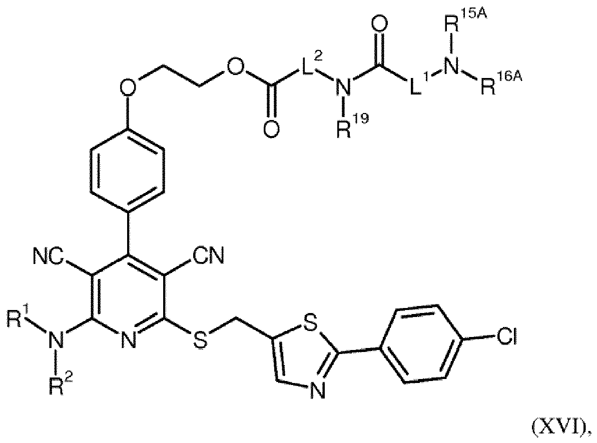 dans lesquelles L¹, L², R¹, R², R⁸, R⁹, R¹⁰ R¹¹, R¹², R^{13A}, R^{14A}, R^{15A}, R^{16A} et R¹⁹ ont chacun les significations indiquées précédemment,
puis les groupes protecteurs éventuellement présents sont clivés pour obtenir un composé de formule (I-D), (I-E), (I-F) ou (I-G) 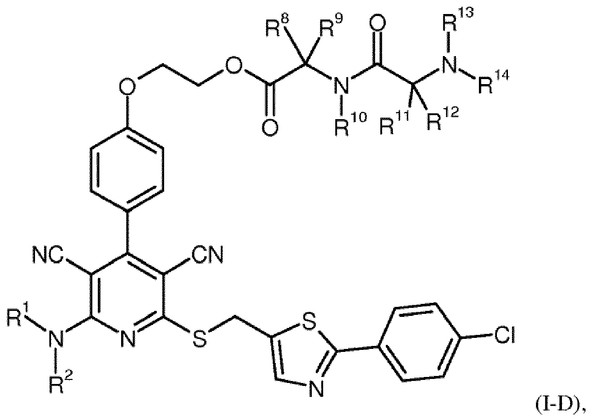 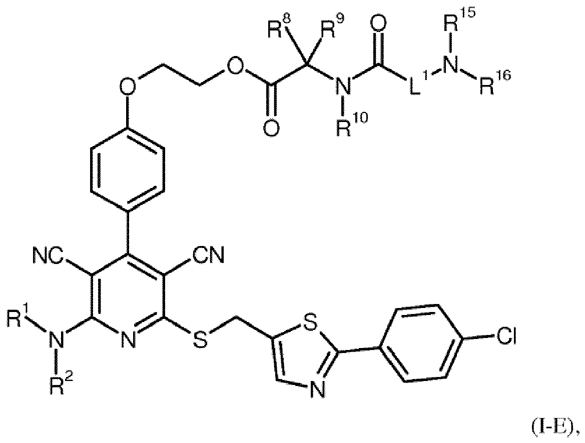 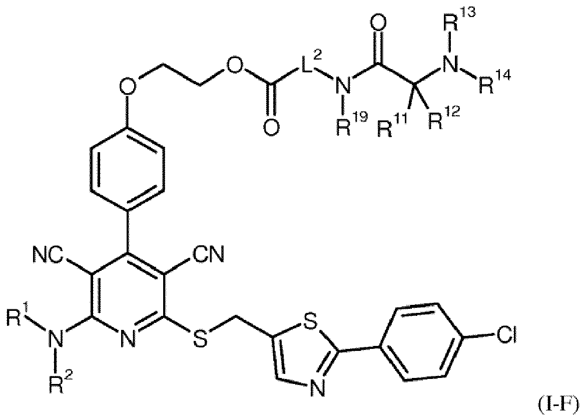 ou 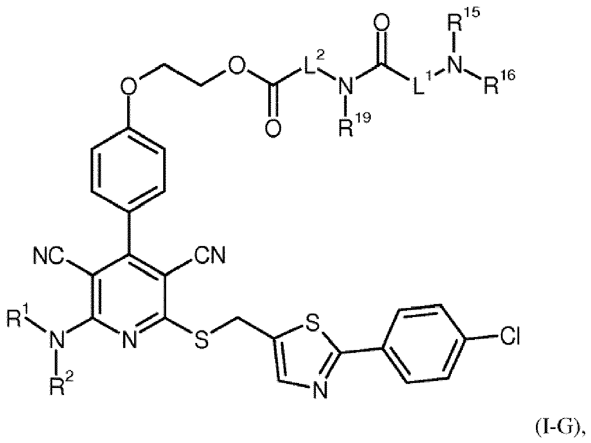 dans lesquelles L¹, L², R¹, R², R⁸, R⁹, R¹⁰, R¹¹, R¹², R¹³, R¹⁴, R¹⁵, R¹⁶ et R¹⁹ ont chacun les significations indiquées dans les revendications 1, 2, 4, 5, 6 et 7,
ou
[C] à partir d'un composé de formule (VII-1) ou (VIII-1) 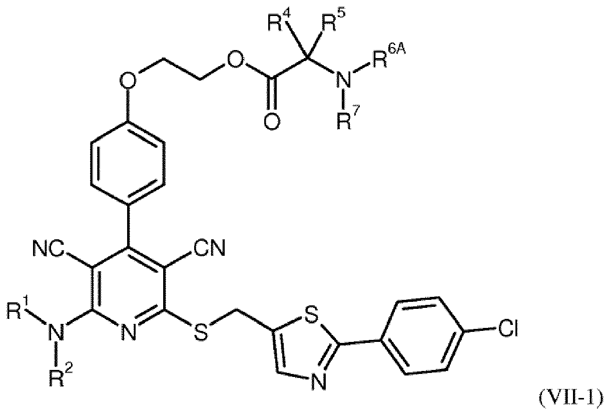 ou 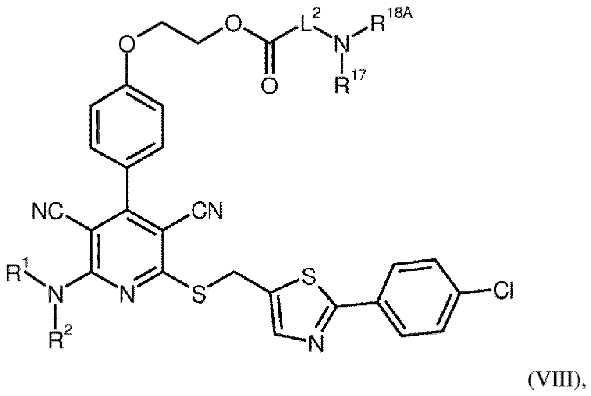 dans lesquelles L², R¹, R², R⁴, R⁵, R⁷ et R¹⁷ ont chacun les significations indiquées dans les revendications 1, 2, 4, 5, 6 et 7,
et
R^{6A} et R^{18A} représentent un groupe protecteur amino, par exemple tert.-butoxycarbonyle,
le groupe protecteur amino est clivé selon les méthodes standard pour obtenir un composé de formule (I-B-1) ou (I-C-1) 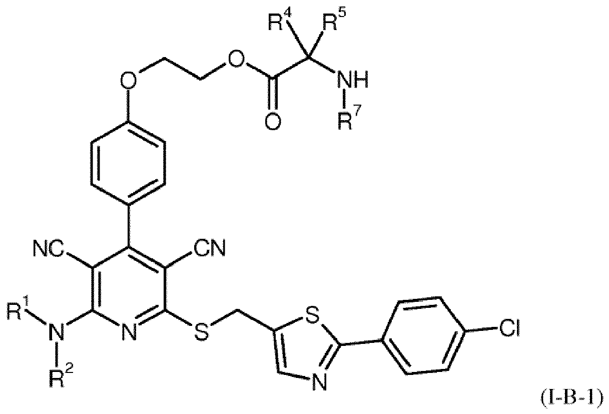 ou 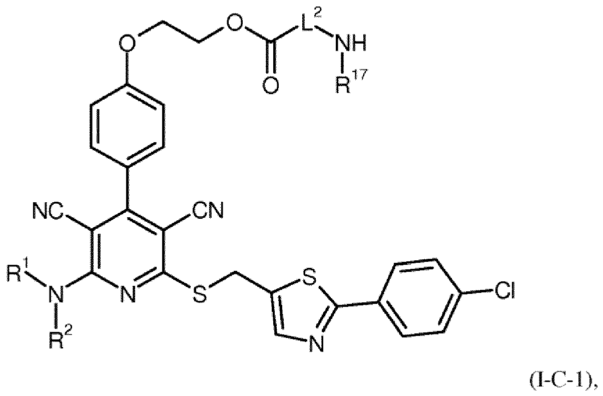 dans lesquelles L², R¹, R², R⁴, R⁵, R⁷ et R¹⁷ ont chacun les significations indiquées dans les revendications 1, 2, 4, 5, 6 et 7,
et celui-ci est tout d'abord couplé dans un solvant inerte en présence d'un agent de condensation avec un acide carboxylique de formule (XVII) ou (XVIII) 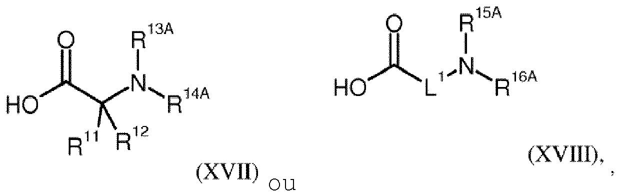 dans lesquelles L¹, R¹¹ et R¹² ont chacun les significations indiquées dans les revendications 1, 2, 4, 5, 6 et 7,
et
R^{13A}, R^{14A}, R^{15A} et R^{16A} ont respectivement les significations indiquées pour R¹³, R¹⁴, R¹⁵ et R¹⁶ ou représentent un groupe protecteur amino, tel que par exemple tert.-butoxycarbonyle,
en composés de formule (XIII), (XIV), (XV) ou (XVI), puis les groupes protecteurs éventuellement présents sont de nouveau clivés pour obtenir les composés (I-D), (I-E), (I-F) ou (I-G) résultants,
et les composés (I-B), (I-C), (I-D), (I-E), (I-F) et (I-G) résultants sont éventuellement transformés avec les (i) solvants et/ou (ii) bases ou acides appropriés en leurs solvates, sels et/ou solvates des sels.

23. Agonistes du récepteur d'adénosine A1 de formule (I), tels que définis dans l'une quelconque des revendications 1 à 20, destinés à une utilisation pour le traitement et/ou la prévention de maladies.

24. Agonistes du récepteur d'adénosine A1 de formule (I), tels que définis dans l'une quelconque des revendications 1 à 20, destinés à une utilisation dans un procédé de traitement et/ou de prophylaxie de la maladie coronarienne, du syndrome coronarien aigu, de l'angine de poitrine, de l'insuffisance cardiaque, de l'infarctus du myocarde et la fibrillation atriale.

25. Agonistes du récepteur d'adénosine A1 de formule (I), tels que définis dans l'une quelconque des revendications 1 à 20, destinés à une utilisation dans un procédé de traitement et/ou de prophylaxie du diabète, du syndrome métabolique et des dyslipidémies.

26. Agonistes du récepteur d'adénosine A1 de formule (I), selon la revendication 1, destinés à une utilisation dans un procédé de traitement et/ou de prophylaxie des maladies rénales.

27. Utilisation d'un agoniste du récepteur d'adénosine A1 de formule (I), tel que défini dans l'une quelconque des revendications 1 à 20, pour la fabrication d'un médicament pour le traitement et/ou la prévention de la maladie coronarienne, du syndrome coronarien aigu, de l'angine de poitrine, de l'insuffisance cardiaque, de l'infarctus du myocarde et de la fibrillation atriale.

28. Utilisation d'un agoniste du récepteur d'adénosine A1 de formule (I), tel que défini dans l'une quelconque des revendications 1 à 20, pour la fabrication d'un médicament pour le traitement et/ou la prévention des manifestations aigues et chroniques de l'insuffisance cardiaque, de l'insuffisance cardiaque décompensée, de l'insuffisance cardiaque droite, de l'insuffisance cardiaque gauche, de l'insuffisance globale, de la cardiomyopathie ischémique, de la cardiomyopathie dilatative, des cardiopathies congénitales, des vices valvulaires, de l'insuffisance cardiaque en cas de vices valvulaires, de la sténose mitrale, de l'insuffisance mitrale, de la sténose aortique, de l'insuffisance aortique, de la sténose tricuspidienne, de l'insuffisance tricuspidienne, de la sténose pulmonaire, de l'insuffisance pulmonaire, des vices valvulaires combinés, de l'inflammation du muscle cardiaque (myocardite), de la myocardite chronique, de la myocardite aigue, de la myocardite virale, de l'insuffisance cardiaque diabétique, de la cardiomyopathie alcoolique, des maladies cardiaques de stockage, de l'insuffisance cardiaque diastolique et systolique, et des phases aigues de la détérioration d'une insuffisance cardiaque chronique existante (worsening heart failure).

29. Utilisation d'un agoniste du récepteur d'adénosine A1 de formule (I), tel que défini dans la revendication 1, pour la fabrication d'un médicament pour le traitement et/ou la prévention des maladies rénales.

30. Utilisation d'un agoniste du récepteur d'adénosine A1 de formule (I), tel que défini dans l'une quelconque des revendications 1 à 20, pour la fabrication d'un médicament pour le traitement et/ou la prévention du diabète, du syndrome métabolique et des dyslipidémies.

31. Médicament contenant un agoniste du récepteur d'adénosine A1 de formule (I), tel que défini dans l'une quelconque des revendications 1 à 20, en combinaison avec un adjuvant pharmaceutiquement approprié, non toxique et inerte.

32. Médicament contenant un agoniste du récepteur d'adénosine A1 de formule (I), tel que défini dans l'une quelconque des revendications 1 à 20, en combinaison avec un ou plusieurs agents actifs supplémentaires choisis dans le groupe constitué par les agents actifs modifiant le métabolisme lipidique, les antidiabétiques, les agents actifs abaissant la tension artérielle et les agents actifs antithrombotiques.

33. Médicament selon la revendication 31 ou 32 destiné à une utilisation pour le traitement et/ou la prévention de la maladie coronarienne, du syndrome coronarien aigu, de l'angine de poitrine, de l'insuffisance cardiaque, de l'infarctus du myocarde et de la fibrillation atriale.

34. Médicament selon la revendication 31 ou 32 destiné à une utilisation pour le traitement et/ou la prévention des manifestations aigues et chroniques de l'insuffisance cardiaque, de l'insuffisance cardiaque décompensée, de l'insuffisance cardiaque droite, de l'insuffisance cardiaque gauche, de l'insuffisance globale, de la cardiomyopathie ischémique, de la cardiomyopathie dilatative, des cardiopathies congénitales, des vices valvulaires, de l'insuffisance cardiaque en cas de vices valvulaires, de la sténose mitrale, de l'insuffisance mitrale, de la sténose aortique, de l'insuffisance aortique, de la sténose tricuspidienne, de l'insuffisance tricuspidienne, de la sténose pulmonaire, de l'insuffisance pulmonaire, des vices valvulaires combinés, de l'inflammation du muscle cardiaque (myocardite), de la myocardite chronique, de la myocardite aigue, de la myocardite virale, de l'insuffisance cardiaque diabétique, de la cardiomyopathie alcoolique, des maladies cardiaques de stockage, de l'insuffisance cardiaque diastolique et systolique, et des phases aigues de la détérioration d'une insuffisance cardiaque chronique existante (worsening heart failure).

35. Médicament selon la revendication 31 ou 32 destiné à une utilisation pour le traitement et/ou la prévention du diabète, du syndrome métabolique et des dyslipidémies.

36. Médicament selon la revendication 31 ou 32 destiné à une utilisation pour le traitement et/ou la prévention des maladies rénales.
